# EUROPEAN PATENT SPECIFICATION

(11) **EP 1 809 750 B1**
(45) Date of publication and mention of the grant of the patent: **21.03.2012**
(21) Application number: 05810935.6
(22) Date of filing: 07.11.2005
(51) Int. Cl.: C12N 15/67, C12N 5/10, C12N 15/79, C12N 15/09, C12N 15/11, C12N 15/85

(54) **SELECTION OF HOST CELLS EXPRESSING PROTEIN AT HIGH LEVELS**
SELEKTION VON WIRTSZELLEN MIT PROTEINEXPRESSION AUF HOHEM NIVEAU
SELECTION DE CELLULES HOTES EXPRIMANT UNE PROTEINE A DES NIVEAUX ELEVES

(30) Priority: 08.11.2004 EP 04105593; 08.11.2004 US 626301 P; 05.07.2005 US 696610 P
(43) Date of publication of application: 25.07.2007
(73) Proprietor: ChromaGenics B.V., 2333 CN Leiden (NL)
(72) Inventor: OTTE, Arie, Pieter, NL-3823 SG Amersfoort (NL); VAN BLOKLAND, Henricus Johannes Maria, NL-1456 NH Wijdewormer (NL); KWAKS, Theodorus Hendrikus Jacobus, NL-1022 XW Amsterdam (NL); SEWALT, Richard George Antonius Bernardus, NL-6814 HD Arnhem (NL)
(74) Representative: Verhage, Richard Abraham
(86) International application number: PCT/EP2005/055794
(87) International publication number: WO 2006/048459

(56) References cited:
- WO-A-01/32901
- WO-A-03/083077
- WO-A-03/106684
- WO-A-2004/055215
- WO-A-2004/056986
- US-A- 5 527 701
- US-A- 6 107 477
- US-A1- 2002 155 540
- KOZAK M: "Pushing the limits of the scanning mechanism for initiation of translation" GENE: AN INTERNATIONAL JOURNAL ON GENES AND GENOMES, ELSEVIER SCIENCE PUBLISHERS, BARKING, GB, vol. 299, no. 1-2, 16 October 2002 (2002-10-16), pages 1-34, XP004395302 ISSN: 0378-1119 cited in the application
- HELLEN CHRISTOPHER U T ET AL: "Internal ribosome entry sites in eukaryotic mRNA molecules" GENES AND DEVELOPMENT, vol. 15, no. 13, 1 July 2001 (2001-07-01), pages 1593-1612, XP002332841 ISSN: 0890-9369
- KOZAK M: "Initiation of translation in prokaryotes and eukaryotes" GENE, ELSEVIER BIOMEDICAL PRESS. AMSTERDAM, NL, vol. 234, no. 2, 8 July 1999 (1999-07-08), pages 187-208, XP004173091 ISSN: 0378-1119
- KUEHN R ET AL: "FUNCTIONAL ANALYSIS OF THE INTERNAL TRANSLATION INITIATION SITE OF FOOT-AND-MOUTH DISEASE VIRUS" JOURNAL OF VIROLOGY, vol. 64, no. 10, 1990, pages 4625-4631, XP009049317 ISSN: 0022-538X
- LOPEZ DE QUINTO S ET AL: "Parameters influencing translational efficiency in aphthovirus IRES-based bicistronic expression vectors" GENE, ELSEVIER BIOMEDICAL PRESS. AMSTERDAM, NL, vol. 217, no. 1-2, September 1998 (1998-09), pages 51-56, XP004149318 ISSN: 0378-1119
- DE BOER H A ET AL: "Portable Shine-Dalgarno regions; nucleotides between the Shine-Dalgarno sequence and the start codon affect the translation efficiency." GENE AMPLIFICATION AND ANALYSIS. 1983, vol. 3, 1983, pages 103-116, XP009049298 ISSN: 0275-2778
- DUMMITT BENJAMIN ET AL: "N-terminal methionine removal and methionine metabolism in Saccharomyces cerevisiae." JOURNAL OF CELLULAR BIOCHEMISTRY, vol. 89, no. 5, 1 August 2003 (2003-08-01), pages 964-974, XP002375872 ISSN: 0730-2312

## Description

The invention relates to the field of molecular biology and biotechnology. More specifically the present invention relates to means and methods for improving the selection of host cells that express proteins at high levels.

Proteins can be produced in various host cells for a wide range of applications in biology and biotechnology, for instance as biopharmaceuticals. Eukaryotic and particularly mammalian host cells are preferred for this purpose for expression of many proteins, for instance when such proteins have certain posttranslational modifications such as glycosylation. Methods for such production are well established, and generally entail the expression in a host cell of a nucleic acid (also referred to as 'transgene') encoding the protein of interest. In general, the transgene together with a selectable marker gene is introduced into a precursor cell, cells are selected for the expression of the selectable marker gene, and one or more clones that express the protein of interest at high levels are identified, and used for the expression of the protein of interest.

One problem associated with the expression of transgenes is that it is unpredictable, stemming from the high likelihood that the transgene will become inactive due to gene silencing (McBurney et al., 2002), and therefore many host cell clones have to be tested for high expression of the transgene.

Methods to select recombinant host cells expressing relatively high levels of desired proteins are known.

One method describes the use of selectable marker proteins with mutations in their coding sequence that diminish, but not destroy the function of the marker (e.g. WO 01/32901). The rationale is that higher levels of the mutant marker expression are required when selection conditions are employed and therefore selection for high expression of the marker is achieved, therewith concomitantly selecting host cells that also express the gene of interest at high levels.

Another method makes use of a selection marker gene under control of a promoter sequence that has been mutated such that the promoter has an activity level substantially below that of its corresponding wild type (US patent 5,627,033).

Another method describes the use of an impaired dominant selectable marker sequence, such as neomycin phosphotransferase with an impaired consensus Kozak sequence, to decrease the number of colonies to be screened and to increase the expression levels of a gene of interest that is co-linked to the dominant selectable marker (US patents 5,648,267 and 5,733,779). In preferred embodiments therein, the gene of interest is placed within an (artificial) intron in the dominant selectable marker. The gene of interest and the dominant selectable marker are in different transcriptional cassettes and each contains its own eukaryotic promoter in this method (US patents 5,648,267 and 5,733,779).

Another method describes the use of a monocistronic transcription unit comprising a non-optimal translational start site (Kozak) attached to a selectable marker to impair translation of the selectable marker by the non-optimal translational start to identify cellular gene sequences that could overcome such impairment by allowing over-production of the selectable marker (US patent 6,107,477).

Another method uses the principle of a selectable marker gene containing an intron that does not naturally occur within the selectable gene, wherein the intron is capable of being spliced in a host cell to provide mRNA encoding a selectable protein and wherein the intron in the selectable gene reduces the level of selectable protein produced from the selectable gene in the host cell (European Patent 0724639 B1).

In yet another method, DNA constructs are used comprising a selectable gene positioned within an intron defined by a 5' splice donor site comprising an efficient splice donor sequence such that the efficiency of splicing an mRNA having said splice donor site is between about 80-99%, and a 3' splice acceptor site, and a product gene encoding a product of interest downstream of 3' splice acceptor site, the selectable gene and the product gene being controlled by the same transcriptional regulatory region (US patent 5,561,053).

In certain methods, use is made of polycistronic expression vector constructs. An early report of use of this principle describes a polycistronic expression vector, containing sequences coding for both the desired protein and a selectable protein, which coding sequences are governed by the same promoter and separated by a translational stop and start signal codons (US patent 4,965,196). In preferred embodiments in US 4,965,196, the selectable marker is the amplifiable DHFR gene. In a particularly preferred embodiment of the system described in US 4,965,196, the sequence coding for the selectable marker is downstream from that coding for the desired polypeptide, such that procedures designed to select for the cells transformed by the selectable marker will also select for particularly enhanced production of the desired protein.

In further improvements based on the concept of multicistronic expression vectors, bicistronic vectors have been described for the rapid and efficient creation of stable mammalian cell lines that express recombinant protein. These vectors contain an internal ribosome entry site (IRES) between the upstream coding sequence for the protein of interest and the downstream coding sequence of the selection marker (Rees et al, 1996). Such vectors are commercially available, for instance the pIRES1 vectors from Clontech (CLONTECHniques, October 1996). Using such vectors for introduction into host cells, selection of sufficient expression of the downstream marker protein then automatically selects for high transcription levels of the multicistronic mRNA, and hence a strongly increased probability of high expression of the protein of interest is envisaged using such vectors.

Preferably in such methods, the IRES used is an IRES which gives a relatively low level of translation of the selection marker gene, to further improve the chances of selecting for host cells with a high expression level of the protein of interest by selecting for expression of the selection marker protein (see e.g. international publication WO 03/106684).

The present invention aims at providing improved means and methods for selection of host cells expressing high levels of proteins of interest.

### Summary of the invention

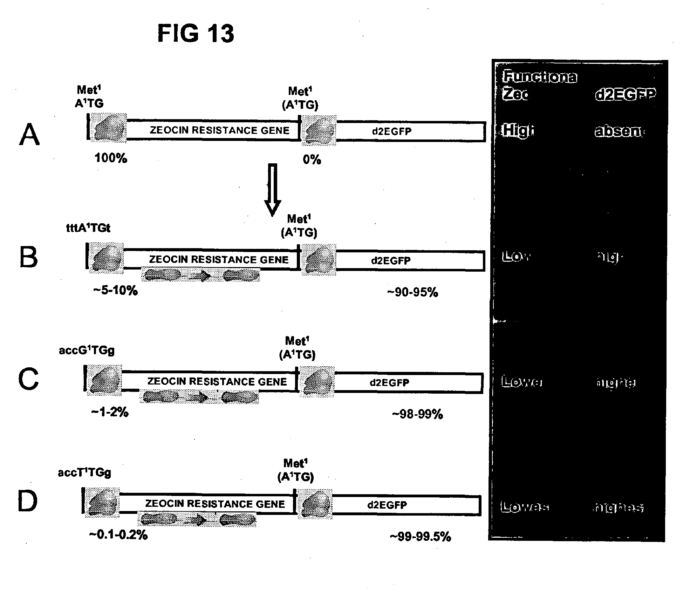

The present invention uses a novel and unique concept for selecting host cells expressing high levels of polypeptides of interest, the concept referred to herein as 'reciprocal interdependent translation'. It is unique with respect to the prior art approaches in that an extra level of regulation is used to fine-tune the amount of translation products from a multicistronic transcript, whereby the level of translation of a selectable marker polypeptide is lowered, thereby increasing the level of translation of the polypeptide of interest coded on the same multicistronic transcription unit, i.e. the expression level of the polypeptide of interest is directly and more or less reciprocally dependent from the expression level of the selectable marker polypeptide (see Fig. 13 for a schematic view). In contrast, approaches using an IRES between the coding sequences for the polypeptide of interest and the selectable marker polypeptide (e.g. Rees et al, 1996), involve independent translation of both polypeptides, so that in those approaches there is no direct effect of the translation efficiency of the selectable marker polypeptide on that of the sequence coding for the polypeptide of interest.

In one aspect, the invention provides a DNA molecule comprising a multicistronic transcription unit coding for i) a selectable marker polypeptide capable of being selected for in a eukaryotic host cell, and for ii) a polypeptide of interest, the polypeptide of interest having a translation initiation sequence (and startcodon) separate from that of the selectable marker polypeptide, characterized in that the coding sequence for the polypeptide of interest is downstream from the coding sequence for the selectable marker in said multicistronic transcription unit, and the sequence encoding the selectable marker has no ATG in the coding strand following the start codon of the selectable marker polypeptide up to the start codon of the polypeptide of interest, and characterized in that the translation start sequence in the coding strand for the selectable marker polypeptide is chosen from the group consisting of:
a) a translation start sequence comprising a mutation in the nucleotides in positions -3 to -1 and/or +4 of the consensus sequence RCCATGG (wherein R is A or G, and wherein the A of the ATG startcodon is nt +1);
b) a GTG startcodon;
c) a TTG startcodon;
d) a CTG startcodon;
e) an ATT startcodon; and
f) an ACG startcodon.
Preferably the translation *initiation* efficiency of the selectable marker is decreased.
According to the invention, the decreased translation initiation efficiency is brought about by having a non-optimal translation start sequence of the selectable marker polypeptide.

In preferred embodiments thereof, the translation start sequence in the coding strand for the selectable marker polypeptide comprises an ATG sequence defining a startcodon, said ATG sequence being in a non-optimal context for translation initiation. This results in a decreased use of this ATG as startcodon, when compared to an ATG startcodon in an optimal context.

In a more preferred embodiment, the translation start sequence in the coding strand for the selectable marker polypeptide comprises a startcodon different from an ATG startcodon, such as one of GTG, TTG, CTG, ATT, or ACG sequence, the first two thereof being the most preferred. Such non-ATG startcodons preferably are flanked by sequences providing for relatively good recognition of the non-ATG sequences as startcodons, such that at least some ribosomes start translation from these startcodons, i.e. the translation start sequence preferably comprises the sequence ACC[non-ATG startcodon]G or GCC[non-ATG startcodon]G.

According to the invention, the sequence encoding the selectable marker polypeptide has no ATG sequence in the coding strand following the sequence coding for the startcodon of the selectable marker polypeptide up to the sequence encoding the stopcodon thereof, and not in any sequences in the same strand between said stopcodon and the startcodon of the polypeptide of interest (which startcodon preferably is defined by an ATG in the same coding strand).

In certain embodiments thereof, any ATG sequence present in frame and coding for methionine in the sequence coding for the wild-type selectable marker polypeptide has been mutated to code for valine, threonine, isoleucine or leucine.

In preferred embodiments, the selectable marker protein provides resistance against lethal and/or growth-inhibitory effects of a selection agent, such as an antibiotic.

Preferably, the coding sequence of the polypeptide of interest comprises an optimal translation start sequence.

The invention further provides expression cassettes comprising a DNA molecule according to the invention, which expression cassettes further comprise a promoter upstream of the multicistronic expression unit and being functional in a eukaryotic host cell for initiation transcription of the multicistronic expression unit, and said expression cassettes further comprising a transcription termination sequence downstream of the multicistronic expression unit.

In preferred embodiments thereof, such expression cassettes further comprise at least one chromatin control element chosen from the group consisting of a matrix or scaffold attachment region (MAR/SAR), an insulator sequence, a ubiquitous chromatin opener element (UCOE), and an anti-repressor sequence. Anti-repressor sequences are most preferred in this aspect, and in preferred embodiments said anti-repressor sequences are chosen from the group consisting of a) any one SEQ. ID. NO. 1 through SEQ. ID. NO. 66; b) fragments of any one of SEQ. ID. NO. 1 through SEQ. ID. NO. 66, wherein said fragments have anti-repressor activity; c) sequences that are at least 70% identical in nucleotide sequence to a) or b) wherein said sequences have anti-repressor activity; and d) the complement to any one of a) to c). In certain preferred embodiments, said anti-repressor sequences are chosen from the group consisting of: STAR67 (SEQ. ID. NO. 66), STAR7 (SEQ. ID. NO. 7), STAR9 (SEQ. ID. NO. 9), STAR17 (SEQ. ID. NO. 17), STAR27 (SEQ. ID. NO. 27), STAR29 (SEQ. ID. NO. 29), STAR43 (SEQ. ID. NO. 43), STAR44 (SEQ. ID. NO. 44), STAR45 (SEQ. ID. NO. 45), STAR47 (SEQ. ID. NO. 47), STAR61 (SEQ. ID. NO. 61), and functional fragments or derivatives of these STAR sequences. In certain embodiments, the expression cassette comprises STAR67, or a functional fragment or derivative thereof, positioned upstream of the promoter driving expression of the multicistronic gene. In certain embodiments, the multicistronic gene is flanked on both sides by at least one anti-repressor sequence. In certain preferred embodiments, expression cassettes are provided according to the invention, comprising in 5' to 3' order: anti-repressor sequence A - anti-repressor sequence B - [promoter - multicistronic transcription unit according to the invention (encoding the functional selectable marker protein and downstream thereof the polypeptide of interest) - transcription termination sequence] - anti-repressor sequence C, wherein A, B and C may be the same or different.

In certain embodiments, the polypeptide of interest is a part of a multimeric protein, for example a heavy or light chain of an immunoglobulin.

The invention also provides DNA molecules comprising a sequence encoding a functional selectable marker polypeptide, capable of being selected for in a eukaryotic host cell, characterized in that said DNA molecule:
i) has a translation start sequence chosen from the group consisting of:
   a) a translation start sequence comprising a mutation in the nucleotides in positions -3 to -1 and/or +4 of the consensus sequence RCCATGG (wherein R is A or G, and wherein the A of the ATG startcodon is nt +1);
   b) a GTG startcodon;
   c) a TTG startcodon;
   d) a CTG startcodon;
   e) an ATT startcodon; and
   f) an ACG startcodon, followed by an otherwise functional selectable marker coding sequence, and
ii) in that the coding strand of the sequence defining the selectable marker polypeptide downstream of the non-optimal startcodon and up to the stopcodon is devoid of ATG sequences.

The invention also provides host cells comprising DNA molecules according to the invention.

The invention further provides methods for generating host cells expressing a polypeptide of interest, the method comprising the steps of: introducing into a plurality of precursor host cells an expression cassette according to the invention, culturing the cells under conditions selecting for expression of the selectable marker polypeptide, and selecting at least one host cell producing the polypeptide of interest.

In a further aspect, the invention provides methods for producing a polypeptide of interest, the methods comprising culturing a host cell, said host cell comprising an expression cassette according to the invention, and expressing the polypeptide of interest from the expression cassette. In preferred embodiments thereof, the polypeptide of interest is further isolated from the host cells and/or from the host cell culture medium.

### Detailed description of the invention

In one aspect, the invention provides a DNA molecule comprising a multicistronic transcription unit coding for i) a selectable marker polypeptide capable of being selected for in a eukaryotic host cell, and for ii) a polypeptide of interest, the polypeptide of interest having a translation initiation sequence separate from that of the selectable marker polypeptide, characterized in that the coding sequence for the polypeptide of interest is downstream from the coding sequence for the selectable marker in said multicistronic transcription unit,and the sequence encoding the selectable marker has no ATG in the coding strand following the start codon of the selectable marker polypeptide up to the start codon of the polypeptide of interest, and and wherein the translation start sequence in the coding strand for the selectable marker polypeptide is chosen from the group consisting of:
a) a translation start sequence comprising a mutation in the nucleotides in positions -3 to -1 and/or +4 of the consensus sequence RCCATGG (wherein R is A or G, and wherein the A of the ATG startcodon is nt +1);
b) a GTG startcodon;
c) a TTG startcodon;
d) a CTG startcodon;
e) an ATT startcodon; and
f) an ACG startcodon.
Such a DNA molecule can be used according to the invention for obtaining eukaryotic host cells expressing high levels of the polypeptide of interest, by selecting for the expression of the selectable marker polypeptide. Subsequently or simultaneously, one or more host cell(s) expressing the polypeptide of interest can be identified, and further used for expression of high levels of the polypeptide of interest.

The term "monocistronic gene" is defined as a gene capable of providing a RNA molecule that encodes one polypeptide. A "multicistronic transcription unit", also referred to as multicistronic gene, is defined as a gene capable of providing an RNA molecule that encodes at least two polypeptides. The term "bicistronic gene" is defined as a gene capable of providing a RNA molecule that encodes two polypeptides. A bicistronic gene is therefore encompassed within the definition of a multicistronic gene. A "polypeptide" as used herein comprises at least five amino acids linked by peptide bonds, and can for instance be a protein or a part, such as a subunit, thereof. Mostly, the terms polypeptide and protein are used interchangeably herein. A "gene" or a "transcription unit" as used in the present invention can comprise chromosomal DNA, cDNA, artificial DNA, combinations thereof, and the like. Transcription units comprising several cistrons are transcribed as a single mRNA. In contrast to approaches used in the prior art, in the multicistronic transcription units of the invention, translation of the second coding region (i.e. that for the protein of interest) present on that RNA is not dependent on the use of translation reinitiation sites or internal ribosome entry sites for translation iniation of the second coding region, but rather it is dependent on the efficiency of translation of the first coding region (i.e. that for the selectable marker protein) in a more or less reciprocal manner: the higher the translation level of the selectable marker, the lower the transcription level of the protein of interest and vice versa.

A multicistronic transcription unit according to the invention preferably is a bicistronic transcription unit coding from 5' to 3' for a selectable marker polypeptide and a polypeptide of interest. Hence, the polypeptide of interest is encoded downstream from the coding sequence for the selectable marker polypeptide.
It is also possible to include more coding sequences on the same transcription unit, downstream of the (first) polypeptide of interest, e.g. coding for a second selectable marker polypeptide or for a second polypeptide of interest. Such an extra coding sequence downstream of the coding sequence for the first polypeptide of interest then preferably is preceded by a sequence encoding a translation reinitiation site or internal ribosome entry site (IRES), such that also the second polypeptide of interest or the second selection marker polypeptide is translated, in this case independently from the earlier two coding sequences. In such an embodiment, it is for instance possible to have the coding sequences for subunits of a multimeric protein, e.g. a heavy and a light chain of an immunoglobulin, encoded as a first and second polypeptide of interest (in any order), to code for subunits of a multimeric protein within a single multicistronic expression unit. It is however preferred to use separate transcription units for the expression of different polypeptides of interest, also when these form part of a multimeric protein (see e.g. example 13: the heavy and light chain of an antibody each are encoded by a separate transcription unit, each of these expression units being a bicistronic expression unit according to the invention).

The DNA molecules of the invention can be present in the form of double stranded DNA, having with respect to the selectable marker polypeptide and the polypeptide of interest a coding strand and a non-coding strand, the coding strand being the strand with the same sequence as the translated RNA, except for the presence of T instead of U. Hence, an AUG startcodon is coded for in the coding strand by an ATG sequence, and the strand containing this ATG sequence corresponding to the AUG startcodon in the RNA is referred to as the coding strand of the DNA. It will be clear that startcodons or translation initiation sequences are in fact present in an RNA molecule, but that these can be considered equally embodied in a DNA molecule coding for such an RNA molecule; hence, wherever the present invention refers to a startcodon or translation initation sequence, the corresponding DNA molecule having the same sequence as the RNA sequence but for the presence of a T instead of a U in the coding strand of said DNA molecule is meant to be included, and vice versa, except where explicitly specified otherwise. In other words, a startcodon is for instance an AUG sequence in RNA, but the corresponding ATG sequence in the coding strand of the DNA is referred to as startcodon as well in the present invention. The same is used for the reference of 'in frame' coding sequences, meaning triplets (3 bases) in the RNA molecule that are translated into an amino acid, but also to be interpreted as the corresponding trinucleotide sequences in the coding strand of the DNA molecule.

The selectable marker polypeptide and the polypeptide of interest encoded by the multicistronic gene each have their own translation initation sequence, and therefore each have their own startcodon (as well as stopcodon), i.e. they are encoded by separate open reading frames.

The term "selection marker" or "selectable marker" is typically used to refer to a gene and/or protein whose presence can be detected directly or indirectly in a cell, for example a polypeptide that inactivates a selection agent and protects the host cell from the agent's lethal or growth-inhibitory effects (e.g. an antibiotic resistance gene and/or protein). Another possibility is that said selection marker induces fluorescence or a color deposit (e.g. green fluorescent protein (GFP) and derivatives (e.g d2EGFP), luciferase, lacZ, alkaline phosphatase, etc.), which can be used for selecting cells expressing the polypeptide inducing the color deposit, e.g. using a fluorescence activated cell sorter (FACS) for selecting cells that express GFP. Preferably, the selectable marker polypeptide according to the invention provides resistance against lethal and/or growth-inhibitory effects of a selection agent. The selectable marker polypeptide is encoded by the DNA of the invention. The selectable marker polypeptide according to the invention must be functional in a eukaryotic host cell, and hence being capable of being selected for in eukaryotic host cells. Any selectable marker polypeptide fulfilling this criterion can in principle be used according to the present invention. Such selectable marker polypeptides are well known in the art and routinely used when eukaryotic host cell clones are to be obtained, and several examples are provided herein. In certain embodiments, a selection marker used for the invention is zeocin. In other embodiments, blasticidin is used. The person skilled in the art will know that other selection markers are available and can be used, e.g. neomycin, puromycin, bleomycin, hygromycin, etc. In other embodiments, kanamycin is used. In yet other embodiments, the DHFR gene is used as a selectable marker, which can be selected for by methotrexate, especially by increasing the concentration of methotrexate cells can be selected for increased copy numbers of the DHFR gene. Similarly, the glutamine synthetase (GS) gene can be used, for which selection is possible in cells having insufficient GS (e.g. NS-0 cells) by culturing in media without glutamine, or alternatively in cells having sufficient GS (e.g. CHO cells) by adding an inhibitor of GS, methionine sulphoximine (MSX). Other selectable marker genes that could be used, and their selection agents, are for instance described in table 1 of US patent 5,561,053; see also Kaufinan, Methods in Enzymology, 185:537-566 (1990), for a review of these.

When two multicistronic transcription units are to be selected for according to the invention in a single host cell, each one preferably contains the coding sequence for a different selectable marker, to allow selection for both multicistronic transcription units. Of course, both multicistronic transcription units may be present on a single nucleic acid molecule or alternatively each one may be present on a separate nucleic acid molecule.

The term "selection" is typically defined as the process of using a selection marker/selectable marker and a selection agent to identify host cells with specific genetic properties (e.g. that the host cell contains a transgene integrated into its genome). It is clear to a person skilled in the art that numerous combinations of selection markers are possible. One antibiotic that is particularly advantageous is zeocin, because the zeocin-resistance protein (zeocin-R) acts by binding the drug and rendering it harmless. Therefore it is easy to titrate the amount of drug that kills cells with low levels of zeocin-R expression, while allowing the high-expressors to survive. All other antibiotic-resistance proteins in common use are enzymes, and thus act catalytically (not 1:1 with the drug). Hence, the antibiotic zeocin is a preferred selection marker. However, the invention also works with other selection markers.

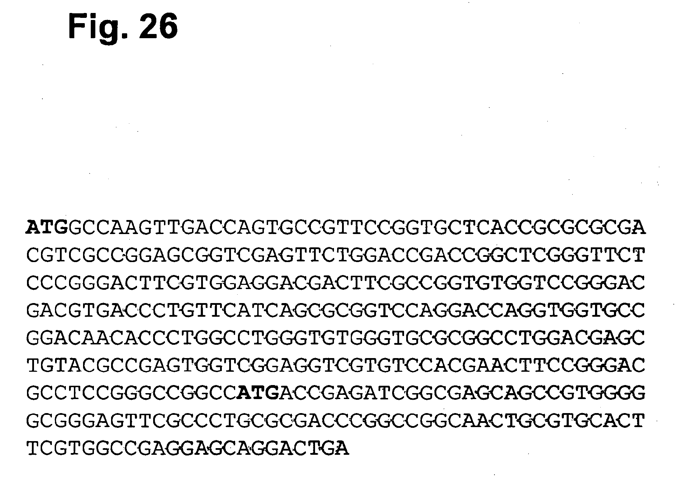

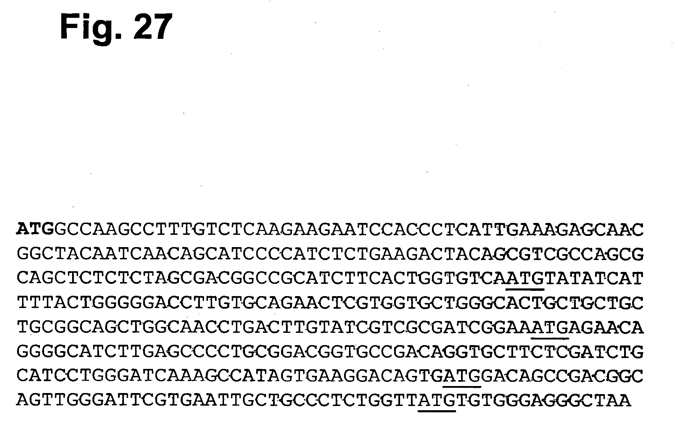

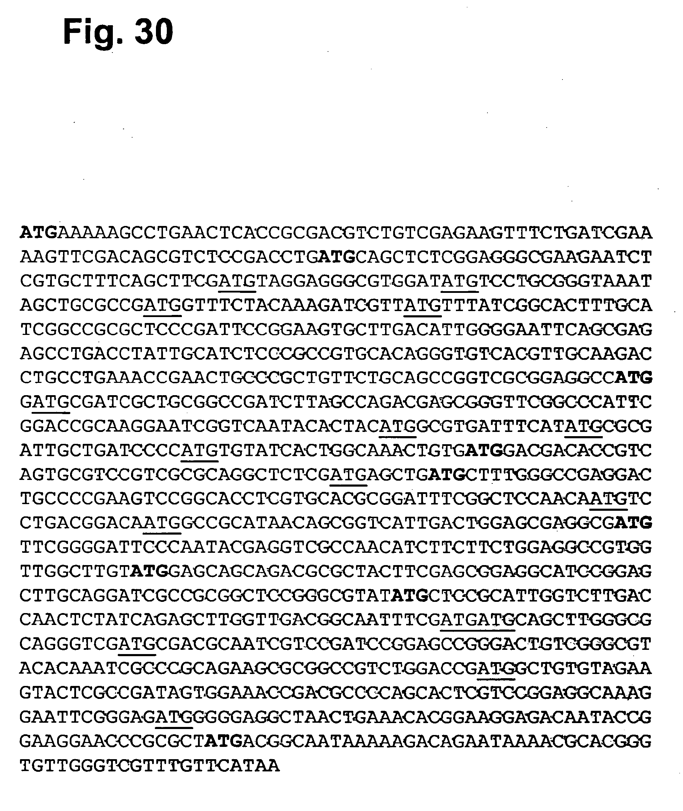

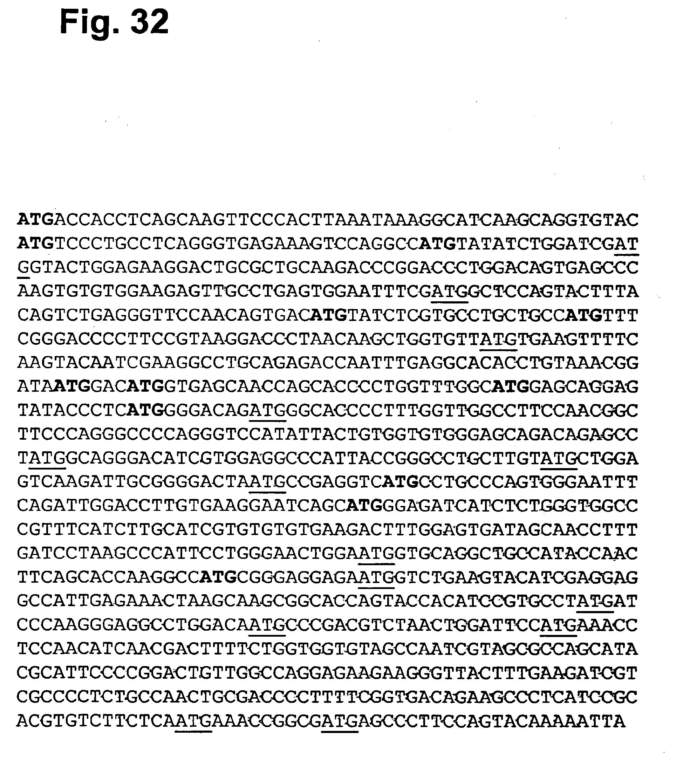

A selectable marker polypeptide according to the invention is the protein that is encoded by the nucleic acid of the invention, which polypeptide can be detected, for instance because it provides resistance to a selection agent such as an antibiotic. Hence, when an antibiotic is used as a selection agent, the DNA encodes a polypeptide that confers resistance to the selection agent, which polypeptide is the selectable marker polypeptide. DNA sequences coding for such selectable marker polypeptides are known, and several examples of wild-type sequences of DNA encoding selectable marker proteins are provided herein (Figs. 26-32). It will be clear that mutants or derivatives of selectable markers can also be suitably used according to the invention, and are therefore included within the scope of the term 'selectable marker polypeptide', as long as the selectable marker protein is still functional. For convenience and as generally accepted by the skilled person, in many publications as well as herein, often the gene and protein encoding the resistance to a selection agent is referred to as the 'selectable agent (resistance) gene' or 'selection agent (resistance) protein', respectively, although the official names may be different, e.g. the gene coding for the protein conferring restance to neomycin (as well as to G418 and kanamycin) is often referred to as neomycin (resistance) (or neo^{r}) gene, while the official name is aminoglycoside 3'-phosphotransferase gene.

For the present invention, it is beneficial to have low levels of expression of the selectable marker polypeptide, so that stringent selection is possible. In the present invention this is brought about by using a selectable marker coding sequence with a non-optimal translation efficiency. Upon selection, only cells that have nevertheless sufficient levels of selectable marker polypeptide will be selected, meaning that such cells must have sufficient transcription of the multicistronic transcription unit and sufficient translation of the selectable marker polypeptide, which provides a selection for cells where the multicistronic transcription unit has been integrated or otherwise present in the host cells at a place where expression levels from this transcription unit are high. According to the invention the (more or less) reciprocally interdependent nature of the translation of the polypeptide of interest from that of the translation efficiency of the marker, ensures high translation levels of the polypeptide of interest. This high level of translation is superimposed on the high transcription levels selected for by the stringent selection conditions (and resulting from the use of a strong promoter in the host cells to drive transcription of the multicistronic transcription unit).

The DNA molecules according to the invention have the coding sequence for the selectable marker polypeptide upstream of the coding sequence for the polypeptide of interest, to provide for a multicistronic transcript with reciprocally interdependent translation of the polypeptide of interest and the selectable marker polypeptide. Hence, the multicistronic transcription unit comprises in the 5' to 3' direction (both in the transcribed strand of the DNA and in the resulting transcribed RNA) the coding sequence for the selectable marker polypeptide and the sequence encoding the polypeptide of interest.

According to the invention, the coding regions of the multicistronic gene are preferably translated from the cap-dependent ORF, and therefore the selectable marker polypeptide would in principle also be produced in abundance. To prevent such high translation of the selectable marker cistron, according to the invention the nucleic acid sequence coding for the selectable marker polypeptide comprises a mutation (within the open reading frame (ORF) or in the translation initiation sequences preceding the ORF, or in both) that decreases the translation efficiency of the selectable marker polypeptide in a eukaryotic host cell. The translation efficiency should be lower than that of the corresponding wild-type sequence in the same cell, i.e. the mutation should result in less polypeptide per cell per time unit, and hence less selectable marker polypeptide. This can be detected using routine methods known to the person skilled in the art. For instance in the case of antibiotic selection the mutation will result in less resistance than obtained with the sequence having no such mutation and hence normal translation efficiency, which difference can easily be detected by determining the number of surviving colonies after a normal selection period, which will be lower when a translation efficiency decreasing mutation is present. As is well known to the person skilled in the art there are a number of parameters that indicate the expression level marker polypeptide such as, the maximum concentration of selection agent to which cells are still resistant, number of surviving colonies at a given concentration, growth speed (doubling time) of the cells in the presence of selection agent, combinations of the above, and the like.

According to the invention, the mutation that decreases the translation efficiency, is a mutation that decreases the translation initiation efficiency. This can for example be established according to the invention by providing the selectable marker polypeptide coding sequence with a non-optimal translation start sequence.

According to the invention, the translation initiation efficiency of the selectable marker gene in eukaryotic cells can be suitably decreased according to the invention by mutating the startcodon and/or the nucleotides in positions -3 to -1 and +4 (where the A of the ATG startcodon is nt +1), for instance in the coding strand of the corresponding DNA sequence, to provide a non-optimal translation start sequence. A translation start sequence is often referred to in the field as 'Kozak sequence', and an optimal Kozak sequence is RCCATGG, the startcodon underlined, R being a purine, i.e. A or G (see Kozak M, 1986, 1987, 1989, 1990, 1997, 2002). Hence, besides the startcodon itself, the context thereof, in particular nucleotides -3 to -1 and +4, are relevant, and an optimal translation startsequence comprises an optimal startcodon (i.e. ATG) in an optimal context (i.e. the ATG directly preceded by RCC and directly followed by G). A non-optimal translation start sequence is defined herein as any sequence that gives at least some detectable translation in a eukaryotic cell (detectable because the selection marker polypeptide is detectable), and not having the consensus sequence RCCATGG (startcodon underlined). Translation by the ribosomes starts preferably at the first startcodon it encounters when scanning from the 5'-cap of the mRNA (scanning mechanism), and is most efficient when an optimal Kozak sequence is present (see Kozak M, 1986, 1987, 1989, 1990, 1997, 2002). However, in a small percentage of events, non-optimal translation initiation sequences are recognized and used by the ribosome to start translation. The present invention makes use of this principle, and allows for decreasing and even fine-tuning of the amount of translation and hence expression of the selectable marker polypeptide, which can therefore be used to increase the stringency of the selection system.

In a first embodiment of the invention, the ATG startcodon of the selectable marker polypeptide (in the coding strand of the DNA, coding for the corresponding AUG startcodon in the RNA transcription product) is left intact, but the positions at - 3 to -1 and +4 are mutated such that they do not fulfill the optimal Kozak sequence any more, e.g. by providing the sequence TTTATGT as the translation start site (ATG startcodon underlined). It will be clear that other mutations around the startcodon at positions -3 to -1 and/or +4 could be used with similar results using the teaching of the present invention, as can be routinely and easily tested by the person skilled in the art. The idea of this first embodiment is that the ATG startcodon is placed in a 'non-optimal' context for translation initiation.

In a second and preferred embodiment, the ATG startcodon itself of the selectable marker polypeptide is mutated. This will in general lead to even lower levels of translation initiation than the first embodiment. The ATG startcodon in the second embodiment is mutated into another codon, which has been reported to provide some translation initiation, for instance to GTG, TTG, CTG, ATT, or ACG (collectively referred to herein as 'non-optimal start codons'). In preferred embodiments, the ATG startcodon is mutated into a GTG startcodon. This provides still lower expression levels (lower translation) than with the ATG startcodon intact but in a non-optimal context. More preferably, the ATG startcodon is mutated to a TTG startcodon, which provides even lower expression levels of the selectable marker polypeptide than with the GTG startcodon (Kozak M, 1986, 1987, 1989, 1990, 1997, 2002; see also examples 9-13 herein).

For the second embodiment, i.e. where a non-ATG startcodon is used, it is strongly preferred to provide an optimal context for such a startcodon, i.e. the non-optimal startcodons are preferably directly preceded by nucleotides RCC in positions -3 to -1 and directly followed by a G nucleotide (position +4). However, it has been reported that using the sequence TTTGTGG (startcodon underlined), some initiation is observed at least in vitro, so although strongly preferred it may not be absolutely required to provide an optimal context for the non-optimal startcodons.

ATG sequences within the coding sequence for a polypeptide, but excluding the ATG startcodon, are referred to as 'internal ATGs', and if these are in frame with the ORF and therefore code for methionine, the resulting methionine in the polypeptide is referred to as an 'internal methionine'. According to the invention the coding region (following the startcodon, not necessarily including the startcodon) coding for the selectable marker polypeptide preferably is devoid of any ATG sequence in the coding strand of the DNA, up to (but not including) the startcodon of the polypeptide of interest (obviously, the startcodon of the polypeptide of interest may be, and in fact preferably is, an ATG startcodon). This can be established by mutating any such ATG sequence within the coding sequence of the selectable marker polypeptide, following the startcodon thereof (as is clear from the teaching above, the startcodon of the selectable marker polypeptide itself may be an ATG sequence, but not necessarily so). To this purpose preferably, the degeneracy of the genetic code is used to avoid mutating amino acids in the selectable marker polypeptide wherever possible. Hence, wherever an ATG is present in the coding strand of the DNA sequence encoding the selectable marker polypeptide, which ATG is not in frame with the selectable marker polypeptide ORF, and therefore does not code for an internal methionine in the selectable marker polypeptide, the ATG can be mutated such that the resulting polypeptide has no mutations in its internal amino acid sequence. Where the ATG is an in-frame codon coding for an internal methionine, the codon can be mutated, and the resulting mutated polypeptide can be routinely checked for activity of the selectable marker polypeptide. In this way a mutation can be chosen which leads to a mutated selectable marker polypeptide that is still active as such (quantitative differences may exist, but those are less relevant, and in fact it could even be beneficial to have less active variants for the purpose of the present invention; the minimum requirement is that the selectable marker polypeptide can still be selected for in eukaryotic cells). Amino acids valine, threonine, isoleucine and leucine are structurally similar to methionine, and therefore codons that code for one of these amino acids are good starting candidates to be tested in place of methione within the coding sequence after the startcodon. Of course, using the teachings of the present invention, the skilled person may test other amino acids as well in place of internal methionines, using routine molecular biology techniques for mutating the coding DNA, and routine testing for functionality of the selectable marker polypeptide. Besides routine molecular biology techniques for mutating DNA, it is at present also possible to synthesise at will (if required using subcloning steps) DNA sequences that have sufficient length for an ORF of a selectable marker polypeptide, and such synthetic DNA sequences can nowadays be ordered commercially from various companies. Hence, using the teachings of the present invention, the person skilled in the art may design appropriate sequences according to the invention encoding a selectable marker polypeptide (with a mutation decreasing translation initiation, and preferably having no internal ATGs), have this sequence synthesized, and test the DNA molecule for functionality of the encoded selectable marker by introducing the DNA molecule in eukaryotic host cells and test for expression of functional selectable marker polypeptide. The commercial availability of such sequences also makes feasible to provide without undue burden for selection marker coding sequences lacking internal ATG sequences, where the wild-type coding sequence of the selection marker polypeptide comprises several such internal ATGs.

By providing a coding sequence for a selectable marker polypeptide lacking any internal ATG sequence, the chances of inadvertent translation initiation by ribosomes that passed the (first, non-optimal) translation start sequence of the selectable marker polypeptide at a subsequent internal ATG trinucleotide is diminished, so that the ribosomes will continue to scan for the first optimal translation start sequence, i.e. that of the polypeptide of interest.

It will be clear that it is not necessarily strictly required to remove all internal ATG sequences from the coding sequence of the selectable marker, as long as the vast majority of ribosomes will not use such internal ATG sequences as a translation initiation start site, for instance because such internal ATGs usually are not in an optimal context for translation initiation. However, also in view of the fact that even an ATG sequence in a non-optimal context gives rise to significant translation initiation events (in general much higher than from a non-ATG startcodon in an optimal context), it will also be clear that if several internal ATG sequences are present in the coding region of the selectable marker, each of these might subtract a fraction of ribosomes that start translation at those ATGs, preventing them from reaching the downstream translation unit encoding the polypeptide of interest, and hence result in less expression of the polypeptide of interest. This is the reason why it is preferred to have a sequence encoding the selectable marker polypeptide, which sequence is free of internal ATG sequences. Possibly, if desired, the ratio of ribosomes translating the polypeptide of interest might even be further enhanced by mutating any non-optimal potential translation initiation sites within the sequence coding for the selectable marker polypeptide.

Clearly, any sequence present in the coding strand between the stopcodon of the selectable marker and the startcodon of the polypeptide of interest can easily be designed such that it does not comprise ATG sequences, or other non-optimal potential translation start codons.

Clearly, it is strongly preferred according to the present invention, that the translation start sequence of the polypeptide of interest comprises an optimal translation start sequence, i.e. having the consensus sequence RCCATGG (startcodon underlined). This will result in a very efficient translation of the polypeptide of interest.

As a consequence of these measures, the reciprocally interdependent translation mechanism of the invention is provided: ribosomes scan from the cap of the mRNA containing the multicistronic transcription unit, and a small percentage of ribosomes will translate the first open reading frame, i.e. the selectable marker protein, because this is provided with a mutation decreasing the translation efficiency, while the great majority of the ribosomes will pass this open reading frame and start translation at the optimal translation initiation site of the polypeptide of interest, resulting in low levels of selectable marker protein and high levels of polypeptide of interest. By providing the coding sequence of the marker with different mutations leading to several levels of decreased translation efficiency, the stringency of selection is increased and reciprocally the expression levels of the polypeptide of interest go up, so that fine-tuning is possible: for instance using a TTG startcodon for the selection marker polypeptide, only very few ribosomes will translate from this startcodon, resulting in very low levels of selectable marker protein, and hence a high stringency of selection, and at the same time almost all ribosomes will translate the polypeptide of interest.

It is demonstrated herein that this can be used in a very robust selection system, leading to a very large percentage of clones that express the polypeptide of interest at high levels, as desired. In addition, the expression levels obtained for the polypeptide of interest appear to be significantly higher than those obtained when an even larger number of colonies are screened using selection systems hitherto known.

In addition to a decreased translation initiation efficiency, it could be beneficial to also provide for decreased translation elongation efficiency of the selectable marker polypeptide, e.g. by mutating the coding sequence thereof so that it comprises several non-preferred codons of the host cell, in order to further decrease the translation levels of the marker polypeptide and allow still more stringent selection conditions, if desired. In certain embodiments, besides the mutation(s) that decrease the translation efficiency according to the invention, the selectable marker polypeptide further comprises a mutation that reduces the activity of the selectable marker polypeptide compared to its wild-type counterpart. This may be used to increase the stringency of selection even further. As non-limiting examples, proline at position 9 in the zeocin resistance polypeptide may be mutated, e.g. to Thr or Phe, and for the neomycin resistance polypeptide, amino acid residue 182 or 261 or both may further be mutated (see e.g. WO 01/32901).

In some embodiments of the invention, a so-called spacer sequence is placed downstream of the sequence encoding the startcodon of the selectable marker polypeptide, which spacer sequence preferably is a sequence in frame with the startcodon and encoding a few amino acids, and that does not contain a secondary structure (Kozak, 1990), and does not contain the sequence ATG. Such a spacer sequence can be used to further decrease the translation initiation frequency if a secondary structure is present in the RNA (Kozak, 1990) of the selectable marker polypeptide (e.g. for zeocin, possibly for blasticidin), and hence increase the stringency of the selection system according to the invention.

The invention also provides a DNA molecule comprising the sequence encoding a functional selectable marker protein according to the invention, which DNA molecule
i) has a translation start sequence chosen from the group consisting of:
   a) a translation start sequence comprising a mutation in the nucleotides in positions -3 to -1 and/or +4 of the consensus sequence RCCATGG (wherein R is A or G, and wherein the A of the ATG startcodon is nt +1);
   b) a GTG startcodon;
   c) a TTG startcodon;
   d) a CTG startcodon;
   e) an ATT startcodon; and
   f) an ACG startcodon, followed by an otherwise functional selectable marker coding sequence, and
ii) in that the coding strand of the sequence defining the selectable marker polypeptide downstream of the non-optimal startcodon and up to the stopcodon is devoid of ATG sequences. Such DNA molecules encompass a useful intermediate product according to the invention. These molecules can be prepared first, introduced into eukaryotic host cells and tested for functionality (for some markers this is even possible in prokaryotic host cells), if desired in a (semi-) quantitative manner, of the selectable marker polypeptide. They may then be further used to prepare a DNA molecule according to the invention, comprising the multicistronic transcription unit.

It is a preferred aspect of the invention to provide an expression cassette comprising the DNA molecule according to the invention, having the multicistronic transcription unit. Such an expression cassette is useful to express sequences of interest, for instance in host cells. An 'expression cassette' as used herein is a nucleic acid sequence comprising at least a promoter functionally linked to a sequence of which expression is desired. Preferably, an expression cassette further contains transcription termination and polyadenylation sequences. Other regulatory sequences such as enhancers may also be included. Hence, the invention provides an expression cassette comprising in the following order: 5'- promoter- multicistronic transcription unit according to the invention, coding for a selectable marker polypeptide and downstream thereof a polypeptide of interest - transcription termination sequence -3'. The promoter must be capable of functioning in a eukaryotic host cell, i.e. it must be capable of driving transcription of the multicistronic transcription unit. The expression cassette may optionally further contain other elements known in the art, e.g. splice sites to comprise introns, and the like. In some embodiments, an intron is present behind the promoter and before the sequence encoding the selectable marker polypeptide.

To obtain expression of nucleic acid sequences encoding protein, it is well known to those skilled in the art that sequences capable of driving such expression, can be functionally linked to the nucleic acid sequences encoding the protein, resulting in recombinant nucleic acid molecules encoding a protein in expressible format. In the present invention, the expression cassette comprises a multicistronic transcription unit. In general, the promoter sequence is placed upstream of the sequences that should be expressed. Much used expression vectors are available in the art, e.g. the pcDNA and pEF vector series of Invitrogen, pMSCV and pTK-Hyg from BD Sciences, pCMV-Script from Stratagene, etc, which can be used to obtain suitable promoters and/or transcription terminator sequences, polyA sequences, and the like.

Where the sequence encoding the polypeptide of interest is properly inserted with reference to sequences governing the transcription and translation of the encoded polypeptide, the resulting expression cassette is useful to produce the polypeptide of interest, referred to as expression. Sequences driving expression may include promoters, enhancers and the like, and combinations thereof. These should be capable of functioning in the host cell, thereby driving expression of the nucleic acid sequences that are functionally linked to them. The person skilled in the art is aware that various promoters can be used to obtain expression of a gene in host cells. Promoters can be constitutive or regulated, and can be obtained from various sources, including viruses, prokaryotic, or eukaryotic sources, or artificially designed. Expression of nucleic acids of interest may be from the natural promoter or derivative thereof or from an entirely heterologous promoter (Kaufman, 2000). Some well-known and much used promoters for expression in eukaryotic cells comprise promoters derived from viruses, such as adenovirus, e.g. the E1A promoter, promoters derived from cytomegalovirus (CMV), such as the CMV immediate early (IE) promoter (referred to herein as the CMV promoter) (obtainable for instance from pcDNA, Invitrogen), promoters derived from Simian Virus 40 (SV40) (Das et al, 1985), and the like. Suitable promoters can also be derived from eukaryotic cells, such as methallothionein (MT) promoters, elongation factor 1α (EF-1α) promoter (Gill et al., 2001), ubiquitin C or UB6 promoter (Gill et al., 2001; Schorpp et al, 1996), actin promoter, an immunoglobulin promoter, heat shock promoters, and the like. Some preferred promoters for obtaining expression in eukaryotic cells, which are suitable promoters in the present invention, are the CMV-promoter, a mammalian EF1-alpha promoter, a mammalian ubiquitin promoter such as a ubiquitin C promoter, or a SV40 promoter (e.g. obtainable from pIRES, cat.no. 631605, BD Sciences). Testing for promoter function and strength of a promoter is a matter of routine for a person skilled in the art, and in general may for instance encompass cloning a test gene such as lacZ, luciferase, GFP, etc. behind the promoter sequence, and test for expression of the test gene. Of course, promoters may be altered by deletion, addition, mutation of sequences therein, and tested for functionality, to find new, attenuated, or improved promoter sequences. According to the present invention, strong promoters that give high transcription levels in the eukaryotic cells of choice are preferred.

In certain embodiments, a DNA molecule according to the invention is part of a vector, e.g. a plasmid. Such vectors can easily be manipulated by methods well known to the person skilled in the art, and can for instance be designed for being capable of replication in prokaryotic and/or eukaryotic cells. In addition, many vectors can directly or in the form of isolated desired fragment therefrom be used for transformation of eukaryotic cells and will integrate in whole or in part into the genome of such cells, resulting in stable host cells comprising the desired nucleic acid in their genome.

Conventional expression systems are DNA molecules in the form of a recombinant plasmid or a recombinant viral genome. The plasmid or the viral genome is introduced into (eukaryotic host) cells and preferably integrated into their genomes by methods known in the art. In preferred embodiments, the present invention also uses these types of DNA molecules to deliver its improved transgene expression system. A preferred embodiment of the invention is the use of plasmid DNA for delivery of the expression system. A plasmid contains a number of components: conventional components, known in the art, are an origin of replication and a selectable marker for propagation of the plasmid in bacterial cells; a selectable marker that functions in eukaryotic cells to identify and isolate host cells that carry an integrated transgene expression system; the protein of interest, whose high-level transcription is brought about by a promoter that is functional in eukaryotic cells (e.g. the human cytomegalovirus major immediate early promoter/enhancer, pCMV (Boshart et al., 1985); and viral transcriptional terminators (e.g. the SV40 polyadenylation site (Kaufman & Sharp, 1982) for the transgene of interest and the selectable marker.

The vector used can be any vector that is suitable for cloning DNA and that can be used for transcription of a nucleic acid of interest. When host cells are used it is preferred that the vector is an integrating vector. Alternatively, the vector may be an episomally replicating vector.

It is widely appreciated that chromatin structure and other epigenetic control mechanisms may influence the expression of transgenes in eukaryotic cells (e.g. Whitelaw et al, 2001). The multicistronic expression units according to the invention form part of a selection system with a rather rigourous selection regime. This generally requires high transcription levels in the host cells of choice. To increase the chance of finding clones of host cells that survive the rigorous selection regime, and possibly to increase the stability of expression in obtained clones, it will generally be preferable to increase the predictability of transcription. Therefore, in preferred embodiments, an expression cassette according to the invention further comprises at least one chromatin control element. A 'chromatin control element' as used herein is a collective term for DNA sequences that may somehow have an effect on the chromatin structure and therewith on the expression level and/or stability of expression of transgenes in their vicinity (they function 'in cis', and hence are placed preferably within 5 kb, more preferably within 2 kb, still more preferably within 1 kb from the transgene) within eukaryotic cells. Such elements have sometimes been used to increase the number of clones having desired levels of transgene expression. The mechanisms by which these elements work may differ for and even within different classes of such elements, and are not completely known for all types of such elements. However, such elements have been described, and for the purpose of the present invention chromatin control elements are chosen from the group consisting of matrix or scaffold attachment regions (MARs/SARs) (e.g. Phi-Van et al, 1990; WO 02/074969, WO 2005/040377), insulators (West et al, 2002) such as the beta-globin insulator element (5' HS4 of the chicken beta-globin locus), scs, scs', and the like (e.g. Chung et al, 1993, 1997; Kellum and Schedl, 1991; WO 94/23046, WO 96/04390, WO 01/02553, WO 2004/027072), a ubiquitous chromatin opening element (UCOE) (WO 00/05393, WO 02/24930, WO 02/099089, WO 02/099070), and anti-repressor sequences(also referred to as 'STAR' sequences) (Kwaks et al, 2003; WO 03/004704). Non-limiting examples of MAR/SAR sequences that could be used in the current invention are the chicken lysosyme 5' MAR (Phi-Van et al, 1990) or fragments thereof, e.g. the B, K and F regions as described in WO 02/074969); DNA sequences comprising at least one bent DNA element and at least one binding site for a DNA binding protein, preferably containing at least 10% of dinucleotide TA, and/or at least 12% of dinucleotide AT on a stretch of 100 contiguous base pairs, such as a sequence selected from the group of comprising the sequences SEQ ID Nos 1 to 27 in WO 2005/040377, fragments of any one of SEQ ID Nos 1 to 27 in WO 2005/040377 being at least 100 nucleotides in length and having MAR activity, sequences that are at least 70% identical in nucleotide sequence to any one of SEQ ID Nos 1 to 27 in WO 2005/040377 or fragments thereof and having MAR activity, wherein MAR activity is defined as being capable of binding to nuclear matrices/scaffolds in vitro and/or of altering the expression of coding sequences operably linked to a promoter; sequences chosen from any one of SEQ ID NO: 1 to 5 in WO 02/074969, fragments of any one of any one of SEQ ID NO: 1 to 5 in WO 02/074969 and having MAR activity, sequences that are at least 70% identical in nucleotide sequence to any one of SEQ ID NO: 1 to 5 in WO 02/074969 or fragments thereof and having MAR activity; sequences chosen from SEQ ID NO: 1 and SEQ ID NO: 2 in WO 2004/027072, functional fragments thereof and sequences being at least 70% identical thereto. A non-limiting example of insulator sequences that could be used in the present invention is a sequence that comprises SEQ ID NO:1 of WO 01/02553. Non-limiting examples of UCOEs that could be used in the present invention are sequences depicted in Figures 2 and 7 of WO 02/24930, functional fragments thereof and sequences being at least 70% identical thereto while still retaining activity; sequences comprising SEQ ID NO: 28 of US 2005/181428, functional fragments thereof and sequences being at least 70% identical thereto while still retaining activity.

Preferably, said chromatin control element is an anti-repressor sequence, preferably chosen from the group consisting of: a) any one SEQ. ID. NO. 1 through SEQ. ID. NO. 66; b) fragments of any one of SEQ. ID. NO. 1 through SEQ. ID. NO. 66, wherein said fragments have anti-repressor activity ('functional fragments'); c) sequences that are at least 70% identical in nucleotide sequence to a) or b) wherein said sequences have anti-repressor activity ('functional derivatives'); and d) the complement to any one of a) to c). Preferably, said chromatin control element is chosen from the group consisting of STAR67 (SEQ. ID. NO. 66), STAR7 (SEQ. ID. NO. 7), STAR9 (SEQ. ID. NO. 9), STAR 17 (SEQ. ID. NO. 17), STAR27 (SEQ. ID. NO. 27), STAR29 (SEQ. ID. NO. 29), STAR43 (SEQ. ID. NO. 43), STAR44 (SEQ. ID. NO. 44), STAR45 (SEQ. ID. NO. 45), STAR47 (SEQ. ID. NO. 47), STAR61 (SEQ. ID. NO. 61), or a functional fragment or derivative of said STAR sequences. In a particularly preferred embodiment, said STAR sequence is STAR 67 (SEQ. ID. NO. 66) or a functional fragment or derivative thereof. In certain preferred embodiments, STAR 67 or a functional fragment or derivative thereof is positioned upstream of a promoter driving expression of the multicistronic transcription unit. In other preferred embodiments, the expression cassettes according to the invention are flanked on both sides by at least one anti-repressor sequence.

Sequences having anti-repressor activity as used herein are sequences that are capable of at least in part counteracting the repressive effect of HP 1 or HPC2 proteins when these proteins are tethered to DNA. Sequences having anti-repressor activity (sometimes also referred to as anti-repressor sequences or anti-repressor elements herein) suitable for the present invention, have been disclosed in WO 03/004704, and were coined "STAR" sequences therein (wherever a sequence is referred to as a STAR sequence herein, this sequence has anti-repressor activity according to the invention). As a non-limiting example, the sequences of 66 anti-repressor elements, named STAR1-65 (see WO 03/004704) and STAR67, are presented herein as SEQ. ID. NOs. 1-65 and 66, respectively.

According to the invention, a functional fragment or derivative of a given anti-repressor element is considered equivalent to said anti-repressor element, when it still has anti-repressor activity. The presence of such anti-repressor activity can easily be checked by the person skilled in the art, for instance by the assay described below. Functional fragments or derivatives can easily be obtained by a person skilled in the art of molecular biology, by starting with a given anti-repressor sequence, and making deletions, additions, substitutions, inversions and the like (see e.g. WO 03/004704). A functional fragment or derivative also comprises orthologs from other species, which can be found using the known anti-repressor sequences by methods known by the person skilled in the art (see e.g. WO 03/004704). Hence, the present invention encompasses fragments of the anti-repressor sequences, wherein said fragments still have anti-repressor activity. The invention also encompasses sequences that are at least 70% identical in nucleotide sequence to said sequences having anti-repressor activity or to functional fragments thereof having anti-repressor activity, as long as these sequences that are at least 70% identical still have the anti-repressor activity according to the invention. Preferably, said sequences are at least 80% identical, more preferably at least 90% identical and still more preferably at least 95% identical to the reference native sequence or functional fragment thereof. For fragments of a given sequence, percent identity refers to that portion of the reference native sequence that is found in the fragment.

Sequences having anti-repressor activity according to the invention can be obtained by various methods, including but not limited to the cloning from the human genome or from the genome of another organism, or by for instance amplifying known anti-repressor sequences directly from such a genome by using the knowledge of the sequences, e.g. by PCR, or can in part or wholly be chemically synthesized.

Sequences having anti-repressor activity, and functional fragments or derivatives thereof, are structurally defined herein by their sequence and in addition are functionally defined as sequences having anti-repressor activity, which can be determined with the assay described below.

Any sequence having anti-repressor activity according to the present invention should at least be capable of surviving the following functional assay (see WO 03/004704, example 1).

Human U-2 OS cells (ATCC HTB-96) are stably transfected with the pTet-Off plasmid (Clontech K1620-A) and with nucleic acid encoding a LexA-repressor fusion protein containing the LexA DNA binding domain and the coding region of either HP1 or HPC2 (Drosophila Polycomb group proteins that repress gene expression when tethered to DNA; the assay works with either fusion protein) under control of the Tet-Off transcriptional regulatory system (Gossen and Bujard, 1992). These cells are referred to below as the reporter cells for the anti-repressor activity assay. A reporter plasmid, which provides hygromycin resistance, contains a polylinker sequence positioned between four LexA operator sites and the SV40 promoter that controls the zeocin resistance gene. The sequence to be tested for anti-repressor activity can be cloned in said polylinker. Construction of a suitable reporter plasmid, such as pSelect, is described in example 1 and Fig. 1 of WO 00/004704. The reporter plasmid is transfected into the reporter cells, and the cells are cultured under hygromycin selection (25 µg/ml; selection for presence of the reporter plasmid) and tetracycline repression (doxycycline, 10 ng/ml; prevents expression of the LexA-repressor fusion protein). After 1 week of growth under these conditions, the doxycycline concentration is reduced to 0.1 ng/ml to induce the LexA-repressor gene, and after 2 days zeocin is added to 250 µg/ml. The cells are cultured for 5 weeks, until the control cultures (transfected with empty reporter plasmid, i.e. lacking a cloned anti-repressor sequence in the polylinker) are killed by the zeocin (in this control plasmid, the SV40 promoter is repressed by the LexA-repressor fusion protein that is tethered to the LexA operating sites, resulting in insufficient zeocin expression in such cells to survive zeocin selection). A sequence has anti-repressor activity according to the present invention if, when said sequence is cloned in the polylinker of the reporter plasmid, the reporter cells survive the 5 weeks selection under zeocin. Cells from such colonies can still be propagated onto new medium containing zeocin after the 5 weeks zeocin selection, whereas cells transfected with reporter plasmids lacking anti-repressor sequences cannot be propagated onto new medium containing zeocin. Any sequence not capable of conferring such growth after 5 weeks on zeocin in this assay, does not qualify as a sequence having anti-repressor activity, or functional fragment or functional derivative thereof according to the present invention. As an example, other known chromatin control elements such as those tested by Van der Vlag et al (2000), including Drosophila scs (Kellum and Schedl, 1991), 5'-HS4 of the chicken β-globin locus (Chung et al, 1993, 1997) or Matrix Attachment Regions (MARs) (Phi-Van et al., 1990), do not survive this assay.

In addition, it is preferred that the anti-repressor sequence or functional fragment or derivative thereof confers a higher proportion of reporter over-expressing clones when flanking a reporter gene (e.g. luciferase, GFP) which is integrated into the genome of U-2 OS or CHO cells, compared to when said reporter gene is not flanked by anti-repressor sequences, or flanked by weaker repression blocking sequences such as Drosophila scs. This can be verified using for instance the pSDH vector, or similar vectors, as described in example 1 and Fig. 2 of WO 03/004704. Anti-repressor elements can have at least one of three consequences for production of protein: (1) they increase the predictability of identifying host cell lines that express a protein at industrially acceptable levels (they impair the ability of adjacent heterochromatin to silence the transgene, so that the position of integration has a less pronounced effect on expression); (2) they result in host cell lines with increased protein yields; and/or (3) they result in host cell lines that exhibit more stable protein production during prolonged cultivation.
Any STAR sequence can be used in the expression cassettes according to the present invention, but the following STAR sequences are particularly useful: STAR67 (SEQ. ID. NO. 66), STAR7 (SEQ. ID. NO. 7), STAR9 (SEQ. ID. NO. 9), STAR 17 (SEQ. ID. NO. 17), STAR27 (SEQ. ID. NO. 27), STAR29 (SEQ. ID. NO. 29), STAR43 (SEQ. ID. NO. 43), STAR44 (SEQ. ID. NO. 44), STAR45 (SEQ. ID. NO. 45), STAR47 (SEQ. ID. NO. 47), STAR61 (SEQ. ID. NO. 61), or functional fragments or derivatives of these STAR sequences.

In certain embodiments said anti-repressor sequence, preferably STAR67, is placed upstream of said promoter, preferably such that less than 2kb are present between the 3' end of the anti-repressor sequence and the start of the promoter sequence. In preferred embodiments, less than 1 kb, more preferably less than 500 nucleotides (nt), still more preferably less than about 200, 100, 50, or 30 nt are present between the 3' end of the anti-repressor sequence and the start of the promoter sequence. In certain preferred embodiments, the anti-repressor sequence is cloned directly upstream of the promoter, resulting in only about 0-20 nt between the 3' end of the anti-repressor sequence and the start of the promoter sequence.

For the production of multimeric proteins, two or more expression cassettes can be used. Preferably, both expression cassettes are multicistronic expression cassettes according to the invention, each coding for a different selectable marker protein, so that selection for both expression cassettes is possible. This embodiment has proven to give good results, e.g. for the expression of the heavy and light chain of antibodies. It will be clear that both expression cassettes may be placed on one nucleic acid molecule or both may be present on a separate nucleic acid molecule, before they are introduced into host cells. An advantage of placing them on one nucleic acid molecule is that the two expression cassettes are present in a single predetermined ratio (e.g. 1: 1) when introduced into host cells. On the other hand, when present on two different nucleic acid molecules, this allows the possibility to vary the molar ratio of the two expression cassettes when introducing them into host cells, which may be an advantage if the preferred molar ratio is different from 1:1 or when it is unknown beforehand what is the preferred molar ratio, so that variation thereof and empirically finding the optimum can easily be performed by the skilled person. According to the invention, preferably at least one of the expression cassettes, but more preferably each of them, comprises a chromatin control element, more preferably an anti-repressor sequence.
In another embodiment, the different subunits or parts of a multimeric protein are present on a single expression cassette.

Instead of or in addition to the presence of a STAR sequence placed upstream of a promoter in an expression cassette, it has proven highly beneficial to provide a STAR sequence on both sides of an expression cassette, such that expression cassette comprising the transgene is flanked by two STAR sequences, which in certain embodiments are essentially identical to each other.

It is shown herein that the combination of a first anti-repressor element upstream of a promoter and flanking the expression cassette by two other anti-repressor sequences provides superior results.

As at least some anti-repressor sequences can be directional (WO 00/004704), the anti-repressor sequences flanking the expression cassette (anti-repressor A and B) may beneficially placed in opposite direction with respect to each other, such that the 3' end of each of these anti-repressor sequences is facing inwards to the expression cassette (and to each other). Hence, in preferred embodiments, the 5' side of an anti-repressor element faces the DNA/chromatin of which the influence on the transgene is to be diminished by said anti-repressor element. For an anti-repressor sequence upstream of a promoter in an expression cassette, the 3' end faces the promoter. The sequences of the anti-repressor elements in the sequence listing (SEQ. ID. NOs. 1-66) are given in 5' to 3' direction, unless otherwise indicated.
In certain embodiments, transcription units or expression cassettes according to the invention are provided, further comprising: a) a transcription pause (TRAP) sequence upstream of the promoter that drives transcription of the multicistronic transcription unit, said TRAP being in a 5' to 3' direction; or b) a TRAP sequence downstream of said open reading frame of the polypeptide of interest and preferably downstream of the transcription termination sequence of said multicistronic transcription unit, said TRAP being in a 3' to 5' orientation; or c) both a) and b); wherein a TRAP sequence is functionally defined as a sequence which when placed into a transcription unit, results in a reduced level of transcription in the nucleic acid present on the 3' side of the TRAP when compared to the level of transcription observed in the nucleic acid on the 5' side of the TRAP. Non-limiting examples of TRAP sequences are transcription termination and/or polyadenylation signals. One non-limiting example of a TRAP sequence is given in SEQ. ID. NO. 142. Examples of other TRAP sequences, methods to find these, and uses thereof have been described in WO 2004/055215.

DNA molecules comprising multicistronic transcription units and/or expression cassettes according to the present invention can be used for improving expression of nucleic acid, preferably in host cells. The terms "cell"/"host cell" and "cell line"/"host cell line" are respectively typically defined as a cell and homogeneous populations thereof that can be maintained in cell culture by methods known in the art, and that have the ability to express heterologous or homologous proteins.

Prokaryotic host cells can be used to propagate and/or perform genetic engineering with the DNA molecules of the invention, especially when present on plasmids capable of replicating in prokaryotic host cells such as bacteria.

A host cell according to the present invention preferably is a eukaryotic cell, more preferably a mammalian cell, such as a rodent cell or a human cell or fusion between different cells. In certain non-limiting embodiments, said host cell is a U-2 OS osteosarcoma, CHO (Chinese hamster ovary), HEK 293, HuNS-1 myeloma, WERI-Rb-1 retinoblastoma, BHK, Vero, non-secreting mouse myeloma Sp2/0-Ag 14, non-secreting mouse myeloma NS0, NCI-H295R adrenal gland carcinomal or a PER.C6^{®} cell. In certain embodiments of the invention, a host cell is a cell expressing at least E1A, and preferably also E1B, of an adenovirus. As non-limiting examples, such a cell can be derived from for instance human cells, for instance from a kidney (example: HEK 293 cells, see Graham et al, 1977), lung (e.g. A549, see e.g. WO 98/39411) or retina (example: HER cells marketed under the trade mark PER.C6^{®}, see US patent 5,994,128), or from amniocytes (e.g. N52.E6, described in US patent 6,558,948), and similarly from other cells. Methods for obtaining such cells are described for instance in US patents 5,994,128 and US 6,558,948. PER.C6 cells for the purpose of the present invention means cells from an upstream or downstream passage or a descendent of an upstream or downstream passage of cells as deposited under ECACC no. 96022940, i.e. having the characteristics of those cells. It has been previously shown that such cells are capable of expression of proteins at high levels (e.g. WO 00/63403, and Jones et al, 2003). In other preferred embodiments, the host cells are CHO cells, for instance CHO-K1, CHO-S, CHO-DG44, CHO-DUKXB11, and the like. In certain embodiments, said CHO cells have a dhfr⁻ phenotype. Such eukaryotic host cells can express desired polypeptides, and are often used for that purpose. They can be obtained by introduction of a DNA molecule of the invention, preferably in the form of an expression cassette, into the cells. Preferably, the expression cassette is integrated in the genome of the host cells, which can be in different positions in various host cells, and selection will provide for a clone where the transgene is integrated in a suitable position, leading to a host cell clone with desired properties in terms of expression levels, stability, growth characteristics, and the like. Alternatively the multicistronic transcription unit may be targeted or randomly selected for integration into a chromosomal region that is transcriptionally active, e.g. behind a promoter present in the genome. Selection for cells containing the DNA of the invention can be performed by selecting for the selectable marker polypeptide, using routine methods known by the person skilled in the art. When such a multicistronic transcription unit is integrated behind a promoter in the genome, an expression cassette according to the invention can be generated in situ, i.e. within the genome of the host cells.

Preferably the host cells are from a stable clone that can be selected and propagated according to standard procedures known to the person skilled in the art. A culture of such a clone is capable of producing polypeptide of interest, if the cells comprise the multicistronic transcription unit of the invention. Cells according to the invention preferably are able to grow in suspension culture in serum-free medium.

In preferred embodiments, the DNA molecule comprising the multicistronic transcription unit of the invention, preferably in the form of an expression cassette, is integrated into the genome of the eukaryotic host cell according to the invention. This will provide for stable inheritance of the multicistronic transcription unit.

Selection for the presence of the selectable marker polypeptide, and hence for expression, can be performed during the initial obtaining of the cells, and could be lowered or stopped altogether after stable clones have been obtained. It is however also possible to apply the selection agent during later stages continuously, or only occasionally, possibly at lower levels than during initial selection of the host cells.

A polypeptide of interest according to the invention can be any protein, and may be a monomeric protein or a (part of a) multimeric protein. A multimeric protein comprises at least two polypeptide chains. Non-limiting examples of a protein of interest according to the invention are enzymes, hormones, immunoglobulin chains, therapeutic proteins like anti-cancer proteins, blood coagulation proteins such as Factor VIII, multi-functional proteins, such as erythropoietin, diagnostic proteins, or proteins or fragments thereof useful for vaccination purposes, all known to the person skilled in the art.

In certain embodiments, an expression cassette of the invention encodes an immunoglobulin heavy or light chain or an antigen binding part, derivative and/or analogue thereof. In a preferred embodiment a protein expression unit according to the invention is provided, wherein said protein of interest is an immunoglobulin heavy chain. In yet another preferred embodiment a protein expression unit according to the invention is provided, wherein said protein of interest is an immunoglobulin light chain. When these two protein expression units are present within the same (host) cell a multimeric protein and more specifically an immunoglobulin, is assembled. Hence, in certain embodiments, the protein of interest is an immunoglobulin, such as an antibody, which is a multimeric protein. Preferably, such an antibody is a human or humanized antibody. In certain embodiments thereof, it is an IgG, IgA, or IgM antibody. An immunoglobulin may be encoded by the heavy and light chains on different expression cassettes, or on a single expression cassette. Preferably, the heavy and light chain are each present on a separate expression cassette, each having its own promoter (which may be the same or different for the two expression cassettes), each comprising a multicistronic transcription unit according to the invention, the heavy and light chain being the polypeptide of interest, and preferably each coding for a different selectable marker protein, so that selection for both heavy and light chain expression cassette can be performed when the expression cassettes are introduced and/or present in a eukaryotic host cell.

The polypeptide of interest may be from any source, and in certain embodiments is a mammalian protein, an artificial protein (e.g. a fusion protein or mutated protein), and preferably is a human protein.

Obviously, the configurations of the expression cassettes of the present invention may also be used when the ultimate goal is not the production of a polypeptide of interest, but the RNA itself, for instance for producing increased quantities of RNA from an expression cassette, which may be used for purposes of regulating other genes (e.g. RNAi, antisense RNA), gene therapy, in vitro protein production, etc.

In one aspect, the invention provides a method for generating a host cell expressing a polypeptide of interest, the method comprising the steps of: a) introducing into a plurality of precursor cells an expression cassette according to the invention, and b) culturing the generated cells under conditions selecting for expression of the selectable marker polypeptide, and c) selecting at least one host cell producing the polypeptide of interest. This novel method provides a very good result in terms of the ratio of obtained clones versus clones with high expression of the desired polypeptide. Using the most stringent conditions, i.e. the weakest translation efficiency for the selectable marker polypeptide (using the weakest translation start sequence), far fewer colonies are obtained using the same concentration of selection agent than with known selection systems, and a relatively high percentage of the obtained clones produces the polypeptide of interest at high levels. In addition, the obtained levels of expression appear higher than those obtained when an even larger number of clones using the known selection systems are used.

It is an additional advantage that the selection system is swift because it does not require copy number amplification of the transgene. Hence, cells with low copy numbers of the multicistronic transcription units already provide high expression levels. High transgene copy numbers of the transgene may be prone to genetic instability and repeat-induced silencing (e.g. Kim et al, 1998; McBurney et al, 2002). Therefore, an additional advantage of the embodiments of the invention with relatively low transgene copy numbers is that lower copy numbers are anticipated to be less prone to recombination and to repeat-induced silencing, and therefore less problems in this respect are anticipated when using host cells with a limited number of copies of the transgene compared to host cells obtained using an amplification system where hundreds or even thousands of copies of the selectable marker and protein of interest coding sequences may be present in the genome of the cell. The present invention provides examples of high expression levels, using the multicistronic transcription unit selection system, while the copy number of the transgene is relatively low, i.e less than 30 copies per cell, or even less than 20 copies per cell. Hence, the present invention allows the generation of host cells according to the invention, comprising less than 30 copies of the multicistronic transcription unit in the genome of the host cells, preferably less than 25, more preferably less than 20 copies, while at the same time providing sufficient expression levels of the polypeptide of interest for commercial purposes, e.g. more than 15, preferably more than 20 pg/cell/day of an antibody.

While clones having relatively low copy numbers of the multicistronic transcription units and high expression levels can be obtained, the selection system of the invention nevertheless can be combined with amplification methods to even further improve expression levels. This can for instance be accomplished by amplification of a co-integrated dhfr gene using methotrexate, for instance by placing dhfr on the same nucleic acid molecule as the multicistronic transcription unit of the invention, or by cotransfection when dhfr is on a separate DNA molecule.

In one aspect, the invention provides a method for producing a polypeptide of interest, the method comprising culturing a host cell, said host cell comprising a DNA molecule comprising a multicistronic expression unit or an expression cassette according to the invention, and expressing the polypeptide of interest from the coding sequence for the polypeptide of interest.

The host cell for this aspect is a eukaryotic host cell, preferably a mammalian cell, such as a CHO cell, further as described above.

Introduction of nucleic acid that is to be expressed in a cell, can be done by one of several methods, which as such are known to the person skilled in the art, also dependent on the format of the nucleic acid to be introduced. Said methods include but are not limited to transfection, infection, injection, transformation, and the like. Suitable host cells that express the polypeptide of interest can be obtained by selection as described above.

In certain embodiments, selection agent is present in the culture medium at least part of the time during the culturing, either in sufficient concentrations to select for cells expressing the selectable marker polypeptide or in lower concentrations. In preferred embodiments, selection agent is no longer present in the culture medium during the production phase when the polypeptide is expressed.

Culturing a cell is done to enable it to metabolize, and/or grow and/or divide and/or produce recombinant proteins of interest. This can be accomplished by methods well known to persons skilled in the art, and includes but is not limited to providing nutrients for the cell. The methods comprise growth adhering to surfaces, growth in suspension, or combinations thereof. Culturing can be done for instance in dishes, roller bottles or in bioreactors, using batch, fed-batch, continuous systems such as perfusion systems, and the like. In order to achieve large scale (continuous) production of recombinant proteins through cell culture it is preferred in the art to have cells capable of growing in suspension, and it is preferred to have cells capable of being cultured in the absence of animal- or human-derived serum or animal- or human-derived serum components.

The conditions for growing or multiplying cells (see e.g. Tissue Culture, Academic Press, Kruse and Paterson, editors (1973)) and the conditions for expression of the recombinant product are known to the person skilled in the art. In general, principles, protocols, and practical techniques for maximizing the productivity of mammalian cell cultures can be found in Mammalian Cell Biotechnology: a Practical Approach (M. Butler, ed., IRL Press, 1991).

In a preferred embodiment, the expressed protein is collected (isolated), either from the cells or from the culture medium or from both. It may then be further purified using known methods, e.g. filtration, column chromatography, etc, by methods generally known to the person skilled in the art.

The selection method according to the present invention works in the absence of chromatin control elements, but improved results are obtained when the multicistronic expression units are provided with such elements. The selection method according to the present invention works particularly well when an expression cassette according to the invention, comprising at least one anti-repressor sequence is used. Depending on the selection agent and conditions, the selection can in certain cases be made so stringent, that only very few or even no host cells survive the selection, unless anti-repressor sequences are present. Hence, the combination of the novel selection method and anti-repressor sequences provides a very attractive method to obtain only limited numbers of colonies with a greatly improved chance of high expression of the polypeptide of interest therein, while at the same time the obtained clones comprising the expression cassettes with anti-repressor sequences provide for stable expression of the polypeptide of interest, i.e. they are less prone to silencing or other mechanisms of lowering expression than conventional expression cassettes.

In certain embodiments, almost no clones are obtained when no anti-repressor sequence is present in the expression cassette according to the invention, providing for very stringent selection. The novel selection system disclosed herein therefore also provides the possibility to test parts of anti-repressor elements for functionality, by analyzing the effects of such sequences when present in expression cassettes of the invention under selection conditions. This easy screen, which provides an almost or even complete black and white difference in many cases, therefore can contribute to identifying functional parts or derivatives from anti-repressor sequences. When known anti-repressor sequences are tested, this assay can be used to characterize them further. When fragments of known anti-repressor sequences are tested, the assay will provide functional fragments of such known anti-repressor sequences.
The practice of this invention will employ, unless otherwise indicated, conventional techniques of immunology, molecular biology, microbiology, cell biology, and recombinant DNA, which are within the skill of the art. See e.g. Sambrook, Fritsch and Maniatis, Molecular Cloning: A Laboratory Manual, 2nd edition, 1989; Current Protocols in Molecular Biology, Ausubel FM, et al, eds, 1987; the series Methods in Enzymology (Academic Press, Inc.); PCR2: A Practical Approach, MacPherson MJ, Hams BD, Taylor GR, eds, 1995; Antibodies: A Laboratory Manual, Harlow and Lane, eds, 1988.

The invention is further explained in the following examples. The examples do not limit the invention in any way. They merely serve to clarify the invention.

### Examples

Examples 1-7 describe the testing of STAR67 and other STAR sequences in various configurations and under various circumstances. Examples 8 and further describe the novel selection system of the invention.

### Example 1: Construction of STAR67 vectors

A novel anti-repressor (STAR) sequence was isolated using a genetic screen as described in WO 03/004704, and this novel sequence was coined STAR67. The effects of STAR67 on expression of transgenes in mammalian cell lines were tested. Here we describe the construction of the various constructs.

### Materials and Methods

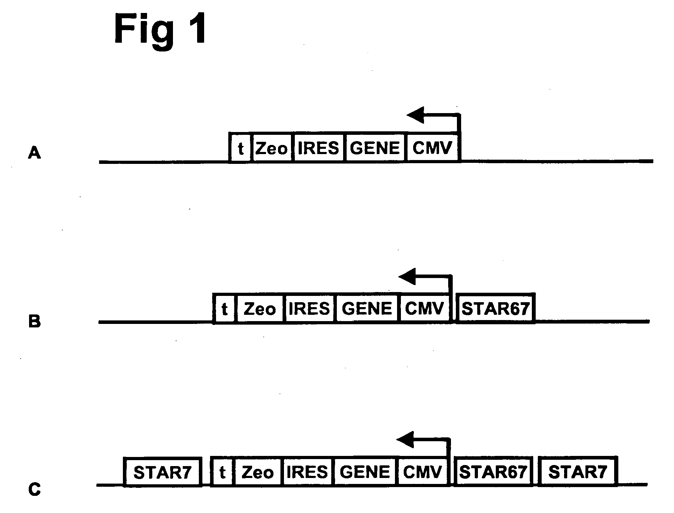

Three plasmids were created (Fig. 1):
A) CMV-d2EGFP-ires-Zeo (CMV Control),
B) STAR67-CMV-d2EGFP-ires-Zeo (CMV-STAR67),
C) STAR7-STAR67-CMV-d2EGFP-ires-Zeo-STAR7 (CMV-STAR67 7/7)

The construction of construct A is described below. Plasmid pd2EGFP (Clontech 6010-1) was modified by insertion of a linker at the *Bsi*WI site to yield pd2EGFP-link. The linker, made by annealing oligonucleotides
GTACGGATATCAGATCTTTAATTAAG (SEQ. ID. NO. 67) and
GTACCTTAATTAAAGATCTGATAT (SEQ. ID. NO. 68), introduced sites for the *Pac*I*, Bgl*II*,* and *Eco*RV restriction endonucleases. This created the multiple cloning site MCSII for insertion of STAR elements. Then primers
GATCAGATCTGGCGCGCCATTTAAATCGTCTCGCGCGTTTCGGTGATGACG G (SEQ. ID. NO. 69) and
(AGGCGGATCCGAATGTATTTAGAAAAATAAACAAATAGGGG (SEQ. ID. NO. 70) were used to amplify a region of 0.37 kb from pd2EGFP, which was inserted into the *Bgl*II site of pIRES (Clontech 6028-1) to yield pIRES-stuf. This introduced sites for the *Asc*I and SwaI restriction endonucleases at MCSI, and acts as a "stuffer fragment" to avoid potential interference between STAR elements and adjacent promoters. pIRES-stuf was digested with *Bgl*II and *Fsp*I to liberate a DNA fragment composed of the stuffer fragment, the CMV promoter, the IRES element (flanked by multiple cloning sites MCS A and MCS B), and the SV40 polyadenylation signal. This fragment was ligated with the vector backbone of pd2EGFP-link produced by digestion with *BamHI* and *Stu*I*,* to yield pIRES-link.

The open reading frame of the zeocin-resistance gene was inserted into *Bam*HI/*Not*I sites downstream of the pIRES as follows: the zeocin-resistance ORF was amplified by PCR with primers
GATCGGATCCTTCGAAATGGCCAAGTTGACCAGTGC (SEQ. ID. NO. 71) and AGGCGCGGCCGCAATTCTCAGTCCTGCTCCTC (SEQ. ID. NO. 72) from plasmid pCMV/zeo (Invitrogen, cat.no. V50120), digested with *Bam*HI and *Not*I*,* and ligated with BamHI/NotI-digested pIRES-link to yield pIRES-link-zeo. The d2EGFP reporter ORF was introduced into pIRES-link-zeo by amplification of pd2EGFP (Clontech 6010-1) with primers
GATCGAATTCTCGCGAATGGTGAGCAAGCAGATCCTGAAG (SEQ. ID. NO. 73) and
AGGCGAATTCACCGGTGTTTAAACTTACACCCACTCGTGCAGGCTGCCCA GG (SEQ. ID. NO. 74), and insertion of the *Eco*RI-digested d2EGFP cassette into the *Eco*RI site in the pIRES-link-zeo plasmid. This created construct A, CMV-d2EGFP-IRES-Zeo (CMV Control).

STAR67 was cloned upstream of the CMV promoter, in the AscI site (about 15 nt remaining between STAR67 and the promoter). This created construct B, STAR67-CMV-d2EGFP-ires-Zeo (CMV-STAR67).

STAR67 was also tested in the context of a cassette that contains also the STAR7, cloned directionally in the 5' SalI and XbaI sites and 3' BglII and PacI sites to flank the entire cassette with STAR7. This is construct C (CMV-STAR67 7/7).

### Example 2: STAR67 enhances the expression level from CMV, EF1α and UB6 promoters in stably transfected CHO cells.

We tested whether the presence of STAR67 adjacent to the CMV, EF1α and UB6 promoters influences the expression level of these promoters in CHO cells. The constructs A and B (Fig. 1) described in Example 1 are used for this purpose, modified for the respective promoters:
1 CMV-d2EGFP-ires-Zeo (CMV Control)
2 STAR67-CMV-d2EGFP-ires-Zeo (CMV-STAR67)
3 EF1α-d2EGFP-ires-Zeo (EF1α Control)
4 STAR67-EF1α-d2EGFP-ires-Zeo (EF1α-STAR67)
5 UB6-d2EGFP-ires-Zeo (UB6 Control)
6 STAR67-UB6-d2EGFP-ires-Zeo (UB6-STAR67).

### Materials and Methods

The UB6 and EF1α promoters were exchanged for the CMV promoter in the plasmids described in Fig. 1. The UB6 promoter was cloned as follows. A DNA 0.37 kb stuffer from the pd2EGFP plasmid was amplified by PCR, as described in example 1, using primers identified by SEQ. ID. NOs. 69 and 70. The resulting DNA stuffer was cloned in the BglII site of pUB6N5-His [Invitrogen V250-20], creating pUB6-stuf. From pUB6-stuf an AscI-SacI fragment was cloned into CMV-d2EGFP-IRES-Zeo, from which the CMV promoter was removed.

The EF1α promoter was amplified by PCR with pEF1α/V5-His [Invitrogen V920-20] as template, using primers
GATCGGCGCGCCATTTAAATCCGAAAAGTGCCACCTGACG (SEQ. ID. NO. 79) and
AGGCGGGACCCCCTCACGACACCTGAAATGGAAG (SEQ. ID. NO. 80). The PCR fragment was cloned in the AscI and PpuMI sites of CMV-d2EGFP-IRES-Zeo, from which the CMV promoter was removed.

The Chinese Hamster Ovary cell line CHO-K1 (ATCC CCL-61) was cultured in HAMS-F12 medium + 10% Fetal Calf Serum containing 2 mM glutamine, 100 U/ml penicillin, and 100 micrograms/ml streptomycin at 37°C/5% CO₂. Cells were transfected with the plasmids using Lipofectamine 2000 (Invitrogen) as described by the manufacturer. Briefly, cells were seeded to culture vessels and grown overnight to 70-90% confluence. Lipofectamine reagent was combined with plasmid DNA at a ratio of 6 microliters per microgram (e.g. for a 10 cm Petri dish, 20 micrograms DNA and 120 microliters Lipofectamine) and added to the cells. After overnight incubation the transfection mixture was replaced with fresh medium, and the transfected cells were incubated further. After overnight cultivation, cells were trypsinized and seeded into fresh culture vessels with fresh medium. After another overnight incubation zeocin was added to a concentration of 50 µg/ml and the cells were cultured further. After another three days the medium was replaced by fresh medium containing zeocin (100 µg/ml) and cultured further. When individual colonies became visible (approximately ten days after transfection) medium was removed and replaced with fresh medium without zeocin. Individual clones were isolated and transferred to 24-well plates in medium without zeocin. One day after isolation of the colonies zeocin was added to the medium. Expression of the d2EGFP reporter gene was assessed approximately 3 weeks after transfection. d2EGFP expression levels in the colonies were measured after periods of two weeks. After the initial two weeks after transfection when the first d2EGFP measurements were performed, the colonies were cultured in medium without zeocin or other antibiotics. This continued for the remainder of the experiment.

### Results

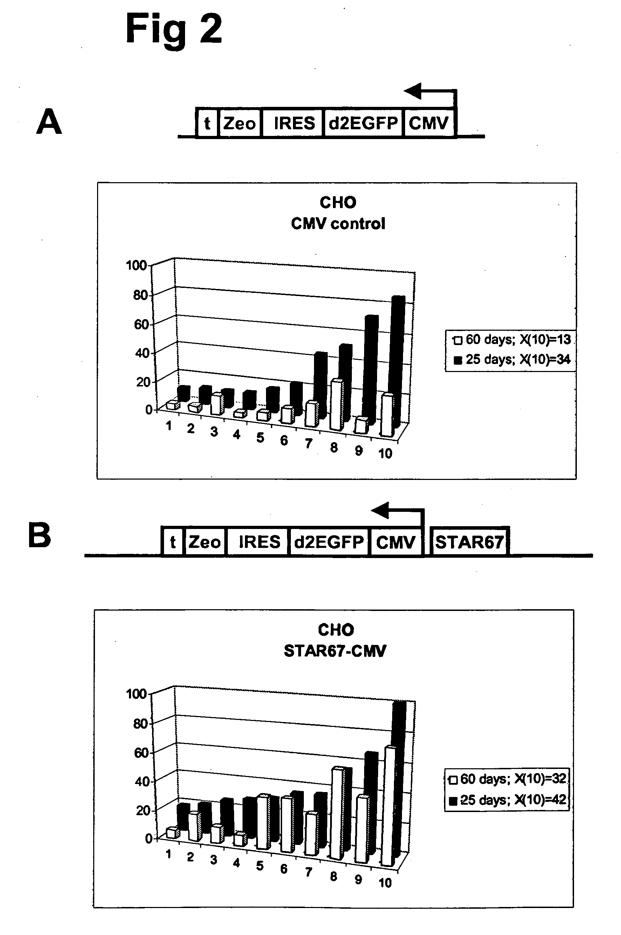

Fig. 2 shows that transfection of the construct that contains STAR67 cloned upstream of the CMV promoter resulted in a number of CHO colonies that express significantly higher levels of d2EGFP protein, as compared to the "empty" control without STAR67, CMV Control. The average of the d2EGFP signal in the 10 colonies transfected with the CMV Control plasmid was 34, when measured 25 days after transfection. 60 days after transfection the average of the d2EGFP signal in these 10 colonies was reduced to 13, indicating that expression is not stable over time. In comparison, the average of the d2EGFP signal in the 10 colonies transfected with the STAR67-CMV plasmid was 42 when measured after 25 days and 32 measured 60 days after transfection. Hence 60 days after transfection, a STAR67-encompassing CMV construct conveyed a factor 2.5 higher CMV promoter driven expression level of the reporter protein in stably transfected clones. Importantly, 25 days after transfection, after the first measurement, selection pressure was removed by culturing the colonies in medium without zeocin. Hence, colonies containing the STAR67 construct are more stable over time in the absence of selection pressure than colonies that do not contain a STAR67 construct.

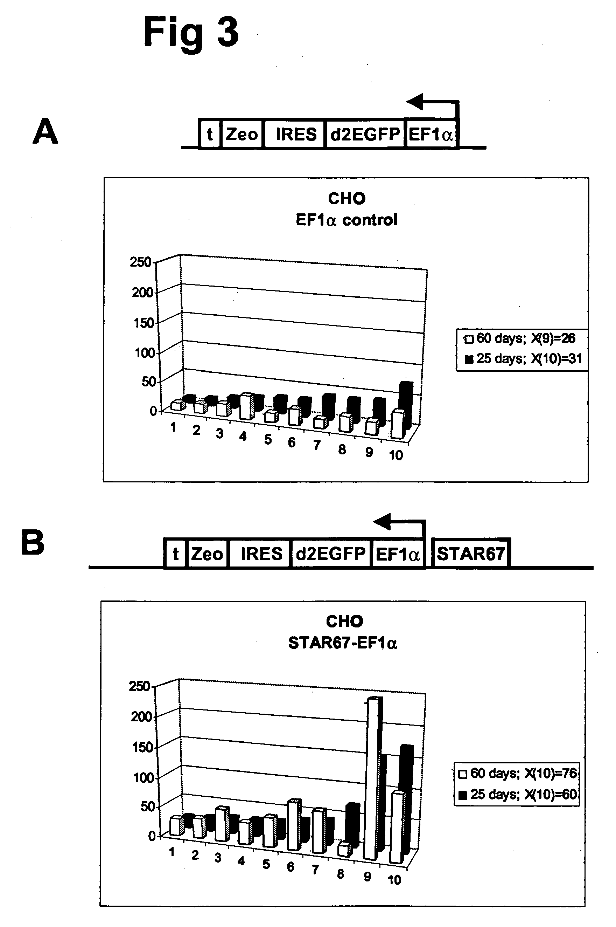

Fig. 3 shows that transfection of the construct that contains STAR67 cloned upstream of the EF1α- promoter resulted in a number of CHO colonies that express significantly higher levels of d2EGFP protein, as compared to the "empty" control without STAR67, EF1α Control. The average of the d2EGFP signal in the 10 colonies transfected with the EF1α Control plasmid was 31, when measured 25 days after transfection. 60 days after transfection the average of the d2EGFP signal in these 10 colonies was 26. In comparison, the average of the d2EGFP signal in the 10 colonies transfected with the EF1α-STAR67 plasmid was 60 when measured after 25 days and 76 measured 60 days after transfection. Hence, both after 25 and 60 days after transfection, a STAR67-encompassing EF1α construct conveyed a factor 2.9 higher EF1α promoter driven expression level of the reporter protein in stably transfected clones.

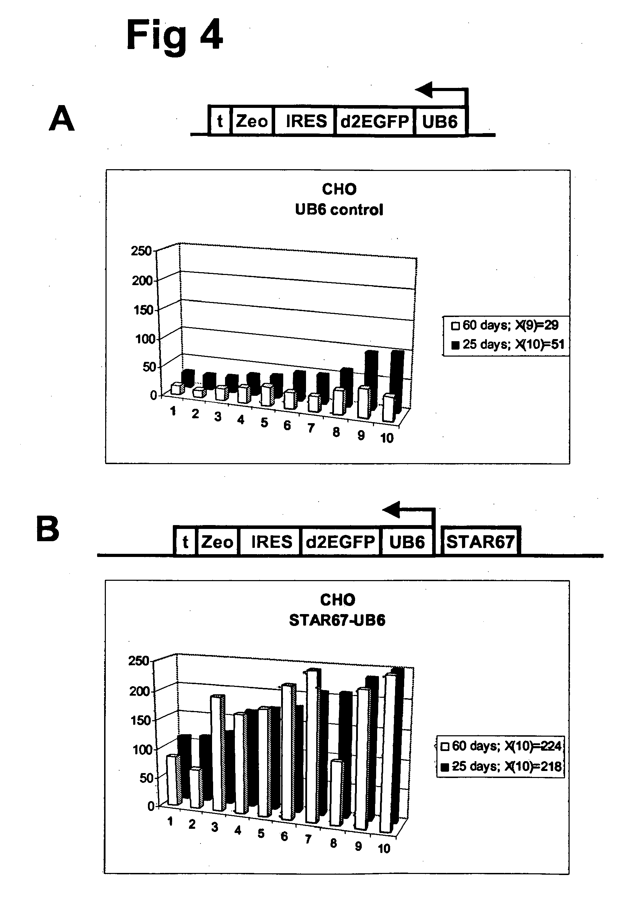

Fig. 4 shows that transfection of the construct that contains STAR67 cloned upstream of the UB6 promoter resulted in a number of CHO colonies that express significantly higher levels of d2EGFP protein, as compared to the "empty" control without STAR67, UB6 Control. The average of the d2EGFP signal in the 10 colonies transfected with the UB6 Control plasmid was 51, when measured 25 days after transfection. 60 days after transfection the average of the d2EGFP signal in these 10 colonies was 29, indicating that the expression was not stable over time. In comparison, the average of the d2EGFP signal in the 10 colonies transfected with the UB6-STAR67 plasmid was 218 when measured after 25 days and 224 measured 60 days after transfection. Hence, 25 days after transfection, a STAR67-encompassing UB6 construct conveyed a factor 4.3 higher UB6 promoter driven expression level of the reporter protein in stably transfected clones. After 60 days this factor was 7.7, due to instability of expression in the control colonies and stability in the UB6-STAR67 colonies. Importantly, 25 days after transfection, after the first measurement, selection pressure was removed by culturing the colonies in medium without zeocin. Hence, colonies containing the STAR67 construct are more stable over time in the absence of selection pressure than colonies that do not contain a STAR67 construct.

In conclusion, STAR67 increases expression from three different, unrelated promoters.

### Example 3: STAR67 enhances the expression level from CMV, EF1α and UB6 promoters in stably transfected PER.C6 cells.

We tested whether the presence of STAR67 adjacent of the CMV, EF1α and UB6 promoters influences the expression level of these promoters in another cell type than CHO cells, namely human PER.C6 cells. The same constructs as in example 1 were used.

### Materials and Methods

### Transfection, culturing and analysis of PER.C6 cells

PER.C6^{®} cells were cultured in DMEM medium + pyridoxine + 9% Foetal Bovine Serum (Non-Heat Inactivated), 8.9 mM MgCl₂ 100 U/ml penicillin, and 100 micrograms/ml streptomycin at 37°C/10% CO₂. Cells were transfected with the plasmids using Lipofectamine 2000 (Invitrogen) as described by the manufacturer. Briefly, cells were seeded to 6-wells and grown overnight to 70-90% confluence. Lipofectamine reagent was combined with plasmid DNA at a ratio of 15 microliters per 3 microgram (e.g. for a 10 cm Petri dish, 20 micrograms DNA and 120 microliters Lipofectamine) and added after 30 minutes incubation at 25°C to the cells. After 6-hour incubation the transfection mixture was replaced with fresh medium, and the transfected cells were incubated further. After overnight cultivation, cells were trypsinized and seeded (1:15, 1:30, 1:60, 1:120 dilutions) into fresh petri dishes (90 mm) with fresh medium with zeocin added to a concentration of 100 µg/ml and the cells were cultured further. When colonies became visible, individual clones were isolated by scraping and transferred to 24-well plates in medium with zeocin. When grown to ~70% confluence, cells were transferred to 6-well plates. Stable colonies were expanded for 2 weeks in 6-well plates before the d2EGFP signal was determined on a XL-MCL Beckman Coulter flowcytometer. The mean of the d2EGFP signal was taken as measure for the level of d2EGFP expression. Colonies were measured for a second time after 2 weeks. Thereafter colonies were further cultured in the absence of zeocin.

### Results

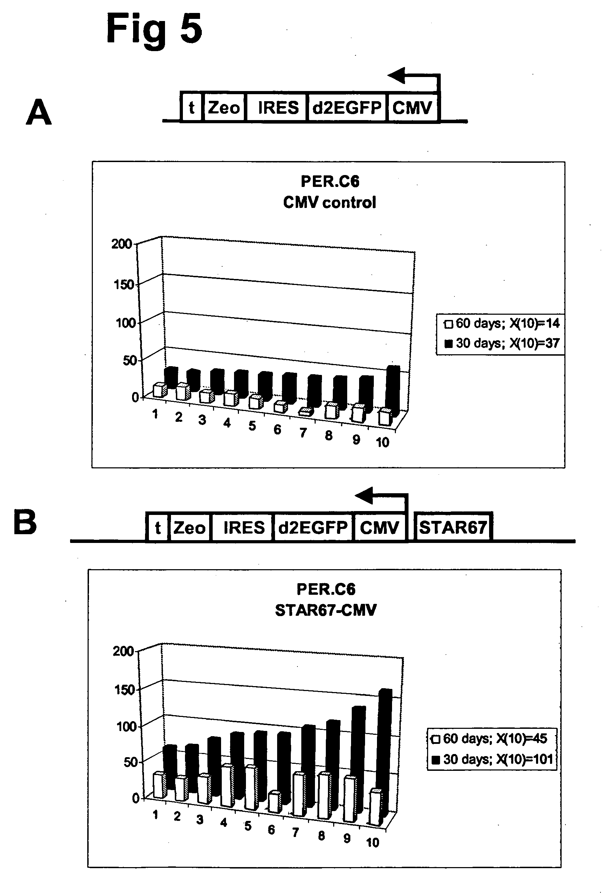

Fig. 5 shows that transfection of the construct that contains STAR67 cloned upstream of the CMV promoter resulted in a number of PER.C6 colonies that express significantly higher levels of d2EGFP protein, as compared to the "empty" control without STAR67, CMV Control. The average of the d2EGFP signal in the 10 colonies transfected with the CMV Control plasmid was 37, when measured 30 days after transfection. 60 days after transfection the average of the d2EGFP signal in these 10 colonies was reduced to 14, indicating that expression was not stable over time. In comparison, the average of the d2EGFP signal in the 10 colonies transfected with the STAR67-CMV plasmid was 101 when measured after 30 days and 45 measured 60 days after transfection. Hence 60 days after transfection, a STAR67-encompassing CMV construct conveyed a factor 3.2 higher CMV promoter driven expression level of the reporter protein in stably transfected clones.

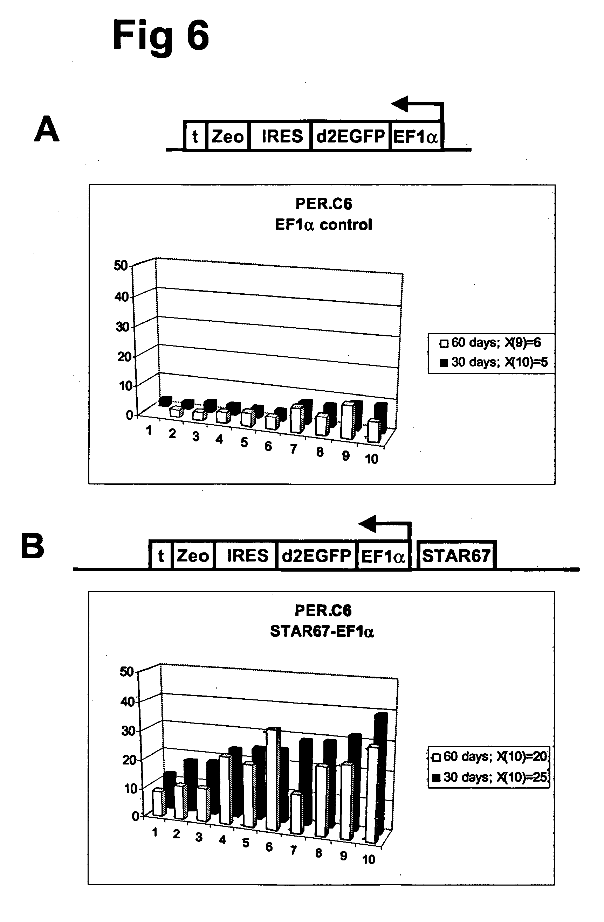

Fig. 6 shows that transfection of the construct that contains STAR67 cloned upstream of the EF1α- promoter resulted in a number of PER.C6 colonies that express significantly higher levels of d2EGFP protein, as compared to the "empty" control without STAR67, EF1α Control. The average of the d2EGFP signal in the 10 colonies transfected with the EF1α Control plasmid was 5, when measured 30 days after transfection. 60 days after transfection the average of the d2EGFP signal in these 10 colonies was 6. In comparison, the average of the d2EGFP signal in the 10 colonies transfected with the EF1α-STAR67 plasmid was 25 when measured after 30 days and 20 measured 60 days after transfection. Hence, both after 30 and 60 days after transfection, a STAR67-encompassing EF1α construct conveyed a factor 4 higher EF1α promoter driven expression level of the reporter protein in stably transfected clones.

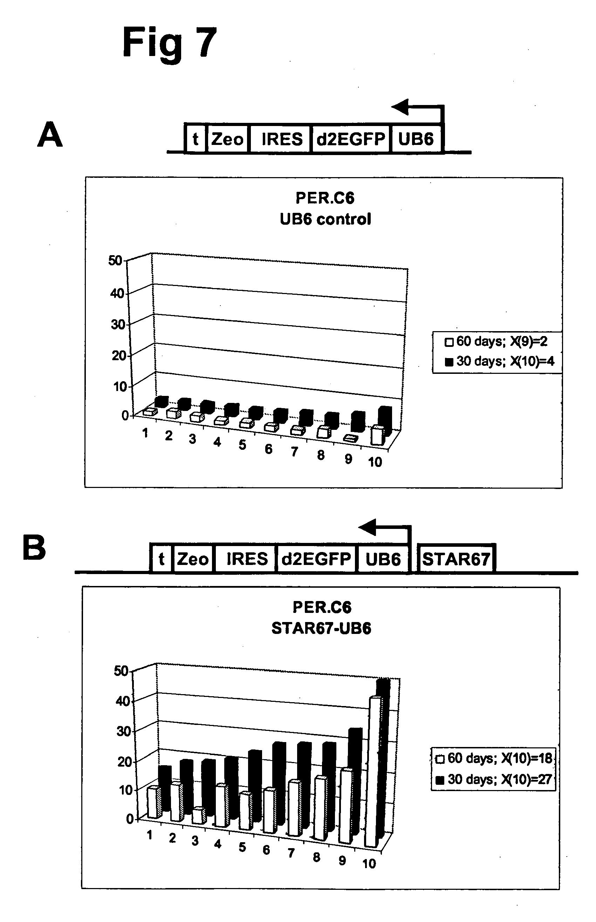

Fig. 7 shows that transfection of the construct that contains STAR67 cloned upstream of the UB6 promoter resulted in a number of PER.C6 colonies that express significantly higher levels of d2EGFP protein, as compared to the "empty" control without STAR67, UB6 Control. The average of the d2EGFP signal in the 10 colonies transfected with the UB6 Control plasmid was 4, when measured 30 days after transfection. 60 days after transfection the average of the d2EGFP signal in these 10 colonies was 2. In comparison, the average of the d2EGFP signal in the 10 colonies transfected with the UB6-STAR67 plasmid was 27 when measured after 30 days and 18 measured 60 days after transfection. Hence, both after 30 and 60 days after transfection, a STAR67-encompassing UB6 construct conveyed a factor 7 to 9 fold higher UB6 promoter driven expression level of the reporter protein in stably transfected clones.

Hence placing STAR67 upstream of the promoter resulted in significantly higher protein expression levels in comparison with STAR67-less constructs, also in PER.C6 cells. Hence STAR67 functions in different, unrelated cell types.

### Example 4: Novel configuration of STAR67 combined with other STAR elements to enhance the SV40 promoter in CHO cells.

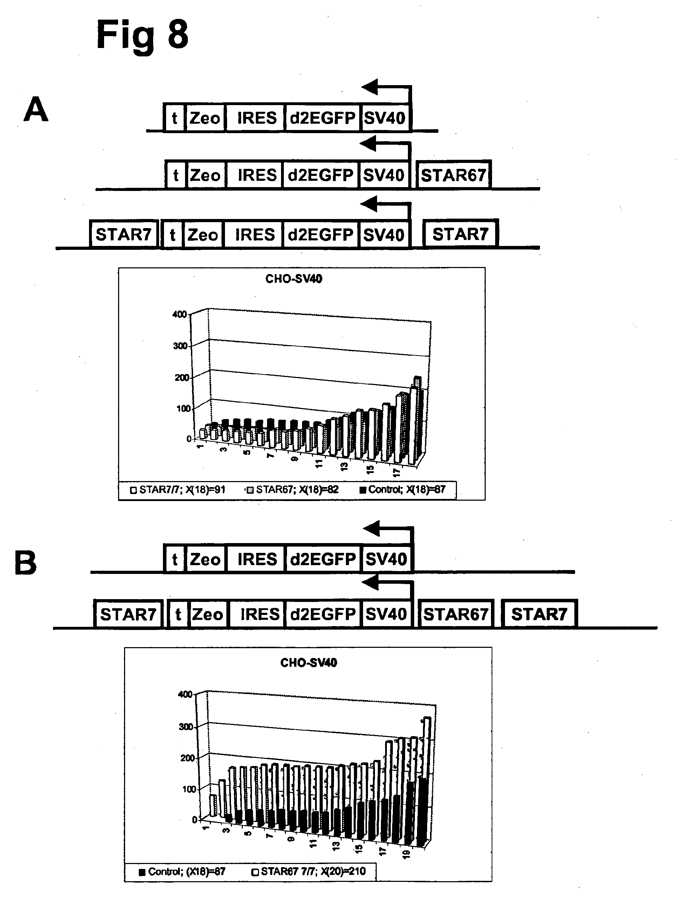

We tested whether the presence of STAR67 adjacent of the SV40 promoter influences the expression level of this promoter, either alone or in combination with another STAR element, in this example STAR7. The constructs that were used for this purpose (see Fig. 8), are:
1 SV40-d2EGFP-ires-Zeo (SV40 Control)
2 STAR67-SV40-d2EGFP-ires-Zeo (SV40-STAR67)
3 STAR7-SV40-d2EGFP-ires-Zeo-STAR7 (SV40-STAR7/7)
4 STAR7- STAR67-SV40--d2EGFP-ires-Zeo-STAR7 (SV40-STAR67 7/7)

### Materials and Methods

The SV40 promoter was amplified by PCR with pIRES as template using primers and The PCR fragment was cloned in the AscI and SacI sites of CMV-d2EGFP-IRES-Zeo, from which the CMV promoter was removed.

CHO cells were transfected, colonies were isolated and propagated and analysed as in Example 2.

### Results

Fig. 8 shows that transfection of the construct that either contains STAR67 cloned upstream of the SV40 promoter (SV40-STAR67) or STAR 7 cloned to flank the entire construct (SV40-STAR 7/7) did not result in CHO colonies that express significantly higher levels of d2EGFP protein, as compared to the "empty" control without STARs (SV40 Control). The average of the d2EGFP signal in the 18 colonies transfected with the SV40 Control plasmid was 86, when measured 40 days after transfection. In comparison, the average of the d2EGFP signal in the 18 colonies transfected with the SV40-STAR67 plasmid was 82 when measured after 40 days and the average of the d2EGFP signal in the 18 colonies transfected with the SV40-STAR 7/7 plasmid was 91 when measured after 40 days. Hence, no significant effect of these STAR elements on the SV40 promoter in CHO cells was observed.

It appears that the expression levels from the SV40 promoter in these cells are already quite high, and even much higher than those observed with the CMV promoter, which was considered to be a very strong promoter. This high background expression in the absence of a STAR element using the SV40 promoter in CHO cells, may explain why no significant effect of STAR67 alone, or STAR7 flanking the transgene, was observed.

However, the average of the d2EGFP signal in the 18 colonies transfected with the SV40-STAR67 7/7 plasmid was 209 when measured after 40 days colonies, which is a factor 2.4 higher than the average of the 18 control colonies (86). Hence, when the STAR67 element is used in combination with another STAR element, this results in a number of stably transfected CHO colonies that show significantly higher d2EGFP expression levels.

Therefore in this novel configuration (5'- STAR sequence A - STAR sequence C - promoter - nucleic acid encoding a protein of interest - STAR sequence B-3', wherein in the present example STAR sequences A and B are STAR 7 and STAR sequence C is STAR67) STAR elements appear to function even better than in hitherto disclosed configurations.

We have done experiments in which the flanking STAR7 elements were replaced by flanking STAR6 elements or by flanking STAR4 elements, in combination with STAR67 upstream of the SV40 promoter (SV40-STAR67 6/6 and SV40-STAR67 4/4, respectively, using the same nomenclature as above), and observed improved expression also with these combinations. This proves that the flanking STAR7 elements can indeed be exchanged for other STAR sequences, and still the improvement of the novel configuration of the expression cassette with the STAR sequences is observed.

### Example 5: A combination of STAR67 and STAR7 enhance UB6-driven antibody expression levels in stably transfected CHO cells.

In example 4 we showed that the combination of STAR67 and STAR7 enhanced the expression levels of d2EGFP protein in CHO cells. Here we tested whether the combination of STAR67 and STAR7 could be used for the production of an antibody. We chose an antibody against the EpCAM molecule (Huls et al, 1999) as test protein and used the UB6 promoter.

### Materials and Methods

### Plasmids

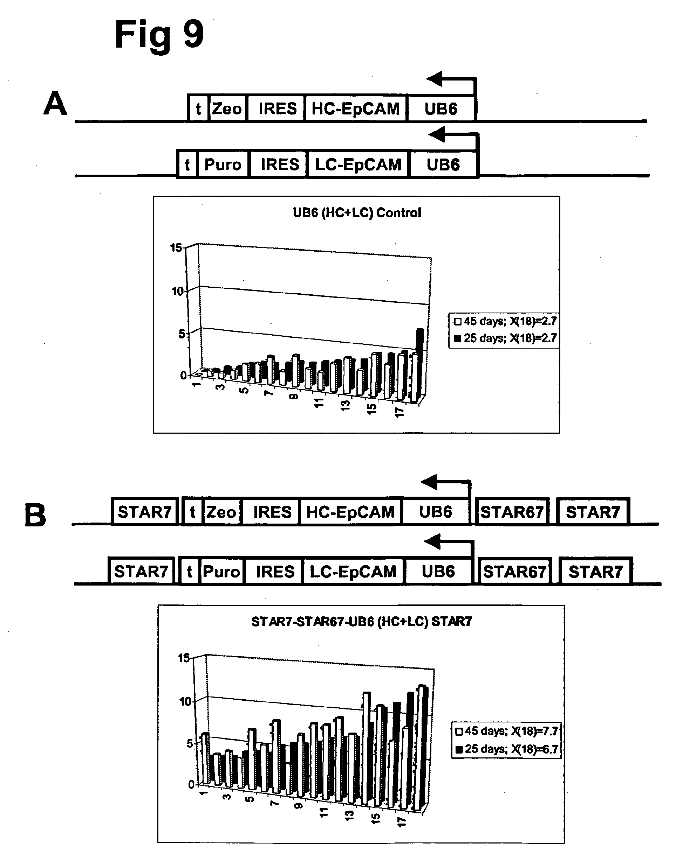

The heavy chain cDNA (HC-EpCAM) was cloned in a construct encompassing the UB6 promoter. The HC-EpCAM was coupled to the Zeocin resistance gene by an IRES sequence. The light chain cDNA (LC-EpCAM) was also cloned in a construct encompassing the UB6 promoter. The LC-EpCAM was coupled to the puromycin resistance gene by an IRES sequence. Together these two plasmids represent the UB6 (HC+LC) Control (Fig. 9)

To test the effects of STAR67 and STAR7, STAR67 was cloned in both HC+LC constructs, upstream of the UB6 promoters. STAR7 was cloned to flank the entire cassettes, both at the 5' and 3' end (Fig. 9). These two plasmids represent STAR7-STAR67-UB6 (HC+LC) STAR7.

### Transfection and culture of CHO cells

The Chinese Hamster Ovary cell line CHO-K1 (ATCC CCL-61) was transfected and cultured as in example 2, using zeocin (100 µg/ml) and puromycin (2.5 µg/ml) for selection. One day after transfection, zeocin was added to the culture medium. When first colonies became visible (approximately 7 days after addition of zeocin) the culture medium was removed and replaced with culture medium containing puromycin. After approximately 7 days, colonies were isolated and transferred to 24 wells plates, in culture medium containing zeocin only.

### Results

Fig. 9 shows that that transfection of antibody constructs that contain STAR67 cloned upstream of the UB6 promoter and two STAR7 elements cloned to flank the entire cassettes resulted in a number of CHO colonies that express significantly higher levels of EpCAM antibody (measured by ELISA using an anti-human IgG antibody) as compared to the "empty" control without STAR67 and STAR7, UB6 (HC+LC) Control. The average of the EpCAM production in the 18 colonies transfected with the UB6 (HC+LC) Control plasmid was 2.7 pg/cell/day, when measured 25 days after transfection. Selection agents zeocin and puromycin were removed after 25 days. 45 days after transfection the average of the EpCAM production in these 18 colonies was 2.7 pg/cell/day. In comparison, the average of the EpCAM production signal in the 18 colonies transfected with the STAR7-STAR67-UB6 (HC+LC)-STAR7 plasmid was 6.7 when measured after 25 days and 7.7 pg/cell/day, when measured 45 days after transfection. Hence, both after 30 and 45 days after transfection, a STAR67/STAR7-encompassing UB6 construct conveyed a factor 2.5 to 2.9 fold higher UB6 promoter driven EpCAM expression level in stably transfected CHO clones.

Hence placing STAR67 upstream of the promoter and two STAR7 elements to flank the cassettes resulted in significantly higher EpCAM antibody expression levels in CHO, in comparison with STAR67/STAR7-less constructs.

### Example 6: STAR67 is not an enhancer blocker, whereas STAR6 and STAR7 are.

All hitherto known STAR elements that were tested for that property, including STAR6 and STAR7, are enhancer blockers (WO 03/004704, Kwaks et al, 2003). Enhancer blocker activity is tested by placing a STAR element between a strong enhancer and a promoter. Here we tested whether also STAR67 is an enhancer blocker.

### Materials and Methods

The d2EGFP gene was PCR-amplified using primers
TTGGTTGGTCATGAATGGTGAGCAAGGGCGAGGAGCTGTTC (SEQ.ID.NO. 75) and ATTCTCTAGACTACACATTGATCCTAGCAGAAGCAC (SEQ.ID.NO. 76) and cloned into plasmid pGL3-promoter (Promega) using the NcoI and XbaI restriction sites to replace the Luciferase gene to create plasmid pGL3-promoter-GFP. A linker (created by annealing oligo's
CGATATCTTGGAGATCTACTAGTGGCGCGCCTTGGGCTAGCT (SEQ.ID.NO. 77) and
GATCAGCTAGCCCAAGGCGCGCCACTAGTAGATCTCCAAGATATCGAGCT (SEQ.ID.NO. 78), was cloned in the SacI and BglII sites to create multiple cloning sites. The original BglII site was destroyed upon ligation of the linker, creating a new unique BglII site within the linker DNA. The SV40-enhancer was cut from plasmid pGL3-basic (Promega) using BsaBI and BamHI and cloned into the pGL3-linker-promoter-GFP using the EcoRV and BglII sites creating plasmid pGL3-enhancer-promoter-GFP. The STAR40 element was placed upstream of the SV40 enhancer using KpnI and SacI sites to prevent action of the enhancer on upstream sequences. Finally, STAR elements 6, 7 and 67 were placed in between the SV40 enhancer and the SV40 minimal promoter using the SpeI and AscI restriction sites.

### Transfection and culture of CHO cells

The Chinese Hamster Ovary cell line CHO-K1 (ATCC CCL-61) is transfected as in example 2. One day after the transfection, d2EGFP levels are measured on an Epics XL flowcytometer (Beckman Coulter)

### Results

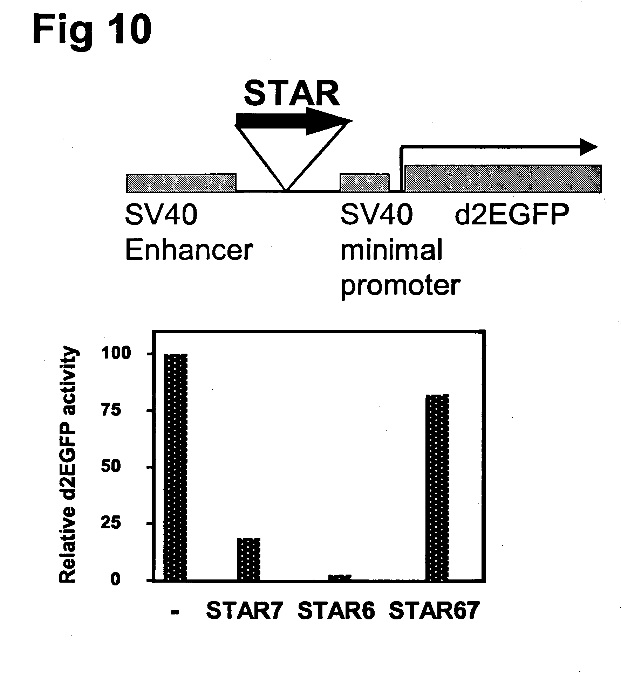

Fig. 10 shows that STAR67 is not an enhancer blocker, whereas STAR6 and STAR7 act enhancer blockers in the same assay. STAR6, STAR7 and STAR67 were cloned between the SV40 enhancer and a minimal SV40-promoter upstream of the d2EGFP gene. When no STAR element was cloned between the enhancer and the promoter strong transcriptional activation occurred (arbitrarily set at 100%). When STAR6 or STAR7 was placed between the enhancer and the promoter, transcription dropped to background levels, indicating that STAR6 and STAR7 are potent enhancer blockers. In contrast, when STAR67 was cloned between the enhancer and the promoter relative transcription levels were still 80% of the control, indicating that STAR67 is not a good enhancer blocker, this in contrast with STAR6 and STAR 7, as well as other STAR elements, as previously described (WO 03/004704, Kwaks et al, 2003).

### Example 7: STAR67 enhances UB6 and CMV-driven antibody expression levels in stably transfected CHO cells.

In example 5 we showed that the combination of STAR67 and STAR7 enhanced the expression levels of EpCAM antibody in CHO cells, in the context of two distinct plasmids, which contained the heavy and light chains. In this example we tested whether STAR67 could be used for the production of EpCAM antibody when both heavy and light chains are placed on one plasmid. We used simultaneous selection for each selectable marker.

### Materials and Methods

### Plasmids

The heavy chain cDNA (HC-EpCAM) is under the control of the UB6 promoter and coupled to the Zeocin resistance gene by an IRES sequence. The light chain cDNA (LC-EpCAM) is under control of the CMV promoter and coupled to the puromycin resistance gene by an IRES sequence. Basically these are the constructs used in example 5. These two expression cassettes were placed on one plasmid, in such a manner that transcription of the two expression units had opposite directions. In the control plasmid the UB6 and CMV promoters were separated by a stuffer of 500 bp(EpCAM Control) (Fig. 10). In another plasmid STAR67 was placed between the UB6 and CMV promoter (EpCAM STAR67) (Fig. 10).

### Transfection and culture of CHO cells

The Chinese Hamster Ovary cell line CHO-K1 (ATCC CCL-61) was transfected and cultured as in example 2, using zeocin (100 µg/ml) and puromycin (2.5 µg/ml) for selection. In example 5, consecutive selection for both selection markers was used. In contrast, here both selection agents were present in the culture medium simultaneously. One day after transfection, zeocin and puromycin were added to the culture medium. The selection medium was present until colonies were isolated (approximately 14 days after transfection). After colonies were isolated and transferred to 24-wells plates, the cells were cultured in the presence of zeocin and puromycin.

### Results

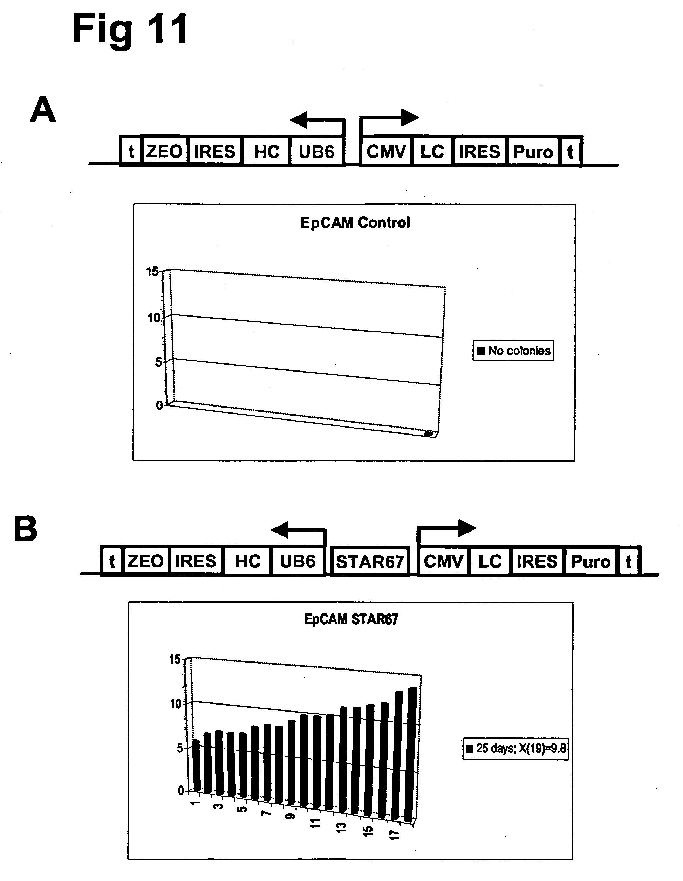

Fig. 11 shows that that transfection of the antibody construct that contains STAR67 cloned between the UB6 and CMV promoters resulted in a number of CHO colonies that express EpCAM antibody (measured by ELISA using an anti-human IgG antibody). The average of the EpCAM production in the 19 colonies transfected with the EpCAM STAR67 plasmid was 9.8 pg/cell/day, when measured 25 days after transfection.

In contrast, surprisingly no colonies survived of the transfection with the EpCAM Control plasmid. When selection was performed with either zeocin or puromycin alone, EpCAM Control colonies survived. However, when the selection pressure was increased by placing selection pressure on both the heavy and light chain, these conditions allowed only colonies to survive that have a STAR67 present in the transfected plasmid.

The results also show that incorporation of STAR67 has a beneficial effect on two promoters, the UB6 and CMV promoters, that are placed upstream and downstream of one STAR67 element. This indicates that STAR67 may operate in a bi-directional fashion.

The difference between the EpCAM control and EpCAM STAR67 plasmid is black-white in the sense that only transfection of EpCAM STAR67 results in the establishment of colonies, when the selection pressure is high. This opens an opportunity to use this plasmid configuration for identifying the region in STAR67 that is responsible for mediating this effect. Smaller, overlapping portions of STAR67 are placed between the UB6 and CMV promoters, driving the EpCAM molecule. When a portion of STAR67 is functional, colonies will survive when both zeocin and puromycin are simultaneously used as selection agent. When a portion of STAR67 is not functional, no colonies will survive under identical selection conditions.

Hence placing STAR67 upstream of the promoter resulted in significantly higher EpCAM antibody expression levels in CHO, in comparison with STAR-less constructs.

### Example 8: Construction and testing of a Zeocin resistance gene product with no internal methionine

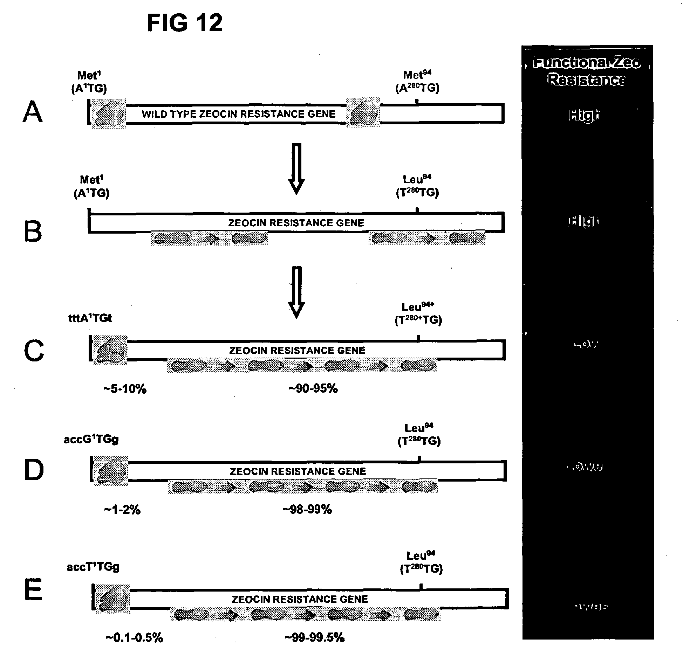

The basic idea behind the development of a novel selection system is to place the gene encoding the resistance gene upstream of a gene of interest, and one promoter drives the expression of this bicistronic mRNA. The translation of the bicistronic mRNA is such that only in a small percentage of translation events the resistance gene will be translated into protein and that most of the time the downstream gene of interest will be translated into protein. Hence the translation efficiency of the upstream resistance gene must be severely hampered in comparison to the translation efficiency of the downstream gene of interest. To achieve this, three steps can be taken according to the invention:
1) within the resistance gene on the mRNA, the searching ribosome preferably should not meet another AUG, since any downstream AUG may serve as translation start codon, resulting in a lower translation efficiency of the second, downstream gene of interest. Hence, preferably any AUG in the resistance gene mRNA will have to be replaced. In case this AUG is a functional codon that encodes a methionine, this amino acid will have to be replaced by a different amino acid, for instance by a leucine (Fig. 12A and B);
2) the start codon of the resistance gene must have a bad context (be part of a non-optimal translation start sequence); i.e. the ribosomes must start translation at this start codon only in a limited number of events, and hence in most events continue to search for a better, more optimal start codon (Fig. 12C-E). Three different stringencies can be distinguished: a) the normal ATG startcodon, but placed in a bad context (*TTT*ATG*T*) (called ATGmut) (Fig. 12C), b) preferably when placed in an optimal context, GTG can serve as startcodon (*ACC*GTG*G*) (Fig. 12D) and c) preferably when placed in an optimal context, TTG can serve as startcodon (*ACC*TTG*G*) (Fig. 12E). The most stringent translation condition is the TTG codon, followed by the GTG codon (Fig. 12). The Zeo mRNA with a TTG as start codon is expected to produce the least Zeocin resistance protein and will hence convey the lowest functional Zeocin resistance to cells (Figs. 12, 13).
3) preferably, the normal start codon (ATG) of the downstream gene of interest should have an optimal translation context (e.g. *ACC*ATG*G*)(Fig. 13A-D). This warrants that, after steps 1 and 2 have been taken, in most events the start codon of the gene of interest will function as start codon of the bicistronic mRNA.

In example 8, step 1 is performed, that is, in the Zeocin resistance gene one existing internal methionine is replaced by another amino acid (Fig. 12B-E). It is important that after such a change the Zeo protein still confers Zeocin resistance to the transfected cells. Since it is not known beforehand which amino acid will fulfill this criterium, three different amino acids have been tried: leucine, threonine and valine. The different constructs with distinct amino acids have than been tested for their ability to still confer Zeocin resistance to the transfected cells.

### Materials and methods

### Construction of the plasmids

The original Zeo open reading frame has the following sequence around the startcodon: AA*ACC**ATG**G*CC (startcodon in bold; SEQ. ID. NO. 83). This is a startcodon with an optimal translational context (Fig. 12A). First the optimal context of the start codon of the Zeo open reading frame was changed through amplification from plasmid pCMV-zeo [Invitrogen V50120], with primer pair ZEOforwardMUT (SEQ. ID. NO. 84): GATCTCGCGATACAGGA*TTT**ATG**T*TGGCCAAGTTGACCAGTGCCGTTCCG and ZEO-WTreverse (WT=Wild type; SEQ. ID. NO. 85):
AGGCGAATTCAGTCCTGCTCCTCGGC, using pCMV-ZEO (Invitrogen; V50120) as a template. The amplified product was cut with NruI-EcoRI, and ligated into pcDNA3, resulting in pZEOATGmut.

The original Zeo open reading frame contains an in frame ATG, encoding methionine at amino acid position 94 (out of 124). This internal ATG, encoding the methionine at position 94 was changed in such a way that the methionine was changed into leucine, threonine or valine respectively:
1) To replace the internal codon for methionine in the Zeo open reading frame with the codon for leucine (Fig. 12B), part of the Zeo open reading frame was amplified using primer pair ZEOforwardMUT (SEQ. ID. NO. 84) and ZEO-LEUreverse (SEQ. ID. NO. 86):
   AGGCCCCGCCCCCACGGCTGCTCGCCGATCTCGGT**CAA**GGCCGGC.The PCR product was cut with BamHI-BgII and ligated into pZEOATGmut. This resulted in pZEO(leu).
   To replace the internal codon for methionine in the Zeo open reading frame with the codon for threonine (not shown, but as in Fig. 12B), part of the Zeo open reading frame was amplified using primer pair ZEOforwardMUT (SEQ. ID. NO. 84) and ZEO-THRreverse (SEQ. ID. NO. 87):
   AGGCCCCGCCCCCACGGCTGCTCGCCGATCTC**GGT**GGTGGCCGGC. The PCR product was cut with BamHI-BglI and ligated into pZEOATGmut. This resulted in pZEO(thr).
   To replace the internal codon for methionine in the Zeo open reading frame with the codon for valine (not shown, but as in Fig. 12B)(GTG), part of the Zeo open reading frame was amplified using primer pair ZEOforwardMUT (SEQ. ID. NO. 84) and ZEO-VALreverse (SEQ. ID. NO. 88):
   AGGCCCCGCCCCCACGGCTGCTCGCCGATCTCGGTC**CAC**GCCGG. The PCR product was cut with BamHI-BgII and ligated into pZEOATGmut. This resulted in pZEO(val).

### Transfection and culturing of cells

The Chinese Hamster Ovary cell line CHO-K1 (ATCC CCL-61) was cultured in HAMS-F12 medium + 10% Fetal Calf Serum containing 2 mM glutamine, 100 U/ml penicillin, and 100 micrograms/ml streptomycin at 37°C/5% CO₂. Cells were transfected with the plasmids using Lipofectamine 2000 (Invitrogen) as described by the manufacturer. Briefly, cells were seeded to culture vessels and grown overnight to 70-90% confluence. Lipofectamine reagent was combined with plasmid DNA at a ratio of 6 microliters per microgram (e.g. for a 10 cm Petri dish, 20 micrograms DNA and 120 microliters Lipofectamine) and added to the cells. After overnight incubation the transfection mixture was replaced with fresh medium, and the transfected cells were incubated further. After overnight cultivation, cells were trypsinized and seeded into fresh culture vessels with fresh medium containing zeocin (100 µg/ml). When individual colonies became visible (approximately ten days after transfection) colonies were counted.

### Results

Four plasmids were transfected to CHO-K1 cells, 1) pZEO(WT), 2) pZEO(leu), 3) pZEO(thr), and 4) pZEO(val). The cells were selected on 100 µg/ml zeocine. Transfection of pZEO(leu) resulted in an equal number of zeocin resistant colonies in comparison with the control pZEO (WT). pZEO(thr) and pZEO(val) gave less colonies, but the differences were not in the order of a magnitude. Hence it was concluded that changes of the internal methionine into leucine, threonine or valine all resulted in a Zeocin resistance protein that is still able to confer zeocin resistance to the transfected cells. Rather arbitrarily, pZEO(leu) was chosen as starting point for creating different startcodons on the Zeo open reading frame. Hence in the examples below the start as well as internal methionines are always replaced by leucine, for zeocin, but also for other selectable marker genes, as will be clear from further examples.

### Example 9: Creation and testing of Zeocin-d2EGFP bicistronic constructs with differential translation efficiencies.

To create a bicistronic mRNA encompassing a mutated Zeocin resistance mRNA with less translational efficiency, and the d2EGFP gene as downstream gene of interest, the start codon of the d2EGFP gene was first optimized (step 3 in example 8). After that, the different versions of the Zeocin resistance gene were created. The differences between these versions are that they have different start codons, with distinct translational efficiency (step 2 in Example 9, Figure 12C-E). These different Zeocin resistance gene versions were cloned upstream of the modified d2EGFP gene (Fig. 13).

### Materials and methods

### Creation of plasmids

The d2EGFP reporter ORF was introduced into pcDNA3. The sequence around the startcodon of this d2EGFP cDNA is GAA*TTC**ATG**G*G (startcodon in bold; SEQ. ID. NO. 89), which is not optimal. As a first step, d2EGFP was amplified from pd2EGFP (Clontech 6010-1) with primers d2EGFPforwardBamHI (SEQ. ID. NO. 90):
GATCGGATCCTATGAGGAATTCGCCACCA TGGTGAGCAAGGGCGAGGAG and d2EGFPreverseNotI (SEQ. ID. NO. 91):
AAGGAAAAAAGCGGCCGCCTACACATTGATCCTAGCAGAAG. This product contains now a startcodon with an optimal translational context (*ACC**ATG**G*). This created pd2EGFP and subsequently, the Zeo open reading frame was ligated into pd2EGFP, resulting in pZEO-d2EGFP. It is pointed out here that the optimization of the translational start sequence of the gene of interest (here: EGFP as a model gene) is not essential but preferred in order to skew the translation initiation frequency towards the gene of interest still further.

Now three classes of constructs were made:
1) ATG as a start codon in the Zeo resistance gene, but in a bad context (*TTT**ATG**T*) (not shown, but as in Fig. 13B) and followed by spacer sequence, instead of the optimal ATG (Fig. 13A).
   The spacer sequence is placed downstream of the ATG sequence. In the zeocin (and possibly in the blasticidin) RNA, a secondary structure is present, causing the ribosome to be temporarily delayed. Because of this, a poor startcodon can in some cases be used by the ribosome, despite being a bad startcodon or being in a non-optimal context for translation initiation. This causes the chance of translation to increase, and in case of the current invention therefore renders the stringency for selection lower. To decrease this effect, and hence to further decrease the translation initation efficiency, a spacer sequence is introduced that does not contain a secondary structure (Kozak, 1990). Hence, the term 'space' is introduced, and used in the plasmid and primer names to indicate the presence of such a spacer sequence. The spacer removes the 'ribosome delaying sequence' from the neighbourhoud of the initiation codon, therewith causing the ribosome to start translating less frequently, and hence increasing the stringency of the selection according to the invention. The spacer introduces some extra amino acids in the coding sequence. This has been done in some cases for both zeocin and for blasticidin, as will be apparent from the examples. The nomenclature of the plasmids and primers in general in the following is along these lines: the name of the selectable marker polypeptide is referred to by abbreviation (e.g. Zeo, Blas, etc); the startcodon is mentioned (e.g. ATG, GTG, TTG); when this startcodon is placed in a non-optimal context for translation initiation, the addition "mut" is used (this is usually only done for ATG startcodons, as combining a non-optimal context with a non-ATG startcodon usually does not result in sufficient translation initiation to allow for selection); when a spacer sequence is used behind the startcodon, the addition "space" is used (this is done usually for "ATGmut" startcodons for Zeo or Blas selectable markers).
   The Zeo open reading frame was amplified with primer pair ZEOforwardBamHI-**ATG**mut/space (SEQ. ID. NO. 93): (wherein the sequence following the underlined sequence comprises the spacer sequence), and ZEOWTreverse (SEQ. ID. NO. 85), the PCR product was cut with EcoRI-BamHI, and ligated into pd2EGF, cut with EcoRI-BamHI, creating pZEO**ATGmut/space**-d2EGFP.
2) GTG as a start codon in the Zeo resistance gene, instead of ATG (Fig. 13C).
   The Zeo open reading frame was amplified with primer pair ZEOforwardBamHI-**GTG** (SEQ. ID. NO. 94):
   GATCGGATCC*ACC**GTG***GCCAAGTTGACCAGTGCCGTTC
   and ZEOWTreverse (SEQ. ID. NO. 85), the PCR product was cut with EcoRI-BamHI, and ligated into pd2EGFP, cut with EcoRI-BamHI, creating pZEO-GTG-d2EGFP.
3) TTG as a start codon in the Zeo resistance gene, instead of ATG (Fig. 13D). The Zeo open reading frame was amplified with primer pair ZEOforwardBamHI-**TTG**:
   GATCGGATCC*ACC**TTG**G*CCAAGTTGACCAGTGCCGTTC (SEQ. ID. NO. 95) and ZEOWTreverse (SEQ. ID. NO. 85), the PCR product was cut with EcoRI-BamHI, and ligated into pd2EGFP, cut with EcoRI-BamHI, creating pZEO-**TTG-**d2EGFP.

### Transfection, culturing and analysis of CHO cells

The Chinese Hamster Ovary cell line CHO-K1 (ATCC CCL-61) was cultured in HAMS-F12 medium + 10% Fetal Calf Serum containing 2 mM glutamine, 100 U/ml penicillin, and 100 micrograms/ml streptomycin at 37°C/5% CO₂. Cells were transfected with the plasmids using Lipofectamine 2000 (Invitrogen) as described by the manufacturer. Briefly, cells were seeded to culture vessels and grown overnight to 70-90% confluence. Lipofectamine reagent was combined with plasmid DNA at a ratio of 15 microliters per 3 microgram (e.g. for a 10 cm Petri dish, 20 micrograms DNA and 120 microliters Lipofectamine) and added after 30 minutes incubation at 25⁰C to the cells. After overnight incubation the transfection mixture was replaced with fresh medium, and the transfected cells were incubated further. After overnight cultivation, cells were trypsinized and seeded into fresh culture vessels with fresh medium. After another overnight incubation zeocin was added to a concentration of 50 µg/ml and the cells were cultured further. After another three days the medium was replaced by fresh medium containing zeocin (100 µg/ml) and cultured further. When individual colonies became visible (approximately ten days after transfection) medium was removed and replaced with fresh medium without zeocin. Individual clones were isolated and transferred to 24-well plates in medium without zeocin. One day after isolation of the colonies zeocin was added to the medium. Expression of the d2EGFP reporter gene was assessed approximately 3 weeks after transfection. d2EGFP expression levels in the colonies were measured after periods of two weeks.

### Results

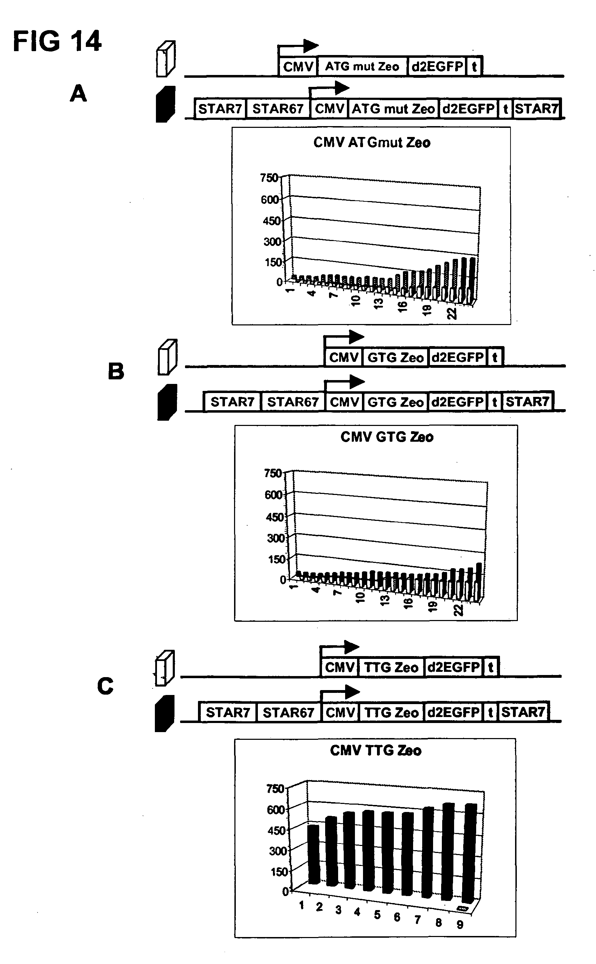

CHO-K1 cells were transfected with constructs that contain the ATGmut/space Zeo (Fig. 13B), GTG Zeo (Fig. 13C) and TTG Zeo (Fig. 13D) genes as selection gene, all being cloned upstream of the d2EGFP reporter gene. These three constructs were without STAR elements (Control) or with STAR elements 7 and 67 upstream of the CMV promoter and STAR 7 downstream from the d2EGFP gene (Fig. 14). Fig. 14 shows that both the control (without STAR elements) constructs with ATGmut/space Zeo (A) and GTG Zeo (B) gave colonies that expressed d2EGFP protein. The average d2EGFP expression level of 24 ATGmut/space Zeo colonies was 46 and of GTG Zeo colonies was 75. This higher average expression level in GTG Zeo colonies may reflect the higher stringency of GTG, in comparison with ATGmut/space (example 8). Addition of STAR elements 7 and 67 to the constructs resulted in colonies that had higher average d2EGFP expression levels. Transfection of the ATGmut/space Zeo STAR 7/67/7 construct resulted in colonies with an average d2EGFP expression level of 118, which is a factor 2.6 higher than the average in the control cells (46). Addition of STAR elements to the GTG Zeo construct resulted in an average d2EGFP expression level of 99, which is a factor 1.3 higher than the average in the control cells (75).

Importantly, no colonies were established when the TTG Zeo construct was transfected. However, the construct with TTG Zeo, flanked with STARs 7 and 67 resulted in the establishment of 6 colonies, with an average d2EGFP expression level of 576 (Fig. 14C). Thus the highest translation stringency, brought about by the TTG startcodon (Fig. 12) yields to the highest d2EGFP expression levels, as predicted in Fig. 13. The results also indicate that the stringency of the TTG Zeo alone (without STAR elements) is at least in some experiments too high for colonies to survive. However, in later independent experiments (see below), some colonies were found with this construct without STAR elements, indicating that the stringency of the selection system with the TTG startcodon in the zeocin selection marker not necessarily precludes the finding of colonies when no STAR elements are present, and that the number of colonies obtained may vary between experiments.

It is concluded that the use of STAR elements in combination with the stringent selection system according to the invention allows to readily identify high producers of the gene of interest.

### Example 10: Establishment of a higher number of TTG Zeo STAR colonies and comparison with an IRES-Zeo construct.

The results in example 9 indicate that the TTG Zeo has extremely stringent translation efficiency, which might be to high to convey Zeocin resistance to the cells. The transfection was scaled up to test whether there would be some colonies that have such high expression levels that they survive. Scaling up the experiment could also address the question whether the high average of TTG Zeo STAR 7/67/7 would become higher when more colonies were analyzed.

### Materials and methods

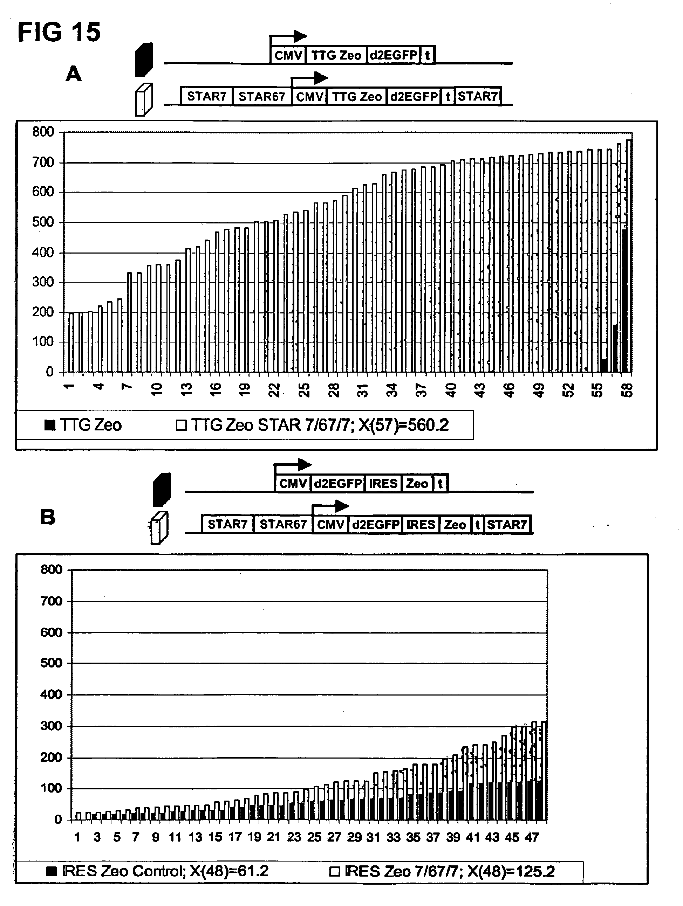

CHO-K1 cells were transfected with the constructs that have the TTG Zeo gene as selection marker, with and without STAR elements 7 and 67 (Fig. 15). Transfections, selection, culturing etc were as in example 9, except that 6 times more cells, DNA and Lipofectamine 2000 were used. Transfections and selection were done in Petri dishes.

### Results

Fig. 15A shows that transfection with the TTG Zeo STAR 7/67/7 construct resulted in the generation of many colonies with an average d2EGFP signal of 560. This is as high as in example 9, except that now 58 colonies were analyzed. When compared to a construct with the Zeocin resistance gene placed behind an IRES sequence (Fig 15B), the average d2EGFP expression level was 61, and when STAR elements 7 and 67 were added to such a construct, the average d2EGFP expression level was 125, a factor 2 above the control (Fig. 15B). The average of the TTG Zeo STAR 7/67/7 colonies was therefore a factor 9.2 higher than the STAR-less IRES-Zeo colonies and a factor 4.5 higher than the STAR7/67/7 IRES Zeo colonies.

An observation is that the form of the curve of all expressing colonies differs between the TTG Zeo STAR7/67/7 and IRES-Zeo STAR 7/67/7. In the first case (TTG Zeo) the curve levels off, whereas in the second case (IRES-Zeo) the curve has a more 'exponential' shape. The plateau in the TTG Zeo curve could indicate that the cells have reached a maximum d2EGFP expression level, above which the d2EGFP expression levels become toxic and the cells die. However, it later appeared that the high values were close to the maximum value that could be detected with the settings of the detector of the FACS analyser. In later experiments, the settings of the FACS analyser were changed to allow for detection of higher values, and indeed in some instances higher values than obtained here were measured in later independent experiments (see below).

Due to up-scaling of the transfections three colonies with the STAR-less TTG Zeo construct could be picked. The d2EGFP expression levels of these colonies were 475, 158 and 43. The last colony died soon after the first measurement. This result indicates that the TTG Zeo construct can convey Zeocin resistance, resulting in colonies that also can give high expression levels in some instances. Hence, the novel selection method according to the invention can be applied with expression cassettes that do not contain chromatin control elements, although it is clearly preferred to use expression cassettes comprising at least one such element, preferably a STAR element.

The results indicate that STAR elements allow a more stringent selection system according to the invention, such as exemplified in this example, resulting in the picking of colonies that have a very high average protein expression level.

### Example 11: Creation and testing of Blasticidin-d2EGFP bicistronic constructs with differential translation efficiencies.

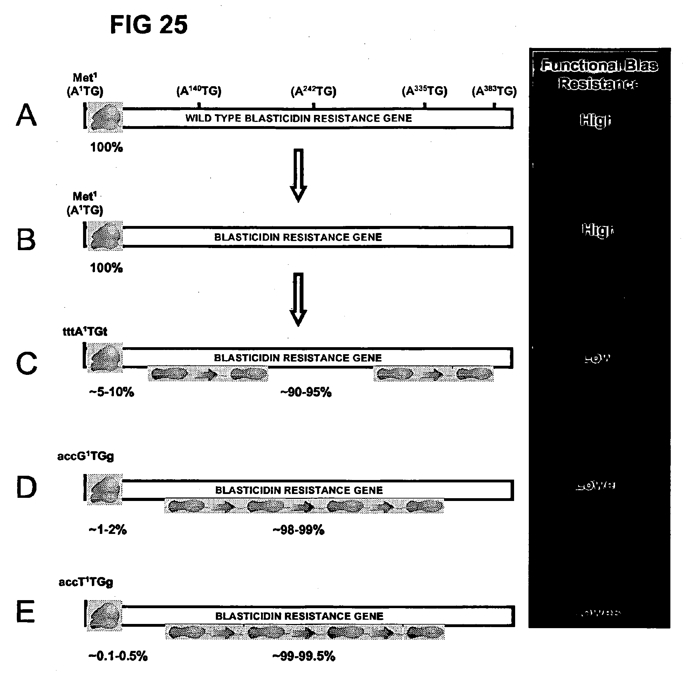

There are four internal ATGs in the blasticidine resistance gene, none of which codes for a methionine (Fig. 25A). These ATGs have to be eliminated though (Fig. 25B), since they will serve as start codon when the ATG startcodon (or the context thereof) has been modified, and this will result in peptides that do not resemble blasticidine resistance protein. More importantly, these ATGs will prevent efficient translation of the gene of interest, as represented by d2EGFP in this example for purposes of illustration. To eliminate the internal ATGs, the blasticidine resistance protein open reading frame was first amplified with 4 primer pairs, generating 4 blasticidine resistance protein fragments. The primer pairs were:
A) BSDBamHIforward (SEQ. ID. NO. 96):
   GATCGGATCCACCATGGCCAAGCCTTTGTCTCAAG
   BSD 150reverse (SEQ. ID. NO. 97):
   GTAAAATGATATACGTTGACACCAG
B) BSD 150forward (SEQ. ID. NO. 98): CTGGTGTCAACGTATATCATTTTAC
   BSD250reverse (SEQ. ID. NO. 99): GCCCTGTTCTCGTTTCCGATCGCG
C) BSD250forward (SEQ. ID. NO. 100):
   CGCGATCGGAAACGAGAACAGGGC
   BSD350reverse (SEQ. ID. NO. 101): GCCGTCGGCTGTCCGTCACTGTCC
D) BSD350forward (SEQ. ID. NO. 102):
   GGACAGTGACGGACAGCCGACGGC
   BSD399reverse (SEQ. ID. NO. 103):
   GATCGAATTCTTAGCCCTCCCACACGTAACCAGAGGGC

Fragments A to D were isolated from an agarose gel and mixed together. Next, only primers BSDBamHIforward and BSD399reverse were used to create the full length blasticidine resistance protein cDNA, but with all internal ATGs replaced. The reconstituted blasticidine was then cut with EcoRI-BamHI, and cloned into pZEO-**GTG**-d2EGFP, cut with EcoRI-BamHI (which releases Zeo), resulting in pBSDmut-d2EGFP. The entire blasticidine resistance protein open reading frame was sequenced to verify that all ATGs were replaced.

With this mutated gene encoding blasticidine resistance protein (Blas), three classes of constructs are made (Fig. 25C-E):
1) ATG as a start codon, but in a bad context and followed by spacer sequence. The mutated blasticidine resistance protein open reading frame in pBSD-d2EGFP was amplified using primers BSDforwardBamHIAvrII-**ATGmut/space** (SEQ. ID. NO. 104): and BSD399reverseEcoRIAvrII (SEQ. ID. NO. 105):
   GATCGAATTCCCTAGGTTAGCCCTCCCACACGTAACCAGAGGGC,
   the PCR product is cut with BamHI-EcoRI, and ligated into pZEO-**GTG**-d2EGFP, cut with EcoRI-BamHI. This results in pBSD-**ATGmut/space**-d2EGFP.
2) GTG as a start codon instead of ATG. The mutated blasticidine resistance protein open reading frame in pBSD-d2EGFP was amplified using primers BSDforwardBamHIAvrII-**GTG** (SEQ. ID. NO. 106): GATCGGATCCTAGG*ACC**GTG**G*CCAAGCCTTTGTCTCAAGAAG
   and BSD399reverseEcoRIAvrII (SEQ. ID. NO. 105), the PCR product was cut with BamHI-EcoRI, and ligated into pZEO-**GTG**-d2EGFP, cut with EcoRI-BamHI. This results in pBSD-**GTG**-d2EGFP.
3) TTG as a start codon instead of ATG. The mutated blasticidine open reading frame in pBSD-d2EGFP was amplified using primers BSDforwardBamHIAvrII-**TTG** (SEQ. ID. NO. 107):
   GATCGGATCCTAGG*ACC**TTG**G*CCAAGCCTTTGTCTCAAGAAG
   and BSD399reverseEcoRIAvrII (SEQ. ID. NO. 105), the PCR product was cut with BamHI-EcoRI, and ligated into pZEO-**GTG**-d2EGFP, cut with EcoRI-BamHI. This results in pBSD-**TTG**-d2EGFP.

### Results

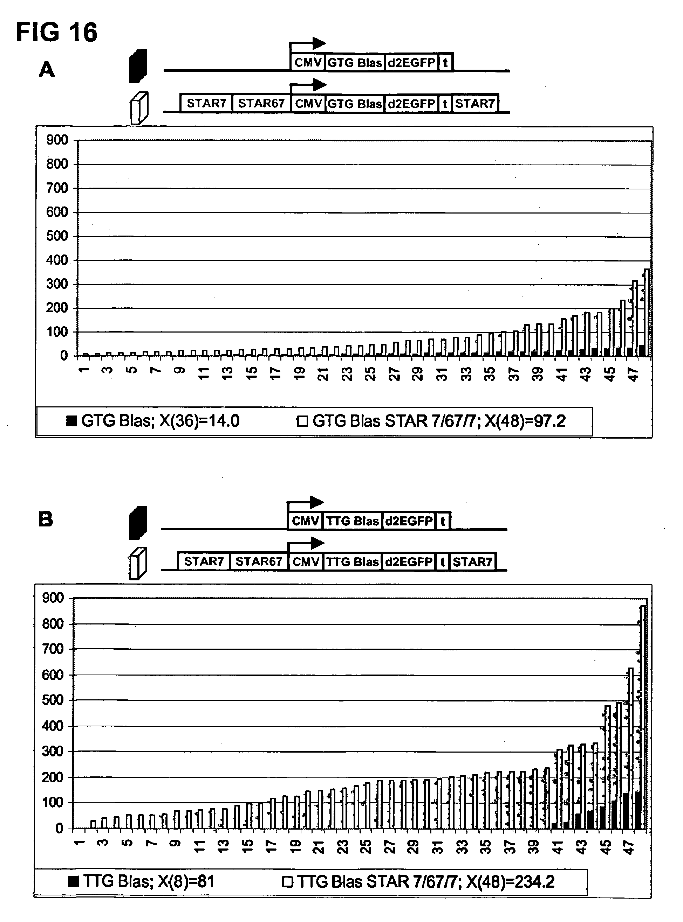

CHO-K1 cells were transfected with constructs that contain the GTG Blas (Fig. 16A) and TTG Blas (Fig. 16B) genes as selection gene, all being cloned upstream of the d2EGFP reporter gene. Selection took place in the presence of 20 µg/ ml Blasticidine. The two constructs were without STAR elements (Control) or with STAR elements 7 and 67 upstream of the CMV promoter and STAR7 downstream from the d2EGFP gene (Fig. 16). Fig. 16 shows that both the control (without STAR elements) constructs with GTG Blas (A) and TTG Blas (B) gave colonies that expressed d2EGFP protein. The average d2EGFP signal of 24 GTG Blas colonies was 14.0 (Fig 16A) and of TTG Blas colonies was 81 (Fig 16B). This higher average expression level in TTG Blas colonies may reflect the higher stringency of TTG, in comparison with GTG (see also example 9). However, only 8 colonies survived under the more stringent TTG conditions.

Addition of STAR elements 7 and 67 to the constructs resulted in colonies that had higher average d2EGFP expression levels. Transfection of the GTG Blas STAR 7/67/7 construct resulted in colonies with an average d2EGFP expression level of 97.2 (Fig 16A), which is a factor 6.9 higher than the average in the control cells (14.0). Addition of STAR elements to the TTG Blas construct resulted in an average d2EGFP signal of 234.2 (Fig 16B), which is a factor 2.9 higher than the average in the control cells (81). However, note again that only 8 colonies survived the harsh selection conditions of TTG Blas, whereas 48 colonies survived with TTG Blas STAR 7/67/7. When only the five highest values are compared, the average of the five highest TTG Blas was 109.1 and the average of the five highest TTG Blas STAR 7/67/7 was 561.2, which is a factor 5.1 higher.

The results indicate that STAR elements allow a more stringent selection system, resulting in the picking of colonies that have a very high average protein expression level. They also show that this selection is not restricted to the Zeocin resistance protein alone, but that also other selection marker polypeptides, in this case the blasticidine resistance protein, can be used.

### Example 12: Stability of d2EGFP expression in the novel selection system

Colonies described in example 10 were further cultured under several conditions to assess the stability of d2EGFP expression over an extended time period.

### Results

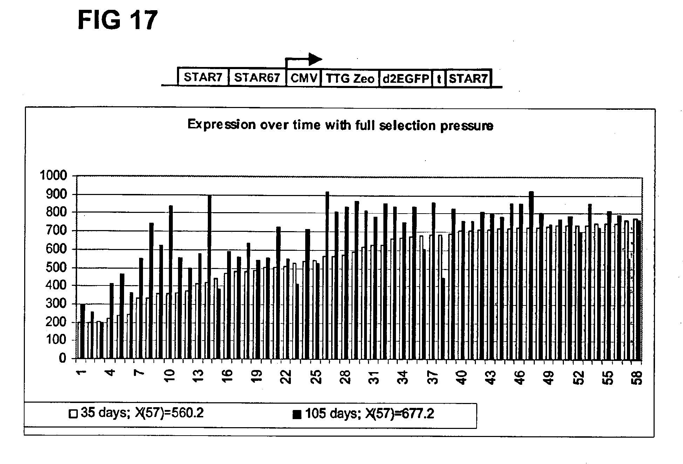

The TTG Zeo STAR 7/67/7 containing colonies in Fig 15A were cultured for an additional 70 days in the presence of 100 µg/ml Zeocin. As shown in Fig 17, the average d2EGFP signal rose from 560.2 after 35 days to 677.2 after 105 days. Except for some rare colonies all colonies had a higher d2EGFP expression level.

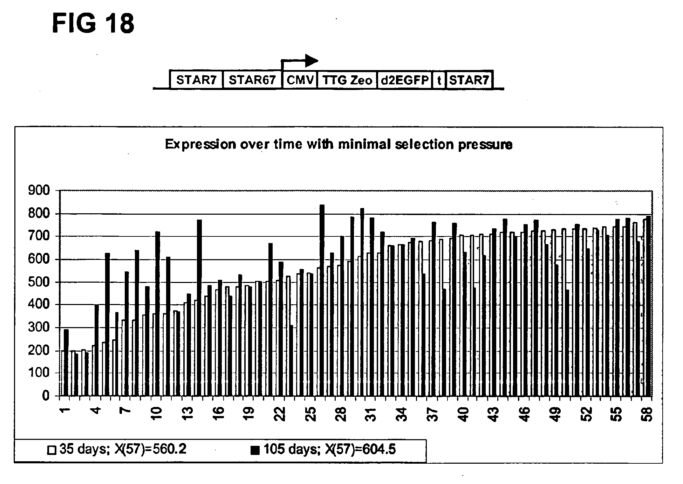

When the level of Zeocin was lowered to 20 µg/ml Zeocin, there was still an increase in the average d2EGFP expression level, from 560.2 after 35 days to 604.5 after 105 days (Fig. 18).

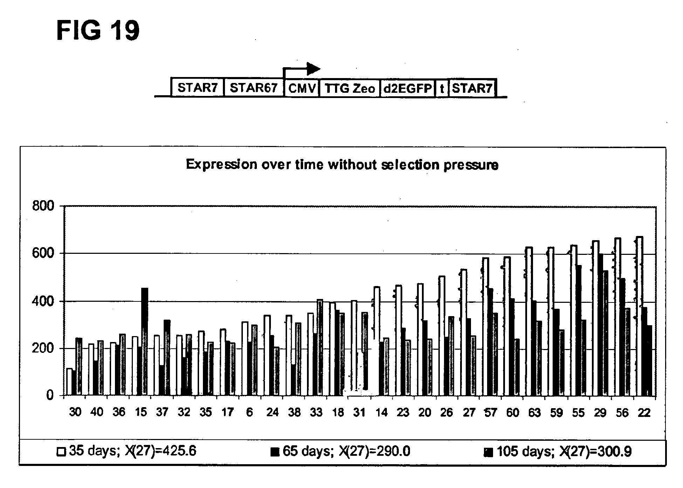

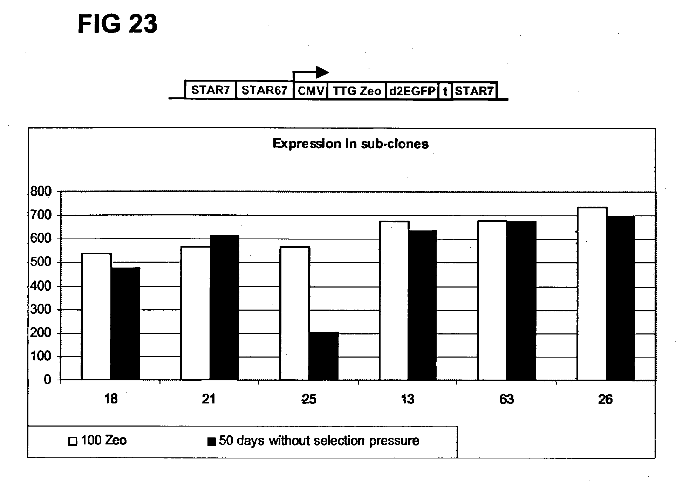

When no selection pressure was present at all due to removal of the Zeocin from the culture medium, approximately 50% of the colonies became mosaic, that is, within one colony non-d2EGFP expressing cells became apparent. This resulted in lowering of d2EGFP expression levels to less than 50% of the original levels. If the signal became less than 67% (decrease of at least one-third) from the original signal, the colony was considered to be unstable in respect to d2EGFP expression. Of the 57 original colonies 27 colonies remained stable according to this criterion; the average d2EGFP signal of these colonies after 35 days (while still under selection pressure) was 425.6, whereas the average d2EGFP signal without selection pressure after 65 days was 290.0. When measured after 105 days, the average signal in the 27 colonies was 300.9. Hence, after an initial decrease, the expression levels in the 27 colonies remained stable according to this criterion (Fig. 19).
Six of the colonies were subjected to one round of sub-cloning. Cells were sown in 96-wells plates as such that each well contained approximately 0.3 cells. No Zeocin was present in the medium so that from the start the sub clones grew without selection pressure. Of each original colony six sub clones were randomly isolated and grown in 6-wells plates till analysis. In Fig. 23 we compared the original values of the original clones, as already shown in Fig. 15A, with one of the sub clones. In one of the six clones (clone 25), no sub clone was present with d2EGFP signal in the range of the original clone. However, in five out of six cases at least one the sub clones had equal d2EGFP expression levels as the parent clone. These expression levels were determined after 50 days without selection pressure. We conclude that one round of sub cloning is sufficient to obtain a high number of colonies that remain stable for high expression in the absence of selection pressure. This has been confirmed in a similar experiment (not shown).

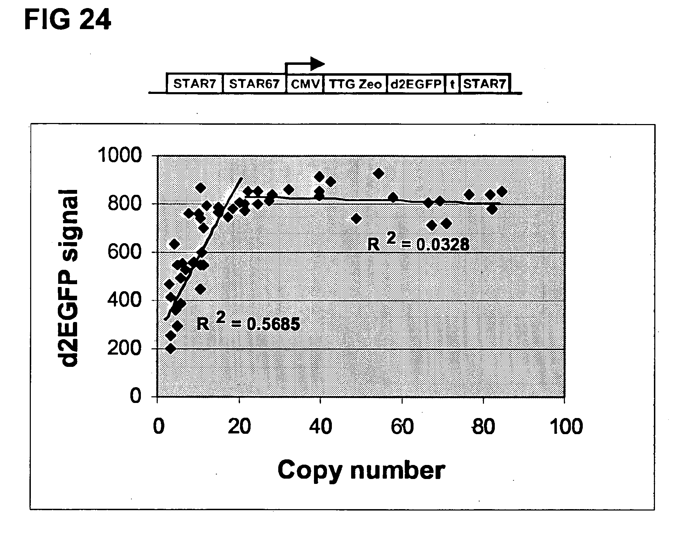

We compared the number of copies that integrated in the TTG Zeo STAR 7/67/7 colonies. DNA was isolated when colonies were 105 days under Zeocin selection pressure (see Fig. 17). As shown in Fig 24 two populations could be distinguished. In Fig 24 the cut off was made at 20 copies and the R² value is calculated and shown. Also the R² value from data with higher than 20 copies is shown. In the range from 100 to 800 d2EGFP signal there was a high degree of copy number dependency, as signified by a relatively high R² of 0.5685 (Fig 24). However, in the population of colonies that fluctuate around a d2EGFP signal of 800 a high variation in copy number was observed (Fig 24), as signified with a low R² of 0.0328. Together the data show that in the novel selection system, in colonies that contain TTG Zeo STAR 7/67/7 constructs there is copy number dependent d2EGFP expression up to - 20 copies. Also, although copy number dependency is lost when >20 copies are present, still a substantial proportion of the colonies with high (>800) d2EGFP signal have no more than 30 copies (Fig. 24). This combination between high d2EGFP expression and a relatively low copy number (between 10 and 30) may be important for identifying colonies that remain relatively stable without selection pressure. It is an advantage to have clones with relatively low copy numbers (less than about 30, more preferably less than about 20) that give high expression levels, because such clones are believed to be less amenable to genetic instability. The present selection system allows to generate such clones, including from CHO cells.

### Example 13: Creation and testing of Zeocin-Blasticidin-EpCAM bicistronic constructs with differential translation efficiencies.

To test the selection system on the production of an antibody, the anti-EpCAM antibody (see also example 5) was taken as example.

### Results

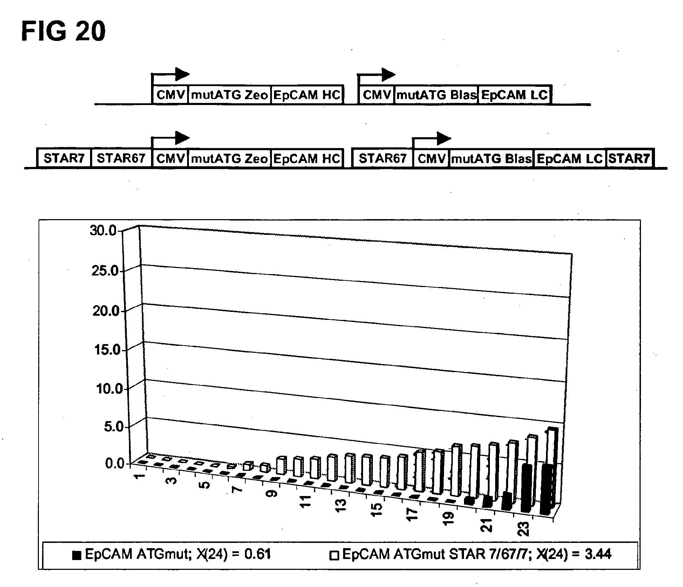

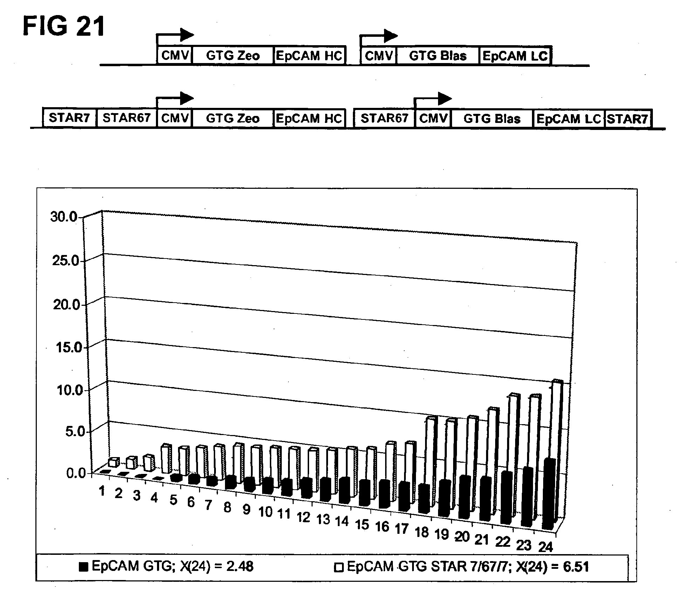

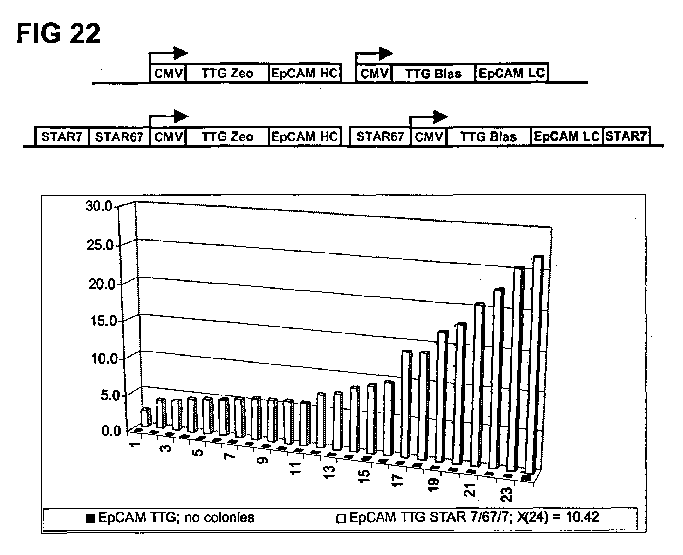

A plasmid was created on which both the heavy chain (HC) and light chain (LC) were placed, each in a separate transcription unit (Fig 20-22). Expression of both chains was driven by the CMV promoter. Upstream of the EpCAM heavy chain the Zeocin resistance gene was placed, either with the ATGmut/space (Fig 20), GTG (Fig 21) or TTG (Fig 22) as startcodon (see example 9). Upstream of the EpCAM light chain the Blasticidine resistance gene was placed, either with the ATGmut/space (Fig 20), GTG (Fig 21) or TTG (Fig 22) as startcodon (see example 11). Two types of constructs were made, one construct without STAR elements (Control) and one construct with a combination of STAR 7 and 67 elements. The STAR elements were placed as follows: upstream of each CMV promoter (i.e. one for the transcription unit comprising HC and one for the transcription unit comprising LC) STAR 67 was placed and the resulting construct was flanked with a 5' and 3' STAR 7 element (Figs 20-22). All constructs were transfected to CHO-K1 cells and selected on 100 µg/ml Zeocin and 20 µg/ml Blasticidin (at the same time). After selection independent colonies were isolated and propagated under continuous selection pressure (using 100 µg/ml zeocin and 20 µg/ml blasticidin). Fig 20 shows that the STAR 7/67/7 combination had a beneficial effect on EpCAM production. The ATGmut/space Zeo and ATGmut/space Blas had no effect on the number of colonies that were formed with plasmids containing STAR elements or not. However, the average EpCAM expression levels of either 24 control versus STAR 7/67/7 colonies ranged from 0.61 pg/cell/day in the control to 3.44 pg/cell/day in the STAR7/67/7 construct (Fig 20). This is a factor 5.6 increase. Since there were many colonies in the ATGmut/space control with 0 pg/cell/day, also the average EpCAM production in the highest five colonies was compared. In the control ATGmut/space this was 3.0 pg/cell/day, versus 7.8 pg/cell/day with the ATGmut/space STAR 7/67/7 construct, an increase of a factor 2.6.

Fig 21 also shows that the STAR 7/67/7 combination had a beneficial effect on EpCAM production, using the GTG startcodon for the markers. With the GTG Zeo and GTG Blas STAR 7/67/7 construct approximately 2 times more colonies were formed. Also, the average EpCAM expression levels of either 24 control versus STAR 7/67/7 colonies ranged from 2.44 pg/cell/day in the control to 6.51 pg/cell/day in the STAR7/67/7 construct (Fig 21). This is a factor 2.7 increase. Also the average EpCAM production in the highest five colonies was compared. In the control GTG this was 5.7 pg/cell/day, versus 13.0 pg/cell/day with the GTG STAR 7/67/7 construct, an increase of a factor 2.3. Also note that the average EpCAM production mediated by the GTG start codon for the selection markers was significantly higher than with the ATGmut/space start codon.

Fig 22 shows that with the TTG Zeo and TTG Blas control construct no colonies were formed, similar as in example 9. With the STAR 7/67/7 TTG construct colonies were formed. The average EpCAM expression levels of the STAR 7/67/7 TTG colonies was 10.4 pg/cell/day (Fig 22). This is again higher than with the ATGmut/space and GTG as start codon (see Figs 20, 21 for comparison). The average EpCAM production in the highest five TTG STAR 7/67/7 colonies was 22.5 pg/cell/day.

The results show that the selection system can also be applied to two simultaneously produced polypeptides, in this case two polypeptides of a multimeric protein, *casu quo* an antibody. The EpCAM production closely follows the results obtained with d2EGFP. The TTG as start codon is more stringent than the GTG start codon, which in turn is more stringent than the ATGmut/space (Figs 12 and 13). Higher stringency results in a decreasing number of colonies, with no colonies in the case of the TTG control that has no STAR elements, and higher stringency of the selection marker is coupled to higher expression of the protein of interest.

### Example 14: Creation and testing of additional GTG Zeocin-d2EGFP bicistronic constructs with differential translation efficiencies.

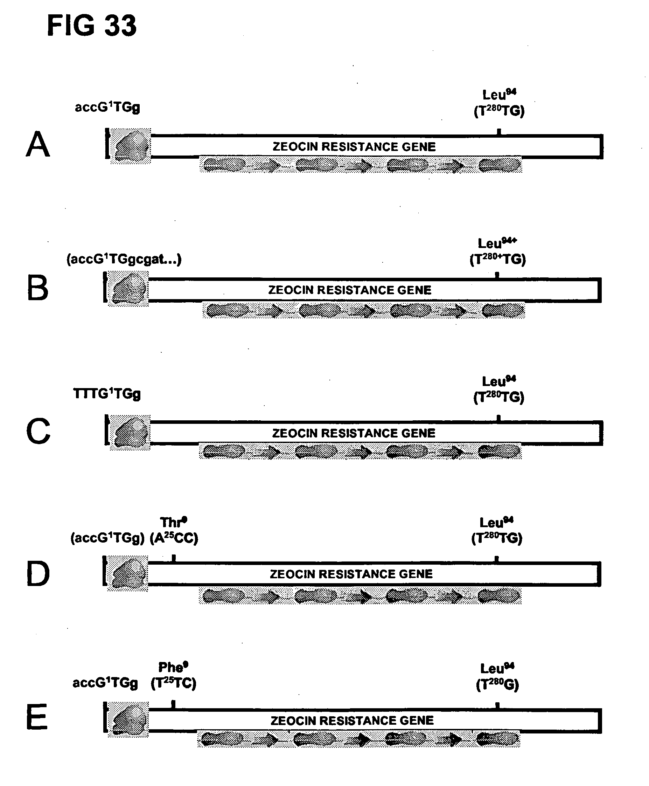

Different versions of the Zeocin resistance gene with mutated startcodons were described in Example 8. Besides the described GTG codons (Example 8, Fig. 33A), additional modified startcodons with distinct translational efficiency are possible. These different Zeocin resistance gene versions were created (Fig. 33) and cloned upstream of the modified d2EGFP gene, as in Example 9.

### Materials and methods

### Creation of plasmids

Four additional GTG constructs were made:
1) GTG as a start codon in the Zeo resistance gene (Fig. 33A), but followed by a spacer sequence (Fig. 33B). The mutspace-Zeo open reading frame was amplified with primer pair GTGspaceBamHIF (SEQ. ID. NO. 122):
   GAATTCGGATCCACC**GTG**GCGATCCAAAGACTGCCAAATCTAG and
   (wherein the sequence following the underlined sequence comprises the spacer sequence), and ZEOWTreverse (SEQ. ID. NO. 85), the PCR product was cut with EcoRI-BamHI, and ligated into pd2EGFP, cut with EcoRI-BamHI, creating pZEO-**GTGspace**-d2EGFP.
2) GTG as a start codon in the Zeo resistance gene, but in a bad context (***TTTGTG***) (Fig. 33C). The Zeo open reading frame was amplified with primer pair ZEOTTTGTGBamHIF (SEQ. ID. NO. 123):
   GAATTCGGATCC**TTTGTG**GCCAAGTTGACCAGTGCCGTTCCG and
   ZEOWTreverse (SEQ. ID. NO. 85), the PCR product was cut with EcoRI-BamHI, and ligated into pd2EGFP, cut with EcoRI-BamHI, creating pZEO(leu)-**TTTGTG-**d2EGFP.
3) GTG as a start codon in the Zeo resistance gene, instead of ATG (Fig. 33A), but with an additional mutation in the Zeo open reading frame at Pro9, which was replaced with threonine (Thr) (Fig. 33D). The Thr9 mutation was introduced by amplifying the Zeo open reading with primer pair ZEOForwardGTG-Thr9 (SEQ. ID. NO. 124):
   AATTGGATCCACC**GTG**GCCAAGTTGACCAGTGCCGTT***ACC***GTGCTC
   and ZEOWTreverse (SEQ. ID. NO. 85), the PCR product was cut with EcoRI-BamHI, and ligated into pd2EGFP, cut with EcoRI-BamHI, creating pZEO-**GTG-**Thr9-d2EGFP.
4) GTG as a start codon in the Zeo resistance gene, instead of ATG (Fig. 33A), but with an additional mutation in the Zeo open reading frame at Pro9, with was replaced with Phenylalanine (Phe) (Fig. 33E). The Phe9 mutation was introduced by amplifying the Zeo open reading with primer pair ZEOForward GTG-Phe9 (SEQ. ID. NO. 125):
   AATTGGATCCACC**GTG**GCCAAGTTGACCAGTGCCGTT***TTC***GTGCTC
   and ZEOWTreverse (SEQ. ID. NO. 85), the PCR product was cut with EcoRI-BamHI, and ligated into pd2EGFP, cut with EcoRI-BamHI, creating pZEO-**GTG-**Phe9-d2EGFP.

### Transfection, culturing and analysis of CHO cells

Transfection, culturing and analysis of CHO-K1 cells was performed as in Example 8.

### Results

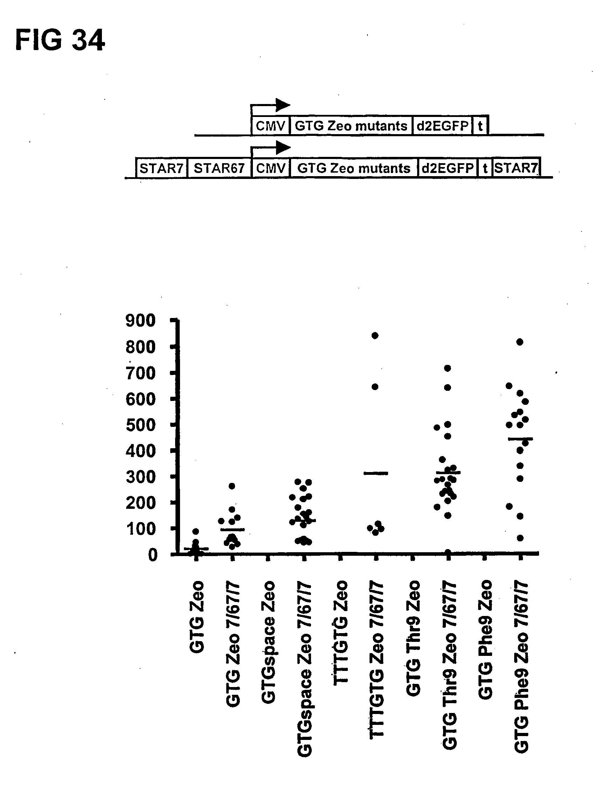

CHO-K1 cells were transfected with constructs that contain the GTG Zeo (Fig. 33A), GTGspace Zeo (Fig. 33B), TTT GTG Zeo (also called: GTGmut Zeo) (Fig. 33C), GTG Thr9 Zeo(leu) (Fig. 33D) and GTG Phe9 Zeo(leu) (Fig. 33D) genes as selection gene, all being cloned upstream of the d2EGFP reporter gene. These five constructs were without STAR elements (Control) or with STAR elements 7 and 67 upstream of the CMV promoter and STAR 7 downstream from the d2EGFP gene (Fig. 33). Fig. 34 shows that of the control constructs without STAR elements only the GTG Zeo construct without STAR elements gave colonies that expressed d2EGFP protein. In contrast, all constructs containing STAR elements gave colonies that expressed d2EGFP protein. The mean d2EGFP fluorescence signal of 11 GTG Zeo Control colonies was 20.3, of 13 GTG Zeo colonies with STARs 7/67/7 104.9, of 24 GTG space Zeo 7/67/7 colonies 201.5, of 6 TTT GTG Zeo 7/67/7 colonies 310.5, of 22 GTG Thr9 Zeo 7/67/7 colonies 423, and of 16 GTG Phe9 Zeo colonies 550.2 (Fig. 34).

The higher stringencies of the novel GTG mutations correlate with higher mean fluorescence signals (Fig. 34). The TTT GTG Zeo 7/67/7, however, gave only two high expressing colonies and a few low expressing colonies. This may indicate that this mutation is at the brink of the stringency that these cells can bear with a fixed concentration of Zeocin added to the culture medium.

The Thr9 and Phe9 mutations do not influence the translation efficiency of the Zeo mutants. Instead they reduce the functionality of the Zeocin resistance protein, by preventing an optimal interaction between the two halves of the Zeocin resistance protein (Dumas et al, 1994). This implies that more of the protein has to be produced to achieve resistance against the Zeocin in the culture medium. As a consequence, the entire cassette has to be transcribed at a higher level, eventually resulting in a higher d2EGFP expression level.

It is concluded that the use of the described translation efficiencies of the Zeocin resistance mRNA result in higher expression levels of the d2EGFP protein, this in combination with STAR elements.

This example further demonstrates the possibility to provide for fine-tuning of the stringency of the selection system of the invention, to achieve optimal expression levels of a protein of interest. Clearly, the person skilled in the art will be capable of combining these and other possibilities within the concepts disclosed herein (e.g. mutate the zeocin at position 9 to other amino acids, or mutate it in other positions; use a GTG or other startcodon in a non-optimal translation initition context for zeocin or other selection markers; or mutate other selection markers to reduce their functionality, for instance use a sequence coding for a neomycin resistance gene having a mutation at amino acid residue 182 or 261 or both, see e.g. WO 01/32901), and the like, to provide for such fine-tuning, and by simply testing determine a suitable combination of features for the selection marker, leading to enhanced expression of the polypeptide of interest.

### Example 15: Creation and testing of additional TTG Zeocin-d2EGFP bicistronic constructs with differential translation efficiencies.

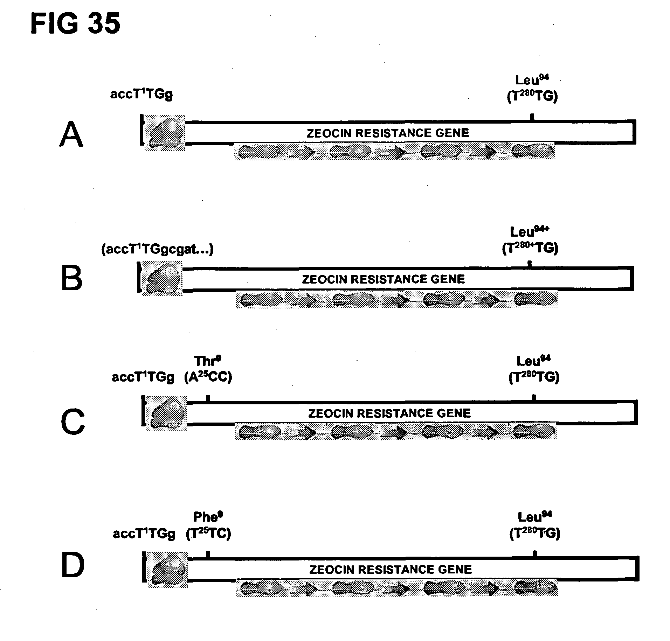

Different versions of the Zeocin resistance gene with mutated startcodons were described in Example 8. Besides the described TTG codons (Fig. 35A) additional modified startcodons with distinct translational efficiency are possible. These different Zeocin resistance gene versions were created and cloned upstream of the modified d2EGFP gene (Fig. 35).

### Materials and methods

### Creation of plasmids

Three additional TTG constructs were made:
1) TTG as a start codon in the Zeo resistance gene (Fig. 35A), but followed by a spacer sequence (Fig. 35B). The Zeo open reading frame (with the spacer sequence) was amplified with primer pair TTGspaceBamHIF (SEQ. ID. NO. 126):
   GAATTCGGATCCACC**TTG**GCGATCCAAAGACTGCCAAATCTAG and
   ZEOWTreverse(SEQ. ID. NO. 85), the PCR product was cut with EcoRI-BamHI, and ligated into pd2EGFP, cut with EcoRI-BamHI, creating pZEO-**TTGspace**-d2EGFP.
2) TTG as a start codon in the Zeo resistance gene, instead of ATG (Fig. 35A), but with an additional mutation in the Zeo open reading frame at Pro9, with was replaced with threonine (Thr) (Fig. 35C). The Thr9 mutation was introduced by amplifying the Zeo open reading with primer pair ZEOForwardTTG-Thr9 (SEQ. ID. NO. 127):
   AATTGGATCCACC**TTG**GCCAAGTTGACCAGTGCCGTT***ACC***GTGCTC
   and ZEOWTreverse (SEQ. ID. NO. 85), the PCR product was cut with EcoRI-BamHI, and ligated into pd2EGFP, cut with EcoRI-BamHI, creating pZEO-**TTG-**Thr9-d2EGFP.
3) TTG as a start codon in the Zeo resistance gene, instead of ATG (Fig. 35A), but with an additional mutation in the Zeo open reading frame at Pro9, with was replaced with Phenylalanine (Phe) (Fig. 35D). The Phe9 mutation was introduced by amplifying the Zeo open reading with primer pair ZEOForwardTTG-Phe9 (SEQ. ID. NO. 128):
   AATTGGATCCACC**TTG**GCCAAGTTGACCAGTGCCGTT***TTC***GTGCTC
   and ZEOWTreverse (SEQ. ID. NO. 85), the PCR product was cut with EcoRI-BamHI, and ligated into pd2EGFP, cut with EcoRI-BamHI, creating pZEO-**TTG-**Phe9-d2EGFP.

### Results

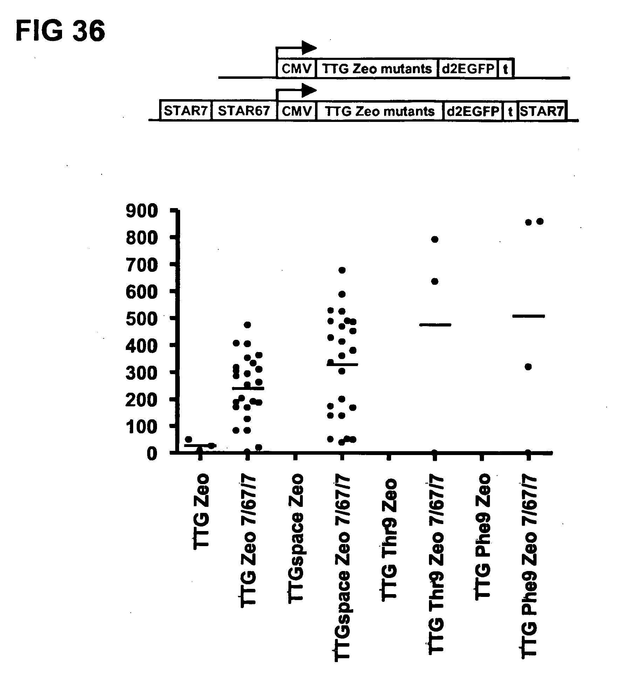

CHO-K1 cells were transfected with constructs that contain the TTG Zeo (Fig. 35A), TTGspace Zeo (Fig. 35B), TTG Thr9 Zeo (Fig. 35C) and TTG Phe9 Zeo (Fig. 35D) genes as selection gene, all being cloned upstream of the d2EGFP reporter gene. These four constructs were without STAR elements (Control) or with STAR elements 7 and 67 upstream of the CMV promoter and STAR 7 downstream from the d2EGFP gene (Fig. 35). Fig. 36 shows that of the control constructs without STAR elements only the TTG Zeo construct without STAR elements gave colonies that expressed d2EGFP protein. In contrast, all constructs containing STAR elements gave colonies that expressed d2EGFP protein. The mean d2EGFP fluorescence signal of 3 TTG Zeo Control colonies was 26.8, of 24 TTG Zeo colonies with STARs 7/67/7 426.8, of 24 TTGspace Zeo 7/67/7 colonies 595.7, of 2 TTG Thr9 Zeo 7/67/7 colonies 712.1, and of 3 TTG Phe9 Zeo colonies 677.1 (Fig. 36).

The higher stringencies of the novel TTG mutations correlate with higher mean fluorescence signals (Fig. 36). The TTG Thr9 Zeo 7/67/7 and TTG Phe9 Zeo 7/67/7 constructs, however, gave only two high expressing colonies each and a few low expressing colonies. This may indicate that these mutations are at the brink of the stringency that the cells can bear with a fixed concentration of Zeocin added to the culture medium.

It is concluded that the use of the described translation efficiencies of the Zeocin resistance mRNA result in higher expression levels of the d2EGFP protein, this in combination with STAR elements.

### Example 16: Creation and testing of Puromycin-d2EGFP bicistronic constructs with differential translation efficiencies.

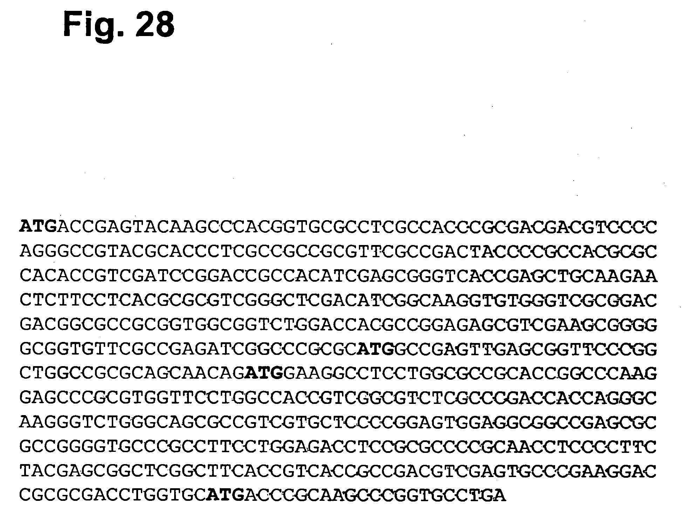

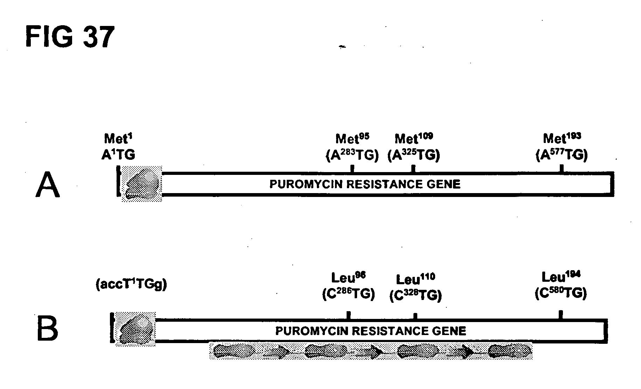

There are three internal ATGs in the puromycin resistance gene, each of which codes for a methionine (Fig. 28, Fig. 37A). These ATGs have to be eliminated (Fig. 37B, C), since they will serve as start codon when the ATG startcodon (or the context thereof) has been modified, and this will result in peptides that do not resemble puromycin resistance protein. More importantly, these ATGs will prevent efficient translation of the gene of interest, as represented by d2EGFP in this example for purposes of illustration. The methionines were changed into leucine, like in the zeocin resistance protein (example 8). However, instead of using the TTG codon for leucine (for instance in Zeocin in example 8), now the CTG codon for leucine was chosen (in humans, for leucine the CTG codon is used more often than the TTG codon). To eliminate the internal ATGs, the puromycin resistance protein open reading frame was first amplified with 4 primer pairs, generating 4 puromycin resistance protein fragments. The primer pairs were:
PURO BamHI F (SEQ. ID. NO. 129):
   GATCGGATCCATGGTTACCGAGTACAAGCCCACGGT,
PURO300 R LEU (SEQ. ID. NO. 130):
   CAGCCGGGAACCGCTCAACTCGGCCAGGCGCGGGC;and
PURO300FLEU (SEQ. ID. NO. 131):
   CGAGTTGAGCGGTTCCCGGCTGGCCGCGCAGCAACAGCTGGAAGGCCTC,
PURO600RLEU (SEQ. ID. NO. 132):
   AAGCTTGAATTCAGGCACCGGGCTTGCGGGTCAGGCACCAGGTC.
This generates two PCR products, corresponding to the 5' and 3' part of the puromycin resistance gene. The two products were added together and amplified with PURO BamHI F (SEQ. ID. NO. 129) - PURO600RLEU (SEQ. ID. NO. 132). The resulting PCR product was cut with BamHI-EcoRI and ligated, creating pCMV-ATGPURO (leu). Sequencing of this clone verified that all three internal ATGs had been converted. The entire puromycin open reading frame was then amplified with PUROBamHI TTG1F (SEQ. ID. NO. 133):
GAATTCGGATCCACC**TTG**GTTACCGAGTACAAGCCCACGGTG and
PURO600RLEU (SEQ. ID. NO. 132). This primer introduces an extra codon (GTT) directly after the TTG startcodon, because the 'G' at nucleotide +4 is introduced for an optimal context, and hence two more nucleotides are introduced to preserve the reading frame.

### Results

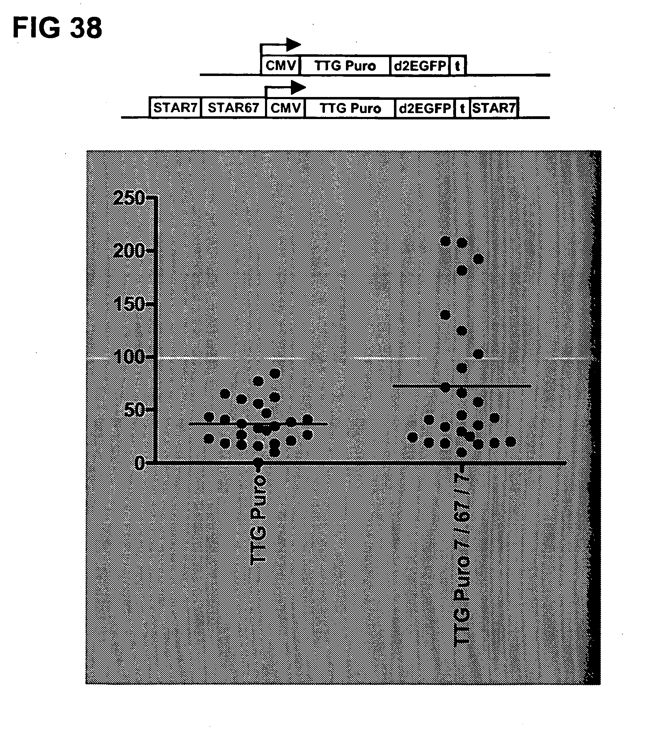

CHO-K1 cells were transfected with the construct that contains the TTG Puro (Fig. 38) gene as selection gene, cloned upstream of the d2EGFP reporter gene. Selection was under 10 µg/ml puromycin. The construct was without STAR elements (Control) or with STAR elements 7 and 67 upstream of the CMV promoter and STAR 7 downstream from the d2EGFP gene (Fig. 38). Fig. 38 shows that the average d2EGFP fluorescence signal of 24 TTG Puro Control colonies was 37.9, of 24 TTG Puro colonies with STARs 7/67/7 75.5. Moreover, when the average of the five highest values is taken, the d2EGFP fluorescence signal of TTG Puro Control colonies was 69.5, and of TTG Puro colonies with STARs 7/67/7 186.1, an almost three-fold increase in d2EGFP fluorescence signal. This shows that the described, modified translation efficiency of the Puromycin resistance mRNA result in higher expression levels of the d2EGFP protein, this in combination with STAR elements.

This experiment demonstrates that the puromycin resistance gene can be mutated to remove the ATG sequences therefrom, while remaining functional. Moreover it is concluded that the selection method of the invention also works with yet another selection marker, puromycin.

### Example 17: Creation and testing of neomycin constructs with differential translation efficiencies.

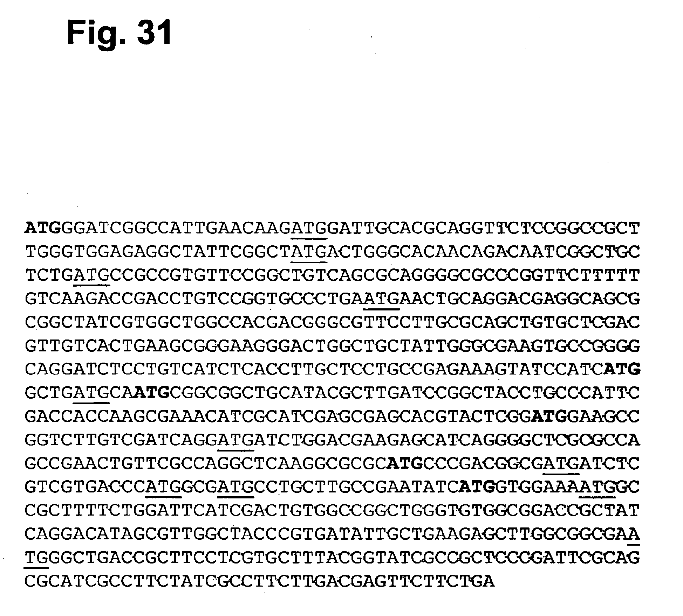

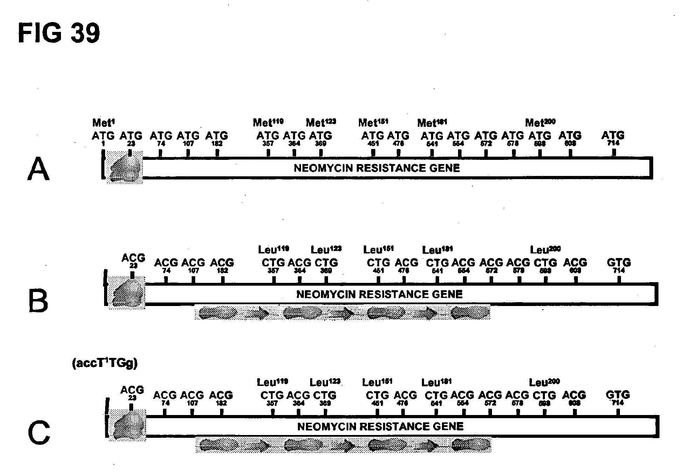

There are sixteen internal ATGs in the neomycin resistance gene, five of which code for a methionine in the neomycin open reading frame (Fig. 31, Fig. 39A). All these sixteen ATGs have to be eliminated (Fig. 39B, C), since they will serve as start codon when the ATG startcodon (or the context thereof) has been modified, and this will result in peptides that do not resemble neomycin resistance protein, and this will decrease the translation from the downstream open reading frame coding for the polypeptide of interest in the transcription units of the invention. To eliminate the internal ATGs, the neomycin resistance protein open reading frame was entirely synthesized by a commercial provider (GeneArt, Germany), wherein all internal coding ATGs (for Met) where replaced by CTGs (coding for Leu), and non-coding ATGs were replaced such that a degenerated codon was used and hence no mutations in the protein sequence resulted; the synthesised sequence of the neomycin is given in SEQ. ID. NO. 134. In order to replace the ATG start codon with GTG (Fig. 39B) or TTG Fig. 39C), the synthesized neomycin gene was amplified with primer pairs NEO-F-HindIII (SEQ. ID. NO. 136):
GATCAAGCTTTTGGATCGGCCATTGAAACAAGACGGATTG and NEO EcoRI 800R (SEQ. ID. NO. 137):
AAGCTTGAATTCTCAGAAGAACTCGTCAAGAAGGCG.

### Results

*E.coli* bacteria were used to test the functionality of the neomycin resistance protein from which all ATGs were removed. E.coli bacteria were transformed with the constructs that contain the GTG Neo (Fig. 39B) or TTG Neo (Fig. 39C) gene as selection gene. Selection took place by growing the bacteria on kanamycin. Only a functional neomycin resistance gene can give resistance against kanamycin. Transformation with either modified Neo gene resulted in the formation of E.coli colonies, from which the plasmid containing the gene could be isolated. This shows that the described, modified translation efficiencies of the Neomycin resistance mRNAs, as well as the removal of all ATGs from the Neo open reading frame result in the production of functional neomycin resistance protein.

The mutated neomycin resistance genes are incorporated in a multicistronic transcription unit of the invention, and used for selection with G418 or neomycin in eukaryotic host cells.

### Example 18: Creation and testing of dhfr constructs with differential translation efficiencies.

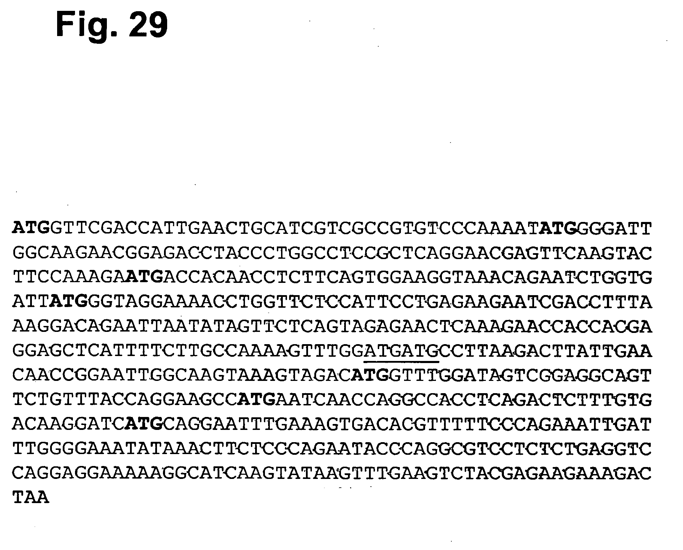

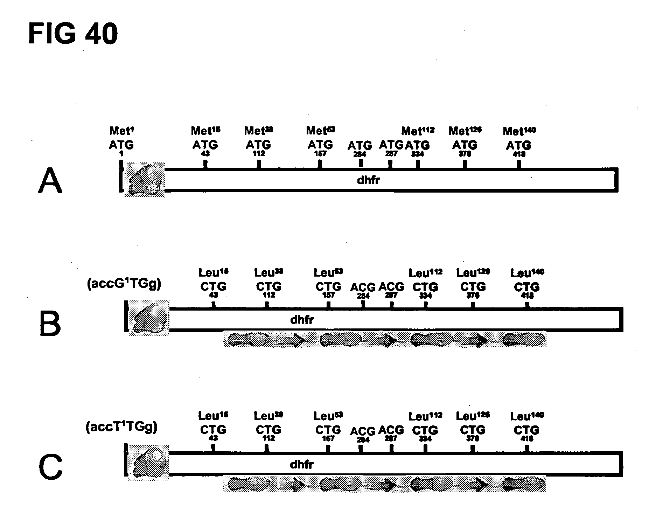

There are eight internal ATGs in the dhfr gene, six of which code for a methionine in the dhfr open reading frame (Fig. 29, Fig. 40A). All these ATGs have to be eliminated (Fig. 40B, C), since they will serve as start codon when the ATG startcodon (or the context thereof) has been modified, and this will result in peptides that do not resemble dhfr protein, and will decrease the translation from the downstream open reading frame coding for the polypeptide of interest in the transcription units of the invention. To eliminate the internal ATGs, the dhfr protein open reading frame was entirely synthesized (SEQ. ID. NO. 138), as described above for neomycin. In order to replace the ATG start codon with GTG (Fig. 40B) or TTG (Fig. 40C), the synthesized DHFR gene was amplified with primers DHFR-F-HindIII (SEQ. ID. NO. 140): GATCAAGCTTTTGTTCGACCATTGAACTGCATCGTC and DHFR-EcoRI-600-R (SEQ. ID. NO. 141):
AGCTTGAATTCTTAGTCTTTCTTCTCGTAGACTTC.

### Results

*E.coli* bacteria were used to test the functionality of the dhfr protein from which all ATGs were removed. *E.coli* was transformed with the constructs that contain the GTG dhfr (Fig. 40B) or TTG dhfr (Fig. 40C) gene. Selection took place by growing the bateria on trimethoprim (Sigma T7883-56). Only a functional dhfr gene can give resistance against trimethoprim. Transformation with either modified dhfr gene resulted in the formation of E.coli colonies, from which the plasmid containing the gene could be isolated. This shows that the described, modified translation efficiencies of the dhfr mRNAs, as well as the removal of all ATGs from the dhfr open reading frame result in the production of functional dhfr protein.

The mutated dhfr genes are incorporated in a multicistronic transcription unit of the invention, and used for selection with methotrexate in eukaryotic host cells.

### Example 20: Testing of Zeocin- and Blasticidin constructs with differential translation efficiencies in PER.C6 cells.

Various Zeocin and blasticidin genes with mutated startcodons, all cloned upstream of the d2EGFP gene were tested in the PER.C6 cell line.

### Results

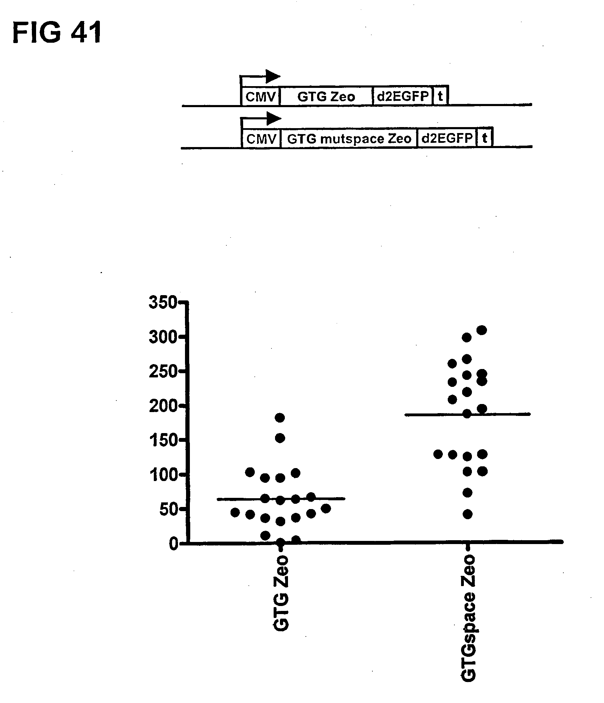

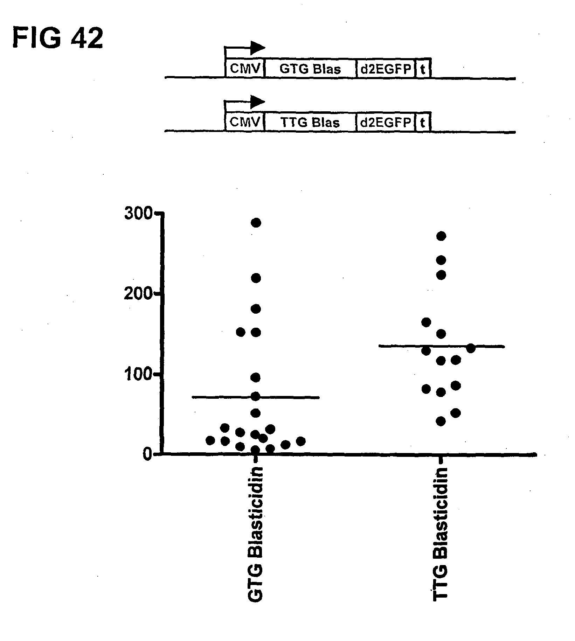

The GTG Zeocin and GTGspace Zeocin resistance gene modifications (see also Example 14; Fig. 41) and the GTG blasticidin and TTG blasticidin resistance gene modifications (see also Example 11; Fig. 42), all cloned upstream of the d2EGFP gene were transfected to PER.C6 cells. As shown in Fig. 41, transfection with both the GTG Zeocin and GTGspace Zeocin gene resulted in colonies that expressed d2EGFP. The average d2EGFP fluorescence signal of 20 GTG Zeo colonies was 63.8, while the average d2EGFP signal of 20 GTGspace Zeo colonies was 185, demonstrating that also in PER.C6 cells the GTGspace Zeo has a higher translation stringency than the GTG Zeo mRNA.

As shown in Fig. 42, transfection with both the GTG Blasticidin and TTG Blasticidin gene resulted in colonies that expressed d2EGFP. The average d2EGFP fluorescence signal of 20 GTG Blasticidin colonies was 71.4, while the average d2EGFP fluorescence signal of 20 TTG Blasticidin colonies was 135, demonstrating that also in PER.C6 cells the TTG Blasticidin has a higher translation stringency than the GTG Blasticidin mRNA.

This example demonstrates that the selection system of the invention can also be used in other cells than CHO cells.

### Example 21: Testing of the addition of a transcriptional pause signal to a TTG Zeocin-d2EGFP construct.

A TRAnscription Pause (TRAP) sequence is thought to, at least in part, prevent formation of antisense RNA or, to at least in part, prevent transcription to enter said protein expression unit (see WO 2004/055215). A TRAP sequence is functionally defined as a sequence which when placed into a transcription unit, results in a reduced level of transcription in the nucleic acid present on the 3' side of the TRAP when compared to the level of transcription observed in the nucleic acid on the 5' side of the TRAP, and non-limiting examples of TRAP sequences are transcription termination signals. In order to function to prevent or decrease transcription to enter the transcription unit, the TRAP is to be placed upstream of a promoter driving expression of the transcription unit and the TRAP should be in a 5' to 3' direction. In order to prevent at least in part formation of antisense RNA, the TRAP should be located downstream of the open reading frame in a transcription unit and present in a 3' to 5' direction (that is, in an opposite orientation as the normal orientation of a transcriptional termination sequence that is usually present behind the open reading frame in a transcription unit). A combination of a TRAP upstream of the promoter in a 5' to 3' orientation and a TRAP downstream of the open reading frame in a 3' to 5' oreintation is preferred. Adding a TRAP sequence to a STAR element improves the effects of STAR elements on transgene expression (see WO 2004/055215). Here we test the effects of the TRAP sequence in the context of the TTG Zeo resistance gene.

### Results

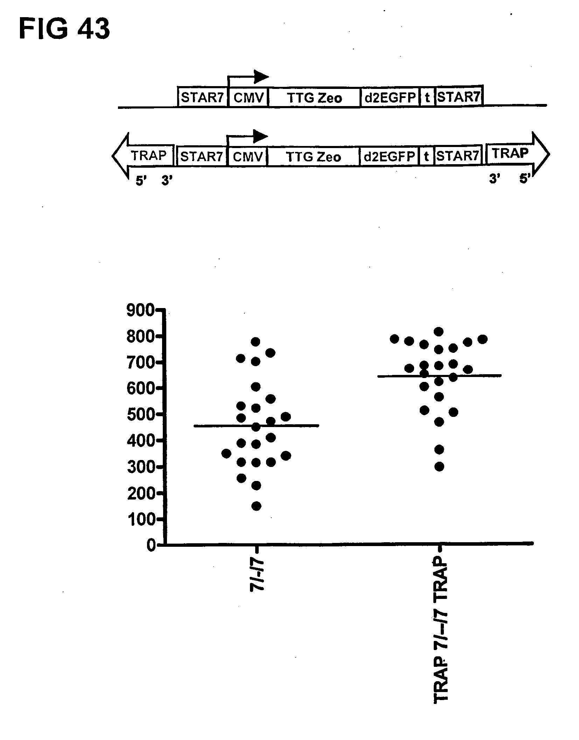

The TTG Zeocin-d2EGFP cassette that was flanked with STAR7 elements (Fig. 43) was modified by the addition of the SPA/pause TRAP sequence (see WO 2004/055215); SEQ. ID. NO. 142), both upstream of the 5' STAR7 (in 5' to 3' direction) and downstream of the 3' STAR7 (in 3' to 5' direction) (Fig. 43). Both STAR 7/7 and TRAP-STAR 7/7-TRAP containing vectors were transfected to CHO-K1. Stable colonies were isolated and the d2EGFP fluorescence intensities were measured. As shown in Fig. 43 the average d2EGFP fluorescence signal of 23 TTG Zeo STAR 7/7 colonies was 455.1, while the average d2EGFP fluorescence signal of 23 TTG Zeo TRAP-STAR 7/7-TRAP colonies was 642.3. The average d2EGFP fluorescence signal in highest 5 TTG Zeo STAR 7/7 colonies was 705.1, while the average d2EGFP fluorescence signal of 5 TTG Zeo TRAP-STAR 7/7-TRAP colonies was 784.7.

This result indicates that the addition of TRAPs does not enhance the d2EGFP fluorescence signal in the highest colonies, but that there is a significant raise in the number of high expressing colonies. Whereas only 5 TTG Zeo STAR 7/7 colonies had d2EGFP signal above 600, 17 TTG Zeo TRAP-STAR 7/7-TRAP colonies had a d2EGFP fluorescence signal above 600.

In the experiment 3 µg DNA of each plasmid was transfected. However, whereas the transfection efficiency was similar, the total number of colonies with the TTG Zeo STAR 7/7 plasmid was 62, while the total number of colonies with the TTG Zeo TRAP-STAR 7/7-TRAP plasmid was 116, almost a doubling.

We conclude that addition of TRAP elements to the STAR containing plasmids with modified Zeocin resistance gene translation codons results in a significantly higher overall number of colonies and that more colonies are present with the highest expression levels.

### Example 22: Copy-number dependency of expression

We analyzed the EpCAM antibody expression levels in relation to the number of integrated EpCAM DNA copies.

### Results

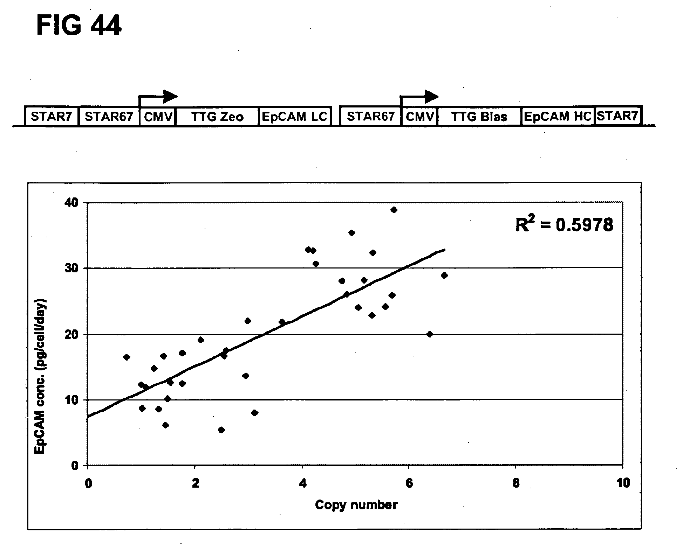

The construct that was tested was TTG-Zeo-Light Chain (LC)-TTG-Blas-Heavy Chain (HC), both expression units being under the control of the CMV promoter (see Fig. 44). This construct contained STAR 7 and 67 (see Fig. 44). Selection conditions were such that with 200 µg/ml Zeocin and 20 µg/ml Blasticidin in the culture medium no control colonies (no STARs) survived and only STAR 7/67/7 colonies survived.

DNA was isolated when colonies were 60 days under Zeocin and Blasticidin selection pressure (see Fig. 44). The R² value is calculated and shown. In the entire range from 5 to 40 pg/cell/day EpCAM there was a high degree of copy number dependency, as signified by a relatively high R² of 0.5978 (Fig 44). The data show that in the novel selection system, in colonies that contain TTG Zeo-TTG Blas EpCAM STAR 7/67/7 constructs there is copy number dependent EpCAM expression.

### Example 23: Methotrexate induction of higher EpCAM expression

We analyzed EpCAM antibody expression levels after incubation of clones with methotrexate (MTX). The purpose of this experiment was to determine whether amplification of a STAR-containing construct would result in higher EpCAM expression. MTX acts through inhibition of the dhfr gene product. While some CHO strains that are dhfr-deficient have been described, CHO-K1 is dhfr⁺. Therefore relatively high concentrations of MTX in the culture medium have to be present to select for amplification by increased MTX concentrations in CHO-K1 cells.

### Results

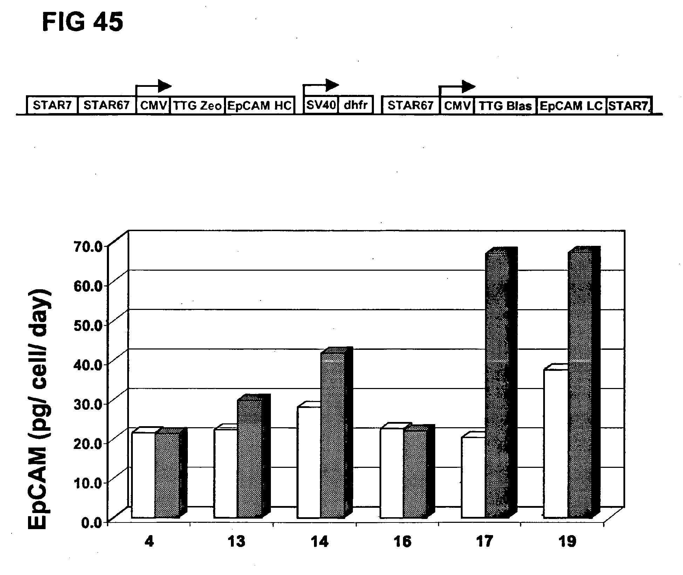

The construct that was tested was TTG-Zeo-Heavy Chain (HC)-TTG-Blas-Light Chain (LC), both expression units being under the control of the CMV promoter. Upstream of each CMV promoter STAR67 was positioned and STAR7 was used to flank the entire cassette (see also Example 13, Fig. 22 for such a construct). This construct was further modified by placing an SV40-dhfr cassette (a mouse dhfr gene under control of an SV40 promoter) between the HC and LC cassettes, upstream of the second STAR67 (Fig. 45). CHO-K1 cells were transfected. Selection was done with 100 µg/ml Zeocin and 10 µg/ml Blasticidin in the culture medium. No control colonies (without STAR elements) survived and only colonies with constructs containing the STAR elements survived. Colonies were isolated and propagated before measuring EpCAM expression levels. Six colonies that produced between 20 and 35 pg/cell/day were transferred to medium containing 100 nM MTX. This concentration was raised to 500 nM, 1000 nM and finally to 2000 nM with two weeks periods in between each step. After two weeks on 2000 nM MTX, EpCAM concentrations were measured. As shown in Fig. 45, four colonies showed enhanced EpCAM production. Colony 13: from 22 to 30; colony 14: from 28 to 42; colony 17: from 20 to 67 and colony 19: from 37 to 67 pg/ cell/ day. Colonies 4 and 16 showed no enhanced EpCAM expression. We conclude that addition of methotrexate to the culture medium of CHO-K1 colonies created with the selection system of the invention can result in enhanced protein expression. Hence, STAR elements and the selection method of the invention can be combined with and are compatible with MTX-induced enhancement of protein expression levels.

### Example 24: TTG-Zeo selection operates in the context of different promoters

We analyzed d2EGFP expression levels in the context of the TTG Zeo selection marker and different promoters. We compared the action of STAR elements in the context of the CMV enhancer/promoter, the SV40 enhancer/promoter and the CMV enhancer/β-actin promoter.

### Results

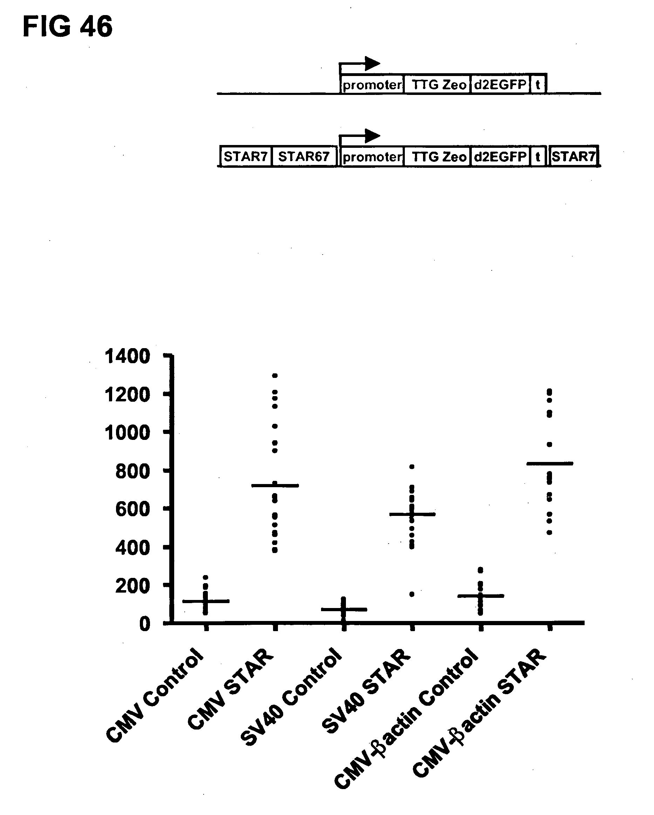

In Fig. 46 we indicate the promoters we tested in the context of the TTG Zeo selection marker. The tested plasmids consisted of the indicated control constructs with three different promoters and STAR constructs which were flanked with STAR 7 and STAR 67 at the 5' end and STAR 7 at the 3' end. The constructs were transfected to CHO-K1 cell and selection was performed with 200 µg/ml Zeocin in the culture medium. Up to 23 independent colonies were isolated and propagated before analysis of d2EGFP expression levels. As shown in Fig. 46, incorporation of STAR elements in constructs with the CMV enhancer/promoter, the SV40 enhancer/promoter or the CMV enhancer/β-actin promoter all resulted in the formation of colonies with higher d2EGFP expression levels than with the corresponding control constructs. This shows that the selection system of the invention, in combination with STAR elements, operates well in the context of different promoters. Further analysis showed that the mean of CMV-driven d2EGFP values was significantly higher than the mean of SV40-driven d2EGFP values (p<0.05). In contrast, the mean of CMV-driven d2EGFP values did not significantly differ from CMV/β actin-driven d2EGFP values (p=0.2).

### Example 25: Comparison of different STAR elements in the TTG-Zeo selection system.

We analyzed d2EGFP expression levels in the context of the CMV promoter-TTG Zeo selection marker and 53 different STAR elements, to obtain more insight in which STAR elements give the best results in this context.

### Results

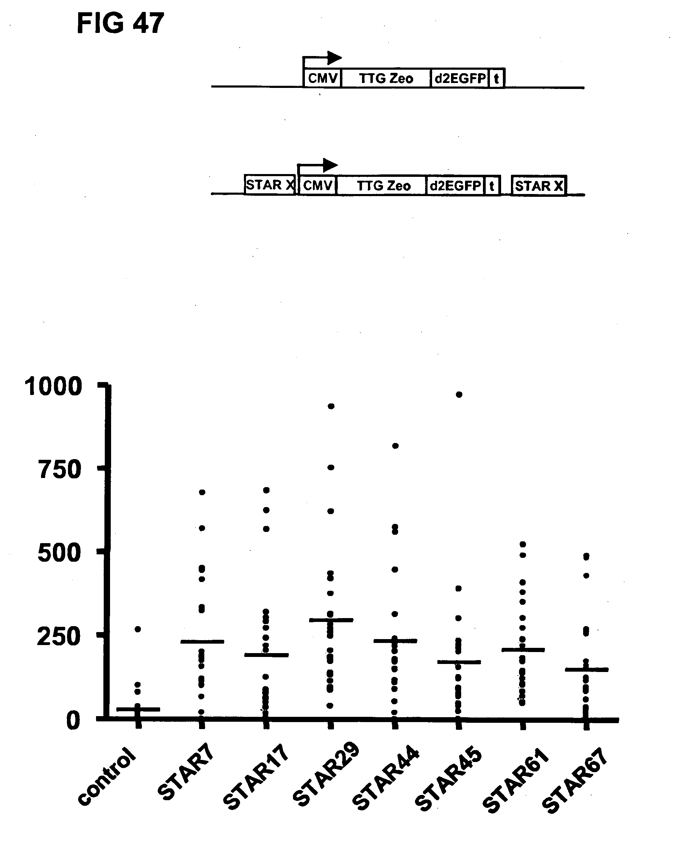

We cloned 53 STAR elements up-and downstream of the CMV promoter-TTG Zeo-d2EGFP cassette. The following STAR elements were tested in such constructs: STAR2-12, 14, 15, 17-20, 26-34, 36, 37, 39, 40, 42-49, 51, 52, 54, 55, 57-62, 64, 65, 67. The constructs were transfected to CHO-K1 cells and selection was performed with 200 µg/ml Zeocin in the culture medium. Up to 24 independent colonies were isolated and propagated before analysis of d2EGFP expression levels. Incorporation of STAR elements in the constructs resulted in different degrees of enhanced d2EGFP expression, as compared to the control. Incorporation of STAR elements 14, 18 and 55 in this experiment did not result in an increase of average d2EGFP expression over the control (no STAR element). Although some constructs (with STAR elements 2, 3, 10, 42, 48 and 49) in this experiment gave rise to only a few colonies, all tested STAR elements except 14, 18 and 55 resulted in average d2EGFP expression levels higher than for the control. It should be noted that some STAR elements may act in a more cell type specific manner and that it is well possible that STAR 14, 18 and 55 work better in other cell types, with other promoters, other selection markers, or in different context or configuration than in the particular set of conditions tested here. Addition of 10 STAR elements, namely STAR elements 7, 9, 17, 27, 29, 43, 44, 45, 47 and 61, induced average d2EGFP expression levels higher than 5 times the average d2EGFP expression level of the control. We retransformed the control and 7 constructs with STAR elements and repeated the experiment. The results are shown in Fig. 47. Incorporation of STAR elements in the constructs resulted in different degrees of enhanced d2EGFP expression, as compared to the control (Fig. 47). The average d2EGFP expression level in colonies transfected with the control construct was 29. The averages from d2EGFP expression levels in colonies with the 7 different STAR constructs ranged between 151 (STAR 67) and 297 (STAR 29). This is a factor of 5 to 10-fold higher than the average in the control colonies.
We conclude that a) the vast majority of STAR elements have a positive effect on gene expression levels, b) there is variation in the degree of positive effects induced by the different STAR elements, and c) 10 out of 53 tested STAR elements induce more than 5-fold average d2EGFP expression levels, as compared to the control, and that STAR elements can induce a 10-fold higher average d2EGFP expression level, as compared to the control.

### Example 26: Other chromatin control elements in the context of a selection system of the invention

DNA elements such as the HS4 hypersensitive site in the locus control region of the chicken β-globin locus (Chung et al, 1997), matrix attachment regions (MAR) (Stief et al, 1989) and a ubiquitous chromatin opening element (UCOE) (Williams et al, 2005) have been reported to have beneficial effects on gene expression when these DNA elements are incorporated in a vector. We combined these DNA elements with the selection system of the invention.

### Results

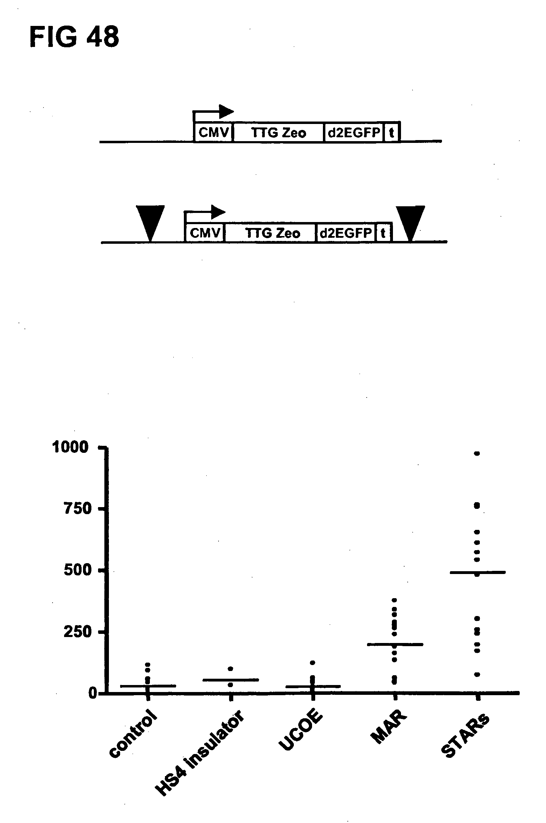

The 1.25 kb HS4 element was cloned into the cassette encompassing the CMV promoter, TTG Zeo and d2EGFP by a three way ligation step to obtain a construct with a tandem of 2 HS4 elements (Chung et al, 1997). This step was done both for the 5' and 3' of the cassette encompassing the CMV promoter, TTG Zeo and d2EGFP. The 2959 bp long chicken lysozyme MAR (Stief et al, 1989) was cloned 5' and 3' of the cassette encompassing the CMV promoter, TTG Zeo and d2EGFP.
The 2614 bp long UCOE (Williams et al, 2005) was a NotI-KpnI fragment, excised from a human BAC clone (RP11-93D5), corresponding to nucleotide 29449 to 32063. This fragment was cloned 5' of the CMV promoter.
The STAR construct contained STAR7 and STAR67 5' of the CMV promoter and STAR7 3' of the cassette. These four constructs, as well as the control construct without flanking chromatin control DNA elements, were transfected to CHO-K1 cells. Selection was performed by 200 µg/ml Zeocin in the culture medium. Colonies were isolated, propagated and d2EGFP expression levels were measured. As shown in Fig. 48 constructs with all DNA elements resulted in the formation of d2EGFP expressing colonies. However, incorporation of 2xHS4 elements and the UCOE did not result in the formation of colonies that displayed higher d2EGFP expression levels, in comparison with the control colonies. In contrast, incorporation of the lysozyme MAR resulted in the formation of colonies that expressed d2EGFP significantly higher. The mean expression level induced by MAR containing constructs was four-fold higher than in the control colonies. Best results were obtained, however, by incorporating STAR 7 and 67 in the construct. An almost ten-fold increase in the mean d2EGFP expression level was observed, as compared to the control colonies. We conclude that other chromatin control DNA elements such as MARs can be used in the context of the selection system of the invention. However, the best results were obtained when STAR elements were used as chromatin control elements.

### DESCRIPTION OF FIGURES

**Fig. 1****.** Schematic diagram of transcription units without and with STAR elements.
   A) bicistronic gene containing (from 5' to 3') a transgene (encoding for example the d2EGFP gene), an IRES, and a selectable marker (zeo, conferring zeocin resistance) under control of the CMV promoter. The expression unit has the SV40 transcriptional terminator at its 3' end (t). The name of the construct is CMV-d2EGFP-ires-Zeo (CMV Control).
   B) construct as in A, but now STAR 67 is cloned upstream of the CMV promoter. The name of the construct is STAR67-CMV-d2EGFP-ires-Zeo (CMV-STAR67).
   C) construct as in B, but upstream and downstream STAR7 elements are cloned to flank the entire construct. The name of the construct is STAR7-STAR67-CMV-d2EGFP-ires-Zeo-STAR7 (CMV-STAR67 7/7)
**Fig. 2****.** STAR67 improves CMV driven d2EGFP expression in CHO cells.
   The mean of the d2EGFP signal for 10 independent stable colonies is plotted for the indicated constructs in CHO cells. A) CMV Control; B) STAR67-CMV. X(10): average d2EGFP expression levels of the 10 colonies. See example 2 for details.
**Fig. 3****.** STAR67 improves EF1α driven d2EGFP expression in CHO cells. Same as Fig. 2, but now with EF1α promoter. A) EF1α Control; B) STAR67- EF1α.
**Fig. 4****.** STAR67 improves UB6 driven d2EGFP expression in CHO cells. Same as Figs. 2 and 3, but now with UB6 promoter. A) UB6 Control; B) STAR67-UB6.
**Fig. 5****.** STAR67 improves CMV driven d2EGFP expression in PER.C6 cells. Same as Fig. 2, but now in PER.C6 cells. A) CMV Control; B) STAR67-CMV.
**Fig. 6**. STAR67 improves EF1α driven d2EGFP expression in PER.C6 cells. Same as Fig. 3, but now in PER.C6 cells. A) EF1α Control; B) STAR67- EF1α.
**Fig. 7****.** STAR67 improves UB6 driven d2EGFP expression in PER.C6 cells. Same as Fig. 4, but now in PER.C6 cells. A) UB6 Control; B) STAR67-UB6.
**Fig. 8****.** STAR67 improves SV40 driven d2EGFP expression in CHO-K1 cells in combination with another STAR element. Similar as Fig. 2, but now using the SV40 promoter in CHO cells, and the indicated constructs. The mean of the d2EGFP signal is plotted for 18-20 independent stable colonies 60 days after transfection. A) SV40 Control, SV40-STAR67, SV40-STAR7/7; B) SV40 Control and SV40-STAR67 7/7. See example 4 for details.
**Fig. 9****.** STAR67 improves UB6 driven expression levels of the EpCAM antibody in CHO-K1 cells. See example 5 for details. A) constructs without STAR elements; B) constructs with STAR67 upstream of promoter and flanking STAR7 elements. The anti-EpCAM antibody concentration is presented as pg/cell/day. X(18): average production level of the 18 colonies.
**Fig. 10****.** STAR67 is not an enhancer blocker, whereas STAR 6 and 7 are. See example 6 for details.
**Fig. 11****.** STAR67 enhances UB6 and CMV-driven antibody expression levels in stably transfected CHO cells. See example 7 for details. A) single DNA molecule containing anti-EpCAM heavy chain (HC) and light chain (LC), each behind a promoter, and each linked to a different selectable marker gene (simultaneous selection was used for both markers): construct without STAR elements. No colonies were found; B) same construct with STAR67 between the two promoters. The anti-EpCAM antibody concentration is presented as pg/cell/day. X(19): average production level of the 19 colonies.
**Fig. 12****.** Schematic representation of the use of a selection marker gene (zeocin resistance gene) according to the invention. A. wild-type zeocin resitance gene, having its normal translation initation site (ATG startcodon) and one internal ATG codon, which codes for methionine. B. mutant zeocin resistance gene, wherein the internal ATG has been mutated into a codon for leucine; this mutant is a functional zeocin resistance gene. C. same as B, but comprising a mutated translation initiation site, wherein the context of the ATG startcodon has been mutated to decrease the translation initiation. D. same as B, but comprising a mutated startcodon (GTG). E. same as B, but with a TTG startcodon. The numbers under the figures C-E schematically indicate a relative amount of initiation frequency (under the startcodon) and 'scan-through' frequency (under the coding sequence) by the ribosomes, but only in a semi-quantitative manner, i.e. they indicate the efficiency of translation initiation compared to each other, but the qualitative numbers may differ completely: the numbers only serve to explain the invention. See example 8 for details.
**Fig. 13****.** Schematic representation of a multicistronic transcription unit according to the invention, with more or less reciprocal interdependent translation efficiency. Explanation as for Fig. 12, but now a dEGFP gene (here exemplifying a gene of interest) has been placed downstream of the selectable marker polypeptide coding sequence. The Zeocin resistance gene comprises the internal Met→Leu mutation (see Fig. 12B). See example 9 for details.
**Fig. 14****.** Results of selection systems according to the invention, with and without STAR elements. A. zeocin resistance gene with ATG startcodon in bad context (referred to as "ATGmut" in the picture, but including a spacer sequence behind the ATG in the bad context, so in the text generally referred to as "ATGmut/space"). B. zeocin resistance gene with GTG startcodon. C. zeocin resistance gene with TTG startcodon. d2EGFP signal for independent colonies is shown on the vertical axis. See example 9 for details.
**Fig. 15****.** Results of selection system according to the invention in upscaled experiment (A), and comparison with selection system according to prior art using an IRES (B). d2EGFP signal for independent colonies is shown on the vertical axis. See example 10 for details.
**Fig. 16****.** Results of selection system with multicistronic transcription unit according to the invention, using blasticidin as a selectable marker. A. blasticidin resistance gene mutated to comprise a GTG startcodon. B. blasticidin resistance gene mutated to comprise a TTG startcodon. The blasticidin resistance gene has further been mutated to remove all internal ATG sequences. d2EGFP signal for independent colonies is shown on the vertical axis. See example 11 for details.
**Fig. 17****.** Stability of expression of several clones with a multicistronic transcription unit according to the invention (including a zeocin with TTG startcodon). Selection pressure (100 µg/ml zeocin) was present during the complete experiment. d2EGFP signal for independent colonies is shown on the vertical axis. See example 12 for details.
**Fig. 18****.** As Fig. 17, but zeocin concentration was lowered to 20 µg/ml after establishment of clones.
**Fig. 19****.** As Fig. 17, but zeocin was absent from culture medium after establishment of clones.
**Fig. 20****.** Expression of an antibody (anti-EpCAM) using the selection system with the multicistronic transcription unit according to the invention. The heavy chain (HC) and light chain (LC) are the polypeptide of interest in this example. Each of these is present in a separate transcription unit, which are both on a single nucleic acid molecule in this example. The HC is preceded by the zeocin resistance gene coding for a selectable marker polypeptide, while the LC is preceded by the blasticidin resistance gene coding for a selectable marker polypeptide. Both resistance genes have been mutated to comprise an ATG startcodon in a non-optimal context ("mutATG" in Figure, but including a spacer sequence, and hence in the text generally referred to as "ATGmut/space"). Each of the multicistronic transcription units is under control of a CMV promoter. Constructs with STAR sequences as indicated were compared to constructs without STAR sequences. The antibody levels obtained when these constructs were introduced into host cells are given on the vertical axis in pg/cell/day for various independent clones. See example 13 for details.
**Fig. 21****.** As Fig. 20, but both the selection marker genes have been provided with a GTG startcodon. See example 13 for details.
**Fig. 22****.** As Fig. 20, but both the selection marker genes have been provided with a TTG startcodon. See example 13 for details.
**Fig. 23****.** Stability of expression in sub-clones in the absence of selection pressure (after establishing colonies under selection pressure, some colonies where sub-cloned in medium containing no zeocin). See example 12 for details.
**Fig. 24****.** Copy-number dependency of expression levels of an embodiment of the invention. See example 12 for details.
**Fig. 25****.** As Fig. 12, but for the blasticidin resistance gene. None of the 4 internal ATG's in this gene are in frame coding for a methionine, and therefore the redundancy of the genetic code was used to mutate these ATG's without mutating the internal amino acid sequence of the encoded protein.
**Fig. 26****.** Coding sequence of the wild-type zeocin resistance gene (SEQ. ID. NO. 108). Bold ATG's code for methione. The first bold ATG is the startcodon.
**Fig. 27****.** Coding sequence of the wild-type blasticidin resistance gene (SEQ. ID. NO. 110). Bold ATG's code for methione. The first bold ATG is the startcodon. Other ATG's in the sequence are underlined: these internal ATG's do not code for methionine, because they are not in frame.
**Fig. 28****.** Coding sequence of the wild-type puromycin resistance gene (SEQ. ID. NO. 112). Bold ATG's code for methione. The first bold ATG is the startcodon.
**Fig. 29****.** Coding sequence of the wild-type mouse DHFR gene (SEQ. ID. NO. 114). Bold ATG's code for methione. The first bold ATG is the startcodon. Other ATG's in the sequence are underlined: these internal ATG's do not code for methionine, because they are not in frame.
**Fig. 30****.** Coding sequence of the wild-type hygromycin resistance gene (SEQ. ID. NO. 116). Bold ATG's code for methione. The first bold ATG is the startcodon. Other ATG's in the sequence are underlined: these internal ATG's do not code for methionine, because they are not in frame.
**Fig. 31****.** Coding sequence of the wild-type neomycin resistance gene (SEQ. ID. NO. 118). Bold ATG's code for methione. The first bold ATG is the startcodon. Other ATG's in the sequence are underlined: these internal ATG's do not code for methionine, because they are not in frame.
**Fig. 32****.** Coding sequence of the wild-type human glutamine synthase (GS) gene (SEQ. ID. NO. 120). Bold ATG's code for methione. The first bold ATG is the startcodon. Other ATG's in the sequence are underlined: these internal ATG's do not code for methionine, because they are not in frame.
**Fig. 33****.** Schematic representation of some further modified zeocin resistance selection marker genes with a GTG startcodon according to the invention, allowing for further fine-tuning of the selection stringency. See example 14 for details.
**Fig. 34****.** Results with expression systems containing the further modified zeocin resistance selection marker genes. See example 14 for details. Dots indicate individual data points; lines indicate the average expression levels; used constructs (see also Fig. 33) are indicated on the horizontal axis (the addition of 7/67/7 at the end of the construct name indicates the presence of STAR sequences 7 and 67 upstream of the promoter and STAR7 downstream of the transcription termination site), and schematically depicted above the graph; vertical axis indicates d2EGFP signal.
**Fig. 35****.** Schematic representation of some further modified zeocin resistance selection marker genes with a TTG startcodon according to the invention, allowing for further fine-tuning of the selection stringency. See example 15 for details.
**Fig. 36****.** Results with expression systems containing the further modified zeocin resistance selection marker genes. See example 15 for details. Dots indicate individual data points; lines indicate the average expression levels; used constructs are indicated on the horizontal axis, and schematically depicted above the graph; vertical axis indicates d2EGFP signal.
**Fig. 37****.** As Fig. 12, but for the puromycin resistance gene. All three internal ATG's code for methione (panel A), and are replaced by CTG sequences coding for leucine (panel B). See example 16 for details.
**Fig. 38****.** Results with expression constructs containing the puromycin resistance gene with a TTG startcodon and no internal ATG codons. See example 16 for details. Dots indicate individual data points; lines indicate the average expression levels; used constructs are indicated on the horizontal axis, and schematically depicted above the graph; vertical axis indicates d2EGFP signal.
**Fig. 39****.** As Fig. 12, but for the neomycin resistance gene. See Example 17 for details. A. wild-type neomycin resistance gene; ATG sequences are indicated, ATGs coding for methionine are indicated by Met above the ATG. B. neomycin resistance gene without ATG sequences, and with a GTG startcodon. C. neomycin resistance gene without ATG sequences, and with a TTG startcodon.
**Fig. 40****.** As Fig. 12, but for the dhfr gene. See Example 18 for details. A. wild-type dhfr gene; ATG sequences are indicated, ATGs coding for methionine are indicated by Met above the ATG. B. dhfr gene without ATG sequences, and with a GTG startcodon. C. dhfr gene without ATG sequences, and with a TTG startcodon.
**Fig. 41****.** Results with expression constructs (zeocin selectable marker) according to the invention in PER.C6 cells. See Example 20 for details. Dots indicate individual data points; lines indicate the average expression levels; used constructs are indicated on the horizontal axis, and schematically depicted above the graph; vertical axis indicates d2EGFP signal.
**Fig. 42****.** Results with expression constructs (blasticidin selectable marker) according to the invention in PER.C6 cells. See Example 20 for details. Dots indicate individual data points; lines indicate the average expression levels; used constructs are indicated on the horizontal axis, and schematically depicted above the graph; vertical axis indicates d2EGFP signal.
**Fig. 43****.** Results with expression constructs according to the invention, further comprising a transcription pause (TRAP) sequence. See Example 21 for details. Dots indicate individual data points; lines indicate the average expression levels; used constructs are indicated on the horizontal axis, and schematically depicted above the graph; vertical axis indicates d2EGFP signal.
**Fig. 44****.** Copy-number dependency of expression of an antibody using transcription units according to the invention. See Example 22 for details.
**Fig. 45****.** Antibody expression from colonies containing expression constructs according to the invention, wherein the copy number of the expression constructs is amplified by methotrexate. See Example 23 for details. White bars: selection with zeocin and blasticidin; black bars: selection with zeocin, blasticidin and methotrexate (MTX). Numbers of tested colonies are depicted on the horizontal axis.
**Fig. 46****.** Results with different promoters. See Example 24 for details. Dots indicate individual data points; lines indicate the average expression levels; used constructs are indicated on the horizontal axis, and schematically depicted above the graph; vertical axis indicates d2EGFP signal.
**Fig. 47****.** Results with different STAR elements. See example 25 for details. Dots indicate individual data points; lines indicate the average expression levels; used constructs are indicated on the horizontal axis, and schematically depicted above the graph; vertical axis indicates d2EGFP signal.
**Fig. 48****.** Results with other chromatin control elements. See Example 26 for details. Dots indicate individual data points; lines indicate the average expression levels; used constructs are indicated on the horizontal axis, and schematically depicted above the graph (black triangles indicate different tested chromatin control elements); vertical axis indicates d2EGFP signal.

### References

Boshart, M, Weber, F, Jahn, G, Dorsch-Hasler, K, Fleckenstein, B, and Schaffner, W. (1985) A very strong enhancer is located upstream of an immediate early gene of human cytomegalovirus Cell 41, 521-530.
Chung JH, Whiteley M, and Felsenfeld G. (1993) A 5' element of the chicken beta-globin domain serves as an insulator in human erythroid cells and protects against position effect in Drosophila. Cell 74: 505-514.
Chung JH, Bell AC, Felsenfeld G. (1997). Characterization of the chicken beta-globin insulator. Proc Natl Acad Sci USA 94: 575-580.
Das, GC, Niyogi, SK, and Salzman, NP. (1985) SV40 promoters and their regulation Prog Nucleic Acid Res Mol Biol 32, 217-236.
Dumas, P, Bergdoll, M., Cagnon, C and Masson JM. 1994. Crystal structure and site-directed mutagenesis of a bleomycin resistance protein and their significance for drug sequestering. EMBO J 13, 2483-2492.
Gill DR, Smyth SE, Goddard CA, Pringle IA, Higgins CF, Colledge WH, and Hyde SC. (2001) Increased persistence of lung gene expression using plasmids containing the ubiquitin C or elongation factor 1α promoter. Gene Therapy 8: 1539-1546.
Gossen M, and Bujard H. (1992) Tight control of gene expression in mammalian cells by tetracycline-responsive promoters. Proc Natl Acad Sci USA 89: 5547-5551.
Graham FO, Smiley J, Russell W and Nairn R. (1977). Characteristics of a human cell line transformed by DNA from human adenovirus type 5. J. Gen. Virol. 36, 59-72.
Huls GA, Heijnen IAFM, Cuomo ME, Koningsberger JC, Wiegman L, Boel E, van der Vuurst-de Vries A-R, Loyson SAJ, Helfrich W, van Berge Henegouwen GP, van Meijer M, de Kruif J, Logtenberg T. (1999). A recombinant, fully human monoclonal antibody with antitumor activity constructed from phage-displayed antibody fragments. Nat Biotechnol. 17, 276-281.
Jones D, Kroos N, Anema R, Van Montfort B, Vooys A, Van Der Kraats S, Van Der Helm E, Smits S, Schouten J, Brouwer K, Lagerwerf F, Van Berkel P, Opstelten D-J, Logtenberg T, Bout A (2003) High-level expression of recombinant IgG in the human cell line PER.C6. Biotechnol. Prog. 19: 163-168.
Kaufman, RJ. (2000) Overview of vector design for mammalian gene expression Mol Biotechnol 16, 151-160.
Kaufman, RJ, and Sharp, PA. (1982) Construction of a modular dihydrofolate reductase cDNA gene: analysis of signals utilized for efficient expression Mol Cell Biol 2, 1304-1319.
Kellum R, and Schedl P. (1991) A position-effect assay for boundaries of higher order chromosomal domains. Cell 64: 941-950.
Kim SJ, Kim Ns, Ryu CJ, Hong HJ, Lee GM. 1998. Characterization of chimeric antibody producing CHO cells in the course of dihydrofolate reductase-mediated gene amplification and their stability in the absence of selective pressure. Biotechnol Bioeng 58: 73-84.
Kozak M. (1986) Point mutations define a sequence flanking the AUG initiator codon that modulates translation by eukaryotic ribosomes. Cell 44: 283-292.
Kozak M. (1987) An analysis of 5'-noncoding sequences from 699 vertebrate messenger RNAs. Nucleic Acids Res. 15: 8125-8148.
Kozak M. (1989) Context effects and inefficient initiation at non-AUG codons in eucaryotic cell-free translation systems. Mol Cell Biol. 9: 5073-5080.
Kozak M. (1990) Downstream secondary structure facilitates recognition of initiator codons by eukaryotic ribosomes. Proc Natl Acad Sci USA 87:8301-8305.
Kozak M. (1997) Recognition of AUG and alternative initiator codons is augmented by G in position +4 but is not generally affected by the nucleotides in positions +5 and +6. EMBO J. 16: 2482-2492.
Kozak M. (2002) Pushing the limits of the scanning mechanism for initiation of translation. Gene 299: 1-34.
Kwaks TH, Barnett P, Hemrika W, Siersma T, Sewalt RG, Satijn DP, Brons JF, van Blokland R, Kwakman P, Kruckeberg AL, Kelder A, Otte AP. (2003) Identification of anti-repressor elements that confer high and stable protein production in mammalian cells. Nat Biotechnol 21, 553-558. Erratum in: Nat Biotechnol 21, 822 (2003).
Phi-Van L, Von Kreis JP, Ostertag W, and Strätling WH. (1990) The chicken lysozyme 5' matrix attachment region increases transcription from a heterologous promoter in heterologous cells and dampens position effects on the expression of transfected genes. Mol. Cell. Biol. 10: 2302-2307.
McBurney, MW, Mai, T, Yang, X, and Jardine, K. (2002) Evidence for repeat-induced gene silencing in cultured Mammalian cells: inactivation of tandem repeats of transfected genes Exp Cell Res 274, 1-8.
Rees, S, Coote, J, Stables, J, Goodson, S, Harris, S, and Lee, MG. (1996) Bicistronic vector for the creation of stable mammalian cell lines that predisposes all antibiotic-resistant cells to express recombinant protein Biotechniques 20, 102-104, 106, 108-110.
Schorpp, M, Jager, R, Schellander, K, Schenkel, J, Wagner, EF, Weiher, H, and Angel, P. (1996) The human ubiquitin C promoter directs high ubiquitous expression of transgenes in mice Nucleic Acids Res 24, 1787-8.
Stief A, Winter DM, Stratling WH, Sippel AE (1989) A nuclear DNA attachment element mediates elevated and position-independent gene activity. Nature 341: 343-345.
Van der Vlag, J, den Blaauwen, JL, Sewalt, RG, van Driel, R, and Otte, AP. (2000) Transcriptional repression mediated by polycomb group proteins and other chromatin-associated repressors is selectively blocked by insulators J Biol Chem 275, 697-704.
West AG, Gaszner M, Felsenfeld G (2002) Insulators: many functions, many mechanisms. Genes Dev. 16: 271-288.
Whitelaw, E, Sutherland, H, Kearns, M, Morgan, H, Weaving, L, and Garrick, D. (2001) Epigenetic effects on transgene expression Methods Mol Biol 158, 351-68.
Williams S, Mustoe T, Mulcahy T, Griffiths M, Simpson D, Antoniou M, Ivine A, Mountain A, Crombie R (2005) CpG-island fragments from the HNRPA2B1/CBX3 genomic locus reduce silencing and enhance transgene expression from the hCMV promoter/enhancer in mammalian cells. BMC Biotechnol. 5:17.

## Claims

1. A DNA molecule comprising a multicistronic transcription unit coding for i) a selectable marker polypeptide capable of being selected for in a eukaryotic host cell, and for ii) a polypeptide of interest,
the polypeptide of interest having a translation initiation sequence separate from that of the selectable marker polypeptide,
wherein:
the coding sequence for the polypeptide of interest is downstream from the coding sequence for the selectable marker in said multicistronic transcription unit, and
the sequence encoding the selectable marker polypeptide has no ATG sequence in the coding strand following the startcodon of the selectable marker polypeptide up to the startcodon of the polypeptide of interest, and wherein the translation start sequence in the coding strand for the selectable marker polypeptide is chosen from the group consisting of:
a) a translation start sequence comprising a mutation in the nucleotides in positions -3 to -1 and/or +4 of the consensus sequence RCCATGG (wherein R is A or G, and wherein the A of the ATG startcodon is nt +1);
b) a GTG startcodon;
c) a GTG startcodon;
d) a CTG startcodon;
e) an ATT startcodon; and
f) an ACT startcodon.

2. A DNA molecule according to claim 1, wherein the resulting translation start sequence in the coding strand for the selectable marker polypeptide comprises a GTG startcodon or a TTT startcodon.

3. A DNA molecule according to claim 1 or 2, wherein any sequence ATG when present in frame and coding for methionine in the sequence coding for the wild type selectable marker polypeptide is mutated to code for valine, threonine,

4. A DNA molecule according to any of the preceding claims, wherein the selectable marker polypeptide provides resistance against lethal or growth-inhibitory effects of a selection agent, for example an antibiotic.

5. A DNA molecule according to claim 4, wherein said selection agent is chosen from the group consisting of: zeocin, puromycin, blasticidin, hygromycin, neomycin, methotrexate, methionine sulphoximine and kanamycin.

6. A DNA molecule according to claim 5, wherein the selection agent is zeocin.

7. A DNA molecule according to claim 5, wherein the selectable marker polypeptide is encoded by the neomycin resistance gene.

8. A DNA molecule according to any one of the preceding claims, wherein the selectable marker polypeptide further comprises a mutation that reduces the activity of the selectable marker polypeptide compared to its wild-type counterpart, for example wherein the selectable marker polypeptide is a zeocin resistance polypeptide wherein proline at position 9 is changed into a different amino acid.

9. A DNA molecule according to any one of the preceding claims, wherein the coding sequence of the polypeptide of interest comprises an optimal translation start sequence.

10. An expression cassette comprising a DNA molecule according to any one of the preceding claims, said expression cassette further comprising a promoter upstream of said multicistronic expression unit and a transcription termination sequences downstream of the multicistronic expression unit.

11. An expression cassette according to claim 10, further comprising at least one chromatin control element, chosen from the group consisting of a matrix or scaffold attachment region (MAR/SAR), an insulator sequence, a universal chromatin opening element (UCOE), and an anti-repressor (STAR) sequence.

12. An expression cassette according to claim 11, wherein said chromatin control element is an anti-repressor sequence chosen from the group consisting of:
a) any one of SEQ. ID. NO. 1 through SEQ. ID. NO. 66;
b) fragments of any one of SEQ. ID. NO. 1 through SEQ. ID. NO. 66, wherein said fragments have anti-repressor activity;
c) sequences that are at least 70% identical in nucleotide sequence to a) or b) wherein said sequences have anti-repressor activity; and
d) the complement to any one of a) to c).

13. An expression cassette according to claim 12, wherein said expression cassette comprises one of:
a) SEQ. ID. NO. 66,
b) a fragment of a) having anti-repressor activity;
c) a sequence that is at least 70% identical in nucleotide sequence to a) or b) wherein said sequence has anti-repressor activity,
wherein a), b) or c) is positioned upstream of the promoter that drives transcription of the multicistronic gene.

14. An expression cassette according to any one of claims 10-13, wherein said multicistronic gene is flanked on both sides by at least one anti-repressor sequence.

15. An expression cassette according to claim 14, comprising: 5' - anti-repressor sequence A - anti-repressor sequence B - promoter - multicistronic gene encoding the functional selectable marker protein and downstream thereof the polypeptide of interest - transcription termination sequence - anti-repressor sequences C - 3',
wherein anti-repressor sequences A and C may be the same or different and are chosen from the group consisting of:
a) any one of SEQ. ID. NO. 1 through SEQ. ID. NO. 65;
b) fragments of any one of the sequences of a), said fragments having anti-repressor activity;
c) sequences that are at least 70% identical in nucleotide sequence to a) or b) wherein said sequences have anti-repressor activity,
and wherein anti-repressor sequence B is chosen from the group consisting of
i) SEQ. ID. NO. 66,
ii) a fragment of i) having anti-repressor activity; and
iii) a sequence that is at least 70% identical in nucleotide sequence to i) or ii) wherein said sequence has anti-repressor activity.

16. An expression cassette according to claim 15, wherein anti-repressor sequences A and C are chosen from:
a) any one of SEQ. ID. NO. 7, SEQ. ID. NO. 9, SEQ. ID. NO. 17, SEQ. ID. NO. 27, SEQ. ID. NO. 29, SEQ. ID. NO. 43, SEQ. ID. NO. 44, SEQ. ID. NO. 45, SEQ. ID. NO. 47, or SEQ. ID. NO. 61;
b) fragments of one of the sequences of a), said fragments having anti-repressor activity; and
c) sequences that are at least 70% identical in nucleotide sequence to a) or b) wherein said sequences have anti-repressor activity.

17. An expression cassette according to any one of claims 10-16, wherein the polypeptide of interest is a part of a multimeric protein.

18. An expression cassette according to claim 17, wherein the polypeptide of interest is an immunoglobulin tight chain or an immunoglobulin heavy chain.

19. An expression cassette according to any one of claims 10-18, further comprising:
a) a transcription pause (TRAP) sequence upstream of said promoter and being in a 5' to 3' direction; or
b) a TRAP sequence downstream of said transcription termination sequence and being in a 3' to 5' orientation; or
c) both a) and b);
wherein a TRAP sequence is a sequence which when placed into a transcription unit, results in a reduced level of transcription in the nucleic acid present on the 3' side of the TRAP when compared to the level of transcription observed in the nucleic acid on the 5' side of the TRAP,
for example wherein said TRAP is SEQ. ID. NO. 142.

20. A DNA molecule comprising a sequence encoding a functional delectable marker polypeptide capable of being selected for in a eukaryotic host cell, **characterized in that** said DNA molecule:
i) has a translation start sequence chosen from the group consisting of:
a) a translation start sequence comprising a mutation in the nucleotides in positions -3 to -1 and/or +4 of the consensus sequence RCCATGG (wherein R is A or G, and wherein the A of the ATG startcodon is nt +1);
b) a GTG startcodon;
c) a TTG startcodon;
d) a CTG startcodon;
e) an ATT startcodon; and
f) an ACG startcodon, followed by an otherwise functional selectable marker coding sequence, and
ii) **in that** the coding strand of the sequence defining the selectable marker polypeptide downstream of the non-optimal startcodon and up to the stopcodon is devoid of ATG sequences.

21. A host cell comprising a DNA molecule or an expression cassette according to any one of the preceding claims.

22. A host cell according to claim 21, which is a eukaryotic host cell, preferably a mammalian host cell.

23. A host cell according to claim 22, being a Chinese Hamster Ovary (CHO) cell.

24. A method for generating a host cell expressing a polypeptide of interest, the method comprising the steps of:
a) introducing into a plurality of precursor cells a DNA molecule according to any one of claims 1-9 or an expression cassette according to any one of claims 10-19, and
b) culturing the generated cells under conditions selecting for expression of the selectable marker polypeptide, and
c) selecting at least one host cell producing the polypeptide of interest.

25. A method for producing a polypeptide of interest, comprising culturing a host cell, said host cell comprising an expression cassette according to any one of claims 10-19 and expressing the polypeptide of interest from the expression cassette.

26. A method according to claim 25, further comprising isolating the polypeptide of interest.

## Patentansprüche

1. DNA-Molekül, umfassend eine multicistronische Transkriptionseinheit, die i) ein selektierbares Markerpolypeptid, das in der Lage ist, in einer eukaryotischen Wirtszelle selektiert zu werden, und ii) ein Polypeptid von Interesse codiert,
wobei das Polypeptid von Interesse eine Translations-Initiationssequenz hat, die getrennt ist von der des selektierbaren Markerpolypeptids,
wobei
die codierende Sequenz des Polypeptids von Interesse stromabwärts von der codierenden Sequenz des selektierbaren Markers in der multicistronischen Transkriptionseinheit liegt, und
die Sequenz, die das Polypeptid des selektierbaren Markers codiert, keine ATG-Sequenz im codierenden Strang hat, der dem Startcodon des Polypeptids des selektierbaren Markers bis zu dem Startcodon des Polypeptids von Interesse folgt, und wobei die Translations-Startsequenz in dem codierenden Strang des selektierbaren Markerpolypeptids ausgewählt ist aus der Gruppe bestehend aus:
a) einer Translations-Startsequenz umfassend eine Mutation in den Nucleotiden an Positionen -3 bis -1 und/oder +4 der Konsensus-Sequenz RCCATGG (wobei R A oder G ist, und wobei das A des Startcodons ATG nt+1 ist);
b) einem GTG-Startcodon;
c) einem TTG-Startcodon;
d) einem CTG-Startcodon;
e) einem ATT-Startcodon; und
f) einem ACG-Startcodon.

2. DNA-Molekül nach Anspruch 1, wobei die sich ergebende Translations-Startsequenz in dem codierenden Strang des selektierbaren Markerpolypeptids ein GTG-Startcodon oder ein TTG-Startcodon umfasst.

3. DNA-Molekül nach Anspruch 1 oder 2, wobei jede ATG-Sequenz, wenn diese im Leserahmen vorliegt und Methionin in der Sequenz codiert, die das selektierbare Markerpolypeptid des Wildtyps codiert, so mutiert ist, dass Valin, Threonin, Isoleucin oder Leucin codiert wird.

4. DNA-Molekül nach einem der vorangegangenen Ansprüche, wobei das selektierbare Markerpolypeptid Resistenz gegenüber tödlichen oder wachstumshemmenden Auswirkungen eines Selektionswirkstoffes, zum Beispiel eines Antibiotikums, bietet.

5. DNA-Molekül nach Anspruch 4, wobei der Selektionswirkstoff ausgewählt ist aus der Gruppe bestehend aus: Zeocin, Puromycin, Blasticidin, Hygromycin, Neomycin, Methotrexat, Methionin, Sulphoximin und Kanamycin.

6. DNA-Molekül nach Anspruch 5, wobei der Selektionswirkstoff Zeocin ist.

7. DNA-Molekül nach Anspruch 5, wobei das selektierbare Markerpolypeptid von dem Neomycin-Resistenzgen codiert wird.

8. DNA-Molekül nach einem der vorangegangenen Ansprüche, wobei das selektierbare Markerpolypeptid zudem eine Mutation umfasst, die die Aktivität des selektierbaren Markerpolypeptids reduziert, verglichen mit dem entsprechenden Wildtyp-Gegenstück, wobei zum Beispiel das selektierbare Markerpolypeptid ein Zeocin-Resistenzpolypeptid ist, wobei Prolin an Position 9 gegen eine andere Aminosäure ausgetauscht ist.

9. DNA-Molekül nach einem der vorangegangenen Ansprüche, wobei die codierende Sequenz des Polypeptids von Interesse eine optimale Translations-Startsequenz umfasst.

10. Expressionskassette umfassend ein DNA-Molekül nach einem der vorangegangenen Ansprüche, wobei die Expressionskassette zudem einen Promotor stromaufwärts der multicistronischen Expressionseinheit und eine Transkriptions-Terminationssequenz stromabwärts der multicistronischen Expressionseinheit umfasst.

11. Expressionskassette nach Anspruch 10, die zudem mindestens ein Chromatin-Kontrollelement umfasst, ausgewählt aus der Gruppe bestehend aus einer Matrix- oder Gerüst-Attachment-Region (MAR/SAR), einer Isolator-Sequenz, einem universellen Chromatin-Opening-Element (UCOE) und einer Anti-Repressor-Sequenz (STAR).

12. Expressionkassette nach Anspruch 11, wobei das Chromatin-Kontrollelement eine Anti-Repressor-Sequenz ist, ausgewählt aus der Gruppe bestehend aus:
a) einer der Sequenzen von SEQ ID NO. 1 bis SEQ ID NO. 66;
b) Fragmenten einer der Sequenzen von SEQ ID NO. 1 bis SEQ ID NO. 66, wobei die Fragmente eine Anti-Repressor-Aktivität haben;
c) Sequenzen, die bezüglich der Nucleotidsequenz zu mindestens 70% identisch zu a) oder b) sind, wobei die Sequenzen eine Anti-Repressor-Aktivität haben; und
d) dem Komplement zu einer Sequenz nach a) bis c).

13. Expressionskassette nach Anspruch 12, wobei die Expressionskassette eine der folgenden Sequenzen umfasst:
a) SEQ ID NO. 66,
b) ein Fragment von a), das eine Anti-Repressor-Aktivität hat;
c) eine Sequenz, die bezüglich der Nucleotidsequenz zu mindestens 70% identisch ist zu a) oder b), wobei die Sequenz eine Anti-Repressor-Aktivität hat;
wobei a), b) oder c) stromaufwärts des Promotors positioniert ist, der die Transkription des multicistronischen Gens antreibt.

14. Expressionskassette nach einem der Ansprüche 10 bis 13, wobei das multicistronische Gen auf beiden Seiten von mindestens einer Anti-Repressor-Sequenz flankiert ist.

15. Expressionskassette nach Anspruch 14, umfassend: 5' - Anti-Repressor-Sequenz A - Anti-Repressor-Sequenz B - Promotor - multicistronisches Gen, das das funktionelle selektierbare Markerprotein und stromabwärts davon das Polypeptid von Interesse codiert - Transkriptions-Terminationssequenz - Anti-Repressor-Sequenz C - 3',
wobei die Anti-Repressor-Sequenzen A und C dieselben oder verschieden sein können und ausgewählt sind aus der Gruppe bestehend aus:
a) einer der Sequenzen von SEQ ID NO. 1 bis SEQ ID NO. 65;
b) Fragmenten einer der Sequenzen von a), wobei die Fragmente eine Anti-Repressor-Aktivität haben;
c) Sequenzen, die bezüglich der Nucleotidsquenz zu mindestens 70% identisch sind zu a) oder b), wobei die Sequenzen eine Anti-Repressor-Aktivität haben;
und wobei Anti-Repressor-Sequenz B ausgewählt ist aus der Gruppe bestehend aus:
i) SEQ ID NO. 66;
ii) einem Fragment von i), das Anti-Repressor-Aktivität hat; und
iii) einer Sequenz, die bezüglich der Nucleotidsequenz zu mindestens 70% identisch ist zu i) oder ii), wobei die Sequenz eine Anti-Repressor-Aktivität hat.

16. Expressionskassette nach Anspruch 15, wobei die Anti-Repressor-Sequenzen A und C ausgewählt sind aus:
a) einer der Sequenzen SEQ ID NO. 7, SEQ ID NO. 9, SEQ ID NO. 17, SEQ ID NO. 27, SEQ ID NO. 29, SEQ ID NO. 43, SEQ ID NO. 44, SEQ ID NO. 45, SEQ ID NO. 47 oder SEQ ID NO. 61;
b) Fragmenten einer der Sequenzen von a), wobei die Fragmente eine Anti-Repressor-Aktivität haben; und
c) Sequenzen, die bezüglich der Nucleotidsequenz zu mindestens 70% identisch sind zu a) oder b), wobei die Sequenzen eine Anti-Repressor-Aktivität haben.

17. Expressionskassette nach einem der Ansprüche 10 bis 16, wobei das Polypeptid von Interesse Teil eines multimeren Proteins ist.

18. Expressionskassette nach Anspruch 17, wobei das Polypeptid von Interesse ein leichte Kette eines Immunglobulins oder eine schwere Kette eines Immunglobulins ist.

19. Expressionskassette nach einem der Ansprüche 10 bis 18, die zudem umfasst:
a) eine Transkriptions-Pausen-Sequenz (TRAP), die stromaufwärts von dem Promotor liegt und eine 5' nach 3'-Richtung hat; oder
b) eine TRAP-Sequenz, die stromabwärts der Transkriptions-Terminationssequenz liegt und eine 3' nach 5'-Orientierung hat;
c) sowohl a) als auch b);
wobei eine TRAP-Sequenz eine Sequenz ist, die, wenn diese in eine Transkriptionseinheit eingebracht wird, dazu führt, dass der Spiegel der Transkription der Nucleinsäure, die auf der 3'-Seite der TRAP-Sequenz liegt, reduziert ist, wenn dieser mit dem Spiegel der Transkription verglichen wird, der bei der Nucleinsäure, die auf der 5'-Seite der TRAP-Sequenz liegt, festgestellt wird,
wobei zum Beispiel die TRAP-Sequenz SEQ ID NO. 142 ist.

20. DNA-Molekül umfassend eine Sequenz, die ein funktionelles selektierbares Markerpolypeptid codiert, das in der Lage ist, in einer eukaryotischen Wirtszelle selektiert zu werden, **dadurch gekennzeichnet, dass** das DNA-Molekül:
i) eine Translations-Startsequenz hat, die ausgewählt ist aus der Gruppe bestehend aus:
a) einer Translations-Startsequenz umfassend eine Mutation in den Nucleotiden an Positionen -3 bis -1 und/oder +4 der Konsensus-Sequenz RCCATGG (wobei R A oder G ist, und wobei das A des Startcodons ATG nt+1 ist);
b) einem GTG-Startcodon;
c) einem TTG-Startcodon;
d) einem CTG-Startcodon;
e) einem ATT-Startcodon; und
f) einem ACG-Startcodon, gefolgt von einer codierenden Sequenz eines ansonsten funktionellen selektierbaren Markers, und
ii) dass der codierende Strang der Sequenz, die das selektierbare Markerpolypeptid stromabwärts des nicht-optimalen Startcodons und bis zum Stopcodon definiert, ohne ATG-Sequenzen ist.

21. Wirtszelle umfassend ein DNA-Molekül oder eine Expressionskassette nach einem der vorangegangenen Ansprüche.

22. Wirtszelle nach Anspruch 21, die eine eukaryotische Wirtszelle, bevorzugt eine Säuger-Wirtszelle, ist.

23. Wirtszelle nach Anspruch 22, wobei die Wirtszelle eine Ovarialzelle des Chinesischen Hamsters (CHO) ist.

24. Verfahren zur Herstellung einer Wirtszelle, die ein Polypeptid von Interesse exprimiert, wobei das Verfahren die Schritte umfasst:
a) das Einbringen eines DNA-Moleküls nach einem der Ansprüche 1 bis 9 oder einer Expressionskassette nach einem der Ansprüche 10 bis 19 in eine Vielzahl von Vorläuferzellen, und
b) das Züchten der erzeugten Zellen unter Bedingungen zur Selektion der Expression des selektierbaren Markerpolypeptids, und
c) das Auswählen mindestens einer Wirtszelle, die das Polypeptid von Interesse produziert.

25. Verfahren zur Herstellung eines Polypeptids von Interesse, umfassend das Züchten einer Wirtszelle, wobei die Wirtszelle eine Expressionskassette nach einem der Ansprüche 10 bis 19 umfasst, und das Exprimieren des Polypeptids von Interesse von der Expressionskassette.

26. Verfahren nach Anspruch 25, das zudem die Isolierung des Polypeptids von Interesse umfasst.

## Revendications

1. Molécule d'ADN comprenant une unité de transcription multicistronique codant pour i) un polypeptide marqueur sélectionnable capable d'être sélectionné dans une cellule hôte eucaryote, et pour ii) un polypeptide d'intérêt,
le polypeptide d'intérêt ayant une séquence d'initiation de la traduction séparée de celle du polypeptide marqueur sélectionnable,
dans laquelle :
la séquence codante pour le polypeptide d'intérêt est située en aval de la séquence codante pour le marqueur sélectionnable dans ladite unité de transcription multicistronique, et
la séquence codant le polypeptide marqueur sélectionnable ne possède pas de séquence ATG dans le brin codant suivant le codon initiateur du polypeptide marqueur sélectionnable jusqu'au codon initiateur du polypeptide d'intérêt, et la séquence d'initiation de la traduction dans le brin codant pour le polypeptide marqueur sélectionnable étant choisie parmi le groupe constitué de :
a) une séquence d'initiation de la traduction comprenant une mutation au niveau des nucléotides des positions -3 à -1 et/ou +4 de la séquence consensus RCCATGG (dans laquelle R est A ou G, et dans laquelle la A du codon initiateur ATG est nt + 1) ;
b) un codon initiateur GTG ;
c) un codon initiateur TTG ;
d) un codon initiateur CTG ;
e) un codon initiateur ATT ; et
f) un codon initiateur ACG.

2. Molécule d'ADN selon la revendication 1, la séquence d'initiation de la traduction résultante dans le brin codant pour le polypeptide marqueur sélectionnable comprenant un codon initiateur GTG ou un codon initiateur TTG.

3. Molécule d'ADN selon la revendication 1 ou 2, dans laquelle n'importe quelle séquence ATG lorsque présente dans le cadre et codant pour la méthionine dans la séquence codante pour le polypeptide marqueur sélectionnable de type sauvage est mutée pour coder pour la valine, la thréonine, l'isoleucine ou la leucine.

4. Molécule d'ADN selon l'une quelconque des revendications précédentes, dans laquelle le polypeptide marqueur sélectionnable fournit une résistance contre les effets léthaux ou inhibiteurs de la croissance d'un agent de sélection, par exemple d'un antibiotique.

5. Molécule d'ADN selon la revendication 4, dans laquelle ledit agent de sélection est choisi parmi le groupe constitué de : la zéocine, la puromycine, la blasticidine, l'hygromycine, la néomycine, le méthotrexate, la méthionine sulphoximine et la kanamycine.

6. Molécule d'ADN selon la revendication 5, dans laquelle l'agent de sélection est la zéocine.

7. Molécule d'ADN selon la revendication 5, dans laquelle le polypeptide marqueur sélectionnable est codé par le gène de résistance à la néomycine.

8. Molécule d'ADN selon l'une quelconque des revendications précédentes, dans laquelle le polypeptide marqueur sélectionnable comprend en outre une mutation qui réduit l'activité du polypeptide marqueur sélectionnable comparé à sa contrepartie de type sauvage, par exemple dans laquelle le polypeptide marqueur sélectionnable est un polypeptide de résistance à la zéocine dans lequel la proline à la position 9 est changée en un acide aminé différent.

9. Molécule d'ADN selon l'une quelconque des revendications précédentes, dans laquelle la séquence codante du polypeptide d'intérêt comprend une séquence d'initiation optimale de la traduction.

10. Cassette d'expression comprenant une molécule d'ADN selon l'une quelconque des revendications précédentes, ladite cassette d'expression comprenant en outre un promoteur en amont de ladite unité d'expression multicistronique et une séquence de terminaison de la transcription en aval de l'unité d'expression multicistronique.

11. Cassette d'expression selon la revendication 10, comprenant en outre au moins un élément de contrôle de la chromatine, choisi parmi le groupe constitué d'une région de fixation à une matrice ou échafaudage (MAR/SAR), d'une séquence isolante, d'un élément ubiquiste d'ouverture de la chromatine (UCOE), et d'une séquence anti-répresseur (STAR).

12. Cassette d'expression selon la revendication 11, dans laquelle ledit élément de contrôle de la chromatine est une séquence anti-répresseur choisie parmi le groupe constitué :
a) de n'importe laquelle parmi SEQ ID n°:1 jusqu'à SEQ ID n°:66 ;
b) des fragments de l'une quelconque parmi SEQ ID n°:1 jusqu'à SEQ ID n°:66, dans laquelle lesdits fragments ont une activité anti-répresseur ;
c) de séquences qui sont au moins à 70 % identiques en séquence nucléotidique à a) ou b) lesdites séquences ayant une activité anti-répresseur ; et
d) du complément de l'un quelconque parmi a) à c).

13. Cassette d'expression selon la revendication 12, dans laquelle ladite cassette d'expression comprend l'un(e) parmi :
a) SEQ ID n°:66,
b) un fragment de a) ayant une activité anti-répresseur ;
c) une séquence qui est au moins à 70 % identique en séquence nucléotidique à a) ou b)
dans laquelle ladite séquence a une activité anti-répresseur,
dans laquelle a), b) ou c) est positionné(e) en amont du promoteur qui dirige la transcription du gène multicistronique.

14. Cassette d'expression selon l'une quelconque des revendications 10 à 13, dans laquelle ledit gène multicistronique est flanqué sur les deux côtés d'au moins une séquence anti-répresseur.

15. Cassette d'expression selon la revendication 14, comprenant : 5'-séquence anti-répresseur A - séquence anti-répresseur B - promoteur - gène multicistronique codant pour la protéine marqueur sélectionnable fonctionnelle et en aval de celle-ci le polypeptide d'intérêt - séquence de terminaison de la transcription - séquence anti-répresseur C-3',
dans laquelle les séquences anti-répresseurs A et C peuvent être identiques ou différentes et sont choisies parmi le groupe constitué :
a) de n'importe laquelle parmi SEQ ID n°:1 jusqu'à SEQ ID n°:65 ;
b) des fragments de l'une quelconque des séquences de a), lesdits fragments ayant une activité anti-répresseur ;
c) de séquences qui sont au moins à 70 % identiques en séquence nucléotidique à a) ou b)
dans lesquelles lesdites séquences ont une activité anti-répresseur,
et dans lesquelles la séquence anti-répresseur B est choisie parmi le groupe constitué de :
i) SEQ ID n°:66,
ii) un fragment de i) ayant une activité anti-répresseur ; et
iii) une séquence qui est au moins à 70 % identique en séquence nucléotidique à i) ou ii) dans laquelle ladite séquence a une activité anti-répresseur.

16. Cassette d'expression selon la revendication 15, dans laquelle les séquences anti-répresseurs A et C sont choisies parmi :
a) n'importe laquelle parmi SEQ ID n°:7, SEQ ID n°:9, SEQ ID n°:17, SEQ ID n°:27, SEQ ID n°:29, SEQ ID n°:43, SEQ ID n°:44, SEQ ID n°:45, SEQ ID n°:47, ou SEQ ID n°:61 ;
b) les fragments de l'une des séquences de a), lesdits fragments ayant une activité anti-répresseur ; et
c) les séquences qui sont au moins à 70 % identiques en séquence nucléotidique à a) ou b) lesdites séquences ayant une activité anti-répresseur.

17. Cassette d'expression selon l'une quelconque des revendications 10 à 16, le polypeptide d'intérêt faisant partie d'une protéine multimère.

18. Cassette d'expression selon la revendication 17, le polypeptide d'intérêt étant une chaîne légère d'immunoglobuline ou une chaîne lourde d'immunoglobuline.

19. Cassette d'expression selon l'une quelconque des revendications 10 à 18, comprenant en outre :
a) une séquence de pause de la transcription (TRAP) en amont dudit promoteur étant dans un sens 5' à 3'; ou
b) une séquence TRAP en aval de ladite séquence de terminaison de la transcription et étant dans un sens 3' à 5' ; ou
c) à la fois a) et b) ;
une séquence TRAP étant une séquence qui lorsqu'elle est placée dans une unité de transcription, résulte en un niveau réduit de la transcription dans l'acide nucléique présent sur le côté 3' de la TRAP lorsque comparé au niveau de transcription observé dans l'acide nucléique sur le côté 5' de la TRAP,
par exemple, ladite TRAP étant SEQ ID n°:142.

20. Molécule d'ADN comprenant une séquence codant pour un polypeptide marqueur sélectionnable fonctionnel capable d'être choisi dans une cellule hôte eucaryote **caractérisée en ce que** ladite molécule d'ADN :
i) a une séquence d'initiation de la traduction choisie parmi le groupe constitué de :
a) une séquence d'initiation de la traduction comprenant une mutation dans les nucléotides aux positions -3 à -1 et/ou +4 de la séquence consensus RCCATGG (dans laquelle R est A ou G, et dans laquelle la A du codon initiateur ATG est nt + 1) ;
b) un codon initiateur GTG ;
c) un codon initiateur TTG ;
d) un codon initiateur CTG ;
e) un codon initiateur ATT ; et
f) un codon initiateur ACG, suivi d'une séquence codante marqueur sélectionnable fonctionnelle, et
ii) **en ce que** le brin codant de la séquence définissant le polypeptide marqueur sélectionnable en aval du codon initiateur non optimal et en amont du codon de terminaison est dépourvu des séquences ATG.

21. Cellule hôte comprenant une molécule d'ADN ou une cassette d'expression selon l'une quelconque des revendications précédentes.

22. Cellule hôte selon la revendication 21, qui est une cellule hôte eucaryote, de préférence une cellule hôte de mammifère.

23. Cellule hôte selon la revendication 22, étant une cellule d'ovaire de hamster chinois (CHO).

24. Procédé de génération d'une cellule hôte exprimant un polypeptide d'intérêt, le procédé comprenant les étapes de :
a) introduction dans une pluralité de cellules précurseurs d'une molécule d'ADN selon l'une quelconque des revendications 1 à 9 ou d'une cassette d'expression selon l'une quelconque des revendications 10 à 19, et
b) culture des cellules générées sous des conditions sélectionnant l'expression du polypeptide marqueur sélectionnable, et
c) sélection d'au moins une cellule hôte produisant le polypeptide d'intérêt.

25. Procédé de production d'un polypeptide d'intérêt, comprenant la culture d'une cellule hôte, ladite cellule hôte comprenant une cassette d'expression selon l'une quelconque des revendications 10 à 19 et exprimant le polypeptide d'intérêt à partir de la cassette d'expression.

26. Procédé selon la revendication 25, comprenant en outre l'isolement du polypeptide d'intérêt.


### SEQUENCE LISTING

<110> ChromaGenics B.V.
   Otte, Arie P.
   Kwaks, Theodorus H.J.
   Sewalt, Richard G.A.B.
   van Blokland, Rik
<120> Selection of host cells expressing protein at high levels
<130> 0117 US P01 PRO
<160> 142
<170> PatentIn version 3.2
<210> 1
   <211> 749
   <212> DNA
   <213> Homo sapiens
<220>
   <221> misc_feature
   <223> sequence of STAR1
<400> 1
   atgcggtggg ggcgcgccag agactcgtgg gatccttggc ttggatgttt ggatctttct      60
   gagttgcctg tgccgcgaaa gacaggtaca tttctgatta ggcctgtgaa gcctcctgga     120
   ggaccatctc attaagacga tggtattgga gggagagtca cagaaagaac tgtggcccct     180
   ccctcactgc aaaacggaag tgattttatt ttaatgggag ttggaatatg tgagggctgc     240
   aggaaccagt ctccctcctt cttggttgga aaagctgggg ctggcctcag agacaggttt     300
   tttggccccg ctgggctggg cagtctagtc gaccctttgt agactgtgca cacccctaga     360
   agagcaacta cccctataca ccaggctggc tcaagtgaaa ggggctctgg gctccagtct     420
   ggaaaatctg gtgtcctggg gacctctggt cttgcttctc tcctcccctg cactggctct     480
   gggtgcttat ctctgcagaa gcttctcgct agcaaaccca cattcagcgc cctgtagctg     540
   aacacagcac aaaaagccct agagatcaaa agcattagta tgggcagttg agcgggaggt     600
   gaatatttaa cgcttttgtt catcaataac tcgttggctt tgacctgtct gaacaagtcg     660
   agcaataagg tgaaatgcag gtcacagcgt ctaacaaata tgaaaatgtg tatattcacc     720
   ccggtctcca gccggcgcgc caggctccc                                       749
<210> 2
   <211> 883
   <212> DNA
   <213> Homo sapiens
<220>
   <221> misc_feature
   <223> sequence of STAR2
<400> 2
   gggtgcttcc tgaattcttc cctgagaagg atggtggccg gtaaggtccg tgtaggtggg      60
   gtgcggctcc ccaggccccg gcccgtggtg gtggccgctg cccagcggcc cggcaccccc     120
   atagtccatg gcgcccgagg cagcgtgggg gaggtgagtt agaccaaaga gggctggccc     180
   ggagttgctc atgggctcca catagctgcc ccccacgaag acggggcttc cctgtatgtg     240
   tggggtccca tagctgccgt tgccctgcag gccatgagcg tgcgggtcat agtcgggggt     300
   gccccctgcg cccgcccctg ccgccgtgta gcgcttctgt gggggtggcg ggggtgcgca     360
   gctgggcagg gacgcagggt aggaggcggg gggcagcccg taggtaccct gggggggctt     420
   ggagaagggc gggggcgact ggggctcata cgggacgctg ttgaccagcg aatgcataga     480
   gttcagatag ccaccggctc cggggggcac ggggctgcga cttggagact ggccccccga     540
   tgacgttagc atgcccttgc ccttctgatc ctttttgtac ttcatgcggc gattctggaa     600
   ccagatcttg atctggcgct cagtgaggtt cagcagattg gccatctcca cccggcgcgg     660
   ccggcacagg tagcggttga agtggaactc tttctccagc tccaccagct gcgcgctcgt     720
   gtaggccgtg cgcgcgcgct tggacgaagc ctgccccggc gggctcttgt cgccagcgca     780
   gctttcgcct gcgaggacag agagaggaag agcggcgtca ggggctgccg cggccccgcc     840
   cagcccctga cccagcccgg cccctccttc caccaggccc caa                       883
<210> 3
   <211> 2126
   <212> DNA
   <213> Homo sapiens
<220>
   <221> misc_feature
   <223> sequence of STAR3
<400> 3
   atctcgagta ctgaaatagg agtaaatctg aagagcaaat aagatgagcc agaaaaccat     60
   gaaaagaaca gggactacca gttgattcca caaggacatt cccaaggtga gaaggccata    120
   tacctccact acctgaacca attctctgta tgcagattta gcaaggttat aaggtagcaa    180
   aagattagac ccaagaaaat agagaacttc caatccagta aaaatcatag caaatttatt    240
   gatgataaca attgtctcca aaggaacaag gcagagtcgt gctagcagag gaagcacgtg    300
   agctgaaaac agccaaatct gctttgtttt catgacacag gagcataaag tacacaccac    360
   caactgacct attaaggctg tggtaaaccg attcatagag agaggttcta aatacattgg    420
   tccctcacag gcaaactgca gttcgctccg aacgtagtcc ctggaaattt gatgtccagt    480
   atagaaaagc agagcagtca aaaaatatag ataaagctga accagatgtt gcctgggcaa    540
   tgttagcagc accacactta agatataacc tcaggctgtg gactccctcc ctggggagcg    600
   gtgctgccgg cggcgggcgg gctccgcaac tccccggctc tctcgcccgc cctcccgttc    660
   tcctcgggcg gcggcggggg ccgggactgc gccgctcaca gcggcggctc ttctgcgccc    720
   ggcctcggag gcagtggcgg tggcggccat ggcctcctgc gttcgccgat gtcagcattt    780
   cgaactgagg gtcatctcct tgggactggt tagacagtgg gtgcagccca cggagggcga    840
   gttgaagcag ggtggggtgt cacctccccc aggaagtcca gtgggtcagg gaactccctc    900
   ccctagccaa gggaggccgt gagggactgt gcccggtgag agactgtgcc ctgaggaaag    960
   gtgcactctg gcccagatac tacacttttc ccacggtctt caaaacccgc agaccaggag 1020
   attccctcgg gttcctacac caccaggacc ctgggtttca accacaaaac cgggccattt 1080
   gggcagacac ccagctagct gcaagagttg tttttttttt tatactcctg tggcacctgg 1140
   aacgccagcg agagagcacc tttcactccc ctggaaaggg ggctgaaggc agggaccttt 1200
   agctgcgggc tagggggttt ggggttgagt gggggagggg agagggaaaa ggcctcgtca 1260
   ttggcgtcgt ctgcagccaa taaggctacg ctcctctgct gcgagtagac ccaatccttt 1320
   cctagaggtg gagggggcgg gtaggtggaa gtagaggtgg cgcggtatct aggagagaga 1380
   aaaagggctg gaccaatagg tgcccggaag aggcggaccc agcggtctgt tgattggtat 1440
   tggcagtgga ccctcccccg gggtggtgcc ggaggggggg atgatgggtc gaggggtgtg 1500
   tttatgtgga agcgagatga ccggcaggaa cctgccccaa tgggctgcag agtggttagt 1560
   gagtgggtga cagacagacc cgtaggccaa cgggtggcct taagtgtctt tggtctcctc 1620
   caatggagca gcggcggggc gggaccgcga ctcgggttta atgagactcc attgggctgt 1680
   aatcagtgtc atgtcggatt catgtcaacg acaacaacag ggggacacaa aatggcggcg 1740
   gcttagtcct acccctggcg gcggcggcag cggtggcgga ggcgacggca ctcctccagg 1800
   cggcagccgc agtttctcag gcagcggcag cgcccccggc aggcgcggtg gcggtggcgc 1860
   gcagccaggt ctgtcaccca ccccgcgcgt tcccaggggg aggagactgg gcgggagggg 1920
   ggaacagacg gggggggatt caggggcttg cgacgcccct cccacaggcc tctgcgcgag 1980
   ggtcaccgcg gggccgctcg gggtcaggct gcccctgagc gtgacggtag ggggcggggg 2040
   aaaggggagg agggacaggc cccgcccctc ggcagggcct ctagggcaag ggggcggggc 2100
   tcgaggagcg gaggggggcg gggcgg 2126
<210> 4
   <211> 1625
   <212> DNA
   <213> Homo sapiens
<220>
   <221> misc_feature
   <223> sequence of STAR4
<400> 4
   gatctgagtc atgttttaag gggaggattc ttttggctgc tgagttgaga ttaggttgag 60
   ggtagtgaag gtaaaggcag tgagaccacg taggggtcat tgcagtaatc caggctggag 120
   atgatggtgg ttcagttgga atagcagtgc atgtgctgta acaacctcag ctgggaagca 180
   gtatatgtgg cgttatgacc tcagctggaa cagcaatgca tgtggtggtg taatgacccc 240
   agctgggtag ggtgcatgtg gtgtaacgac ctcagctggg tagcagtgtg tgtgatgtaa 300
   caacctcagc tgggtagcag tgtacttgat aaaatgttgg catactctag atttgttatg 360
   agggtagtgc cattaaattt ctccacaaat tggttgtcac gtatgagtga aaagaggaag 420
   tgatggaaga cttcagtgct tttggcctga ataaatagaa gacgtcattt ccagttaatg 480
   gagacaggga agactaaagg tagggtggga ttcagtagag caggtgttca gttttgaata 540
   tgatgaactc tgagagagga aaaacttttt ctacctctta gtttttgtga ctggacttaa 600
   gaattaaagt gacataagac agagtaacaa gacaaaaata tgcgaggtta tttaatattt 660
   ttacttgcag aggggaatct tcaaaagaaa aatgaagacc caaagaagcc attagggtca 720
   aaagctcata tgccttttta agtagaaaat gataaatttt aacaatgtga gaagacaaag 780
   gtgtttgagc tgagggcaat aaattgtggg acagtgatta agaaatatat gggggaaatg 840
   aaatgataag ttattttagt agatttattc ttcatatcta ttttggcttc aacttccagt 900
   ctctagtgat aagaatgttc ttctcttcct ggtacagaga gagcaccttt ctcatgggaa 960
   attttatgac cttgctgtaa gtagaaaggg gaagatcgat ctcctgtttc ccagcatcag 1020
   gatgcaaaca tttccctcca ttccagttct caaccccatg gctgggcctc atggcattcc 1080
   agcatcgcta tgagtgcacc tttcctgcag gctgcctcgg gtagctggtg cactgctagg 1140
   tcagtctatg tgaccaggag ctgggcctct gggcaatgcc agttggcagc ccccatccct 1200
   ccactgctgg gggcctccta tccagaaggg cttggtgtgc agaacgatgg tgcaccatca 1260
   tcattcccca cttgccatct ttcaggggac agccagctgc tttgggcgcg gcaaaaaaca 1320
   cccaactcac tcctcttcag gggcctctgg tctgatgcca ccacaggaca tccttgagtg 1380
   ctgggcagtc tgaggacagg gaaggagtga tgaccacaaa acaggaatgg cagcagcagt 1440
   gacaggagga agtcaaaggc ttgtgtgtcc tggccctgct gagggctggc gagggccctg 1500
   ggatggcgct cagtgcctgg tcggctgcaa gaggccagcc ctctgcccat gaggggagct 1560
   ggcagtgacc aagctgcact gccctggtgg tgcatttcct gccccactct ttccttctaa 1620
   gatcc 1625
<210> 5
   <211> 1571
   <212> DNA
   <213> Homo sapiens
<220>
   <221> misc_feature
   <223> sequence of STAR5
<400> 5
   cacctgattt aaatgatctg tctggtgagc tcactgggtc tttactcgca tgctgggtcc      60
   acagctccac tgtcctgcag ggtccgtgag tgtgggcccc ttatctattt catcatcata     120
   accctgcgtg tcctcaactc ctggcacata ttgggtggcc ccatccacac acggttgttg     180
   agtgaatcca tgagatgaca aaggctatga tgtagactat atcatgagcc agaaccaggc     240
   tttcctacct ccagacaatc aagggccttg atttgggatt gagggagaaa ggagtagaag     300
   ccaggaagga gaagagattg aggtttacca agggtgcaaa gtcctggccc ctgactgtag     360
   gctgaaaact atagaaatga tagaacaatt ttgcaatgaa atgcagaaga ccctgcatca     420
   actttaggtg ggacttcggg tatttttatg gccacagaac atcctcccat ttacctgcat     480
   ggcccagaca cagacttcaa aacagttgag gccagcaggc tccaggtaag tggtaggatt     540
   ccagaatgcc ctcagagtgt tgtgggaggc agcaggcgat tttcctggac ttctgagttt     600
   atgagaaccc caaaccccaa ttggcattaa cattgaggtc tcaatgtatc atggcaggaa     660
   gcttccgagt ggtgaaaagg aaagtgaaca tcaaagctcg gaagacaaga gggtggagtg     720
   atggcaacca agagcaagac ccttccctct cctgtgatgg ggtggctcta tgtgaagccc     780
   ccaaactgga cacaggtctg gcagaatgag gaacccactg agatttagcg ccaacatcca     840
   gcataaaagg gagactgaca tagaatttga gttagttaaa aataaggcac aatgcttttc     900
   atgtattcct gagttttgtg gactggtgtt caatttgcag cattcttagt tgattaaatc     960
   tgagatgaag aaagagtgtc caacactttc accttggaaa gctctggaaa agcaaaaggg    1020
   agagacaatt agcttcatcc attaactcac ttagtcatta tgcattcatt catgtaacta    1080
   ccaaacacgt actgagtgcc taacactcct gagacactga gaagtttctt gggaatacaa    1140
   agatgaataa aaaccacgcc aggcaggagt tggaggaagg ttctggatgc caccacgctc    1200
   tacctcctgg ctggacacca ggcaatgttg gtaaccttct gcctccaatt tctgcaaata    1260
   cataattaat aaacacaagg ttatcttcta aacagttctt aaaatgagtc aactttgttt    1320
   aaacttgttc tttttagaga aaaatgtatt tttgaaagag ttggttagtg ctaggggaaa    1380
   tgtctgggca cagctcagtc tggtgtgaga gcaggaagca gctctgtgtg tctggggtgg    1440
   gtacgtatgt aggacctgtg ggagaccagg ttgggggaag gcccctcctc atcaagggct    1500
   cctttgcttt ggtttgcttt ggcgtgggag gtgctgtgcc acaagggaat acgggaaata    1560
   agatctctgc t                                                          1571
<210> 6
   <211> 1173
   <212> DNA
   <213> Homo sapiens
<220>
   <221> misc_feature
   <223> sequence of STAR6
<400> 6
   tgacccacca cagacatccc ctctggcctc ctgagtggtt tcttcagcac agcttccaga     60
   gccaaattaa acgttcactc tatgtctata gacaaaaagg gttttgacta aactctgtgt    120
   tttagagagg gagttaaatg ctgttaactt tttaggggtg ggcgagaggg atgacaaata    180
   acaacttgtc tgaatgtttt acatttctcc ccactgcctc aagaaggttc acaacgaggt    240
   catccatgat aaggagtaag acctcccagc cggactgtcc ctcggccccc agaggacact    300
   ccacagagat atgctaactg gacttggaga ctggctcaca ctccagagaa aagcatggag    360
   cacgagcgca cagagcaggg ccaaggtccc agggacagaa tgtctaggag ggagattggg    420
   gtgagggtaa tctgatgcaa ttactgtggc agctcaacat tcaagggagg gggaagaaag    480
   aaacagtccc tgtcaagtaa gttgtgcagc agagatggta agctccaaaa tttgaaactt    540
   tggctgctgg aaagttttag ggggcagaga taagaagaca taagagactt tgagggttta    600
   ctacacacta gacgctctat gcatttattt atttattatc tcttatttat tactttgtat    660
   aactcttata ataatcttat gaaaacggaa accctcatat acccatttta cagatgagaa    720
   aagtgacaat tttgagagca tagctaagaa tagctagtaa gtaaaggagc tgggacctaa    780
   accaaaccct atctcaccag agtacacact cttttttttt ttccagtgta atttttttta    840
   atttttattt tactttaagt tctgggatac atgtgcagaa ggtatggttt gttacatagg    900
   tatatgtgtg ccatagtgga ttgctgcacc tatcaacccg tcatctaggt ttaagcccca    960
   catgcattag ctatttgtcc tgatgctctc cctcccctcc ccacaccaga caggccttgg   1020
   tgtgtgatgt tcccctccct gtgtccatgt gttctcactg ttcagctccc acttatgagt   1080
   gagaacgtgt ggtatttggt tttctgttcc tgtgttagtt tgctgaggat gatggcttcc   1140
   agcttcatcc atgtccctgc aaaggacacg atc                                 1173
<210> 7
   <211> 2101
   <212> DNA
   <213> Homo sapiens
<220>
   <221> misc_feature
   <223> sequence of STAR7
<400> 7
   aggtgggtgg atcacccgag gtcaggagtt caagaccagc ctggccaaca tggtaaaacc    60
   tcgtctctac taaaaaatac gaaaaattag ctggttgtgg tggtgcgtgc ttgtaatccc   120
   agctactcgg gaggctgagg caggagaatc acttgaatct gggaggcaga ggttgcagtg   180
   agctgagata gtgccattgc actccagcct gggcaacaga cggagactct gtctccaaaa   240
   aaaaaaaaaa aaatcttaga ggacaagaat ggctctctca aacttttgaa gaaagaataa   300
   ataaattatg cagttctaga agaagtaatg gggatatagg tgcagctcat gatgaggaag   360
   acttagctta actttcataa tgcatctgtc tggcctaaga cgtggtgagc tttttatgtc   420
   tgaaaacatt ccaatataga atgataataa taatcacttc tgacccccct tttttttcct   480
   ctccctagac tgtgaagcag aaaccccata tttttcttag ggaagtggct acgcactttg   540
   tatttatatt aacaactacc ttatcaggaa attcatattg ttgccctttt atggatgggg   600
   aaactggaca agtgacagag caaaatccaa acacagctgg ggatttccct cttttagatg   660
   atgattttaa aagaatgctg ccagagagat tcttgcagtg ttggaggaca tatatgacct   720
   ttaagatatt ttccagctca gagatgctat gaatgtatcc tgagtgcatg gatggacctc   780
   agttttgcag attctgtagc ttatacaatt tggtggtttt ctttagaaga aaataacaca   840
   tttataaata ttaaaatagg cccaagacct tacaagggca ttcatacaaa tgagaggctc   900
   tgaagtttga gtttgttcac tttctagtta attatctcct gcctgtttgt cataaatgcg   960
   tttagtaggg agctgctaat gacaggttcc tccaacagag tgtggaagaa ggagatgaca  1020
   gctggcttcc cctctgggac agcctcagag ctagtgggga aactatgtta gcagagtgat  1080
   gcagtgacca agaaaatagc actaggagaa agctggtcca tgagcagctg gtgagaaaag  1140
   gggtggtaat catgtatgcc ctttcctgtt ttatttttta ttgggtttcc ttttgcctct 1200
   caattccttc tgacaataca aaatgttggt tggaacatgg agcacctgga agtctggttc 1260
   attttctctc agtctcttga tgttctctcg ggttcactgc ctattgttct cagttctaca 1320
   cttgagcaat ctcctcaata gctaaagctt ccacaatgca gattttgtga tgacaaattc 1380
   agcatcaccc agcagaactt aggttttttt ctgtcctccg tttcctgacc tttttcttct 1440
   gagtgcttta tgtcacctcg tgaaccatcc tttccttagt catctaccta gcagtcctga 1500
   ttcttttgac ttgtctccct acaccacaat aaatcactaa ttactatgga ttcaatccct 1560
   aaaatttgca caaacttgca aatagattac gggttgaaac ttagagattt caaacttgag 1620
   aaaaaagttt aaatcaagaa aaatgacctt taccttgaga gtagaggcaa tgtcatttcc 1680
   aggaataatt ataataatat tgtgtttaat atttgtatgt aacatttgaa taccttcaat 1740
   gttcttattt gtgttatttt aatctcttga tgttactaac tcatttggta gggaagaaaa 1800
   catgctaaaa taggcatgag tgtcttatta aatgtgacaa gtgaatagat ggcagaaggt 1860
   ggattcatat tcagttttcc atcaccctgg aaatcatgcg gagatgattt ctgcttgcaa 1920
   ataaaactaa cccaatgagg ggaacagctg ttcttaggtg aaaacaaaac aaacacgcca 1980
   aaaaccttta ttctctttat tatgaatcaa atttttcctc tcagataatt gttttattta 2040
   tttattttta ttattattgt tattatgtcc agtctcactc tgtcgcctaa gctggcatga 2100
   t 2101
<210> 8
   <211> 1821
   <212> DNA
   <213> Homo sapiens
<220>
   <221> misc_feature
   <223> sequence of STAR8
<400> 8
   gagatcacct cgaagagagt ctaacgtccg taggaacgct ctcgggttca caaggattga 60
   ccgaacccca ggatacgtcg ctctccatct gaggcttgct ccaaatggcc ctccactatt 120
   ccaggcacgt gggtgtctcc cctaactctc cctgctctcc tgagcccatg ctgcctatca 180
   cccatcggtg caggtccttt ctgaagagct cgggtggatt ctctccatcc cacttccttt 240
   cccaagaaag aagccaccgt tccaagacac ccaatgggac attccccttc cacctccttc 300
   tccaaagttg cccaggtgtt catcacaggt tagggagaga agcccccagg tttcagttac 360
   aaggcatagg acgctggcat gaacacacac acacacacac acacacacac acacacacac 420
   acacgactcg aagaggtagc cacaagggtc attaaacact tgacgactgt tttccaaaaa 480
   cgtggatgca gttcatccac gccaaagcca agggtgcaaa gcaaacacgg aatggtggag 540
   agattccaga ggctcaccaa accctctcag gaatattttc ctgaccctgg gggcagaggt 600
   tggaaacatt gaggacattt cttgggacac acggagaagc tgaccgacca ggcattttcc 660
   tttccactgc aaatgaccta tggcgggggc atttcacttt cccctgcaaa tcacctatgg    720
   cgaggtacct ccccaagccc ccacccccac ttccgcgaat cggcatggct cggcctctat    780
   ccgggtgtca ctccaggtag gcttctcaac gctctcggct caaagaagga caatcacagg 840
   tccaagccca aagcccacac ctcttccttt tgttataccc acagaagtta gagaaaacgc 900
   cacactttga gacaaattaa gagtccttta tttaagccgg cggccaaaga gatggctaac 960
   gctcaaaatt ctctgggccc cgaggaaggg gcttgactaa cttctatacc ttggtttagg 1020
   aaggggaggg gaactcaaat gcggtaattc tacagaagta aaaacatgca ggaatcaaaa 1080
   gaagcaaatg gttatagaga gataaacagt tttaaaaggc aaatggttac aaaaggcaac 1140
   ggtaccaggt gcggggctct aaatccttca tgacacttag atataggtgc tatgctggac 1200
   acgaactcaa ggctttatgt tgttatctct tcgagaaaaa tcctgggaac ttcatgcact 1260
   gtttgtgcca gtatcttatc agttgattgg gctcccttga aatgctgagt atctgcttac 1320
   acaggtcaac tccttgcgga agggggttgg gtaaggagcc cttcgtgtct cgtaaattaa 1380
   ggggtcgatt ggagtttgtc cagcattccc agctacagag agccttattt acatgagaag 1440
   caaggctagg tgattaaaga gaccaacagg gaagattcaa agtagcgact tagagtaaaa 1500
   acaaggttag gcatttcact ttcccagaga acgcgcaaac attcaatggg agagaggtcc   1560
   cgagtcgtca aagtcccaga tgtggcgagc ccccgggagg aaaaaccgtg tcttccttag   1620
   gatgcccgga acaagagcta ggcttccgga gctaggcagc catctatgtc cgtgagccgg 1680
   cgggagggag accgccggga ggcgaagtgg ggcggggcca tccttctttc tgctctgctg 1740
   ctgccgggga gctcctggct ggcgtccaag cggcaggagg ccgccgtcct gcagggcgcc 1800
   gtagagtttg cggtgcagag t 1821
<210> 9
   <211> 1929
   <212> DNA
   <213> Homo sapiens
<220>
   <221> misc_feature
   <223> sequence of STAR9
<400> 9
   cacttcctgg gagtggagca gaggctctgc gtggagcatc catgtgcagt actcttaggt     60
   acggaaggga ttgggctaaa ccatggatgg gagctgggaa gggaagggac caacttcagg    120
   ccccactggg acactggagc tgccaccctt tagagccctc ctaaccctac accagaggct    180
   gagggggacc tcagacatca cacacatgct ttcccatgtt ttcagaaatc tggaaacgta    240
   gaacttcagg ggtgagagtg cctagatatt gaatacaagg ctagattggg cttctgtaat    300
   atcccaaagg accctccagc tttttcacca gcacctaatg cccatcagat accaaagaca    360
   cagcttagga gaggttcacc ctgaagctga ggaggaggca gccggattag agttgactga    420
   gcaaggatga ctgccttctc cacctgacga tttcagctgc tgcccttttc ttttcctggg    480
   aatgcctgtc gccatggcct tctgtgtcca caggagagtt tgacccagat actcatggac    540
   caggcaaagg tgctgttcct cccagcccag ggcccaccat gaagcatgcc tgggagcctg 600
   gtaaggaccc agccactcct gggctgttga cattggcttc tcttgcccag cattgtagcc 660
   acgccactgc attgtactgt gagataagtc aaggtgggct caccaggacc tgcactaaat 720
   tgtgaaattc agctccaaag aactttggaa attacccatg catttaagca aaatgaatga 780
   tacctgagca aaccctttca cattggcaca agttacaatc ctgtctcatc ctcttgatta 840
   caaattccat ccaggcaaga gctgtatcac cctgaggtct ccccattcat gttttggtca 900
   ataatattta gtttcctttt gaaaatagat ttttgtgtta ctccattatg atgggcagag 960
   gccagatgct tatattctat ttaaatgact atgtttttct atctgtaact gggtttgtgt 1020
   tcaggtggta aatgcttttt ttttgcagtc agaagattcc tggaaggcga ccagaaatta 1080
   gctggccgct gtcagacctg aagttacttc taaagggcct ttagaaatga attctttttt 1140
   atgccttctc tgaattctga gaagtaggct tgacttcccc taagtgtgga gttgggagtc 1200
   aactcttctg aaaagaaagt ttcagagcat tttccaaagc catggtcagc tgtgggaagg 1260
   gaagacgatg gatagtacag ttgccggaaa acactgatgg aggcggatgc tccagctcag 1320
   ccaaagacct ttgttctgcc caccccagaa atgccccttc ctcaatcgca gaaacgttgc 1380
   cccatggctc ctgatactca gaatgcagcc tctgaccagg accatctgca tcctccagga 1440
   gctcgtaaga aatgcagcat cgtgggacct gctggcacct ggtgaaccca aacctgcagg 1500
   gctcctgggt gtgcttgggg cggctgcagg ggaagaggga gtcagcagcc tcctcctgac 1560
   cttcccgggg gctgcttttc tgagggccca gaatgcaccg gttgaccttg ttgcatcact 1620
   ggcccatgac tggctgcttt ggtcaggtgt aaaaaggtgt ttccagaggg tctgctcctc 1680
   tcactatcgg accaggtttc catggagagc tcagcctccc agcaaggata gagaacttca 1740
   aatggctcaa agaactgaga ggccacacat gtgtgacctg aatagtctct gctgcaaaac 1800
   aaagggtttc ttaatgtaaa acgttctctt cctcacagag gggttcccag ctgctagtgg 1860
   gcatgttgca ggcatttcct gggctgcatc aggttgtcat aagccagagg atcatttttg 1920
   ggggctcat 1929
<210> 10
   <211> 1167
   <212> DNA
   <213> Homo sapiens
<220>
   <221> misc_feature
   <223> sequence of STAR10
<220>
   <221> misc_feature
   <222> (452)..(1143)
   <223> n is a, c, g, or t on various positions
<400> 10
   aggtcaggag ttcaagacca gcctggccaa catggtgaaa ccctgtccct acaaaaaata     60
   caaaaattag ccgggcgtgg tggggggcgc ctataatccc agctactcag gatgctgaga    120
   caggagaatt gtttgaaccc gggaggtgga ggttgcagtg aactgagatc gcgccactgc    180
   actccagcct ggtgacagag agagactccg tctcaacaac agacaaacaa acaaacaaac    240
   aacaacaaaa atgtttactg acagctttat tgagataaaa ttcacatgcc ataaaggtca    300
   ccttctacag tatacaattc agtggattta gtatgttcac aaagttgtac gttgttcacc     360
   atctactcca gaacatttac atcaccccta aaagaagctc tttagcagtc acttctcatt     420
   ctccccagcc cctgccaacc acgaatctac tntctgtctc tattctgaat atttcatata    480
   aaggagtcct atcatatggg ccttttacgt ctaccttctt tcacttagca tcatgttttt    540
   aagattcatc cacagtgtag cacgtgtcag ttaattcatt tcatcttatg gctggataat    600
   gctctattgt atgcatatcc ctcactttgc ttatccattc atcaactgat tgacatttgg    660
   gttatttcta ctttttgact attatgagta atgctgctat gaacattcct gtaccaatcg    720
   ttacgtggac atatgctttc aattctcctg agtatgtaac tagggttgga gttgctgggt    780
   catatgttaa ctcagtgttt catttttttg aagaactacc aaatggtttt ccaaagtgga    840
   tgcaacactt tacattccca ccagcaagat atgaaggttc caatgtctct acatttttgc    900
   caacacttgt gattttcttt tatttattta tttatttatt tatttttgag atggagtctc    960
   actctgtcac ccaggctgga gtgcagtggc acaatttcag ctcactgcaa tctccacctc   1020
   tcgggctcaa gcgatactcc tgcctcaacc tcccgagtaa ctgggattac aggcgcccac   1080
   caccacacca agctaatttt ttgtattttt agtagagacg gggtttcatc atgtcggcca    1140
   ggntgtactc gaactctgac ctcaagt                                       1167
<210> 11
   <211> 1377
   <212> DNA
   <213> Homo sapiens
<220>
   <221> misc_feature
   <223> sequence of STAR11
<400> 11
   aggatcactt gagcccagga gttcaagacc agcctgggca acatagcgag aacatgtctc      60
   aaaaaggaaa aaaatggggg aaaaaaccct cccagggaca gatatccaca gccagtcttg     120
   ataagctcca tcattttaaa gtgcaaggcg gtgcctccca tgtggatgat tatttaatcc     180
   tcttgtactt tgtttagtcc tttgtggaaa tgcccatctt ataaattaat agaattctag     240
   aatctaatta aaatggttca actctacatt ttactttagg ataatatcag gaccatcaca     300
   gaatgtctga gatgtggatt taccctatct gtagctcact tcttcaacca ttcttttagc     360
   aaggctagtt atcttcagtg acaacccctt gctgccctct actatctcct ccctcagatg     420
   gactactctg attaagcttg agctagaata agcatgttat cccgggattt catatggaat     480
   attttataca tgagtgagcc attatgagtt gtttgaaaat ttattatgtt gagggagggt     540
   aaccgctgta acaaccatca ccaaatctaa tcgactgaat acatttgacg tttatttctt     600
   gttcacctga cagttcagtg ttacctaaat ttacatgaag acccagaggc ccacgctcct     660
   tcattttggg ctccaccgac ctccaaggtt tcagggccct ctgccccgcc ttctgcaccc     720
   acaggggaag agagtggagg atgcacacgc ccaggcctgg aagtgacgca tgtggcttcc     780
   ccgtccacag acttcaccca cagtccattg gccttcttaa gtcatggact cctgctgagc     840
   tgccagggtg catgggaaat ccatgtgact gtgtgccctg gaggaagggg agcgtttcgg     900
   tgagcacaca ggagtctttg ccactagacg ctgatgagga ttccccacag gcgatgaagc     960
   atggagactc atcttgtaac aaacagatga gttgttgaca tctcttaagt ttactttgtg    1020
   tgcagttttt attcagatag gaaaggctgt taaaatctta acacctaact ggaagaaggg    1080
   ttttagagaa gtgtggtttt cagtaagcca gttctttcca caatccaaga aacgaaataa    1140
   atttccagca tggagcagtt ggcaggtaag gtttttgttg tggtctcgcc caggcttgag    1200
   tgtaaccggt gtggtcatag ctcactacat tctcaaactc ctggccttaa gtcatcctcc    1260
   tgcctcagcc tcccaaaggc aagtaaggtt aagaataggg gaaaggtgaa gtttcacagc    1320
   ttttctagaa ttctttttat tcaaggaact ctcagatcat caaacccacc cagaatc       1377
<210> 12
   <211> 1051
   <212> DNA
   <213> Homo sapiens
<220>
   <221> misc_feature
   <223> sequence of STAR12
<400> 12
   atcctgcttc tgggaagaga gtggcctccc ttgtgcaggt gactttggca ggaccagcag      60
   aaacccaggt ttcctgtcag gaggaagtgc tcagcttatc tctgtgaagg gtcgtgataa     120
   ggcacgagga ggcaggggct tgccaggatg ttgcctttct gtgccatatg ggacatctca     180
   gcttacgttg ttaagaaata tttggcaaga agatgcacac agaatttctg taacgaatag     240
   gatggagttt taagggttac tacgaaaaaa agaaaactac tggagaagag ggaagccaaa     300
   caccaccaag tttgaaatcg attttattgg acgaatgtct cactttaaat ttaaatggag     360
   tccaacttcc ttttctcacc cagacgtcga gaaggtggca ttcaaaatgt ttacacttgt     420
   ttcatctgcc tttttgctaa gtcctggtcc cctacctcct ttccctcact tcacatttgt     480
   cgtttcatcg cacacatatg ctcatcttta tatttacata tatataattt ttatatatgg     540
   cttgtgaaat atgccagacg agggatgaaa tagtcctgaa aacagctgga aaattatgca     600
   acagtgggga gattgggcac atgtacattc tgtactgcaa agttgcacaa cagaccaagt     660
   ttgttataag tgaggctggg tggtttttat tttttctcta ggacaacagc ttgcctggtg     720
   gagtaggcct cctgcagaag gcattttctt aggagcctca acttccccaa gaagaggaga     780
   gggcgagact ggagttgtgc tggcagcaca gagacaaggg ggcacggcag gactgcagcc     840
   tgcagagggg ctggagaagc ggaggctggc acccagtggc cagcgaggcc caggtccaag     900
   tccagcgagg tcgaggtcta gagtacagca aggccaaggt ccaaggtcag tgagtctaag     960
   gtccatggtc agtgaggctg agacccaggg tccaatgagg ccaaggtcca gagtccagta    1020
   aggccgagat ccagggtcca gggaggtcaa g                                   1051
<210> 13
   <211> 1291
   <212> DNA
   <213> Homo sapiens
<220>
   <221> misc_feature
   <223> sequence of STAR13
<400> 13
   agccactgag gtcctaactg cagccaaggg gccgttctgc acatgtcgct caccctctgt     60
   gctctgttcc ccacagagca aacgcacatg gcaacgttgg tccgctcagc cactggttct    120
   gtggtggaac ggtggatgtc tgcactgtga catcagctga gtaagtaaca acgactgagg    180
   atgccgctga cccagggctg gggaagggga ctcccagctc agacaggctt ggctgtggtt    240
   tgctttggga ggagagtgaa catcacaggg aatggctcat gtcagcccca ggagggtggg    300
   ctggcccctg gtccccgggc tccttctggc cctgcaggcg atagagagcc tcaacctgct    360
   gccgcttctc cttggcccgg gtgatggccg tctggaagag cctgcagtag aggtgcacag    420
   ccagcggaga gtcgtcattg ccgggtacag ggtaggtgat gaggcagggg ttgcagttgg    480
   tgtccacgat gcccactgtg gggatgttca tcttggctgc gtctctcacg gccacgtgtg    540
   gctcaaagat gttgttgagc gtgtgcagga agatgatgag gtccggcagg cggaccgtgg    600
   ggccaaagag gaggcgcgcg ttggtcagca tgccgcccct gaagtagcga gtgtgggcgt    660
   actcgccaca gtcacgggcc atgttctcaa tcaggtacga gaactgccgg ttgcggctta    720
   taaacaagat gatgcccttg cggtaggcca tgtgggcggt gaagttcaag gccagctgga    780
   ggtgcgtggc tgtctgttcc aggtcgatga tgtcgtggtc caggcggctc ccaaagatgt    840
   acggctccat aaacctgcca gagaccccac caaggcaagg gggatgagag ttcacggggc    900
   catctccact ggctccttgc aggaacacag acgcccacca gggactcccg ggctcctctg    960
   tgggggcact atgggctggg aagcacaatt tgcaacgctc cccgtgtgca tggacagcag   1020
   tgcagaccca tccaggccac ccctctgcat gcctcgtctc gtggcttaac ccctcctacc   1080
   ctctacctct tcccgaagga atcctaatag aactgacccc atatggatgt gtggacatcc   1140
   aacatgacgc caaaaggaca ttctgccccg tgcagctcac agggcagccg cctccgtcac   1200
   tgtcctcttc ccgaggcttt gcggatgagg cccctctggg gttggactta gcggggtgct   1260
   ctgggccaaa agcattaagg gatcagggca g                                  1291
<210> 14
   <211> 711
   <212> DNA
   <213> Homo sapiens
<220>
   <221> misc_feature
   <223> sequence of STAR14
<400> 14
   ccctggacca gggtccgtgg tcttggtggg cactggcttc ttcttgctgg gtgttttcct 60
   gtgggtctct ggcaaggcac tttttgtggc gctgcttgtg ctgtgtgcgg gaggggcagg 120
   tgctctttcc tcttggagct ggaccctctg gggcgggtcc ccgtcggcct ccttgtgtgt 180
   tttctgcacc tggtacagct ggatggcctc ctcaatgccg tcgtcgctgc tggagtcgga 240
   cgcctcgggc gcctgtacgg cgctcgtgac tcgctttccc ctccttgcgg tgctggcgtt 300
   ccttttaatc ccacttttat tctgtactgc ttctgaaggg cggtgggggt tgctggcttt 360
   gtgctgccct ccttctcctg cgtggtcgtg gtcgtgacct tggacctgag gcttctgggc 420
   tgcacgtttg tctttgctaa ccgggggagg tctgcagaag gcgaactcct tctggacgcc 480
   catcaggccc tgccggtgca ccacctttgt agccggctct tggtgggatt tcgagagtga 540
   cttcgccgaa ttttcatgtg tgtctggttt cttctccact gacccatcac atttttgggt 600
   ctcatgctgt cttttctcat tcagaaactg ttctatttct gccctgatgc tctgctcaaa 660
   ggagtctgct ctgctcatgc tgactgggga ggcagagccc tggtccttgc t 711
<210> 15
   <211> 1876
   <212> DNA
   <213> Homo sapiens
<220>
   <221> misc_feature
   <223> sequence of STAR15
<400> 15
   gagtccaaga tcaaggtgcc agcatcttgt gagggccttc ttgttacgtc actccctagc    60
   gaaagggcaa agagagggtg agcaagagaa agggggcctg aactcgtcct tgtagaagag   120
   gcccattccc gagacaatgg cattcatcca ttcactccac cctcatggcc tcaccacctc   180
   tcatgaggct ccacctccca gccctggttt gttggggatt aaatttccaa cacatgcctt   240
   ttgggggaca tgttaaaatt atagcacccc aaatgttaca ctatcttttg atgagcggta   300
   gttctgattt taagtctagc tggcctactt tttcttgcac gtgggatgct ttctgcctgt   360
   tccagggcag gcagctcttc tctgtccctc tgctggcccc acctcatcct ctgttgtcct   420
   cttccctcct tctgtgccct ggggtcctgg tgggggtgtg actgtcaact gcgttgggct   480
   aacttttttc cctgctggtg gcccgtaatg aaagaaagct tcttgctccc aagttcctta   540
   aatccaagct catagacaac gcggtctcac agcaggcctg gggccagcct cacgtgagcc   600
   ccttccctgg tgtagtcact ggcatggggg aatgggattt cctgttgccc tactgtgtgg   660
   ctgaggtggg ggttgcttcc tggagccagg ccttgtggaa gggcagtgcc cactgcagtg   720
   gatgctgggc cctgaatctg accccagtgt tcattggctc tgtgagaccc agtgagggca   780
   gggagggaag tggagctggg gtgagaagta gaggccctgc agggcccacg tgccagccac   840
   caggcctcag actaggctca gatgacggag agctgcacac ctgcccaacc caggccctgc   900
   agtgcccaca tgccagccgc tggggcccag acttgctcca gagggcggag agctttacac   960
   cggcccaacc caggccatgg ctccaaatgc gtgacagttt tgctgttgct tcttttagtc  1020
   attgtcaagt tgatgcttgt tttgcagagg accaaggctt tatgaaccta ttaccctgtg  1080
   tgaagagttt caccaggtta tggaaatttc tttaaaacca taccacagtt ttttcattat  1140
   tcatgtatat ttttaaaaat aattactgca ctcagtagaa taacatgaaa atgttgcctg  1200
   ttagcccttt tccagtttgc cccgagaata ctgggggcac ttgtggctgc aatgtttatc  1260
   ctgcggcagc tttgccatga agtatctcac ttttattatt atttttgcat tgctcgagta  1320
   tattgacttt ggaaacaaaa gacatcattc tatttatagc attatgtttt tagtagtggt  1380
   atttccatat acaagataca gtaattttcc gtcaatgaaa atgtcaaatt ctagaaaatg  1440
   taacattcct atgcgtggtg ttaacatcgt tctctaacag ttgttggccg aagattcgtt  1500
   tgatgaatcc gatttttcca aaatagccga ttctgatgat tcagacgatt ctgatgttct  1560
   gtttagaaat aattccaaga acagttttta cattttattt tcacattgaa aatcagtcag  1620
   atttgcttca gcctcaaaga gcacgtttat gtaaaattaa atgagtgctg gcagccagct  1680
   gcgctttgtt tttctaaatg ggaaaagggt taaatttcac tcagctttta aatgacagcg  1740
   cacagcctgt gtcatagagg gttggaggag atgactttaa ctgcctgtgg ttaggatccc  1800
   tttcccccag gaatgtctgg gagcccactg ccgggtttgc tgtccgtctc gtttggactc  1860
   agttctgcat gtactg                                                  1876
<210> 16
   <211> 1282
   <212> DNA
   <213> Homo sapiens
<220>
   <221> misc_feature
   <223> sequence of STAR16
<400> 16
   cgcccacctc ggctttccaa agtgctggga ttacaggcat gagtcactgc gcccatcctg     60
   attccaagtc tttagataat aacttaactt tttcgaccaa ttgccaatca ggcaatcttt    120
   gaatctgcct atgacctagg acatccctct ccctacaagt tgccccgcgt ttccagacca    180
   aaccaatgta catcttacat gtattgattg aagttttaca tctccctaaa acatataaaa    240
   ccaagctata gtctgaccac ctcaggcacg tgttctcagg acctccctgg ggctatggca    300
   tgggtcctgg tcctcagatt tggctcagaa taaatctctt caaatatttt ccagaatttt    360
   actcttttca tcaccattac ctatcaccca taagtcagag ttttccacaa ccccttcctc    420
   agattcagta atttgctaga atggccacca aactcaggaa agtattttac ttacaattac    480
   caatttatta tgaagaactc aaatcaggaa tagccaaatg gaagaggcat agggaaaggt    540
   atggaggaag gggcacaaag cttccatgcc ctgtgtgcac accaccctct cagcatcttc    600
   atgtgttcac caactcagaa gctcttcaaa ctttgtcatt taggggtttt tatggcagtt    660
   ccactatgta ggcatggttg ataaatcact ggtcatcggt gatagaactc tgtctccagc    720
   tcctctctct ctcctcccca gaagtcctga ggtggggctg aaagtttcac aaggttagtt    780
   gctctgacaa ccagccccta tcctgaagct attgaggggt cccccaaaag ttaccttagt    840
   atggttggaa gaggcttatt atgaataaca aaagatgctc ctatttttac cactagggag    900
   catatccaag tcttgcggga acaaagcatg ttactggtag caaattcata caggtagata    960
   gcaatctcaa ttcttgcctt ctcagaagaa agaatttgac caagggggca taaggcagag   1020
   tgagggacca agataagttt tagagcagga gtgaaagttt attaaaaagt tttaggcagg   1080
   aatgaaagaa agtaaagtac atttggaaga gggccaagtg ggcgacatga gagagtcaaa   1140
   caccatgccc tgtttgatgt ttggcttggg gtcttatatg atgacatgct tctgagggtt   1200
   gcatccttct cccctgattc ttcccttggg gtgggctgtc cgcatgcaca atggcctgcc   1260
   agcagtaggg aggggccgca tg                                            1282
<210> 17
   <211> 793
   <212> DNA
   <213> Homo sapiens
<220>
   <221> misc_feature
   <223> sequence of STAR17
<400> 17
   atccgagggg aggaggagaa gaggaaggcg agcagggcgc cggagcccga ggtgtctgcg     60
   agaactgttt taaatggttg gcttgaaaat gtcactagtg ctaagtggct tttcggattg    120
   tcttatttat tactttgtca ggtttcctta aggagagggt gtgttggggg tgggggagga    180
   ggtggactgg ggaaacctct gcgtttctcc tcctcggctg cacagggtga gtaggaaacg    240
   cctcgctgcc acttaacaat ccctctatta gtaaatctac gcggagactc tatgggaagc    300
   cgagaaccag tgtcttcttc cagggcagaa gtcacctgtt gggaacggcc cccgggtccc    360
   cctgctgggc tttccggctc ttctaggcgg cctgatttct cctcagccct ccacccagcg    420
   tccctcaggg acttttcaca cctccccacc cccatttcca ctacagtctc ccagggcaca    480
   gcacttcatt gacagccaca cgagccttct cgttctcttc tcctctgttc cttctctttc    540
   tcttctcctc tgttccttct ctttctctgt cataatttcc ttggtgcttt cgccacctta    600
   aacaaaaaag agaaaaaaat aaaataaaaa aaacccattc tgagccaaag tattttaaga    660
   tgaatccaag aaagcgaccc acatagccct ccccacccac ggagtgcgcc aagacgcacc    720
   caggctccat cacagggccg agagcagcgc cactctggtc gtacttttgg gtcaagagat    780
   cttgcaaaag agg                                                       793
<210> 18
   <211> 492
   <212> DNA
   <213> Homo sapiens
<220>
   <221> misc_feature
   <223> sequence of STAR18
<210> 19
   <211> 1840
   <212> DNA
   <213> Homo sapiens
<220>
   <221> misc_feature
   <223> sequence of STAR19
<400> 19
   tcacttcctg atattttaca ttcaaggcta gctttatgca tatgcaacct gtgcagttgc 60
   acagggcttt gtgttcagaa agactagctc ttggtttaat actctgttgt tgccatcttg 120
   agattcatta taatataatt tttgaatttg tgttttgaac gtgatgtcca atgggacaat 180
   ggaacattca cataacagag gagacaggtc aggtggcagc ctcaattcct tgccaccctt 240
   ttcacataca gcattggcaa tgccccatga gcacaaaatt tgggggaacc atgatgctaa 300
   gactcaaagc acatataaac atgttacctc tgtgactaaa agaagtggag gtgctgacag 360
   cccccagagg ccacagttta tgttcaaacc aaaacttgct tagggtgcag aaagaaggca 420
   atggcagggt ctaagaaaca gcccatcata tccttgttta ttcatgttac gtccctgcat 480
   gaactaatca cttacactga aaatattgac agaggaggaa atggaaagat agggcaaccc 540
   atagttcttt ttccttttag tctttcctta tcagtaaacc aaagatagta ttggtaaaat 600
   gtgtgtgagt taattaatga gttagtttta ggcagtgttt ccactgttgg ggtaagaaca 660
   aaatatatag gcttgtattg agctattaaa tgtaaattgt ggaatgtcag tgattccaag 720
   tatgaattaa atatccttgt atttgcattt aaaattggca ctgaacaaca aagattaaca 780
   gtaaaattaa taatgtaaaa gtttaatttt tacttagaat gacattaaat agcaaataaa 840
   agcaccatga taaatcaaga gagagactgt ggaaagaagg aaaacgtttt tattttagta 900
   tatttaatgg gactttcttc ctgatgtttt gttttgtttt gagagagagg gatgtggggg 960
   cagggaggtc tcattttgtt gcccaggctg gacttgaact cctgggctcc agctatcctg 1020
   ccttagcttc ttgagtagct gggactacag gcacacacca cagtgtctga cattttctgg 1080
   attttttttt tttttttatt ttttttgtga gacaggttct ggctctgtta ctcaggttgc 1140
   agtgcagtgg catgatagcg gctcactgca gcctcaacct cctcagctta agctactctc 1200
   ccacttcagc ctcctgagta gccaggacta cagttgtgtg ccaccacacc tgtggctaat 1260
   ttttgtagag atggggtctc tccacgttgc cgaggctggt ctccaactcc tggtctcaag 1320
   cgaacctcct gacttggcct cccgaagtgc tgggattaca ggcttgagcc actgcatcca 1380
   gcctgtcctc tgtgttaaac ctactccaat ttgtctttca tctctacata aacggctctt 1440
   ttcaaagttc ccatagacct cactgttgct aatctaataa taaattatct gccttttctt 1500
   acatggttca tcagtagcag cattagattg ggctgctcaa ttcttcttgg tatattttct 1560
   tcatttggct tctggggcat cacactctct ttgagttact cattcctcat tgatagcttc 1620
   ttcctagtct tctttactgg ttcttcctct tctccctgac tccttaatat tgtttttctc 1680
   cccaggcttt agttcttagt cctcttctgt tatctattta cacccaattc tttcagagtc 1740
   tcatccagag tcatgaactt aaacctgttt ctgtgcagat aattcacatt attatatctc 1800
   cagcccagac tctcccgcaa actgcagact gatcctactg 1840
<210> 20
   <211> 780
   <212> DNA
   <213> Homo sapiens
<220>
   <221> misc_feature
   <223> sequence of STAR20
<400> 20
   gatctcaagt ttcaatatca tgttttggca aaacattcga tgctcccaca tccttaccta 60
   aagctaccag aaaggctttg ggaactgtca acagagctac agaaaagtca gtaaagacca 120
   atggacccct caaacaaaaa cagccaagct tttctgccaa aaagatgact gagaagactg 180
   ttaaagcaaa aaactctgtt cctgcctcag atgatggcta tccagaaata gaaaaattat 240
   ttcccttcaa tcctctaggc ttcgagagtt ttgacctgcc tgaagagcac cagattgcac 300
   atctcccctt gagtgaagtg cctctcatga tacttgatga ggagagagag cttgaaaagc 360
   tgtttcagct gggcccccct tcacctttga agatgccctc tccaccatgg aaatccaatc 420
   tgttgcagtc tcctttaagc attctgttga ccctggatgt tgaattgcca cctgtttgct 480
   ctgacataga tatttaaatt tcttagtgct ttagagtttg tgtatatttc tattaataaa 540
   gcattatttg tttaacagaa aaaaagatat atacttaaat cctaaaataa aataaccatt 600
   aaaaggaaaa acaggagtta taactaataa gggaacaaag gacataaaat gggataataa 660
   tgcttaatcc aaaataaagc agaaaatgaa gaaaaatgaa atgaagaaca gataaataga 720
   aaacaaatag caatatgaaa gacaaacttg accgggtgtg gtggctgatg cctgtaatcc 780
<210> 21
   <211> 607
   <212> DNA
   <213> Homo sapiens
<220>
   <221> misc_feature
   <223> sequence of STAR21
<400> 21
   gatcaataat ttgtaatagt cagtgaatac aaaggggtat atactaaatg ctacagaaat      60
   tccattcctg ggtataaatc ctagacatat ttatgcatat gtacaccaag atatatctgc     120
   aagaatgttc acagcaaatc tctttgtagt agcaaaaggc caaaaggtct atcaacaaga     180
   aaattaatac attgtggcac ataatggcat ccttatgcca ataaaaatgg atgaaattat     240
   agttaggttc aaaaggcaag cctccagata atttatatca tataattcca tgtacaacat     300
   tcaacaacaa gcaaaactaa acatatacaa atgtcaggga aaatgatgaa caaggttaga     360
   aaatgattaa tataaaaata ctgcacagtg ataacattta atgagaaaaa aagaaggaag     420
   ggcttaggga gggacctaca gggaactcca aagttcatgg taagtactaa atacataatc     480
   aaagcactca aaatagaaaa tattttagta atgttttagc tagttaatat cttacttaaa     540
   acaaggtcta ggccaggcac ggtggctcac acctgtaatc ccagcacttt gggaggctga     600
   ggcgggt                                                               607
<210> 22
   <211> 1380
   <212> DNA
   <213> Homo sapiens
<220>
   <221> misc_feature
   <223> sequence of STAR22
<400> 22
   cccttgtgat ccacccgcct tggcctccca aagtgctggg attacaggcg tgagtcacta 60
   cgcccggcca ccctccctgt atattatttc taagtatact attatgttaa aaaaagttta 120
   aaaatattga tttaatgaat tcccagaaac taggatttta catgtcacgt tttcttatta 180
   taaaaataaa aatcaacaat aaatatatgg taaaagtaaa aagaaaaaca aaaacaaaaa 240
   gtgaaaaaaa taaacaacac tcctgtcaaa aaacaacagt tgtgataaaa cttaagtgcc 300
   tgaaaattta gaaacatcct tctaaagaag ttctgaataa aataaggaat aaaataatca 360
   catagttttg gtcattggtt ctgtttatgt gatggattat gtttattgat ttgtgtatgt 420
   tgaacttatc tcaatagatg cagacaaggc cttgataaaa gtttttaaca ccttttcatg 480
   ttgaaaactc tcaatagact aggtattgat gaaacatatc tcaaaataat agaagctatt 540
   tatgataaac ccatagccaa tatcatactg agtgggcaaa agctggaagc attccctttg 600
   aaaactggca caagacaagg atgccctctc tcaccactcc tattaaatgt agtattggaa 660
   gttctggcca gagcaatcag gcaggagaaa gaaaaggtat taaaatagga agagaggaag 720
   tcaaattgtc tctgtttgca gtaaacatga ttgtatattt agaaaacccc attgtctcat 780
   cctaaaaact ccttaagctg ataaacaact tcagcaaagt ctcaggatac aaaatcaatg 840
   tgcaaaaatc acaagcattc ctatacaccg ataatagaca gcagagagcc aaatcatgag 900
   tgaagtccca ttcacaattg cttcaaagaa aataaaatac ttaggaatac aactttcacg 960
   ggacatgaag gacattttca aggacaacta aaaaccactg ctcaaggaaa tgagagagga 1020
   cacaaagaaa tggaaaaaca ttccatgctc atggaagaat caatatcatg aaaatggcca 1080
   tactgcccaa agtaatttat agattcaatg ctaaccccat caagccacca ttgactttct 1140
   tcacagaact agaaaaaaac tattttaaaa ctcatatgta gtcaaaaaga gtcggtatag 1200
   ccaagacaat cctaagcata aagaacaaag ctggatgcat cacgctgact tcaaaccata 1260
   ctacaaggct acagtaacca aaacagcatg gtactggtac caaaacagat agatagaccg 1320
   atagaacaga acagaggcct cggaaataac accacacatc tacaaccctt tgatcttcaa 1380
<210> 23
   <211> 1246
   <212> DNA
   <213> Homo sapiens
<220>
   <221> misc_feature
   <223> sequence of STAR23
<400> 23
   atcccctcat ccttcagggc agctgagcag ggcctcgagc agctggggga gcctcactta      60
   atgctcctgg gagggcagcc agggagcatg gggtctgcag gcatggtcca gggtcctgca     120
   ggcggcacgc accatgtgca gccgccccca cctgttgctc tgcctccgcc acctggccat     180
   gggcttcagc agccagccac aaagtctgca gctgctgtac atggacaaga agcccacaag     240
   cagctagagg accttgtgtt ccacgtgccc agggagcatg gcccacagcc caaagaccag     300
   tcaggagcag gcaggggctt ctggcaggcc cagctctacc tctgtcttca cacagatggg     360
   agatttctgt tgtgattttg agtgatgtgc ccctttggtg acatccaaga tagttgctga     420
   agcaccgctc taacaatgtg tgtgtattct gaaaacgaga acttctttat tctgaaataa     480
   ttgatgcaaa ataaattagt ttggatttga aattctattc atgtaggcat gcacacaaaa     540
   gtccaacatt gcatatgaca caaagaaaag aaaaagcttg cattccttaa atacaaatat     600
   ctgttaacta tatttgcaaa tatatttgaa tacacttcta ttatgttaca tataatatta     660
   tatgtatatg tatatataat atacatatat atgttacata taatatactt ctattatgtt     720
   acatataata tttatctata agtaaataca taaatataaa gatttgagta gctgtagaac     780
   attgtcttat gtgttatcag ctactactac aaaaatatct cttccactta tgccagtttg     840
   ccatataaat atgatcttct cattgatggc ccagggcaag agtgcagtgg gtacttattc     900
   tctgtgagga gggaggagaa aagggaacaa ggagaaagtc acaaagggaa aactctggtg     960
   ttgccaaaat gtcaagtttc acatattccg agacggaaaa tgacatgtcc cacagaagga    1020
   ccctgcccag ctaatgtgtc acagatatct caggaagctt aaatgatttt tttaaaagaa    1080
   aagagatggc attgtcactt gtttcttgta gctgaggctg tgggatgatg cagatttctg    1140
   gaaggcaaag agctcctgct ttttccacac cgagggactt tcaggaatga ggccagggtg    1200
   ctgagcacta caccaggaaa tccctggaga gtgtttttct tactta                   1246
<210> 24
   <211> 939
   <212> DNA
   <213> Homo sapiens
<220>
   <221> misc_feature
   <223> sequence of STAR24
<400> 24
   acgaggtcac gagttcgaga ccagcctggc caagatggtg aagccctgtc tctactaaaa 60
   atacaacaag tagccgggcg cggtgacggg cgcctgtaat cccagctact caggaggctg 120
   aagcaggaga atctctagaa cccaggaggc ggaggtgcag tgagctgaga ctgccccgct 180
   gcactctagc ctgggcaaca cagcaagact ctgtctcaaa taaataaata aataaataaa 240
   taaataaata aataaataaa tagaaaggga gagttggaag tagatgaaag agaagaaaag 300
   aaatcctaga tttcctatct gaaggcacca tgaagatgaa ggccacctct tctgggccag 360
   gtcctcccgt tgcaggtgaa ccgagttctg gcctccattg gagaccaaag gagatgactt 420
   tggcctggct cctagtgagg aagccatgcc tagtcctgtt ctgtttgggc ttgatcctgt 480
   atcacttgat tgtctctcct ggactttcca tggattccag ggatgcaact gagaagttta 540
   tttttaatgc acttacttga agtaagagtt attttaaaac attttagcaa aggaaatgaa 600
   ttctgacagg ttttgcactg aagacattca catgtgagga aaacaggaaa accactatgc 660
   tagaaaaagc aaatgctgtt gagattgtct cacaaacaca aattgcgtgc cagcaggtag 720
   gtttgagcct caggttgggc acattttacc ttaagcgcac tgttggtgga acttaaggtg 780
   actgtaggac ttatatatac atacatacat ataatatata tacatattta tgtgtatata 840
   cacacacaca cacacacaca cacacagggt cttgctatct tgcccagggt ggtctccaac 900
   tctgggtctc aagcgatcct ctgcctcccc ttcccaaag 939
<210> 25
   <211> 1067
   <212> DNA
   <213> Homo sapiens
<220>
   <221> misc_feature
   <223> sequence of STAR25
<400> 25
   cagcccctct tgtgtttttc tttatttctc gtacacacac gcagttttaa gggtgatgtg 60
   tgtataatta aaaggaccct tggcccatac tttcctaatt ctttagggac tgggattggg 120
   tttgactgaa atatgttttg gtggggatgg gacggtggac ttccattctc cctaaactgg 180
   agttttggtc ggtaatcaaa actaaaagaa acctctggga gactggaaac ctgattggag 240
   cactgaggaa caagggaatg aaaaggcaga ctctctgaac gtttgatgaa atggactctt 300
   gtgaaaatta acagtgaata ttcactgttg cactgtacga agtctctgaa atgtaattaa 360
   aagtttttat tgagcccccg agctttggct tgcgcgtatt tttccggtcg cggacatccc 420
   accgcgcaga gcctcgcctc cccgctgccc tcagcctccg atgacttccc cgcccccgcc    480
   ctgctcggtg acagacgttc tactgcttcc aatcggaggc acccttcgcg ggagcggcca    540
   atcgggagct ccggcaggcg gggaggccgg gccagttaga tttggaggtt caacttcaac    600
   atggccgaag caagtagcgc caatctaggc agcggctgtg aggaaaaaag gcatgagggg    660
   tcgtcttcgg aatctgtgcc acccggcact accatttcga gggtgaagct cctcgacacc    720
   atggtggaca cttttcttca gaagctggtc gccgccggca ggtaaagtgg acgcagccgc    780
   ggtgggagtg tttgttggca ccgaagctca aatcccgcga ggtcaggacg gccgcaggct    840
   ggcgcgcggt gacgtgggtc cgcgttgggg gcggggcagt cggacgaggc gacccagtca    900
   aatcctgagc cttaggagtc agggtattca cgcactgata acctgtagcg gaccgggata    960
   gctagctact ccttcctaca ggaagccccg ttttcactaa aatttcaggt ggttgggagg   1020
   aaagatagag cctttgcaaa ttagagcagg gttttttatt tttttat                1067
<210> 26
   <211> 540
   <212> DNA
   <213> Homo sapiens
<220>
   <221> misc_feature
   <223> sequence of STAR26
<400> 26
   ccccctgaca agccccagtg tgtgatgttc cccactctgt gtccatgcat tctcattgtt     60
   caactcccat ctgtgagtga gaacatgcag tgtttggttt tctgtccttg agatagtttg    120
   ctgagaatga tggtttccag cttcatccat gtccttgcaa aggaagtgaa cttatccttt    180
   tttatggctt catagtattc catggcacat atgtgccaca tttttttaat ccagtctatc    240
   attgatggac atttgggttg gttccaagtc tttgctattg tgaatagcac cacaattaac    300
   atatgtgtgc atgtatacat ctttatagta gcatgattta taatccttcg ggtatatacc    360
   ctgtaatggg atcgctgggt caaatggtat ttctagttct agatccttga ggaatcacca    420
   cactgctttc cacaatggtt gaactaattt acgctcccac cagcagtgta aaagcattcc    480
   tatttctcca cgtcctctcc agtatctgtt gtttcctgac tttttaatga tcatcattct    540
<210> 27
   <211> 1520
   <212> DNA
   <213> Homo sapiens
<220>
   <221> misc_feature
   <223> sequence of STAR27
<400> 27
   cttggccctc acaaagcctg tggccaggga acaattagcg agctgcttat tttgctttgt     60
   atccccaatg ctgggcataa tgcctgccat tatgagtaat gccggtagaa gtatgtgttc    120
   aaggaccaaa gttgataaat accaaagaat ccagagaagg gagagaacat tgagtagagg    180
   atagtgacag aagagatggg aacttctgac aagagttgtg aagatgtact aggcaggggg    240
   aacagcttaa ggagagtcac acaggaccga gctcttgtca agccggctgc catggaggct    300
   gggtggggcc atggtagctt tcccttcctt ctcaggttca gagtgtcagc cttgaacttc    360
   taattcccag aggcatttat tcaatgtttt cttctagggg catacctgcc ctgctgtgga    420
   agactttctt ccctgtgggt cgccccagtc cccagatgag acggtttggg tcagggccag    480
   gtgcaccgtt gggtgtgtgc ttatgtctga tgacagttag ttactcagtc attagtcatt    540
   gagggaggtg tggtaaagat ggagatgctg ggtcacatcc ctagagaggt gttccagtat    600
   gggcacatgg gagggctgga aggataggtt actgctagac gtagagaagc cacatccttt    660
   aacaccctgg cttttcccac tgccaagatc cagaaagtcc ttgtggtttc gctgctttct    720
   cctttttttt tttttttttt tttctgagat ggagtctggc tctgtcgccc aggctggagt    780
   gcagtggcac gatttcggct cactgcaagt tccgcctcct aggttcatac cattctccca    840
   cctcagcctc ccgagtagct gggactacag gcgccaccac acccagctaa ttttttgtat     900
   ttttagtaga gacggcgttt caccatgtta gccaggatgg tcttgatccg cctgcctcag    960
   cctcccaaag tgctgggatt acaggcgtga gccaccgcgc ccggcctgct ttcttctttc   1020
   atgaagcatt cagctggtga aaaagctcag ccaggctggt ctggaactct tgacctcaag   1080
   tgatctgcct gcctcagcct cccaaagtgc tgagattaca ggcatgagcc agtccgaatg   1140
   tggctttttt tgttttgttt tgaaacaagg tctcactgtt gcccaggctg cagtgcagtg   1200
   gcatacctca gctccactgc agcctcgacc tcctgggctc aagcaatcct cccaactgag   1260
   cctccccagt agctggggct acaagcgcat gccaccacgc ctggctattt tttttttttt   1320
   tttttttttt gagaaggagt ttcattcttg ttgcccaggc tggagtgcaa tggcacagtc   1380
   tcagctcact gcagcctccg cctcctgggt tcaagcgatt ctcctgcctc agcctcccga   1440
   gtagctggga ttataggcac ctgccaccat gcctggctaa tttttttgta tttttagtag    1500
   ggatggggtt tcaccatgtt                                               1520
<210> 28
   <211> 961
   <212> DNA
   <213> Homo sapiens
<220>
   <221> misc_feature
   <223> sequence of STAR28
<400> 28
   aggaggttat tcctgagcaa atggccagcc tagtgaactg gataaatgcc catgtaagat     60
   ctgtttaccc tgagaagggc atttcctaac tctccctata aaatgccaag tggagcaccc    120
   cagatgaaat agctgatatg ctttctatac aagccatcta ggactggctt tatcatgacc    180
   aggatattca cccactgaat atggctatta cccaagttat ggtaaatgct gtagttaagg    240
   gggtcccttc cacatggaca ccccaggtta taaccagaaa gggttcccaa tctagactcc    300
   aagagagggt tcttagacct catgcaagaa agaacttggg gcaagtacat aaagtgaaag    360
   caagtttatt aagaaagtaa agaaacaaaa aaatggctac tccataagca aagttatttc    420
   tcacttatat gattaataag agatggatta ttcatgagtt ttctgggaaa ggggtgggca    480
   attcctggaa ctgagggttc ctcccacttt tagaccatat agggtatctt cctgatattg    540
   ccatggcatt tgtaaactgt catggcactg atgggagtgt cttttagcat tctaatgcat    600
   tataattagc atataatgag cagtgaggat gaccagaggt cacttctgtt gccatattgg    660
   tttcagtggg gtttggttgg cttttttttt tttttaacca caacctgttt tttatttatt    720
   tatttattta tttatttatt tatatttttt attttttttt agatggagtc ttgctctgtc    780
   acccaggtta gagtgcagtg gcaccatctc ggctcactgc aagctctgcc tccttggttc    840
   acgccattct gctgcctcag cctcccgagt agctgggact acaggtgcct gccaccatac    900
   ccggctaatt ttttctattt ttcagtagag acggggtttc accgtgttag ccaggatggt    960
   c                                                                    961
<210> 29
   <211> 2233
   <212> DNA
   <213> Homo sapiens
<220>
   <221> misc_feature
   <223> sequence of STAR29
<400> 29
   agcttggaca cttgctgatg ccactttgga tgttgaaggg ccgccctctc ccacaccgct     60
   ggccactttt aaatatgtcc cctctgccca gaagggcccc agaggagggg ctggtgaggg    120
   tgacaggagt tgactgctct cacagcaggg ggttccggag ggaccttttc tccccattgg    180
   gcagcataga aggacctaga agggccccct ccaagcccag ctgggcgtgc agggccagcg    240
   attcgatgcc ttcccctgac tcaggtggcg ctgtcctaaa ggtgtgtgtg ttttctgttc    300
   gccagggggt ggcggataca gtggagcatc gtgcccgaag tgtctgagcc cgtggtaagt    360
   ccctggaggg tgcacggtct cctccgactg tctccatcac gtcaggcctc acagcctgta    420
   ggcaccgctc ggggaagcct ctggatgagg ccatgtggtc atccccctgg agtcctggcc    480
   tggcctgaag aggaggggag gaggaggcca gcccctccct agccccaagg cctgcgaggc    540
   tgcaagcccg gccccacatt ctagtccagg cttggctgtg caagaagcag attgcctggc    600
   cctggccagg cttcccagct aggatgtggt atggcagggg tgggggacat tgaggggctg    660
   ctgtagcccc cacaacctcc ccaggtaggg tggtgaacag taggctggac aagtggacct    720
   gttcccatct gagattcaag agcccacctc tcggaggttg cagtgagccg agatccctcc    780
   actgcactcc agcctgggca acagagcaag actctgtctc aaaaaaacag aacaacgaca    840
   acaaaaaacc cacctctggc ccactgccta actttgtaaa taaagtttta ttggcacata    900
   gacacaccca ttcatttaca tactgctgcg gctgcttttg cattaccctt gagtagacga    960
   cagaccacgt ggccatggaa gccaaaaata tttactgtct ggccctttac agaagtctgc   1020
   tctagaggga gaccccggcc catggggcag gaccactggg cgtgggcaga agggaggcct 1080
   cggtgcctcc acgggcctag ttgggtatct cagtgcctgt ttcttgcatg gagcaccagg 1140
   ggtcagggca agtacctgga ggaggcaggc tgttgcccgc ccagcactgg gacccaggag 1200
   accttgagag gctcttaacg aatgggagac aagcaggacc agggctccca ttggctgggc 1260
   ctcagtttcc ctgcctgtaa gtgagggagg gcagctgtga aggtgaactg tgaggcagag 1320
   cctctgctca gccattgcag gggcggctct gccccactcc tgttgtgcac ccagagtgag 1380
   gggcacgggg tgagatgtca ccatcagccc ataggggtgt cctcctggtg ccaggtcccc 1440
   aagggatgtc ccatcccccc tggctgtgtg gggacagcag agtccctggg gctgggaggg 1500
   ctccacactg ttttgtcagt ggtttttctg aactgttaaa tttcagtgga aaattctctt 1560
   tcccctttta ctgaaggaac ctccaaagga agacctgact gtgtctgaga agttccagct 1620
   ggtgctggac gtcgcccaga aagcccaggt actgccacgg gcgccggcca ggggtgtgtc 1680
   tgcgccagcc atgggcacca gccaggggtg tgtctacgcc ggccaggggt aggtctccgc 1740
   cggcctccgc tgctgcctgg ggagggccgt gcctgacact gcaggcccgg tttgtccgcg 1800
   gtcagctgac ttgtagtcac cctgcccttg gatggtcgtt acagcaactc tggtggttgg 1860
   ggaaggggcc tcctgattca gcctctgcgg acggtgcgcg agggtggagc tcccctccct 1920
   ccccaccgcc cctggccagg gttgaacgcc cctgggaagg actcaggccc gggtctgctg 1980
   ttgctgtgag cgtggccacc tctgccctag accagagctg ggccttcccc ggcctaggag 2040
   cagccgggca ggaccacagg gctccgagtg acctcagggc tgcccgacct ggaggccctc 2100
   ctggcgtcgc ggtgtgactg acagcccagg agcgggggct gttgtaattg ctgtttctcc 2160
   ttcacacaga accttttcgg gaagatggct gacatcctgg agaagatcaa gaagtaagtc 2220
   ccgcccccca ccc 2233
<210> 30
   <211> 1851
   <212> DNA
   <213> Homo sapiens
<220>
   <221> misc_feature
   <223> sequence of STAR30
<400> 30
   gggtgcattt ccacccaggg gacacttggc aatggtggga gacattgctt gttgtcacaa      60
   ctgggcatgg gagtgctgct gcgtctagtg ggtagaggcc agagatgctc ctaatatcct     120
   acaaggcaca gaacagcccc ccacaacaga gaattatcca gcctgaaaat gtccacagtg     180
   ctgaggttgg gaaaccctat tctagagcca acaggctgtg aagcttgact catggttcca     240
   tcaccaatag ctgcgtgacc ttggtgagtt ccttagctgc tctgtgcctc ggattcatgg     300
   taggttttcc ttgttaggtt taaatgagtg aagttataca gagggcctga agtctcatgg     360
   tattttacta gagcctcatt gtgttttagt tataattaga aattgggtaa ggtaaggaca     420
   cagaagaagc catctgatct gggggcttca cacttagaag tgacctcgga gcaattgtat     480
   tggggtggaa agggactaac agccaggagc agagggcaca ttggaattgg ggccagaggg     540
   cacagactgc cttgtccatc aggcatagca atggacagag gaaggggaat gactagttat     600
   ggctgcaagg ccaagtacag gggacttatt tctcatatct atctatctat ctacctaccg     660
   tctatttatc tatcatctat ctacttattt atctatctat ttatgcatgt gtaccaaccg     720
   aaagttttag taaatgcaca aactgcgata taatgaaaat ggaaattttc aaaagaagag     780
   aaatcacctg ccacctgact accttaacaa atgagtggtt ttcatctctc cttccaggcc     840
   tgtcattttt acagtgcttt agtcataaaa caggtcctct attctattgt tttatgtcac     900
   atgaaattgt accataagca ttttccatga tgtgactcca ctgtttcatt ttccattttt     960
   ttccagaatg aagataacct cattgttttt ttcctgattg taaaaatgct ctgtgctctt    1020
   tttttttttt tttaacaatg caggcagtac caaaaagtat gaagaagaat gtaatagttc    1080
   ccatttccca tctcactctt taaggccagc attttggtga acatccatcc gaacaaatct    1140
   ccacgcgttt atcaatttgt tgacttactc cttcttttat gtaaatatga acatgattta    1200
   actgccagtc catttggaac cttaaagtga aggtttttta ttgttggggt ttgctatggt    1260
   ctgaatatgt gtgtcccccc aaaatttatg ttgaatccta acgcccaatg cgattaggag    1320
   gtggggccat taggaggtga ttaagtcatg aagtcatcag ccctaatgaa tgggatttgt    1380
   ggccttgaaa agggacccca gagagctgcc ttgccccttc tgccatgtaa ggacacagtg    1440
   aggagctagg aagggggcct cagcagagac caaatgtgat ggtgcctcga tattggactt    1500
   cccagcctcc agaatgtgag aaatgaattt ctgttgttta taagtcaccc agtctatagt    1560
   attttgttct agcagcccaa acagactaag tcagggttgt tgttttagga agtggggaat    1620
   ggggccatgc atgggtgtac gccagaacaa aggaagccag caagtcctga aagatactgg    1680
   aaaagggaat agtgggcacg tgcagtgtgt tagtttcctg aggctgctat aacaaagcac    1740
   cacaggttgg gtggcttaaa taacagaaat tcattctccc atcattctgg ggaccagacg    1800
   tctgaaatca agactcctat gccatgctcc ttctgaaggc tccaggggag g             1851
<210> 31
   <211> 1701
   <212> DNA
   <213> Homo sapiens
<220>
   <221> misc_feature
   <223> sequence of STAR31
<220>
   <221> misc_feature
   <222> (159)..(1696)
   <223> n is a, c, g, or t on various positions
<400> 31
   cacccgcctt ggccccccag agtgctggga ttacaagtgt aaaccaccat tcctggctag 60
   atttaatttt ttaaaaaata aagagaagta ggaatagttc attttaggga gagcccctta 120
   actgggacag gggcaggaca ggggtgaggc ttcccttant tcaagctcac ctcaaaccca    180
   cccaggactg tgtgtcacat tctccaataa aggaaaggtt gctgcccccg cctgtgagtg    240
   ctgcagtgga gggtagaggg ccgtgggcag agtgcttcat ggactgctca tcaagaaagg    300
   cttcatgaca atcggcccag ctgctgtcat cccacattct acttccagct aggagaaggc    360
   ggcttgccca cagtcaccca gccggcaagt gtcacccctg ggttggaccc agagctatga    420
   tcctgcccag gggtccagct gagaatcagg cccacgttct aggcagaggg gctcacctac    480
   tgggactcca gtagctgtag tgcatggagg catcatggct gcagcagcct ggacctggtc    540
   tcacactggc tgtccctgtg ggcaggccat cctcaatgcc aggtcaggcc caagcatgta    600
   tcccagacaa tgacaatggg gtggaatcct ctcttgtccc agaagccact cctcactgtt    660
   ctacctgagg aaggcagggg catggtggaa tcctgaagcc tgctgtgagg gtctccagcg    720
   aacttgcaca tggtcagccc tgccttctcc tccctgaact agattgagcg agagcaagaa    780
   ggacattgaa ccagcaccca aagaattttg gggaacggcc tctcatccag gtcaggctca    840
   cctccttttt aaaatttaat taattaatta attaattttt ttttagagac agagtcttac    900
   tgtgtggccc aggctgtagt gcagtggcac aatcatagtt cactgcagcc tcaaactccc    960
   cacctcagcc tctggattag ctgagactac aggtgcacca ccaccacacc cagctaatat   1020
   ttttattttt gtagagagag ggtttcacca tcttgcccag gctggtctca aactcctggg   1080
   ctcaagtgat cccgcccagg tctgaaagcc cccaggctgg cctcagactg tggggttttc   1140
   catgcagcca cccgagggcg cccccaagcc agttcatctc ggagtccagg cctggccctg   1200
   ggagacagag tgaaaccagt ggtttttatg aacttaactt agagtttaaa agatttctac   1260
   tcgatcactt gtcaagatgc gccctctctg gggagaaggg aacgtgactg gattccctca   1320
   ctgttgtatc ttgaataaac gctgctgctt catcctgtgg gggccgtggc cctgtccctg   1380
   tgtgggtggg gcctcttcca tttccctgac ttagaaacca cagtccacct agaacagggt   1440
   ttgagaggct tagtcagcac tgggtagcgt tttgactcca ttctcggctt tcttcttttt   1500
   ctttccagga tttttgtgca gaaatggttc ttttgttgcc gtgttagtcc tccttggaag   1560
   gcagctcaga aggcccgtga aatgtcgggg gacaggaccc ccagggaggg aaccccaggc   1620
   tacgcacttt agggttcgtt ctccagggag ggcgacctga cccccgnatc cgtcggngcg   1680
   cgnngnnacn aannnnttcc c                                             1701
<210> 32
   <211> 771
   <212> DNA
   <213> Homo sapiens
<220>
   <221> misc_feature
   <223> sequence of STAR32
<400> 32
   gatcacacag cttgtatgtg ggagctagga ttggaacccc agaagtctgg ccccaggttc      60
   atgctctcac ccactgcata caatggcctc tcataaatca atccagtata aaacattaga     120
   atctgcttta aaaccataga attagtagcg taagtaataa atgcagagac catgcagtga     180
   atggcattcc tggaaaaagc ccccagaagg aattttaaat cagctttcgt ctaatcttga     240
   gcagctagtt agcaaatatg agaatacagt tgttcccaga taatgcttta tgtctgacca     300
   tcttaaactg gcgctgtttt tcaaaaactt aaaaacaaaa tccatgactc ttttaattat     360
   aaaagtgata catgtctact tgggaggctg aggtggtggg aggatggctt gagtttgagg     420
   ctgcagtatg ctactatcat gcctataaat agccgctgca ttccagcttg ggcaacatac     480
   ccaggcccta tctcaaaaaa ataaaaagta atacatctac attgaagaaa attaatttta     540
   ttgggttttt ttgcattttt attatacaca gcacacacag cacatatgaa aaaatgggta     600
   tgaactcagg cattcaactg gaagaacagt actaaatcaa tgtccatgta gtcagcgtga     660
   ctgaggttgg tttgtttttt cttttttctt ctcttctctt ctcttttctt tttttttgag     720
   acggagcttt gctctttttg cccaggcttg attgcaatgg cgtgatctca g             771
<210> 33
   <211> 1368
   <212> DNA
   <213> Homo sapiens
<220>
   <221> misc_feature
   <223> sequence of STAR33
<400> 33
   gcttttatcc tccattcaca gctagcctgg cccccagagt acccaattct ccctaaaaaa      60
   cggtcatgct gtatagatgt gtgtggcttg gtagtgctaa agtggccaca tacagagctc     120
   tgacaccaaa cctcaggacc atgttcatgc cttctcactg agttctggct tgttcgtgac     180
   acattatgac attatgatta tgatgacttg tgagagcctc agtcttctat agcactttta     240
   gaatgcttta taaaaaccat ggggatgtca ttatattcta acctgttagc acttctgttc     300
   gtattaccca tcacatccca acatcaattc tcatatatgc aggtacctct tgtcacgcgc     360
   gtccatgtaa ggagaccaca aaacaggctt tgtttgagca acaaggtttt tatttcacct     420
   gggtgcaggt gggctgagtc tgaaaagaga gtcagtgaag ggagacaggg gtgggtccac     480
   tttataagat ttgggtaggt agtggaaaat tacaatcaaa gggggttgtt ctctggctgg     540
   ccagggtggg ggtcacaagg tgctcagtgg gagagccttt gagccaggat gagccagaag     600
   gaatttcaca aggtaatgtc atcagttaag gcagggactg gccattttca cttcttttgt     660
   ggtggaatgt catcagttaa ggcaggaacc ggccattttc acttcttttg tgattcttca     720
   cttgcttcag gccatctgga cgtataggtg caggtcacag tcacagggga taagatggca     780
   atggcatagc ttgggctcag aggcctgaca cctctgagaa actaaagatt ataaaaatga     840
   tggtcgcttc tattgcaaat ctgtgtttat tgtcaagagg cacttatttg tcaattaaga     900
   acccagtggt agaatcgaat gtccgaatgt aaaacaaaat acaaaacctc tgtgtgtgtg     960
   tgtgtgtgag tgtgtgtgta tgtgtgtgtg tgtgtattag agaggaaaag cctgtatttg    1020
   gaggtgtgat tcttagattc taggttcttt cctgcccacc ccatatgcac ccaccccaca    1080
   aaagaacaaa caacaaatcc caggacatct tagcgcaaca tttcagtttg catattttac    1140
   atatttactt ttcttacata ttaaaaaact gaaaatttta tgaacacgct aagttagatt    1200
   ttaaattaag tttgttttta cactgaaaat aatttaatat ttgtgaagaa tactaataca    1260
   ttggtatatt tcattttctt aaaattctga acccctcttc ccttatttcc ttttgacccg    1320
   attggtgtat tggtcatgtg actcatggat ttgccttaag gcaggagg                 1368
<210> 34
   <211> 755
   <212> DNA
   <213> Homo sapiens
<220>
   <221> misc_feature
   <223> sequence of STAR34
<400> 34
   actgggcacc ctcctaggca ggggaatgtg agaactgccg ctgctctggg gctgggcgcc 60
   atgtcacagc aggagggagg acggtgttac accacgtggg aaggactcag ggtggtcagc 120
   cacaaagctg ctggtgatga ccaggggctt gtgtcttcac tctgcagccc taacacccag 180
   gctgggttcg ctaggctcca tcctgggggt gcagaccctg agagtgatgc cagtgggagc     240
   ctcccgcccc tccccttcct cgaaggccca ggggtcaaac agtgtagact cagaggcctg     300
   agggcacatg tttatttagc agacaaggtg gggctccatc agcggggtgg cctggggagc 360
   agctgcatgg gtggcactgt ggggagggtc tcccagctcc ctcaatggtg ttcgggctgg 420
   tgcggcagct ggcggcaccc tggacagagg tggatatgag ggtgatgggt ggggaaatgg     480
   gaggcacccg agatggggac agcagaataa agacagcagc agtgctgggg ggcaggggga     540
   tgagcaaagg caggcccaag acccccagcc cactgcaccc tggcctccca caagccccct     600
   cgcagccgcc cagccacact cactgtgcac tcagccgtcg atacactggt ctgttaggga 660
   gaaagtccgt cagaacaggc agctgtgtgt gtgtgtgcgt gtatgagtgt gtgtgtgtga 720
   tccctgactg ccaggtcctc tgcactgccc ctggg 755
<210> 35
   <211> 1193
   <212> DNA
   <213> Homo sapiens
<220>
   <221> misc_feature
   <223> sequence of STAR35
<220>
   <221> misc_feature
   <222> (312) .. ( 1191)
   <223> n is a, c, g, or t on various positions
<400> 35
   cgacttggtg atgcgggctc ttttttggtt ccatatgaac tttaaagtag tcttttccaa 60
   ttctgtgaag aaagtcattg gtaggttgat ggggatggca ttgaatctgt aaattacctt 120
   gggcagtatg gccattttca caatgttgat tcttcctatc catgatgatg gaatgttctt 180
   ccattagttt gtatcctctt ttatttcctt gagcagtggt ttgtagttct ccttgaagag 240
   gtccttcaca tcccttgtaa gttggattcc taggtatttt attctctttg aagcaaattg 300
   tgaatgggag tncactcacg atttggctct ctgtttgtct gctgggtgta taaanaatgt 360
   ngtgatnttn gtacattgat ttngtatccn tgagacttng ctgaatttgc ttnatcngct 420
   tnngggaacc ttttgggctg aaacnatggg attttctaaa tatacaatca tgtcgtctgc 480
   aaacagggaa caatttgact tcctcttttc ctaattgaat acactttatc tccttctcct 540
   gcctaattgc cctgggcaaa acttccaaca ctatgntngn aataggagnt ggtgagagag 600
   ggcatccctg ttcttgttgc cagnttttca aagggaatgc ttccagtttt ggcccattca 660
   gtatgatatg ggctgtgggt ngtgtcataa atagctctta tnattttgaa atgtgtccca 720
   tcaataccta atttattgaa agtttttagc atgaangcat ngttgaattt ggtcaaaggc 780
   tttttctgca tctatggaaa taatcatgtg gtttttgtct ttggctcntg tttatatgct 840
   ggatnacatt tattgatttg tgtatatnga acccagcctn ncatcccagg gatgaagccc 900
   acttgatcca agcttggcgc gcngnctagc tcgaggcagg caaaagtatg caaagcatgc 960
   atctcaatta gtcagcaccc atagtccgcc cctacctccg cccatccgcc cctaactcng 1020
   nccgttcgcc cattctcgcc catggctgac taatnttttt annatccaag cggngccgcc 1080
   ctgcttganc attcagagtn nagagnnttg gaggccnagc cttgcaaaac tccggacngn 1140
   ttctnnggat tgaccccnnt taaatatttg gttttttgtn ttttcanngg nga 1193
<210> 36
   <211> 1712
   <212> DNA
   <213> Homo sapiens
<220>
   <221> misc_feature
   <223> sequence of STAR36
<400> 36
   gatcccatcc ttagcctcat cgatacctcc tgctcacctg tcagtgcctc tggagtgtgt      60
   gtctagccca ggcccatccc ctggaactca ggggactcag gactagtggg catgtacact     120
   tggcctcagg ggactcagga ttagtgagcc ccacatgtac acttggcctc agtggactca     180
   ggactagtga gccccacatg tacacttggc ctcaggggac tcaggattag tgagccccca     240
   catgtacact tggcctcagg ggactcagga ttagtgagcc ccacatgtac acttggcctc     300
   aggggactca ggactagtga gccccacatg tacacttggc ctcaggggac tcagaactag     360
   tgagccccac atgtacactt ggcttcaggg gactcaggat tagtgagccc cacatgtaca     420
   cttggacacg tgaaccacat cgatgtgctg cagagctcag ccctctgcag atgaaatgtg     480
   gtcatggcat tccttcacag tggcacccct cgttccctcc ccacctcatc tcccattctt     540
   gtctgtcttc agcacctgcc atgtccagcc ggcagattcc accgcagcat cttctgcagc     600
   acccccgacc acacacctcc ccagcgcctg cttggccctc cagcccagct cccgcctttc     660
   ttccttgggg aagctccctg gacagacacc ccctcctccc agccatggct ttttcctgct     720
   ctgccccacg cgggaccctg ccctggatgt gctacaatag acacatcaga tacagtcctt     780
   cctcagcagc cggcagaccc agggtggact gctcggggcc tgcctgtgag gtcacacagg     840
   tgtcgttaac ttgccatctc agcaactagt gaatatgggc agatgctacc ttccttccgg     900
   ttccctggtg agaggtactg gtggatgtcc tgtgttgccg gccacctttt gtccctggat     960
   gccatttatt tttttccaca aatatttccc aggtctcttc tgtgtgcaag gtattagggc    1020
   tgcagcgggg gccaggccac agatctctgt cctgagaaga cttggattct agtgcaggag    1080
   actgaagtgt atcacaccaa tcagtgtaaa ttgttaactg ccacaaggag aaaggccagg    1140
   aaggagtggg gcatggtggt gttctagtgt tacaagaaga agccagggag ggcttcctgg    1200
   atgaagtggc atctgacctg ggatctggag gaggagaaaa atgtcccaaa agagcagaga    1260
   gcccacccta ggctctgcac caggaggcaa cttgctgggc ttatggaatt cagagggcaa    1320
   gtgataagca gaaagtcctt gggggccaca attaggattt ctgtcttcta aagggcctct    1380
   gccctctgct gtgtgacctt gggcaagtta cttcacctct agtgctttgg ttgcctcatc    1440
   tgtaaagtgg tgaggataat gctatcacac tggttgagaa ttgaagtaat tattgctgca    1500
   aagggcttat aagggtgtct aatactagta ctagtaggta cttcatgtgt cttgacaatt    1560
   ttaatcatta ttattttgtc atcaccgtca ctcttccagg ggactaatgt ccctgctgtt    1620
   ctgtccaaat taaacattgt ttatccctgt gggcatctgg cgaggtggct aggaaagcct    1680
   ggagctgttt cctgttgacg tgccagacta gt                                  1712
<210> 37
   <211> 1321
   <212> DNA
   <213> Homo sapiens
<220>
   <221> misc_feature
   <223> sequence of STAR37
<400> 37
   aggatcacat ttaaggaagt gtgtggggtc cctggatgac accagcaccc agtgcggctc     60
   tgtctggcaa ccgctcccaa ggtggcagga gtgggtgtcc cctgtgtgtc agtgggcagc    120
   tcctgctgag cctacagctc actggggagc ctgacagcgg ggccatgtgc ctgacactcc    180
   tctctgcttg tggacctggc aaggcaggga gcagaaaaca gagccacttg aaggctttct    240
   gtctgcgtct gtgtgcagtg tggatttagt tgtgcttttt tcttgctggg agagcacagc    300
   caccatttac aagcagtgtc accctcatgg gtggcgagga cagaacagga gcctctgctc    360
   tctgtaccta tctgggcccg gtgggctccc ttgtcctggc ttccatctct gtctcagcga    420
   ccattcagcc ctgcgcagga acacatgttg cttagaaaag ccaaattcag cccttgtctc    480
   tgcctcctct ggtctcatga tgtgcatctg ttaccttgaa actggaaacc agtctatcaa    540
   tgtctgtgcc aattttttat tccctcccca acctccttcc ccatacgact ttttatttat    600
   gtaggatgtg tgctgtctaa tgatgggatg accacatttt tccatgttct aaaagtgctc    660
   ctctcccgca gggtcccagg gctggtggtt gctttgggtc tacagctacg tcttacccgc    720
   ctcctgcctc aacagcctgt gtggtggcaa agccggtgtg gggctgggga acgcagcgtt    780
   ctccaggagg gggacccggc tctccttctg cagtgcaggc gaaggcctag atgccagtgt    840
   gacctcccac aaggcgtggc ttccagactc cccggctgga agtgatgctt ttttgcctcc    900
   ggccctgggt ttgaagcagc ctggctttct cttggtaagt ggctggtgtc ttagcagctg    960
   caatctgagc tcagccacct acacaccacc gtggccgaca ctttcattaa aaagtttcct   1020
   gagacgactt gcgtgcatgt tgacttcatg atcagcgccg ctgggaagaa cccctgagcc   1080
   ggtggggtgg ggctggaagc agcaggtgca gtgatggggc tgggtgccca ggaggcctca   1140
   gtgctcaatc aggccaaggt ggccaagccc aggctgcagg gaaggccggc ctgggggttg   1200
   tgggtgagca caggcaggca ccagctgggc agtgttagga tgctggagca gcatccgtaa   1260
   ccccactgag tggggtagtc tggttggggc agggaccgct gttgctttgg cagagagaga   1320
   t                                                                   1321
<210> 38
   <211> 1445
   <212> DNA
   <213> Homo sapiens
<220>
   <221> misc_feature
   <223> sequence of STAR38
<220>
   <221> misc_feature
   <222> (348)..(999)
   <223> n is a, c, g, or t on various positions
<400> 38
   gatctatggg agtagcttcc ttagtgagct ttcccttcaa atactttgca accaggtaga     60
   gaattttgga gtgaaggttt tgttcttcgt ttcttcacaa tatggatatg catcttcttt    120
   tgaaaatgtt aaagtaaatt acctctcttt tcagatactg tcttcatgcg aacttggtat    180
   cctgtttcca tcccagcctt ctataaccca gtaacatctt ttttgaaacc agtgggtgag    240
   aaagacacct ggtcaggaac gcggaccaca ggacaactca ggctcaccca cggcatcaga    300
   ctaaaggcaa acaaggactc tgtataaagt accggtggca tgtgtatnag tggagatgca    360
   gcctgtgctc tgcagacagg gagtcacaca gacacttttc tataatttct taagtgcttt    420
   gaatgttcaa gtagaaagtc taacattaaa tttgattgaa caattgtata ttcatggaat    480
   attttggaac ggaataccaa aaaatggcaa tagtggttct ttctggatgg aagacaaact    540
   tttcttgttt aaaataaatt ttattttata tatttgaggt tgaccacatg accttaagga    600
   tacatataga cagtaaactg gttactacag tgaagcaaat taacatatct accatcgtac    660
   atagttacat ttttttgtgt gacaggaaca gctaaaatct acgtatttaa caaaaatcct    720
   aaagacaata catttttatt aactatagcc ctcatgatgt acattagatc gtgtggttgt    780
   ttcttccgtc cccgccacgc cttcctcctg ggatggggat tcattcccta gcaggtgtcg    840
   gagaactggc gcccttgcag ggtaggtgcc ccggagcctg aggcgggnac tttaanatca    900
   gacgcttggg ggccggctgg gaaaaactgg cggaaaatat tataactgna ctctcaatgc    960
   cagctgttgt agaagctcct gggacaagcc gtggaagtcc cctcaggagg cttccgcgat   1020
   gtcctaggtg gctgctccgc ccgccacggt catttccatt gactcacacg cgccgcctgg   1080
   aggaggaggc tgcgctggac acgccggtgg cgcctttgcc tgggggagcg cagcctggag   1140
   ctctggcggc agcgctggga gcggggcctc ggaggctggg cctggggacc caaggttggg   1200
   cggggcgcag gaggtgggct cagggttctc cagagaatcc ccatgagctg acccgcaggg   1260
   cggccgggcc agtaggcacc gggcccccgc ggtgacctgc ggacccgaag ctggagcagc   1320
   cactgcaaat gctgcgctga ccccaaatgc tgtgtccttt aaatgtttta attaagaata   1380
   attaataggt ccgggtgtgg aggctcaagc cttaatcccc agcacctggc gaggccgagg   1440
   aggga                                                                1445
<210> 39
   <211> 2331
   <212> DNA
   <213> Homo sapiens
<220>
   <221> misc_feature
   <223> sequence of STAR39
<400> 39
   gtgaaataga tcactaaagc tgattcctct tgtctaaatg aaactttcta ccctttgatg      60
   gacagctatg ctttccccat cctctcccgt cccccagccc ttggtaacca tcatcctact     120
   ctctacttgt aggagttcaa cttgtttaga ttttgtgagt gagaacatgt ggtatttgcc     180
   tttagagtcc tctaggttta tccatattgt gttaaatgac aggattccct gcctttttaa     240
   ggctgaatag tatttcattg taatatatat acatacacac acacatatac acacacatat     300
   atatacatat atacatatat gtacatagat acatatatat gtacatatat acacacacat     360
   atacacacat atatacacat atatacatat acatatatac acatatatgt acatatatat     420
   aacttttttt catttatcca ttcacttaat acatatgatg gagggcttta tatatgccag     480
   gctctgtgat gaatgctgga aattcaatag tgagaaagac tcagtctctg cctccaaaga     540
   gcatcatggg ctaggtgctg caacgaggaa ttgccaactg ttgtcatgag agcacagaga     600
   agggactcaa ccagccttga agaatcaggg gaggcttcta agctaatggt gtgtgcctgg     660
   ggatcacatt gtttcaagca gcagtaacag gatgtgctca ggtccagatg tgagagagag     720
   agagagcata tgtcttcaag aaactaacag tagctcccta tagctgaagc aggagtacaa     780
   aatagtgagt ttaagtgatg aggcaagaga tatgaagaag cttgaccatg cagctacacc     840
   gggcagcatg ccctctgaga catctcatgg aagccggaaa tgggagtgcc ttgataccaa     900
   gccagagaaa ttataatact aagtagatag actgagcagc actcctcctg ggaagaatga     960
   gacaagccct gaatttggag gtaagttgtg gattggtgat tagaggagag gtaacaggca 1020
   ccaaagcaag aaatagtatt gatgcaaagc tgaggttaat tggatgacaa aatgaagagc 1080
   ataaggggct cagacacaga ctgagcagaa aacgagtagc atctgaacct agattgagtt 1140
   actaatggat gagaaagagt tcttaaagtt gatgaccacg ggatccatat ataagaatgt 1200
   ccaatctccc caaattgatc cacgagttca gtgcaatgcc aatcaaaatc ccactaacaa 1260
   gtttatttta aaatgtaaat gaaaatacaa aatttttaaa aagcaaagca atattgaaaa 1320
   cccaggaaaa attaggagga cttacacaac ctgatctcaa aacttaccat tatcaagaca 1380
   gagtgttatt gacacaagga gagacaaata gataaacgga atgtggtagt ctggagatgc 1440
   acccacatgt atgtggtcaa ttgatttttg gccaaggcac caagtcaatt caaaggagca 1500
   aggaaagtag tacagaaaca accaaatatt gttttggaaa ataatgacaa agggcttata 1560
   accagaatat aagcatataa atataattct ttcaaatcaa taataagaag gcaaatatct 1620
   aataaaaatg agcaaagact tgaaaagtca cttaaaaagg cttattaatt agaaatatgc 1680
   aaatgttatt agtcttcagt ggaatttaca ttaaaccaca agggatacta ttatatctta 1740
   tgcccactag aataaccaaa ggaaaaaaga cagacaaaac aaaatgctgg tgaggatgtg 1800
   aagcaactgg aactctcata cattattggt ggtaatgtaa aatttataca accattatga 1860
   ataaaggttt ggcagtttct tacaaagttg aatgcacttc tccacgatga ctaggctttt 1920
   cactcatagg cgtctggctc cctagaactg aaaacatatg ttcacaagaa gacttgcaaa 1980
   tatatattct cccacgtcag gagatatttg ctatgcattt aactgacata agattagtgc 2040
   tagagtttat aatgaggttc ttcaaatcta aaagaaaatg caaagcatat aatagtaagg 2100
   ggtgcaggcc aggcgcagtg gctcactctg taatcccagc actttgggag gccgaggtgg 2160
   gcggatcaca aggtcaggag ttcgagacca acctggccaa catagtgaaa ccctgtctct 2220
   actaaaaata caaaaactag ccaggtgcgg tgtcatgcac ctgtagtccc agctactcgg 2280
   gaggccgagg caggagaatc acttgaacct gggaggtgga ggttgcagtg a 2331
<210> 40
   <211> 1071
   <212> DNA
   <213> Homo sapiens
<220>
   <221> misc_feature
   <223> sequence of STAR40
<400> 40
   gctgtgattc aaactgtcag cgagataagg cagcagatca agaaagcact ccgggctcca 60
   gaaggagcct tccaggccag ctttgagcat aagctgctga tgagcagtga gtgtcttgag 120
   tagtgttcag ggcagcatgt taccattcat gcttgacttc tagccagtgt gacgagaggc 180
   tggagtcagg tctctagaga gttgagcagc tccagcctta gatctcccag tcttatgcgg 240
   tgtgcccatt cgctttgtgt ctgcagtccc ctggccacac ccagtaacag ttctgggatc 300
   tatgggagta gcttccttag tgagctttcc cttcaaatac tttgcaacca ggtagagaat 360
   tttggagtga aggttttgtt cttcgtttct tcacaatatg gatatgcatc ttcttttgaa 420
   aatgttaaag taaattacct ctcttttcag atactgtctt catgcgaact tggtatcctg 480
   tttccatccc agccttctat aacccagtaa catctttttt gaaaccagtg ggtgagaaag 540
   acacctggtc aggaacgcgg accacaggac aactcaggct cacccacggc atcagactaa 600
   aggcaaacaa ggactctgta taaagtaccg gtggcatgtg tattagtgga gatgcagcct 660
   gtgctctgca gacagggagt cacacagaca cttttctata atttcttaag tgctttgaat 720
   gttcaagtag aaagtctaac attaaatttg attgaacaat tgtatattca tggaatattt 780
   tggaacggaa taccaaaaaa tggcaatagt ggttctttct ggatggaaga caaacttttc 840
   ttgtttaaaa taaattttat tttatatatt tgaggttgac cacatgacct taaggataca 900
   tatagacagt aaactggtta ctacagtgaa gcaaattaac atatctacca tcgtacatag 960
   ttacattttt ttgtgtgaca ggaacagcta aaatctacgt atttaacaaa aatcctaaag 1020
   acaatacatt tttattaact atagccctca tgatgtacat tagatctcta a 1071
<210> 41
   <211> 1135
   <212> DNA
   <213> Homo sapiens
<220>
   <221> misc_feature
   <223> sequence of STAR41
<400> 41
   cgtgtgcagt ccacggagag tgtgttctcc tcatcctcgt tccggtggtt gtggcgggaa      60
   acgtggcgct gcaggacacc aacatcagtc acgtatttca ttctggaaaa aaaagtagca     120
   caagcctcgg ctggttccct ccagctctta ccaggcagcc taagcctagg ctccattccc     180
   gctcaaggcc ttcctcaggg gcctgctcac cacaggagct gttcccatgc agggactaag     240
   gacatgcagc ctgcatagaa accaagcacc caggaaaaca tgattggatg gagcgggggg     300
   gtgtggtctc tagccttgtc cacctccggt cctcatgggt ctcacacctc ctgagaatgg     360
   gcaccgcaga ggccacagcc catacagcca agatgacaga ctccgtaagt gacagggatc     420
   cacagcagag tgggtgaaat gttccctata aactttacaa aattaatgag ggcaggggga    480
   ggggagaaat gaaaatgaac ccagctcgca gcacatcagc atcagtcact aggtcggcgt     540
   gctctctgac tgcttcctcg tagctgcttg gtgtctcatt gcctcagaag catgtagacc     600
   ctgtcacaag attgtagttc ccctaactgc tccgtagatc acaacttgaa ccttaggaaa     660
   tgctgttttc cctttgagat attcctttgg gtcctgtata ctgatggagc tactgactga     720
   gctgctccga aggaccccac gaggagctga ctaaaccaag agtgcagttt gtacaccctg     780
   atgattacat cccccttgcc ccaccaatca actctcccaa ttttccagcc cctcaccctc    840
   cagtcccctt aaaagcccca gcccaggccg ggcacagtgg ctcatgcctg taatcccagc     900
   actttgggag gccaaggtgg gcagatcacc tgagggcagg aatttgagac cagcctgacc     960
   aacatgaaga aaccccgtct ctattacaaa tacaaaatta gccgggcgtg ttgctgcata    1020
   ctggtaatcc cagctacttg ggagggtgag gcaggagaat cacttgaatc tgggaggcgg    1080
   aggttgcgat gagccgagac agcgccattg cactgcagcc tgggcaacaa gagca          1135
<210> 42
   <211> 735
   <212> DNA
   <213> Homo sapiens
<220>
   <221> misc_feature
   <223> sequence of STAR42
<400> 42
   aagggtgaga tcactaggga gggaggaagg agctataaaa gaaagaggtc actcatcaca 60
   tcttacacac tttttaaaac cttggttttt taatgtccgt gttcctcatt agcagtaagc 120
   cctgtggaag caggagtctt tctcattgac caccatgaca agaccctatt tatgaaacat 180
   aatagacaca caaatgttta tcggatattt attgaaatat aggaattttt cccctcacac 240
   ctcatgacca cattctggta cattgtatga atgaatatac cataatttta cctatggctg 300
   tatatttagg tcttttcgtg caggctataa aaatatgtat gggccggtca cagtgactta 360
   cgcccgtagt cccagaactt tgggaggccg aggcgggtgg atcacctgag gtcgggagtt 420
   caaaaccagc ctgaccaaca tggagaaacc ccgtctctgc taaaaataca aaaattaact 480
   ggacacggtg gcgtatgcct gtaatcccag ctactcggga agctgaggca ggagaactgc 540
   ttgaacccag gaggcggagg ttgtggtgag tcgagattgc gccattgcac tccagcctgg 600
   gcaacaagag cgaaattcca tctcaaaaaa aagaaaaaag tatgactgta tttagagtag 660
   tatgtggatt tgaaaaatta ataagtgttg ccaacttacc ttagggttta taccatttat 720
   gagggtgtcg gtttc 735
<210> 43
   <211> 1227
   <212> DNA
   <213> Homo sapiens
<220>
   <221> misc_feature
   <223> sequence of STAR43
<400> 43
   caaatagatc tacacaaaac aagataatgt ctgcccattt ttccaaagat aatgtggtga 60
   agtgggtaga gagaaatgca tccattctcc ccacccaacc tctgctaaat tgtccatgtc 120
   acagtactga gaccaggggg cttattccca gcgggcagaa tgtgcaccaa gcacctcttg 180
   tctcaatttg cagtctaggc cctgctattt gatggtgtga aggcttgcac ctggcatgga 240
   aggtccgttt tgtacttctt gctttagcag ttcaaagagc agggagagct gcgagggcct 300
   ctgcagcttc agatggatgt ggtcagcttg ttggaggcgc cttctgtggt ccattatctc 360
   cagcccccct gcggtgttgc tgtttgcttg gcttgtctgg ctctccatgc cttgttggct 420
   ccaaaatgtc atcatgctgc accccaggaa gaatgtgcag gcccatctct tttatgtgct    480
   ttgggctatt ttgattcccc gttgggtata ttccctaggt aagacccaga agacacagga    540
   ggtagttgct ttgggagagt ttggacctat gggtatgagg taatagacac agtatcttct    600
   ctttcatttg gtgagactgt tagctctggc cgcggactga attccacaca gctcacttgg    660
   gaaaacttta ttccaaaaca tagtcacatt gaacattgtg gagaatgagg gacagagaag    720
   aggccctaga tttgtacatc tgggtgttat gtctataaat agaatgcttt ggtggtcaac    780
   tagacttgtt catgttgaca tttagtcttg ccttttcggt ggtgatttaa aaattatgta    840
   tatcttgttt ggaatatagt ggagctatgg tgtggcattt tcatctggct ttttgtttag    900
   ctcagcccgt cctgttatgg gcagccttga agctcagtag ctaatgaaga ggtatcctca    960
   ctccctccag agagcggtcc cctcacggct cattgagagt ttgtcagcac cttgaaatga   1020
   gtttaaactt gtttattttt aaaacattct tggttatgaa tgtgcctata ttgaattact   1080
   gaacaacctt atggttgtga agaattgatt tggtgctaag gtgtataaat ttcaggacca   1140
   gtgtctctga agagttcatt tagcatgaag tcagcctgtg gcaggttggg tggagccagg   1200
   gaacaatgga gaagctttca tgggtgg                                        1227
<210> 44
   <211> 1586
   <212> DNA
   <213> Homo sapiens
<220>
   <221> misc_feature
   <223> sequence of STAR44
<400> 44
   cacctgcctc agcctcccaa agtgctgaga ttcaaagaaa ttttcatgga gaggggacag     60
   atggagtcaa ttcttgtggg gtgaacatga gtaccacagt tagactgagg ttgggaaaga    120
   ttttccagac aattggaaga gcatgtgaaa gacacagatt ttgagaaatg ttaagtctag    180
   ggaactgcaa ggcttttggc acaagaaagc cactgtagac tatagaggca ggatgcctag    240
   attcaaatcc caactgctac acttctaagc tttgtaattt tggcaagttt ttaccctcta    300
   ttttcttatc tataaaatat agattttata tatatagata tagatatata gatagataat    360
   aattgtgcat gcctaataaa gttgtcaaag attaaatgtt atatgtgaag tattttgtac    420
   ggtgatagga acccaggaag ggctctatga atattatgta ttattattat tctaaagtag    480
   ctggaataca atgttcaaag gagatagtgg caggagataa gtttgaattg aaagattgag    540
   gccagaacat aaagtgcctc ctatattata ttttacataa ttggaacatc attgaaaaat    600
   ttaagtatta tttatgtgtg tatgtgtgtt ttatataatt aattctagtt catcatttta    660
   aaatatcttt ctgatgtcac tgtgaacaac agatgagaag aagtgaatcc tgagttaagg    720
   agaccagctc tctgattact gccataatcc agggagggta ccataaggat ttcaactgga    780
   agtgaatcca tcatgatgga gaggaaggac agggctgaaa aatacttagg aagtagtatc    840
   agtaggactg gttaagagag agcagaggca ggctacaggg gttggaggtg tcaatcacag    900
   agatagggaa aatgggagga gaagcaggct ttgaaaaagt ggcttgtctt gtaaaattat    960
   gtgctgttaa aacagtacaa gaaattaata tattcaatcc caaaatacag ggacaattct   1020
   ttttgaaaga gttacccaga tagtcttcct tgaagttttc agttaaagaa atttcttgtt   1080
   aacaaataat gtagtcatag aagaaaacac ttaaaacttt attgaataaa gctaataaat   1140
   catttaatat aatttatagg aaattgttac ataacacaca cattcaatac tttttgctaa   1200
   agtataaatt aatggaagga gagcacgcac acagaggttg aattatgttt atgactttat   1260
   tagtcaagaa tacaaaattg agtagctaca tcaagcagaa gcacatgctt tacaatccag   1320
   cacagaatcc cttgacatcc aaactcccga aacagacatg taaatacaga tgacattgtc   1380
   agaacaaaat agggtctcac ccgacctata atgttctttt cttgatataa atatgcacat   1440
   gaattgcata cggtcatatg gttccaatta ccattatttc ctctgggctt agctatccat   1500
   ctaaggggaa tttacaccaa cactgtactt ctacttgcaa gaatatatga aagcatagtt   1560
   aacttctggc ttaggacccc aactca                                        1586
<210> 45
   <211> 1981
   <212> DNA
   <213> Homo sapiens
<220>
   <221> misc_feature
   <223> sequence of STAR45
<400> 45
   atggatcata gggtaaataa atttataatt tcttgagaaa gcttcgtact gttttccaag     60
   atggctgtac taatttccat tcctaccaac agtgtacagg gtttcttttt ctccacatcc    120
   tcaccaacac ttatcttcca tcttttttta taatagccct agtaaaatgt gtgaggtgat    180
   atctcattgt ggcattgatt tgcacttctc tgataattag gaatgtttat gattttttca    240
   tgtacctggt tggccttttg tatgatgtag gaaatgtcta ttctgattct ttgcttattt    300
   tttaataagc atagtttttt tcttattttt gagtaggttg agttgcttat atattattat    360
   atgagcccct tacctgatgt atggtttaaa aatattatcc catttgtggg ttctcttaat    420
   tctatcattg cttcttttcc tgtggaaaag ttttaagttt tatgcagtct catttgtgtg    480
   ttttgctttt gttgcctttt ggaataatct acagaaaatc atagctcagg ccaatgtcat    540
   acagtctcct tctatatttc cttgtagtag ttttacattt aaactttaat tttgatttga    600
   tgcttgtata aagagcaaaa taaaagtcaa attttattct tctgtatgtg gatagtcagt    660
   tttgtctaca ccatttattg aaaataattt tctttcttca ctgtgtattt ttagttattt    720
   tatcaaaaaa tcaattgacc acagacacac ggatttattt acaggttcta tatccctttg    780
   tactgtttta catgtctgtt tttatgccat tgctatgctg ttttaattcc tatagctttg    840
   taatagagtt tggagtcagg tagtctgatg cctccagctt tgttcttttt gttcaagatt    900
   gctttggttg gtccaggtct tttgtggttc catacaaatt ttagcagtaa tttttctatt    960
   tctgtgaaga atgacattgg aatttgatag tggttgcatt taatctgtag attgctttgg   1020
   gtagcattga cacttttaca atactaattt ttgaatccat caatgaagga tgtttctcca   1080
   tttatttatg ccattttaat ttttttcatc aatgtgctat agttttcagt atgtaaatct   1140
   tttatggttt tgattaaatt tactcctgtc ttttatatat ttatatatct gttttgattc   1200
   tattataaat tgaattgcct ttatttttca ggtaatagtt tgtcattagt taatagaaac   1260
   aataatgata tttgtatgtt gattttgtaa ctattaactt tattgaattt cttcatcagc   1320
   tataaccatt tattttggtg gaatctttaa gattttctct atcttaagat tatattttca   1380
   aaaaacagaa acaatcttac ctcttccttc cctatgtgga tttcttttac gtctttgtct   1440
   tgtgtaactg ttctggctag gcaattacac ataatgtttt catcatttat aattttacat   1500
   cacatccatc tattgtggca cattgattgc tacttttcaa gttgtaaacc tggacattta   1560
   tcactactct tcctccaata caggagtcca tggcgtggtg tgggccctac tgtgccacag   1620
   tccagggcac ggctgggctg aggttctctt gtgcaagagt ccgtggctct gcggagcaag   1680
   agttctccag tgccttagtc cagggttagg caggggtggg gctccttcag tagcttagtc   1740
   cagtgcgccg ccctgcgagg gtcctcctga gcaggagtac acgatgaggc agggtcctac   1800
   tgtgccttag cccaggaagc ggggggctgg gtcctctggt gccatagtcc aggctgccgg   1860
   gagctgggtc ctctggtgcc atagctcagg ccggcgggag ctgggtcctc tggtgccgta   1920
   gtccagggtg cagcagaaca ggagtcctgc ggagcagtag tccagggcac gctggggcgt   1980
   g                                                                   1981
<210> 46
   <211> 1859
   <212> DNA
   <213> Homo sapiens
<220>
   <221> misc_feature
   <223> sequence of STAR46
<400> 46
   attgtttttc tcgcccttct gcattttctg caaattctgt tgaatcattg cagttactta 60
   ggtttgcttc gtctccccca ttacaaacta cttactgggt ttttcaaccc tagttccctc 120
   atttttatga tttatgctca tttctttgta cacttcgtct tgctccatct cccaactcat 180
   ggcccctggc tttggattat tgttttggtc ttttattttt tgtcttcttc tacctcaaca 240
   cttatcttcc tctcccagtc tccggtaccc tatcaccaag gttgtcatta acctttcata 300
   ttattcctca ttatccatgt attcatttgc aaataagcgt atattaacaa aatcacaggt 360
   ttatggagat ataattcaca taccttaaaa ttcaggcttt taaagtgtac ctttcatgtg 420
   gtttttggta tattcacaaa gttatgcatt gatcaccacc atctgattcc ataacatgtt 480
   caatacctca aaaagaagtc tgtactcatt agtagtcatt tcacattcac cactccctct 540
   ggctctgggc agtcactgat ctttgtgtct ctatggattt gcctagtcta ggtattttta 600
   tgtaaatggc atcatacaac atgtgacctt ttgtttggct tttttcattt agcaaaatgt 660
   tatcaaggtc tgtccctgtt gtagcatgta ttagcacttc atttcttata tgctgaatga 720
   tatactttat ttgtccatca gttgttcatg ctttatttgt ccatcagttg atgaacattt 780
   gcgtttttgc cactttgggc tattaagaat aatgctactg tgaacaagtg tgtacaagtt 840
   cctctacaaa tttttgtgtg gacatatcct ttcagttctc tcaggtgtat atctgggaat 900
   tgaattgctg ggtcgtgtag tagctatgtt aaacactttg agaaactgct ataatgttct 960
   ccagagctgt accattttaa attctgtgta tgaggattcc acgttctcca cttcctcacc 1020
   agtgtatgga tttgggggta tactttttaa aaagtgggat taggctgggc acagtggctc 1080
   acacctgtaa tcccaacact tcaggaagct gaggtgggag gatcacttga gcctagtagt 1140
   ttgagaccag cctgggcaac atagggagac cctgtctcta caaaaaataa tttaaaataa 1200
   attagctggg cgttgtggca cacacctgta gtcccagcta catgggaggc tgaggtggaa 1260
   ggattccctg agcccagaag tttgaggttg cagtgagcca tgatggcagc actatactgt 1320
   agcctgggtg tcagagcaag actccgtttc agggaagaaa aaaaaaagtg ggatgatatt 1380
   tttgacactt ttcttcttgt tttcttaatt tcatacttct ggaaattcca ttaaattagc 1440
   tggtaccact ctaactcatt gtgtttcatg gctgcatagt aatattgcat aatataaata 1500
   taccattcat tcatcaaagt tagcagatat tgactgttag gtgccaggca ctgctctaag 1560
   cgttaaagaa aaacacacaa aaacttttgc attcttagag tttattttcc aatggagggg 1620
   gtggagggag gtaagaattt aggaaataaa ttaattacat atatagcata gggtttcacc 1680
   agtgagtgca gcttgaatcg ttggcagctt tcttagtagt ataaatacag tactaaagat 1740
   gaaattactc taaatggtgt tacttaaatt actggaatag gtattactat tagtcacttt 1800
   gcaggtgaaa gtggaaacac catcgtaaaa tgtaaaatag gaaacagctg gttaatgtt 1859
<210> 47
   <211> 1082
   <212> DNA
   <213> Homo sapiens
<220>
   <221> misc_feature
   <223> sequence of STAR47
<400> 47
   atcattagtc attagggaaa tgcaaatgaa aaacacaagc agccaccaat atacacctac     60
   taggatgatt taaaggaaaa taagtgtgaa gaaggacgta aagaaattgt aaccctgata    120
   cattgatggt agaaatggat aaagttgcag ccactgtgaa aaacagtctg cagtggctca    180
   gaaggttaaa tatagaaccc ctgttggacc caggaactct actcttaggc accccaaaga    240
   atagagaaca gaaatcaaac agatgtttgt atactaatgt ttgtagcatc acttttcaca    300
   ggagccaaaa ggtggaaata atccaaccat cagtgaacaa atgaatgtaa taaaagcaag    360
   gtggtctgca tgcaatgcta catcatccat ctgtaaaaaa cgaacatcat tttgatagat    420
   gatacaacat gggtggacat tgagaacatt atgcttagtg aaataagcca gacacaaaag    480
   gaatatattg tataattgta attacatgaa gtgcctagaa tagtcaaatt catacaagag    540
   aaagtgggat aggaatcacc atgggctgga aataggggga aggtgctata ctgcttattg    600
   tggacaaggt ttcgtaagaa atcatcaaaa ttgtgggtgt agatagtggt gttggttatg    660
   caaccctgtg aatatattga atgccatgga gtgcacactt tggttaaaag gttcaaatga    720
   taaatattgt gttatatata tttccccacg atagaaaaca cgcacagcca agcccacatg    780
   ccagtcttgt tagctgcctt cctttacctt caagagtggg ctgaagcttg tccaatcttt    840
   caaggttgct gaagactgta tgatggaagt catctgcatt gggaaagaaa ttaatggaga    900
   gaggagaaaa cttgagaatc cacactactc accctgcagg gccaagaact ctgtctccca    960
   tgctttgctg tcctgtctca gtatttcctg tgaccacctc ctttttcaac tgaagacttt   1020
   gtacctgaag gggttcccag gtttttcacc tcggcccttg tcaggactga tcctctcaac   1080
   ta                                                                  1082
<210> 48
   <211> 1242
   <212> DNA
   <213> Homo sapiens
<220>
   <221> misc_feature
   <223> sequence of STAR48
<400> 48
   atcatgtatt tgttttctga attaattctt agatacatta atgttttatg ttaccatgaa 60
   tgtgatatta taatataata tttttaattg gttgctactg tttataagaa tttcattttc 120
   tgtttacttt gccttcatat ctgaaaacct tgctgatttg attagtgcat ccacaaattt 180
   tcttggattt tctatgggta attacaaatc tccacacaat gaggttgcag tgagccaaga 240
   tcacaccact gtactccagc ctgggcgaca gagtgagaca ccatctcaca aaaacacata 300
   aacaaacaaa cagaaactcc acacaatgac aacgtatgtg ctttcttttt ttcttcctct 360
   ttctataata tttctttgtc ctatcttaac tgaactggcc agaaacccca ggacaatgat 420
   aaatacgagc agtgtcaaca gacatctcat tccctttcct agcttttata aaaataacga 480
   ttatgcttca acattacata tggtggtgtc gatggttttg ttatagataa gcttatcagg 540
   ttaagaaatt tgtctgcgtt tcctagtttg gtataaagat tttaatataa atgaatgttg 600
   tattttatca tcttattttt ttcctacatc tgctaaggta atcctgtgtt ttcccctttt 660
   caatctccta atgtggtgaa tgacattaaa ataccttcta ttgttaaaat attcttgcaa 720
   cgctgtatag aaccaatgcc tttattctgt attgctgatg gatttttgaa aaatatgtag 780
   gtggacttag ttttctaagg ggaatagaat ttctaatata tttaaaatat tttgcatgta 840
   tgttctgaag gacattggtg tgtcatttct ataccatctg gctactagag gagccgactg 900
   aaagtcacac tgccggagga ggggagaggt gctcttccgt ttctggtgtc tgtagccatc 960
   tccagtggta gctgcagtga taataatgct gcagtgccga cagttctgga aggagcaaca 1020
   acagtgattt cagcagcagc agtattgcgg gatccccacg atggagcaag ggaaataatt 1080
   ctggaagcaa tgacaatatc agctgtggct atagcagctg agatgtgagt tctcacggtg 1140
   gcagcttcaa ggacagtagt gatggtccaa tggcgcccag acctagaaat gcacatttcc 1200
   tcagcaccgg ctccagatgc tgagcttgga cagctgacgc ct 1242
<210> 49
   <211> 1015
   <212> DNA
   <213> Homo sapiens
<220>
   <221> misc_feature
   <223> sequence of STAR49
<400> 49
   aaaccagaaa cccaaaacaa tgggagtgac atgctaaaac cagaaaccca aaacaatggg      60
   agggtcctgc taaaccagaa acccaaaaca atgggagtga agtgctaaaa ccagaaaccc     120
   aaaacaatgg gagtgtcctg ctacaccaga aacccaaaac gatgggagtg acgtgataaa     180
   accagacacc caaaacaatg ggagtgacgt gctaaaccag aaacccaaaa caatgggagt     240
   gacgtgctaa aacctggaaa cctaaaacaa tgcgagtgag gtgctaacac cagaatccat     300
   aacaatgtga gtgacgtgct aaaccagaac ccaaaacaat gggagtgacg tgctaaaaca     360
   ggaacccaaa acaatgagag tgacgtgcta aaccagaaac ccaaaacaat gggaatgacg     420
   tgctaaaacc ggaacccaaa acaatgggag tgatgtgcta aaccagaaac ccaaaacaat     480
   gggaatgaca tgctaaaact ggaacccaaa acaatggtaa ctaagagtga tgctaaggcc     540
   ctacattttg gtcacactct caactaagtg agaacttgac tgaaaaggag gatttttttt     600
   tctaagacag agttttggtc tgtcccccag agtggagtgc agtggcatga tctcggctca     660
   ctgcaagctc tgcctcccgg gttcaggcca ttctcctgcc tcagcctcct gagtagctgg     720
   gaatacaggc acccgccacc acacttggct aattttttgt atttttagta gagatggggt      780
   ttcaccatat tagcaaggat ggtctcaatc tcctgacctc gtgatctgcc cacctcaggc     840
   tcccaaagtg ctgggattac aggtgtgagc caccacaccc agcaaaaagg aggaattttt     900
   aaagcaaaat tatgggaggc cattgttttg aactaagctc atgcaatagg tcccaacaga     960
   ccaaaccaaa ccaaaccaaa atggagtcac tcatgctaaa tgtagcataa tcaaa         1015
<210> 50
   <211> 2355
   <212> DNA
   <213> Homo sapiens
<220>
   <221> misc_feature
   <223> sequence of STAR50
<400> 50
   caaccatcgt tccgcaagag cggcttgttt attaaacatg aaatgaggga aaagcctagt      60
   agctccattg gattgggaag aatggcaaag agagacaggc gtcattttct agaaagcaat     120
   cttcacacct gttggtcctc acccattgaa tgtcctcacc caatctccaa cacagaaatg     180
   agtgactgtg tgtgcacatg cgtgtgcatg tgtgaaagta tgagtgtgaa tgtgtctata 240
   tgggaacata tatgtgattg tatgtgtgta actatgtgtg actggcagcg tggggagtgc 300
   tggttggagt gtggtgtgat gtgagtatgc atgagtggct gtgtgtatga ctgtggcggg 360
   aggcggaagg ggagaagcag caggctcagg tgtcgccaga gaggctggga ggaaactata 420
   aacctgggca atttcctcct catcagcgag cctttcttgg gcaatagggg cagagctcaa 480
   agttcacaga gatagtgcct gggaggcatg aggcaaggcg gaagtactgc gaggaggggc 540
   agagggtctg acacttgagg ggttctaatg ggaaaggaaa gacccacact gaattccact 600
   tagccccaga ccctgggccc agcggtgccg gcttccaacc ataccaacca tttccaagtg 660
   ttgccggcag aagttaacct ctcttagcct cagtttcccc acctgtaaaa tggcagaagt 720
   aaccaagctt accttcccgg cagtgtgtga ggatgaaaag agctatgtac gtgatgcact 780
   tagaagaagg tctagggtgt gagtggtact cgtctggtgg gtgtggagaa gacattctag 840
   gcaatgagga ctggggagag cctggcccat ggcttccact cagcaaggtc agtctcttgt 900
   cctctgcact cccagccttc cagagaggac cttcccaacc agcactcccc acgctgccag 960
   tcacacatag ttacacacat acaatcacat atatgttccc atatagacac attcacactc 1020
   ataccttcac acatgcacac gcatgtgcac acacagtcac tcatttctgt gttggagatt 1080
   gggtgaggac attcaatggg tgaggaccaa caggtgtgaa gattgctttc tagaaaatga 1140
   ctcctgtctc tctttgccat tcttcccaat ccgatggagc tactaggctt ttccctcatt 1200
   tcatgtttaa taaaccttcc caatggcgaa atgggctttc tcaagaagtg gtgagtgtcc 1260
   catccctgcg gtggggacag gggtggcagc ggacaagcct gcctggaggg aactgtcagg 1320
   ctgattccca gtccaactcc agcttccaac acctcatcct ccaggcagtc ttcattcttg 1380
   gctctaattt cgctcttgtt ttctttttta tttttatcga gaactgggtg gagagctttt 1440
   ggtgtcattg gggattgctt tgaaaccctt ctctgcctca cactgggagc tggcttgagt 1500
   caactggtct ccatggaatt tcttttttta gtgtgtaaac agctaagttt taggcagctg 1560
   ttgtgccgtc cagggtggaa agcagcctgt tgatgtggaa ctgcttggct cagatttctt 1620
   gggcaaacag atgccgtgtc tctcaactca ccaattaaga agcccagaaa atgtggcttg 1680
   gagaccacat gtctggttat gtctagtaat tcagatggct tcacctggga agccctttct 1740
   gaatgtcaaa gccatgagat aaaggacata tatatagtag ctagggtggt ccacttctta 1800
   ggggccatct ccggaggtgg tgagcactaa gtgccaggaa gagaggaaac tctgttttgg 1860
   agccaaagca taaaaaaacc ttagccacaa accactgaac atttgttttg tgcaggttct 1920
   gagtccaggg agggcttctg aggagagggg cagctggagc tggtaggagt tatgtgagat 1980
   ggagcaaggg ccctttaaga ggtgggagca gcatgagcaa aggcagagag gtggtaatgt 2040
   ataaggtatg tcatgggaaa gagtttggct ggaacagagt ttacagaata gaaaaattca 2100
   acactattaa ttgagcctct actacgtgct cgacattgtt ctagtcactg agataggttt 2160
   ggtatacaaa acaaaatcca tcctctatgg acattttagt gactaacaac aatataaata 2220
   ataaaagtga acaaaagctc aaaacatgcc aggcactatt atttatttat ttatttattt   2280
   atttatttat tttttgaaac agagtctcgc tctgttgccc aggctggagt gtagtggtgc   2340
   gatctcggct cactg                                                    2355
<210> 51
   <211> 2289
   <212> DNA
   <213> Homo sapiens
<220>
   <221> misc_feature
   <223> sequence of STAR51
<400> 51
   tcacaggtga caccaatccc ctgaccacgc tttgagaagc actgtactag attgactttc     60
   taatgtcagt cttcattttc tagctctgtt acagccatgg tctccatatt atctagtaca    120
   acacacatac aaatatgtgt gatacagtat gaatataata taaaaatatg tgttataata    180
   taaatataat attaaaatat gtctttatac tagataataa tacttaataa cgttgagtgt    240
   ttaactgctc taagcacttt acctgcagga aacagttttt tttttatttt ggtgaaatac    300
   aactaacata aatttattta caattttaag catttttaag tgtatagttt agtggagtta    360
   atatattcaa aatgttgtgc agccgtcacc atcatcagtc ttcataactc ttttcatatt    420
   gtaaaattaa aagtttatgc tcatttaaaa atgactccca atttcccccc tcctcaacct    480
   ctggaaacta ccattctatt ttctgcctcc gtagttttgc ccactctaag tacctcacat    540
   aagtggaatt tgtcttattt gcctgtttgt gaccggctga tttcatttag tataatgtcc    600
   tcaagtttta ttcacgttat atagcatatg tcataatttt cttcactttt aagcttgagt    660
   aatatttcat cgtatgtatc tcacattttg cttatccatt catctctcag tggacacttg    720
   agttgcttct acattttagc tgttgtgaat actgctgcta tgaacatggg tgtataaata    780
   tctcaagacc tttttatcag ttttttaaaa tatatactca gtagtagttt agctggatta    840
   tatggtaatt ttatttttaa tttttgagga actgtcctac ccttttattc aatagtagct    900
   ataccaattg acaattggca ttcctaccaa cagggcataa gggttctcaa ttctccacat    960
   attccctgat acttgttatt ttcaggtgtt tttttttttt tttttttttt atgggagcca   1020
   tgttaatggg tgtaaggtga tatttcatta tagttttgat ttgcatttcc ctaatgatta   1080
   gtgatgttaa gcatctcttc atgtgcctat tggccatttg tatatcttct ttaaaaatat   1140
   atatatactc attcctttgc ccatttttga attatgttta tttttgttta ttgagtttca   1200
   atacttttct atataaccta ggtattaatc ctttatcaga cttaagattt gcaaatattc   1260
   tctttcattc cacaggttgc taattctctc tgttggtaat atcttttgat gctgttgtgt   1320
   ccagaattga ttcattcctg tgggttcttg gtctcactga cttcaagaat aaagctgcgg   1380
   accctagtgg tgagtgttac acttcttata gatggtgttt ccggagtttg ttccttcaga   1440
   tgtgtccaga gtttcttcct tccaatgggt tcatggtctt gctgacttca ggaatgaagc   1500
   cgcagacctt cgcagtgagg tttacagctc ttaaaggtgg cgtgtccaga gttgtttgtt 1560
   ccccctggtg ggttcgtggt cttgctgact tcaggaatga agccgcagac cctcgcagtg 1620
   agtgttacag ctcataaagg tagtgcggac acagagtgag ctgcagcaag atttactgtg 1680
   aagagcaaaa gaacaaagct tccacagcat agaaggacac cccagcgggt tcctgctgct 1740
   ggctcaggtg gccagttatt attcccttat ttgccctgcc cacatcctgc tgattggtcc 1800
   attttacaga gtactgattg gtccatttta cagagtgctg attggtgcat ttacaatcct 1860
   ttagctagac acagagtgct gattgctgca ttcttacaga gtgctgattg gtgcatttac 1920
   agtcctttag ctagatacag aacgctgatt gctgcgtttt ttacagagtg ctgattggtg 1980
   catttacaat cctttagcta gacacagtgc tgattggtgg gtttttacag agtgctgatt 2040
   ggtgcgtctt tacagagtgc tgattggtgc atttacaatc ctttagctag acacagagtg 2100
   ctgattggtg cgtttataat cctctagcta gacagaaaag ttttccaagt ccccacctga 2160
   ccgagaagcc ccactggctt cacctctcac tgttatactt tggacatttg tccccccaaa 2220
   atctcatgtt gaaatgtaac ccctaatgtt ggaactgagg ccagactgga tgtggctggg 2280
   ccatgggga 2289
<210> 52
   <211> 1184
   <212> DNA
   <213> Homo sapiens
<220>
   <221> misc_feature
   <223> sequence of STAR52
<400> 52
   ctcttctttg tttttttatt ttggggtgtg tgggtacgtg taagatgaga aatgtacaaa 60
   cacaagtatt tcagaaactc caagtaatat tctgtctgtg agttcacggt aaataaataa 120
   aaagggcaaa gtgacagaaa tacaggatta ttaaaagcaa aataatgttc tttgaaatcc 180
   cccccttggt gtatttttta tcttaggatg cagcactttc agcatgccca agtattgaaa 240
   gcagtgtttt tacgctacca cggtaatttt atttagaaac cccatgttca cttttagttt 300
   taaaatggtc tttatgacat aaaattatca gcattcatat ttttgtgttt taatattcct 360
   ttggctactt attgaaacag taaacattac gaaaattagt aaacaaatct ttgatagttg 420
   cttatttttg tttaattgaa tgtttatttt attaggtaaa tatacaatca aatttattta 480
   aaaataatga ggaaaagaat acttttcttt cgctttgcga aagcaaagtg atttttcatt 540
   cttctccgtc cgattccttc tcttccagct gccacagccg actgacaggc tcccggcggc 600
   ctgaggagta gtatgcaaat tttggatgat tgacacctac agtagaagcc aatcacgtca 660
   aagtaggatg ctgattggtt gacaacaata ggcgtaaacc ttgacgtttt aaaaacctga 720
   cacccaatcc aggcgattca tgcaaataaa ggaagggagt cacattacca ggggccagag 780
   agacttgagt acgacctcac gtgttcagtg gtggatattg cacagacgtc tgcaaggtct 840
   atataaacgc tacataatgt tcaactcaat tgcttgcctt ggcctttccc aaacttgtca 900
   ctggaatata aattatccct tttttaaaaa taaaaaaata agaattatgt agtgcacata 960
   tatgatggtt catgtagaaa tctaaatgga cttccaacgc atggaatttt cctatttccc 1020
   cctttcttta aattaatcct cagtgaagga ggctgttttc ccctagattt caaaaggacg 1080
   agatttacag agcctttcct tggagaaacc cgctctaggc acagatggtc agtaaattta 1140
   gcttcttcag cgaagttcca catggcaccg ccagatggca taag 1184
<210> 53
   <211> 1431
   <212> DNA
   <213> Homo sapiens
<220>
   <221> misc_feature
   <223> sequence of STAR53
<400> 53
   ccctgaggaa gatgacgagt aactccgtaa gagaaccttc cactcatccc ccacatccct      60
   gcagacgtgc tattctgtta tgatactggt atcccatctg tcacttgctc cccaaatcat     120
   tcccttctta caattttcta ctgtacagca ttgaggctga acgatgagag atttcccatg     180
   ctctttctac tccctgccct gtatatatcc ggggatcctc cctacccagg atgctgtggg     240
   gtcccaaacc ccaagtaagc cctgatatgc gggccacacc tttctctagc ctaggaattg     300
   ataacccagg cgaggaagtc actgtggcat gaacagatgg ttcacttcga ggaaccgtgg     360
   aaggcgtgtg caggtcctga gatagggcag aatcggagtg tgcagggtct gcaggtcagg     420
   aggagttgag attgcgttgc cacgtggtgg gaactcactg ccacttattt ccttctctct     480
   tcttgcctca gcctcaggga tacgacacat gcccatgatg agaagcagaa cgtggtgacc     540
   tttcacgaac atgggcatgg ctgcggaccc ctcgtcatca ggtgcatagc aagtgaaagc     600
   aagtgttcac aacagtgaaa agttgagcgt catttttctt agtgtgccaa gagttcgatg     660
   ttagcgttta cgttgtattt tcttacactg tgtcattctg ttagatacta acattttcat     720
   tgatgagcaa gacatactta atgcatattt tggtttgtgt atccatgcac ctaccttaga     780
   aaacaagtat tgtcggttac ctctgcatgg aacagcatta ccctcctctc tccccagatg     840
   tgactactga gggcagttct gagtgtttaa tttcagattt tttcctctgc atttacacac     900
   acacgcacac aaaccacacc acacacacac acacacacac acacacacac acacacacac     960
   acacaccaag taccagtata agcatctgcc atctgctttt cccattgcca tgcgtcctgg    1020
   tcaagctccc ctcactctgt ttcctggtca gcatgtactc ccctcatccg attcccctgt    1080
   agcagtcact gacagttaat aaacctttgc aaacgttccc cagttgtttg ctcgtgccat    1140
   tattgtgcac acagctctgt gcacgtgtgt gcatatttct ttaggaaaga ttcttagaag    1200
   tggaattgct gtgtcaaagg agtcatttat tcaacaaaac actaatgagt gcgtcctcgt    1260
   gctgagcgct gttctaggtg ctggagcgac gtcagggaac aaggcagaca ggagttcctg    1320
   acccccgttc tagaggagga tgtttccagt tgttgggttt tgtttgtttg tttcttctag    1380
   agatggtggt cttgctctgt ccaggctaga gtgcagtggc atgatcatag c              1431
<210> 54
   <211> 975
   <212> DNA
   <213> Homo sapiens
<220>
   <221> misc_feature
   <223> sequence of STAR54
<400> 54
   ccataaaagt gtttctaaac tgcagaaaaa tccccctaca gtcttacagt tcaagaattt 60
   tcagcatgaa atgcctggta gattacctga ctttttttgc caaaaataag gcacagcagc 120
   tctctcctga ctctgacttt ctatagtcct tactgaatta tagtccttac tgaattcatt 180
   cttcagtgtt gcagtctgaa ggacacccac atttctcttt tgtctttgtc aattctttgt 240
   gttgtaaggg caggatgttt aaaagttgaa gtcattgact tgcaaaatga gaaatttcag 300
   agggcatttt gttctctaga ccatgtagct tagagcagtg ttcacactga ggttgctgct 360
   aatgtttctg cagttcttac caatagtatc atttacccag caacaggata tgatagagga 420
   cttcgaaaac cccagaaaat gttttgccat atatccaaag ccctttggga aatggaaagg 480
   aattgcgggc tcccattttt atatatggat agatagagac caagaaagac caaggcaact 540
   ccatgtgctt tacattaata aagtacaaaa tgttaacatg taggaagtct aggcgaagtt 600
   tatgtgagaa ttctttacac taattttgca acattttaat gcaagtctga aattatgtca 660
   aaataagtaa aaatttttac aagttaagca gagaataaca atgattagtc agagaaataa 720
   gtagcaaaat cttcttctca gtattgactt ggttgctttt caatctctga ggacacagca 780
   gtcttcgctt ccaaatccac aagtcacatc agtgaggaga ctcagctgag actttggcta 840
   atgttggggg gtccctcctg tgtctcccca ggcgcagtga gcctgcaggc cgacctcact 900
   cgtggcacac aactaaatct ggggagaagc aacccgatgc cagcatgatg cagatatctc 960
   agggtatgat cggcc 975
<210> 55
   <211> 501
   <212> DNA
   <213> Homo sapiens
<220>
   <221> misc_feature
   <223> sequence of STAR55
<400> 55
   cctgaactca tgatccgccc acctcagcct cctgaagtgc tgggattaca ggtgtgagcc 60
   accacaccca gccgcaacac actcttgagc aaccaatgtg tcataaaaga aataaaatgg 120
   aaatcagaaa gtatcttgag acagacaaaa atggaaacac aacataccaa aatttatggg 180
   acacagcaaa agcagtttta ggagggaagt ttatagtgat gaatacctac ctcaaaatca 240
   ttagcctgat tggatgacac tacagtgtat aaatgaattg aaaaccacat tgtgccccat 300
   acatatatac aatttttatt tgttaattaa aaataaaata aaactttaaa aaagaagaaa 360
   gagctcaaat aaacaaccta actttatacc tcaaggaaat agaagagcca gctaagccca 420
   aagttgacag aaggaaaaaa atattggcag aaagaaatga aacagagact agaaagacaa 480
   ttgaagagat cagcaaaact a 501
<210> 56
   <211> 741
   <212> DNA
   <213> Homo sapiens
<220>
   <221> misc_feature
   <223> sequence of STAR56
<400> 56
   acacaggaaa agatcgcaat tgttcagcag agctttgaac cggggatgac ggtctccctc 60
   gttgcccggc aacatggtgt agcagccagc cagttatttc tctggcgtaa gcaataccag 120
   gaaggaagtc ttactgctgt cgccgccgga gaacaggttg ttcctgcctc tgaacttgct 180
   gccgccatga agcagattaa agaactccag cgcctgctcg gcaagaaaac gatggaaaat 240
   gaactcctca aagaagccgt tgaatatgga cgggcaaaaa agtggatagc gcacgcgccc 300
   ttattgcccg gggatgggga gtaagcttag tcagccgttg tctccgggtg tcgcgtgcgc 360
   agttgcacgt cattctcaga cgaaccgatg actggatgga tggccgccgc agtcgtcaca 420
   ctgatgatac ggatgtgctt ctccgtatac accatgttat cggagagctg ccaacgtatg 480
   gttatcgtcg ggtatgggcg ctgcttcgca gacaggcaga acttgatggt atgcctgcga 540
   tcaatgccaa acgtgtttac cggatcatgc gccagaatgc gctgttgctt gagcgaaaac 600
   ctgctgtacc gccatcgaaa cgggcacata caggcagagt ggccgtgaaa gaaagcaatc 660
   agcgatggtg ctctgacggg ttcgagttct gctgtgataa cggagagaga ctgcgtgtca 720
   cgttcgcgct ggactgctgt g 741
<210> 57
   <211> 1365
   <212> DNA
   <213> Homo sapiens
<220>
   <221> misc_feature
   <223> sequence of STAR57
<400> 57
   tccttctgta aataggcaaa atgtatttta gtttccacca cacatgttct tttctgtagg     60
   gcttgtatgt tggaaatttt atccaattat tcaattaaca ctataccaac aatctgctaa    120
   ttctggagat gtggcagtga ataaaaaagt tatagtttct gattttgtgg agcttggact    180
   ttaatgatgg acaaaacaac acattcttaa atatatattt catcaaaatt atagtgggtg    240
   aattatttat atgtgcattt acatgtgtat gtatacataa atgggcggtt actggctgca    300
   ctgagaatgt acacgtggcg cgaacgaggc tgggcggtca gagaaggcct cccaaggagg    360
   tggctttgaa gctgagtggt gcttccacgt gaaaaggctg gaaagggcat tccaagaaaa    420
   ggctgaggcc agcgggaaag aggttccagt gcgctctggg aacggaaagc gcacctgcct    480
   gaaacgaaaa tgagtgtgct gaaataggac gctagaaagg gaggcagagg ctggcaaaag    540
   cgaccgagga ggagctcaaa ggagcgagcg gggaaggccg ctgtggagcc tggaggaagc    600
   acttcggaag cgcttctgag cgggtaaggc cgctgggagc atgaactgct gagcaggtgt    660
   gtccagaatt cgtgggttct tggtctcact gacttcaaga atgaagaggg accgcggacc    720
   ctcgcggtga gtgttacagc tcttaaggtg gcgcgtctgg agtttgttcc ttctgatgtt    780
   cggatgtgtt cagagtttct tccttctggt gggttcgtgg tctcgctggc tcaggagtga    840
   agctgcagac cttcgcggtg agtgttacag ctcataaaag cagggtggac tcaaagagtg    900
   agcagcagca agatttattg caaagaatga aagaacaaag cttccacact gtggaagggg    960
   accccagcgg gttgccactg ctggctccgc agcctgcttt tattctctta tctggcccca   1020
   cccacatcct gctgattggt agagccgaat ggtctgtttt gacggcgctg attggtgcgt   1080
   ttacaatccc tgcgctagat acaaaggttc tccacgtccc caccagatta gctagataga   1140
   gtctccacac aaaggttctc caaggcccca ccagagtagc tagatacaga gtgttgattg   1200
   gtgcattcac aaaccctgag ctagacacag ggtgatgact ggtgtgttta caaaccttgc   1260
   ggtagataca gagtatcaat tggcgtattt acaatcactg agctaggcat aaaggttctc   1320
   caggtcccca ccagactcag gagcccagct ggcttcaccc agtgg                    1365
<210> 58
   <211> 1401
   <212> DNA
   <213> Homo sapiens
<220>
   <221> misc_feature
   <223> sequence of STAR58
<400> 58
   aagtttacct tagccctaaa ttatttcatt gtgattggca ttttaggaaa tatgtattaa     60
   ggaatgtctc ttaggagata aggataacat atgtctaaga aaattatatt gaaatattat    120
   tacatgaact aaaatgttag aactgaaaaa aaattattgt aactccttcc agcgtaggca    180
   ggagtatcta gataccaact ttaacaactc aactttaaca acttcgaacc aaccagatgg    240
   ctaggagatt cacctattta gcatgatatc ttttattgat aaaaaaatat aaaacttcca    300
   ttaaattttt aagctactac aatcctatta aattttaact taccagtgtt ctcaatgcta    360
   cataatttaa aatcattgaa atcttctgat tttaactcct cagtcttgaa atctacttat    420
   ttttagttac atatatatcc aatctactgc cgctagtaga agaagcttgg aatttgagaa    480
   aaaaatcaga cgttttgtat attctcatat tcactaattt attttttaaa tgagtttctg     540
   caatgcatca agcagtggca aaacaggaga aaaattaaaa ttggttgaaa agatatgtgt    600
   gccaaacaat cccttgaaat ttgatgaagt gactaatcct gagttattgt ttcaaatgtg    660
   tacctgttta tacaagggta tcacctttga aatctcaaca ttaaatgaaa ttttataagc    720
   aatttgttgt aacatgatta ttataaaatt ctgatataac attttttatt acctgtttag     780
   agtttaaaga gagaaaagga gttaagaata attacatttt cattagcatt gtccgggtgc    840
   aaaaacttct aacactatct tcaaatcttt ttctccattg ccttctgaac atacccactt    900
   gggtatctca ttagcactgc aaattcaaca ttttcgattg ctaatttttc tccctaaata    960
   tttatttgtt ttctcagctt tagccaatgt ttcactattg accatttgct caagtatagt   1020
   gacgcttcaa tgaccttcag agagctgttt cagtccttcc tggactactt gcatgcttcc   1080
   aacaaaatga agcactcttg atgtcagtca ctcaaataaa tggaaatggg cccatttact   1140
   aggaatgtta acagaataaa aagatagacg tgacaccagt tgcttcagtc catctccatt   1200
   tacttgctta aggcctggcc atatttctca cagttgatat ggcgcagggc acatgtttaa   1260
   atggctgttc ttgtaggatg gtttgactgt tggattcctc atcttccctc tccttaggaa   1320
   ggaaggttac agtagtactg ttggctcctg gaatatagat tcataaagaa ctaatggagt   1380
   atcatctccc actgctcttg t                                             1401
<210> 59
   <211> 866
   <212> DNA
   <213> Homo sapiens
<220>
   <221> misc_feature
   <223> sequence of STAR59
<400> 59
   gagatcacgc cactgcactc cagcctgggg gacagagcaa gactccatct cagaaacaaa 60
   caaacacaca aagccagtca aggtgtttaa ttcgacggtg tcaggctcag gtctcttgac 120
   aggatacatc cagcacccgg gggaaacgtc gatgggtggg gtggaatcta ttttgtggcc 180
   tcaagggagg gtttgagagg tagtcccgca agcggtgatg gcctaaggaa gcccctccgc 240
   ccaagaagcg atattcattt ctagcctgta gccacccaag agggagaatc gggctcgcca 300
   cagaccccac aacccccaac ccaccccacc cccacccctc ccacctcgtg aaatgggctc 360
   tcgctccgtc aggctctagt cacaccgtgt ggttttggaa cctccagcgt gtgtgcgtgg 420
   gttgcgtggt ggggtggggc cggctgtgga cagaggaggg gataaagcgg cggtgtcccg 480
   cgggtgcccg ggacgtgggg cgtggggcgt gggtggggtg gccagagcct tgggaactcg 540
   tcgcctgtcg ggacgtctcc cctcctggtc ccctctctga cctacgctcc acatcttcgc 600
   cgttcagtgg ggaccttgtg ggtggaagtc accatccctt tggactttag ccgacgaagg 660
   ccgggctccc aagagtctcc ccggaggcgg ggccttgggc aggctcacaa ggatgctgac 720
   ggtgacggtt ggtgacggtg atgtacttcg gaggcctcgg gccaatgcag aggtatccat 780
   ttgacctcgg tgggacaggt cagctttgcg gagtcccgtg cgtccttcca gagactcatc 840
   cagcgctagc aagcatggtc ccgagg 866
<210> 60
   <211> 2067
   <212> DNA
   <213> Homo sapiens
<220>
   <221> misc_feature
   <223> sequence of STAR60
<220>
   <221> misc_feature
   <222> (92) .. (1777)
   <223> n is a, c, g, or t on various positions
<400> 60
   agcagtgcag aactggggaa gaagaagagt ccctacacca cttaatactc aaaagtactc      60
   gcaaaaaata acacccctca ccaggtggca tnattactct ccttcattga gaaaattagg     120
   aaactggact tcgtagaagc taattgcttt atccagagcc acctgcatac aaacctgcag     180
   cgccacctgc atacaaacct gtcagccgac cccaaagccc tcagtcgcac caagcctctg     240
   ctgcacaccc tcgtgccttc acactggccg ttccccaagc ctggggcata ctncccagct     300
   ctgagaaatg tattcatcct tcaaagccct gctcatgtgt cctnntcaac aggaaaatct     360
   cccatgagat gctctgctat ccccatctct cctgccccat agcttaggca nacttctgtg     420
   gtggtgagtc ctgggctgtg ctgtgatgtg ttcgcctgcn atgtntgttc ttccccacaa     480
   tgatgggccc ctgaattctc tatctctagc acctgtgctc agtaaaggct tgggaaacca     540
   ggctcaaagc ctggcccaga tgccaccttt tccagggtgc ttccgggggc caccaaccag     600
   agtgcagcct tctcctccac caggaactct tgcagcccca cccctgagca cctgcacccc     660
   attacccatc tttgtttctc cgtgtgatcg tattattaca gaattatata ctgtattctt     720
   aatacagtat ataattgtat aattattctt aatacagtat ataattatac aaatacaaaa     780
   tatgtgttaa tggaccgttt atgttactgg taaagcttta agtcaacagt gggacattag     840
   ttaggttttt ggcgaagtca aaagttatat gtgcattttc aacttcttga ggggtcggta     900
   cntctnaccc ccatgttgtt caanggtcaa ctgtctacac atatcatagc taattcacta     960
   cagaaatgtt agcttgtgtc actagtatct ccccttctca taagcttaat acacatacct    1020
   tgagagagct cttggccatc tctactaatg actgaagttt ttatttatta tagatgtcat    1080
   aataggcata aaactacatt acatcattcg agtgccaatt ttgccacctt gaccctcttt    1140
   tgcaaaacac caacgtcagt acacatatga agaggaaact gcccgagaac tgaagttcct    1200
   gagaccagga gctgcaggcg ttagatagaa tatggtgacg agagttacga ggatgacgag    1260
   agtaaatact tcatactcag tacgtgccaa gcactgctat aagcgctctg tatgtgtgaa    1320
   gtcatttaat cctcacagca tcccacggtg taattatttt cattatcccc atgagggaac    1380
   agaaactcag aacggttcaa cacatatgcg agaagtcgca gccggtcagt gagagagcag    1440
   gttcccgtcc aagcagtcag accccgagtg cacactctcg acccctgtcc agcagactca    1500
   ctcgtcataa ggcggggagt gntctgtttc agccagatgc tttatgcatc tcagagtacc   1560
   caaaccatga aagaatgagg cagtattcan gagcagatgg ngctgggcag taaggctggg    1620
   cttcagaata gctggaaagc tcaagtnatg ggacctgcaa gaaaaatcca ttgtttngat   1680
   aaatagccaa agtccctagg ctgtaagggg aaggtgtgcc aggtgcaagt ggagctctaa   1740
   tgtaaaatcg cacctgagtc tcctggtctt atgagtnctg ggtgtacccc agtgaaaggt   1800
   cctgctgcca ccaagtgggc catggttcag ctgtgtaagt gctgagcggc agccggaccg   1860
   cttcctctaa cttcacctcc aaaggcacag tgcacctggt tcctccagca ctcagctgcg   1920
   aggcccctag ccagggtccc ggcccccggc ccccggcagc tgctccagct tccttcccca   1980
   cagcattcag gatggtctgc gttcatgtag acctttgttt tcagtctgtg ctccgaggtc   2040
   actggcagca ctagccccgg ctcctgt                                       2067
<210> 61
   <211> 1470
   <212> DNA
   <213> Homo sapiens
<220>
   <221> misc_feature
   <223> sequence of STAR61
<220>
   <221> misc_feature
   <222> (130)..(976)
   <223> n is a, c, g, or t on various positions
<400> 61
   cagcccccac atgcccagcc ctgtgctcag ctctgcagcg gggcatggtg ggcagagaca     60
   cagaggccaa ggccctgctt cggggacggt gggcctggga tgagcatggc cttggccttc    120
   gccgagagtn ctcttgtgaa ggaggggtca ggaggggctg ctgcagctgg ggaggagggc    180
   gatggcactg tggcangaag tgaantagtg tgggtgcctn gcaccccagg cacggccagc    240
   ctggggtatg gacccggggc cntctgttct agagcaggaa ggtatggtga ggacctcaaa    300
   aggacagcca ctggagagct ccaggcagag gnacttgaga ggccctgggg ccatcctgtc    360
   tcttttctgg gtctgtgtgc tctgggcctg ggcccttcct ctgctccccc gggcttggag    420
   agggctggcc ttgcctcgtg caaaggacca ctctagactg gtaccaagtc tggcccatgg    480
   cctcctgtgg gtgcaggcct gtgcgggtga cctgagagcc agggctggca ggtcagagtc    540
   aggagaggga tggcagtgga tgccctgtgc aggatctgcc taatcatggt gaggctggag    600
   gaatccaaag tgggcatgca ctctgcactc atttctttat tcatgtgtgc ccatcccaac    660
   aagcagggag cctggccagg agggcccctg ggagaaggca ctgatgggct gtgttccatt    720
   taggaaggat ggacggttgt gagacgggta agtcagaacg ggctgcccac ctcggccgag    780
   agggccccgt ggtgggttgg caccatctgg gcctggagag ctgctcagga ggctctctag    840
   ggctgggtga ccaggnctgg ggtacagtag ccatgggagc aggtgcttac ctggggctgt    900
   ccctgagcag gggctgcatt gggtgctctg tgagcacaca cttctctatt cacctgagtc    960
   ccnctgagtg atgagnacac ccttgttttg cagatgaatc tgagcatgga gatgttaagt   1020
   ggcttgcctg agccacacag cagatggatg gtgtagctgg gacctgaggg caggcagtcc   1080
   cagcccgagg acttcccaag gttgtggcaa actctgacag catgacccca gggaacaccc   1140
   atctcagctc tggtcagaca ctgcggagtt gtgttgtaac ccacacagct ggagacagcc   1200
   accctagccc cacccttatc ctctcccaaa ggaacctgcc ctttcccttc attttcctct   1260
   tactgcattg agggaccaca cagtgtggca gaaggaacat gggttcagga cccagatgga   1320
   cttgcttcac agtgcagccc tcctgtcctc ttgcagagtg cgtcttccac tgtgaagttg   1380
   ggacagtcac accaactcaa tactgctggg cccgtcacac ggtgggcagg caacggatgg   1440
   cagtcactgg ctgtgggtct gcagaggtgg                                    1470
<210> 62
   <211> 1011
   <212> DNA
   <213> Homo sapiens
<220>
   <221> misc_feature
   <223> sequence of STAR62
<400> 62
   agtgtcaaat agatctacac aaaacaagat aatgtctgcc catttttcca aagataatgt 60
   ggtgaagtgg gtagagagaa atgcatccat tctccccacc caacctctgc taaattgtcc 120
   atgtcacagt actgagacca gggggcttat tcccagcggg cagaatgtgc accaagcacc 180
   tcttgtctca atttgcagtc taggccctgc tatttgatgg tgtgaaggct tgcacctggc 240
   atggaaggtc cgttttgtac ttcttgcttt agcagttcaa agagcaggga gagctgcgag 300
   ggcctctgca gcttcagatg gatgtggtca gcttgttgga ggcgccttct gtggtccatt 360
   atctccagcc cccctgcggt gttgctgttt gcttggcttg tctggctctc catgccttgt 420
   tggctccaaa atgtcatcat gctgcacccc aggaagaatg tgcaggccca tctcttttat 480
   gtgctttggg ctattttgat tccccgttgg gtatattccc taggtaagac ccagaagaca 540
   caggaggtag ttgctttggg agagtttgga cctatgggta tgaggtaata gacacagtat 600
   cttctctttc atttggtgag actgttagct ctggccgcgg actgaattcc acacagctca 660
   cttgggaaaa ctttattcca aaacatagtc acattgaaca ttgtggagaa tgagggacag 720
   agaagaggcc ctagatttgt acatctgggt gttatgtcta taaatagaat gctttggtgg 780
   tcaactagac ttgttcatgt tgacatttag tcttgccttt tcggtggtga tttaaaaatt 840
   atgtatatct tgtttggaat atagtggagc tatggtgtgg cattttcatc tggctttttg 900
   tttagctcag cccgtcctgt tatgggcagc cttgaagctc agtagctaat gaagaggtat 960
   cctcactccc tccagagagc ggtcccctca cggctcattg agagtttgtc a 1011
<210> 63
   <211> 1410
   <212> DNA
   <213> Homo sapiens
<220>
   <221> misc_feature
   <223> sequence of STAR63
<400> 63
   ccacagcctg atcgtgctgt cgatgagagg aatctgctct aagggtctga gcggagggag      60
   atgccgaagc tttgagcttt ttgtttctgg cttaaccttg gtggattttc accctctggg     120
   cattacctct tgtccagggg aggggctggg ggagtgcctg gagctgtagg gacagagggc     180
   tgagtggggg ggactgcttg ggctgaccac ataatattct gctgcgtatt aatttttttt     240
   tgagacagtc tttctctgtt gcccaggctg gagtgtaatg gcttgatagc tcactgccac     300
   ctccgcctcc tgggttcaag tgattctcct gcttcagctt ccggagtagc tgggactgca     360
   ggtgcccgcc accatggctg gctaattttt gtatttttat tagcaatggg gttttgctat     420
   gttgcccagg ccggtcccga actcctgccc tcaagtgata cacctgcctc ggcctcccaa     480
   agtgctggga ttagaggctt gagccactgc gcctggccag ctgcatattg ttaattagac     540
   ataaaatgca aaataagatg atataaacac aaaggtgtga aataagatgg acacctgctg     600
   agcgcgcctg tcctgaagca tcgcccctct gcaaaagcag gggtcagcat gtgttctccg     660
   gtccttgctc ttacagagga gtgagctgcc tatgcgtctt ccagccactt cctgggctgc     720
   tcagaggcct ctcacgggtg ttctgggttg ctgccacttg caggggtgct gaggcggggc     780
   tcctcccgtg cggggcatgt ccaggccgcc ctctctgaag gcttggcagg tacaggtggg     840
   agtgggggtc tctgggctgc tgtggggact gggcaggctc ctggaagacc tccctgtgtt     900
   tgggctgaaa gcgcagcccg aggggaggtc cccagggagg ccgctgtcgg gggtgggggc      960
   ttggaggagg gaggggccga ggagccggcg acactccgtg acggcccagg aacgtcccta    1020
   aacaaggcgc cgcgttctcg atggggtggg gtccgctttc ttttctcaaa agctgcagtt    1080
   actccatgct cggaggactg gcgtccgcgc cctgttccaa tgctgccccg gggccctggc    1140
   cttggggaat cggggccttg gactggaccc tgggggcttc gcggagccgg gcctggcggg    1200
   gcgagcggag cagaggctgg gcagccccgg ggaagcgctc gccaaagccg ggcgctgctc    1260
   ccagagcgcg aggtgcagaa ccagaggctg gtcccgcggc gctaacgaga gaagaggaag    1320
   cgcgctgtgt agagggcgcc caccccgtgg ggcgaacccc cttcctcaac tccatggacg    1380
   gggctcatgg gttcccagcg gctcagacgc                                     1410
<210> 64
   <211> 1414
   <212> DNA
   <213> Homo sapiens
<220>
   <221> misc_feature
   <223> sequence of STAR64
<400> 64
   tggatcagat ttgttttata ccctcccttc tactgctctg agagttgtac atcacagtct 60
   actgtatctg tttcccatta ttataatttt tttgcactgt gcttgcctga agggagcctc 120
   aagttcatga gtctccctac cctcctccca aatgagacat ggacctttga atgctttcct 180
   gggaccacca ccccaccttt catgctgctg ttatccagga ttttagttca acagtgtttt 240
   aaccccccaa atgagtcatt tttattgttt cgtatagtga atgtgtattt gggtttgctt 300
   atatggtgac ctgtttattt gctcctcatt gtacctcatg ctctgctctt tccttctaga 360
   ttcagtctct ttcctaatga ggtgtctcgc agcaattctt tacaagacag ccaagatagg 420
   ccagctctca gagcacttgt tgtctgaaaa agtcttgtct tatttaattt ctttttctta 480
   gagatggggt ctcattatgt tacccacact ggtctcaaac ttctggctta aagcggtcct 540
   cccaccttgg cctcccaaag tgctaggatt acaggcgtga gcgacctcgt ccagcctgtc 600
   tgagaaagcg tttgttttgc ccttgctctc agatgacagt ttggggatag aattctaggt 660
   ggacggtttt tttccttcag ccctttgaag agtctgtatt ttcattatct ccctgcatta 720
   gatgttcttt tgcaagtaac gtgtcttttc tctctgggta ttcttaaggt tttctctttg 780
   cctttggtga gctgcagtgg atttgctttt ttcaagaggt caagagaaag gaaagtgtga 840
   ggtttctgtt ttttactgac aatttgtttg ttgatttgtt ttcccaccca gaggttcctt 900
   gccactttgc caggctggaa ggcagacttc ttctggtgtc ctgttcacag acggggcagc 960
   ctgcggaagg ccctgccaca tgcagggcct cggtcctcat tcccttgcat gtggacccgg 1020
   gcgtgactcc tgttcaggct ggcacttccc agagctgagc cccagcctga ccttcctccc 1080
   atactgtctt cacaccccct cctttcttct gatacctgga ggttttcctt tctttcctgt 1140
   cacctccact tggattttaa atcctctgtc tgtggaattg tattcggcac aggaagatgc 1200
   ttgcaagggc caggctcatc agccctgtcc ctgctgctgg aagcagcaca gcagagcctc 1260
   atgctcaggc tgagatggag cagaggcctg cagacgagca cccagctcag ctggggttgg 1320
   cgccgatggt ggagggtcct cgaaagctct ggggacgatg gcagagctat tggcagggga 1380
   gccgcagggt cttttgagcc cttaaaagat ctct 1414
<210> 65
   <211> 1310
   <212> DNA
   <213> Homo sapiens
<220>
   <221> misc_feature
   <223> sequence of STAR65
<400> 65
   gtgaatgttg atggatcaaa tatctttctg tgttgtttat caaagttaaa ataaatgtgg 60
   tcatttaaag gacaaaagat gaggggttgg agtctgttca agcaaagggt atattaggag 120
   aaaagcagaa ttctctccct gtgaagggac agtgactcct attttccacc tcatttttac 180
   taactctcct aactatctgc ttaggtagag atatatccat gtacatttat aaaccacagt 240
   gaatcatttg attttggaat aaagatagta taaaatgtgt cccagtgttg atatacatca 300
   tacattaaat atgtctggca gtgttctaat tttacagttg tccaaagata atgttagggc 360
   atactggcta tggatgaagc tccaatgttc agattgcaaa gaaacttaga attttactaa 420
   tgaaaccaaa tacatcccaa gaaatttttc agaagaaaaa aagagaaact agtagcaaag 480
   taaagaatca ccacaatatc atcagatttt ttttatatgt agaatattta ttcagttctt 540
   ttttcaagta caccttgtct tcattcattg tactttattt tttgtgaagg tttaaattta 600
   tttcttctat gtgtttagtg atatttaaaa tttttattta atcaagttta tcagaaagtt 660
   ctgttagaaa atatgacgag gctttaattc cgccatctat attttccgct attatataaa 720
   gataattgtt ttctcttttt aaaacaactt gaattgggat tttatatcat aattttttaa 780
   tgtctttttt tattatactt taagttctgg gatacatgtg cagaacgtgc aggtgtgtta 840
   catagatata cacgtgccat ggtggtttgc tgcacccact aacctgttat cgacattagg 900
   tatttctcct aatgctatca ccccctattt ccccaccccc cgagaggccc cagtgtgtga 960
   tgttctcctc cctgtgtcca tgtgttctca ttgttcatct cccacttatg gtatctacca 1020
   taaccttgaa attgtcttat gcattcactt gtttggttgt tatatagcct ccatcaggac 1080
   agggatattt gctgctgctt cttttttttt tctttttgag acagtcttgc tccgtcatcc 1140
   aggctggagt gcttctcggc tcaatgcaac ctccacctcc caggtttaag cgattctcca 1200
   acttcagcct cccaaatggc tgggactgca ggcatgcacc actacacctg gctaattttt 1260
   gtatttgtaa tagagacaat gtttcaccat gttggccagg ctggtctcga 1310
<210> 66
   <211> 1917
   <212> DNA
   <213> Homo sapiens
<220>
   <221> misc_feature
   <223> sequence of STAR67
<400> 66
   aggatcctaa aattttgtga ccctagagca agtactaact atgaaagtga aatagagaat 60
   gaaggaatta tttaattaag tccagcaaaa cccaaccaaa tcatctgtaa aatatatttg 120
   ttttcaacat ccaggtattt tctgtgtaaa aggttgagtt gtatgctgac ttattgggaa 180
   aaataattga gttttcccct tcactttgcc agtgagagga aatcagtact gtaattgtta 240
   aaggttaccc atacctacct ctactaccgt ctagcatagg taaagtaatg tacactgtga 300
   agtttcctgc ttgactgtaa tgttttcagt ttcatcccat tgattcaaca gctatttatt 360
   cagcacttac tacaaccatg ctggaaaccc aagagtaaat aggctgtgtt actcaacagg 420
   actgaggtac agccgaactg tcaggcaagg ttgctgtcct ttggacttgc ctgctttctc 480
   tctatgtagg aagaagaaat ggacataccg tccaggaaat agatatatgt tacatttcct 540
   tattccataa ttaatattaa taaccctgga cagaaactac caagtttcta gacccttata 600
   gtaccacctt accctttctg gatgaatcct tcacatgttg atacatttta tccaaatgaa 660
   aattttggta ctgtaggtat aacagacaaa gagagaacag aaaactagag atgaagtttg 720
   ggaaaaggtc aagaaagtaa ataatgcttc tagaagacac aaaaagaaaa atgaaatggt 780
   aatgttggga aagttttaat acattttgcc ctaaggaaaa aaactacttg ttgaaattct 840
   acttaagact ggaccttttc tctaaaaatt gtgcttgatg tgaattaaag caacacaggg 900
   aaatttatgg gctccttcta agttctaccc aactcaccgc aaaactgttc ctagtaggtg    960
   tggtatactc tttcagattc tttgtgtgta tgtatatgtg tgtgtgtgtg tgtgtttgta   1020
   tgtgtacagt ctatatacat atgtgtacct acatgtgtgt atatataaat atatatttac   1080
   ctggatgaaa tagcatatta tagaatattc tttttctttt aaatatatat gtgcatacat   1140
   atgtatatgc acatatatac ataaatgtag atatagctag gtaggcattc atgtgaaaca   1200
   aagaagccta ttacttttta atggttgcat gatattccat cataggagta tagtacaact   1260
   tatgtaacac acatttggct tgttgtaaaa ttttggtatt aataaaatag cacatatcat   1320
   gcaaagacac ccttgcatag gtctattcat tctttgattt ttaccttagg acaaaattta   1380
   aaagtagaat ttctgggtca agcagtatgc tcatttaaaa tgtcattgca tatttccaaa   1440
   ttgtcctcca gaaaagtagt aacagtaaca attgatggac tgcgtgtttt ctaaaacttg   1500
   catttttttc cttattggtg aggtttggca ttttccatat gtttattggc attttaattt   1560
   tttttggttc atgtctttta ttcccttcct gcaaatttgt ggtgtgtctc aactttattt   1620
   atactctcat tttcataatt ttctaaagga atttgacttt aaaaaaataa gacagccaat   1680
   gctttggttt aatttcattg ctgctttttg aagtgactgc tgtgttttta tatactttta   1740
   tattttgttg ttttagcaaa ttcttctata ttataattgt gtatgctgga acaaaaagtt   1800
   atatttctta atctagataa aatatttcaa gatgttgtaa ttacagtccc ctctaaaatc   1860
   atataaatag acgcatagct gtgtgatttg taattagtta tgtccattga tagatcc      1917
<210> 67
   <211> 26
   <212> DNA
   <213> Artificial
<220>
   <223> oligo for making linker containing MCSII of pd2EGFP-link
<400> 67
   gtacggatat cagatcttta attaag 26
<210> 68
   <211> 24
   <212> DNA
   <213> Artificial
<220>
   <223> oligo for making linker containing MCSII of pd2EGFP-link
<400> 68
   gtaccttaat taaagatctg atat 24
<210> 69
   <211> 52
   <212> DNA
   <213> Artificial
<220>
   <223> primer for amplification of 0.37 kb from pd2EGFP
<400> 69
   gatcagatct ggcgcgccat ttaaatcgtc tcgcgcgttt cggtgatgac gg 52
<210> 70
   <211> 41
   <212> DNA
   <213> Artificial
<220>
   <223> primer for amplification of 0.37 kb from pd2EGFP
<400> 70
   aggcggatcc gaatgtattt agaaaaataa acaaataggg g 41
<210> 71
   <211> 36
   <212> DNA
   <213> Artificial
<220>
   <223> primer for amplifying zeocin resistance gene ORF
<400> 71
   gatcggatcc ttcgaaatgg ccaagttgac cagtgc 36
<210> 72
   <211> 32
   <212> DNA
   <213> Artificial
<220>
   <223> primer for amplifying zeocin resistance gene ORF
<400> 72
   aggcgcggcc gcaattctca gtcctgctcc tc 32
<210> 73
   <211> 40
   <212> DNA
   <213> Artificial
<220>
   <223> primer for amplifying d2EGFP ORF
<400> 73
   gatcgaattc tcgcgaatgg tgagcaagca gatcctgaag 40
<210> 74
   <211> 52
   <212> DNA
   <213> Artificial
<220>
   <223> primer for amplifying d2EGFP ORF
<400> 74
   aggcgaattc accggtgttt aaacttacac ccactcgtgc aggctgccca gg 52
<210> 75
   <211> 41
   <212> DNA
   <213> Artificial
<220>
   <223> primer for amplifying d2EGFP gene
<400> 75
   ttggttggtc atgaatggtg agcaagggcg aggagctgtt c 41
<210> 76
   <211> 36
   <212> DNA
   <213> Artificial
<220>
   <223> primer for amplifying d2EGFP gene
<400> 76
   attctctaga ctacacattg atcctagcag aagcac 36
<210> 77
   <211> 42
   <212> DNA
   <213> Artificial
<220>
   <223> oligo for preparing linker for creating MCS in pGL3-promoter-GFP
<400> 77
   cgatatcttg gagatctact agtggcgcgc cttgggctag ct 42
<210> 78
   <211> 50
   <212> DNA
   <213> Artificial
<220>
   <223> oligo for preparing linker for creating MCS in pGL3-promoter-GFP
<400> 78
   gatcagctag cccaaggcgc gccactagta gatctccaag atatcgagct 50
<210> 79
   <211> 40
   <212> DNA
   <213> Artificial
<220>
   <223> primer for amplification of EF-1alfa promoter
<400> 79
   gatcggcgcg ccatttaaat ccgaaaagtg ccacctgacg 40
<210> 80
   <211> 34
   <212> DNA
   <213> Artificial
<220>
   <223> primer for amplification of EF-1alfa promoter
<400> 80
   aggcgggacc ccctcacgac acctgaaatg gaag 34
<210> 81
   <211> 51
   <212> DNA
   <213> Artificial
<220>
   <223> primer for amplification of SV40 promoter
<400> 81
   ttggttgggg cgcgccgcag caccatggcc tgaaataacc tctgaaagag g 51
<210> 82
   <211> 55
   <212> DNA
   <213> Artificial
<220>
   <223> primer for amplification of SV40 promoter
<400> 82
   ttggttggga gctcaagctt tttgcaaaag cctaggcctc caaaaaagcc tcctc 55
<210> 83
   <211> 11
   <212> DNA
   <213> Artificial
<220>
   <223> sequence around startcodon of wild-type zeocin resistance gene
<400> 83
   aaaccatggc c 11
<210> 84
   <211> 50
   <212> DNA
   <213> Artificial
<220>
   <223> primer ZEOforwardMUT
<400> 84
   gatctcgcga tacaggattt atgttggcca agttgaccag tgccgttccg 50
<210> 85
   <211> 26
   <212> DNA
   <213> Artificial
<220>
   <223> primer ZEO-WTreverse
<400> 85
   aggcgaattc agtcctgctc ctcggc 26
<210> 86
   <211> 45
   <212> DNA
   <213> Artificial
<220>
   <223> primer ZEO-LEUreverse
<400> 86
   aggccccgcc cccacggctg ctcgccgatc tcggtcaagg ccggc 45
<210> 87
   <211> 45
   <212> DNA
   <213> Artificial
<220>
   <223> primer ZEO-THRreverse
<400> 87
   aggccccgcc cccacggctg ctcgccgatc tcggtggtgg ccggc 45
<210> 88
   <211> 44
   <212> DNA
   <213> Artificial
<220>
   <223> primer ZEO-VALreverse
<400> 88
   aggccccgcc cccacggctg ctcgccgatc tcggtccacg ccgg 44
<210> 89
   <211> 11
   <212> DNA
   <213> Artificial
<220>
   <223> sequence around startcodon of wt d2EGFP
<400> 89
   gaattcatgg g 11
<210> 90
   <211> 49
   <212> DNA
   <213> Artificial
<220>
   <223> primer d2EGFPforwardBamHI
<400> 90
   gatcggatcc tatgaggaat tcgccaccat ggtgagcaag ggcgaggag 49
<210> 91
   <211> 41
   <212> DNA
   <213> Artificial
<220>
   <223> primer d2EGFPreverseNotI
<400> 91
   aaggaaaaaa gcggccgcct acacattgat cctagcagaa g 41
<210> 92
   <211> 57
   <212> DNA
   <213> Artificial
<220>
   <223> spacer sequence
<400> 92
   tcgatccaaa gactgccaaa tctagatccg agattttcag gagctaagga agctaaa 57
<210> 93
   <211> 99
   <212> DNA
   <213> Artificial
<220>
   <223> primer ZEOforwardBamHI-ATGmut/space
<400> 93
   gatcggatcc ttggtttatg tcgatccaaa gactgccaaa tctagatccg agattttcag 60
   gagctaagga agctaaagcc aagttgacca gtgaagttc 99
<210> 94
   <211> 38
   <212> DNA
   <213> Artificial
<220>
   <223> primer ZEOforwardBamHI-GTG
<400> 94
   gatcggatcc accgtggcca agttgaccag tgccgttc 38
<210> 95
   <211> 38
   <212> DNA
   <213> Artificial
<220>
   <223> primer ZEOforwardBamHI-TTG
<400> 95
   gatcggatcc accttggcca agttgaccag tgccgttc 38
<210> 96
   <211> 35
   <212> DNA
   <213> Artificial
<220>
   <223> primer BSDBamHIforward
<400> 96
   gatcggatcc accatggcca agcctttgtc tcaag 35
<210> 97
   <211> 25
   <212> DNA
   <213> Artificial
<220>
   <223> primer BSD150reverse
<400> 97
   gtaaaatgat atacgttgac accag 25
<210> 98
   <211> 25
   <212> DNA
   <213> Artificial
<220>
   <223> primer BSD150forward
<400> 98
   ctggtgtcaa cgtatatcat tttac 25
<210> 99
   <211> 24
   <212> DNA
   <213> Artificial
<220>
   <223> primer BSD250reverse
<400> 99
   gccctgttct cgtttccgat cgcg 24
<210> 100
   <211> 24
   <212> DNA
   <213> Artificial
<220>
   <223> primer BSD250forward
<400> 100
   cgcgatcgga aacgagaaca gggc 24
<210> 101
   <211> 24
   <212> DNA
   <213> Artificial
<220>
   <223> primer BSD350reverse
<400> 101
   gccgtcggct gtccgtcact gtcc 24
<210> 102
   <211> 24
   <212> DNA
   <213> Artificial
<220>
   <223> primer BSD350forward
<400> 102
   ggacagtgac ggacagccga cggc 24
<210> 103
   <211> 38
   <212> DNA
   <213> Artificial
<220>
   <223> primer BSD399reverse
<400> 103
   gatcgaattc ttagccctcc cacacgtaac cagagggc 38
<210> 104
   <211> 103
   <212> DNA
   <213> Artificial
<220>
   <223> primer BSDforwardBamHIAvrII-ATGmut/space
<400> 104
   gatcggatcc taggttggtt tatgtcgatc caaagactgc caaatctaga tccgagattt 60
   tcaggagcta aggaagctaa agccaagcct ttgtctcaag aag 103
<210> 105
   <211> 44
   <212> DNA
   <213> Artificial
<220>
   <223> primer BSD399reverseEcoRIAvrII
<400> 105
   gatcgaattc cctaggttag ccctcccaca cgtaaccaga gggc 44
<210> 106
   <211> 42
   <212> DNA
   <213> Artificial
<220>
   <223> primer BSDforwardBamHIAvrII-GTG
<400> 106
   gatcggatcc taggaccgtg gccaagcctt tgtctcaaga ag 42
<210> 107
   <211> 42
   <212> DNA
   <213> Artificial
<220>
   <223> primer BSDforwardBamHIAvrII-TTG
<400> 107
   gatcggatcc taggaccttg gccaagcctt tgtctcaaga ag 42
<210> 108
   <211> 375
   <212> DNA
   <213> Artificial
<220>
   <223> wt zeocin resistance gene
<220>
   <221> CDS
   <222> (1)..(375)
<400> 108
   atg gcc aag ttg acc agt gcc gtt ccg gtg ctc acc gcg cgc gac gtc       48
   Met Ala Lys Leu Thr Ser Ala Val Pro Val Leu Thr Ala Arg Asp Val
   1               5                   10                  15
   gcc gga gcg gtc gag ttc tgg acc gac cgg ctc ggg ttc tcc cgg gac       96
   Ala Gly Ala Val Glu Phe Trp Thr Asp Arg Leu Gly Phe Ser Arg Asp
               20                  25                  30
   ttc gtg gag gac gac ttc gcc ggt gtg gtc cgg gac gac gtg acc ctg      144
   Phe Val Glu Asp Asp Phe Ala Gly Val Val Arg Asp Asp Val Thr Leu
           35                  40                  45
   ttc atc agc gcg gtc cag gac cag gtg gtg ccg gac aac acc ctg gcc      192
   Phe Ile Ser Ala Val Gln Asp Gln Val Val Pro Asp Asn Thr Leu Ala
       50                  55                  60
   tgg gtg tgg gtg cgc ggc ctg gac gag ctg tac gcc gag tgg tcg gag      240
   Trp Val Trp Val Arg Gly Leu Asp Glu Leu Tyr Ala Glu Trp Ser Glu
   65                  70                  75                  80
   gtc gtg tcc acg aac ttc cgg gac gcc tcc ggg ccg gcc atg acc gag      288
   Val Val Ser Thr Asn Phe Arg Asp Ala Ser Gly Pro Ala Met Thr Glu
                   85                  90                  95
   atc ggc gag cag ccg tgg ggg cgg gag ttc gcc ctg cgc gac ccg gcc      336
   Ile Gly Glu Gln Pro Trp Gly Arg Glu Phe Ala Leu Arg Asp Pro Ala
               100                 105                 110
   ggc aac tgc gtg cac ttc gtg gcc gag gag cag gac tga                  375
   Gly Asn Cys Val His Phe Val Ala Glu Glu Gln Asp
           115                 120
<210> 109
   <211> 124
   <212> PRT
   <213> Artificial
<220>
   <223> wt zeocin resistance gene
<400> 109
   Met Ala Lys Leu Thr Ser Ala Val Pro Val Leu Thr Ala Arg Asp Val
   1               5                   10                  15
   Ala Gly Ala Val Glu Phe Trp Thr Asp Arg Leu Gly Phe Ser Arg Asp
               20                  25                  30
   Phe Val Glu Asp Asp Phe Ala Gly Val Val Arg Asp Asp Val Thr Leu
           35                  40                  45
   Phe Ile Ser Ala Val Gln Asp Gln Val Val Pro Asp Asn Thr Leu Ala
       50                  55                  60
   Trp Val Trp Val Arg Gly Leu Asp Glu Leu Tyr Ala Glu Trp Ser Glu
   65                  70                  75                  80
   Val Val Ser Thr Asn Phe Arg Asp Ala Ser Gly Pro Ala Met Thr Glu
                   85                  90                  95
   Ile Gly Glu Gln Pro Trp Gly Arg Glu Phe Ala Leu Arg Asp Pro Ala
               100                 105                 110
   Gly Asn Cys Val His Phe Val Ala Glu Glu Gln Asp
           115                 120
<210> 110
   <211> 399
   <212> DNA
   <213> Artificial
<220>
   <223> wt blasticidin resistance gene
<220>
   <221> CDS
   <222> (1)..(399)
<400> 110
   atg gcc aag cct ttg tct caa gaa gaa tcc acc ctc att gaa aga gca      48
   Met Ala Lys Pro Leu Ser Gln Glu Glu Ser Thr Leu Ile Glu Arg Ala
   1               5                   10                  15
   acg gct aca atc aac agc atc ccc atc tct gaa gac tac agc gtc gcc      96
   Thr Ala Thr Ile Asn Ser Ile Pro Ile Ser Glu Asp Tyr Ser Val Ala
               20                  25                  30
   agc gca gct ctc tct agc gac ggc cgc atc ttc act ggt gtc aat gta      144
   Ser Ala Ala Leu Ser Ser Asp Gly Arg Ile Phe Thr Gly Val Asn Val
           35                  40                  45
   tat cat ttt act ggg gga cct tgt gca gaa ctc gtg gtg ctg ggc act      192
   Tyr His Phe Thr Gly Gly Pro Cys Ala Glu Leu Val Val Leu Gly Thr
       50                  55                  60
   gct gct gct gcg gca gct ggc aac ctg act tgt atc gtc gcg atc gga      240
   Ala Ala Ala Ala Ala Ala Gly Asn Leu Thr Cys Ile Val Ala Ile Gly
   65                  70                  75                  80
   aat gag aac agg ggc atc ttg agc ccc tgc gga cgg tgc cga cag gtg      288
   Asn Glu Asn Arg Gly Ile Leu Ser Pro Cys Gly Arg Cys Arg Gln Val
                   85                  90                  95
   ctt ctc gat ctg cat cct ggg atc aaa gcc ata gtg aag gac agt gat      336
   Leu Leu Asp Leu His Pro Gly Ile Lys Ala Ile Val Lys Asp Ser Asp
               100                 105                 110
   gga cag ccg acg gca gtt ggg att cgt gaa ttg ctg ccc tct ggt tat      384
   Gly Gln Pro Thr Ala Val Gly Ile Arg Glu Leu Leu Pro Ser Gly Tyr
           115                 120                 125
   gtg tgg gag ggc taa                                                  399
   Val Trp Glu Gly
       130
<210> 111
   <211> 132
   <212> PRT
   <213> Artificial
<220>
   <223> wt blasticidin resistance gene
<400> 111
   Met Ala Lys Pro Leu Ser Gln Glu Glu Ser Thr Leu Ile Glu Arg Ala
   1               5                   10                  15
   Thr Ala Thr Ile Asn Ser Ile Pro Ile Ser Glu Asp Tyr Ser Val Ala
               20                  25                  30
   Ser Ala Ala Leu Ser Ser Asp Gly Arg Ile Phe Thr Gly Val Asn Val
               35                  40                  45
   Tyr His Phe Thr Gly Gly Pro Cys Ala Glu Leu Val Val Leu Gly Thr
       50                  55                  60
   Ala Ala Ala Ala Ala Ala Gly Asn Leu Thr Cys Ile Val Ala Ile Gly
   65                  70                  75                  80
   Asn Glu Asn Arg Gly Ile Leu Ser Pro Cys Gly Arg Cys Arg Gln Val
                   85                  90                  95
   Leu Leu Asp Leu His Pro Gly Ile Lys Ala Ile Val Lys Asp Ser Asp
               100                 105                 110
   Gly Gln Pro Thr Ala Val Gly Ile Arg Glu Leu Leu Pro Ser Gly Tyr
           115                 120                 125
   Val Trp Glu Gly
       130
<210> 112
   <211> 600
   <212> DNA
   <213> Artificial
<220>
   <223> wt puromycin resistance gene
<220>
   <221> CDS
   <222> (1)..(600)
<400> 112
   atg acc gag tac aag ccc acg gtg cgc ctc gcc acc cgc gac gac gtc        48
   Met Thr Glu Tyr Lys Pro Thr Val Arg Leu Ala Thr Arg Asp Asp Val
   1               5                   10                  15
   ccc agg gcc gta cgc acc ctc gcc gcc gcg ttc gcc gac tac ccc gcc        96
   Pro Arg Ala Val Arg Thr Leu Ala Ala Ala Phe Ala Asp Tyr Pro Ala
               20                  25                  30
   acg cgc cac acc gtc gat ccg gac cgc cac atc gag cgg gtc acc gag       144
   Thr Arg His Thr Val Asp Pro Asp Arg His Ile Glu Arg Val Thr Glu
           35                  40                  45
   ctg caa gaa ctc ttc ctc acg cgc gtc ggg ctc gac atc ggc aag gtg       192
   Leu Gln Glu Leu Phe Leu Thr Arg Val Gly Leu Asp Ile Gly Lys Val
       50                  55                  60
   tgg gtc gcg gac gac ggc gcc gcg gtg gcg gtc tgg acc acg ccg gag       240
   Trp Val Ala Asp Asp Gly Ala Ala Val Ala Val Trp Thr Thr Pro Glu
   65                  70                  75                  80
   agc gtc gaa gcg ggg gcg gtg ttc gcc gag atc ggc ccg cgc atg gcc       288
   Ser Val Glu Ala Gly Ala Val Phe Ala Glu Ile Gly Pro Arg Met Ala
                   85                  90                  95
   gag ttg agc ggt tcc cgg ctg gcc gcg cag caa cag atg gaa ggc ctc       336
   Glu Leu Ser Gly Ser Arg Leu Ala Ala Gln Gln Gln Met Glu Gly Leu
               100                 105                 110
   ctg gcg ccg cac cgg ccc aag gag ccc gcg tgg ttc ctg gcc acc gtc       384
   Leu Ala Pro His Arg Pro Lys Glu Pro Ala Trp Phe Leu Ala Thr Val
           115                 120                 125
   ggc gtc tcg ccc gac cac cag ggc aag ggt ctg ggc agc gcc gtc gtg       432
   Gly Val Ser Pro Asp His Gln Gly Lys Gly Leu Gly Ser Ala Val Val
       130                 135                 140
   ctc ccc gga gtg gag gcg gcc gag cgc gcc ggg gtg ccc gcc ttc ctg       480
   Leu Pro Gly Val Glu Ala Ala Glu Arg Ala Gly Val Pro Ala Phe Leu
   145                 150                 155                 160
   gag acc tcc gcg ccc cgc aac ctc ccc ttc tac gag cgg ctc ggc ttc       528
   Glu Thr Ser Ala Pro Arg Asn Leu Pro Phe Tyr Glu Arg Leu Gly Phe
                   165                 170                 175
   acc gtc acc gcc gac gtc gag tgc ccg aag gac cgc gcg acc tgg tgc       576
   Thr Val Thr Ala Asp Val Glu Cys Pro Lys Asp Arg Ala Thr Trp Cys
               180                 185                 190
   atg acc cgc aag ccc ggt gcc tga                                       600
   Met Thr Arg Lys Pro Gly Ala
           195
<210> 113
   <211> 199
   <212> PRT
   <213> Artificial
<220>
   <223> wt puromycin resistance gene
<400> 113
   Met Thr Glu Tyr Lys Pro Thr Val Arg Leu Ala Thr Arg Asp Asp Val
   1               5                   10                  15
   Pro Arg Ala Val Arg Thr Leu Ala Ala Ala Phe Ala Asp Tyr Pro Ala
               20                  25                  30
   Thr Arg His Thr Val Asp Pro Asp Arg His Ile Glu Arg Val Thr Glu
           35                  40                  45
   Leu Gln Glu Leu Phe Leu Thr Arg Val Gly Leu Asp Ile Gly Lys Val
       50                  55                  60
   Trp Val Ala Asp Asp Gly Ala Ala Val Ala Val Trp Thr Thr Pro Glu
   65                  70                  75                  80
   Ser Val Glu Ala Gly Ala Val Phe Ala Glu Ile Gly Pro Arg Met Ala
                   85                  90                  95
   Glu Leu Ser Gly Ser Arg Leu Ala Ala Gln Gln Gln Met Glu Gly Leu
               100                 105                 110
   Leu Ala Pro His Arg Pro Lys Glu Pro Ala Trp Phe Leu Ala Thr Val
           115                 120                 125
   Gly Val Ser Pro Asp His Gln Gly Lys Gly Leu Gly Ser Ala Val Val
       130                 135                 140
   Leu Pro Gly Val Glu Ala Ala Glu Arg Ala Gly Val Pro Ala Phe Leu
   145                 150                 155                 160
   Glu Thr Ser Ala Pro Arg Asn Leu Pro Phe Tyr Glu Arg Leu Gly Phe
                   165                 170                 175
   Thr Val Thr Ala Asp Val Glu Cys Pro Lys Asp Arg Ala Thr Trp Cys
               180                 185                 190
   Met Thr Arg Lys Pro Gly Ala
           195
<210> 114
   <211> 564
   <212> DNA
   <213> Artificial
<220>
   <223> wt DHFR gene (from mouse)
<220>
   <221> CDS
   <222> (1) .. (569)
<400> 114
   atg gtt cga cca ttg aac tgc atc gtc gcc gtg tcc caa aat atg ggg      48
   Met Val Arg Pro Leu Asn Cys Ile Val Ala Val Ser Gln Asn Met Gly
   1               5                   10                  15
   att ggc aag aac gga gac cta ccc tgg cct ccg ctc agg aac gag ttc      96
   Ile Gly Lys Asn Gly Asp Leu Pro Trp Pro Pro Leu Arg Asn Glu Phe
               20                  25                  30
   aag tac ttc caa aga atg acc aca acc tct tca gtg gaa ggt aaa cag     144
   Lys Tyr Phe Gln Arg Met Thr Thr Thr Ser Ser Val Glu Gly Lys Gln
           35                  40                  45
   aat ctg gtg att atg ggt agg aaa acc tgg ttc tcc att cct gag aag     192
   Asn Leu Val Ile Met Gly Arg Lys Thr Trp Phe Ser Ile Pro Glu Lys
       50                  55                  60
   aat cga cct tta aag gac aga att aat ata gtt ctc agt aga gaa ctc     240
   Asn Arg Pro Leu Lys Asp Arg Ile Asn Ile Val Leu Ser Arg Glu Leu
   65                  70                  75                  80
   aaa gaa cca cca cga gga gct cat ttt ctt gcc aaa agt ttg gat gat     288
   Lys Glu Pro Pro Arg Gly Ala His Phe Leu Ala Lys Ser Leu Asp Asp
                   85                  90                  95
   gcc tta aga ctt att gaa caa ccg gaa ttg gca agt aaa gta gac atg     336
   Ala Leu Arg Leu Ile Glu Gln Pro Glu Leu Ala Ser Lys Val Asp Met
               100                 105                 110
   gtt tgg ata gtc gga ggc agt tct gtt tac cag gaa gcc atg aat caa     384
   Val Trp Ile Val Gly Gly Ser Ser Val Tyr Gln Glu Ala Met Asn Gln
           115                 120                 125
   cca ggc cac ctc aga ctc ttt gtg aca agg atc atg cag gaa ttt gaa     432
   Pro Gly His Leu Arg Leu Phe Val Thr Arg Ile Met Gln Glu Phe Glu
       130                 135                 140
   agt gac acg ttt ttc cca gaa att gat ttg ggg aaa tat aaa ctt ctc     480
   Ser Asp Thr Phe Phe Pro Glu Ile Asp Leu Gly Lys Tyr Lys Leu Leu
   145                 150                 155                 160
   cca gaa tac cca ggc gtc ctc tct gag gtc cag gag gaa aaa ggc atc     528
   Pro Glu Tyr Pro Gly Val Leu Ser Glu Val Gln Glu Glu Lys Gly Ile
                   165                 170                 175
   aag tat aag ttt gaa gtc tac gag aag aaa gac taa                     564
   Lys Tyr Lys Phe Glu Val Tyr Glu Lys Lys Asp
               180                 185
<210> 115
   <211> 187
   <212> PRT
   <213> Artificial
<220>
   <223> wt DHFR gene (from mouse)
<400> 115
   Met Val Arg Pro Leu Asn Cys Ile Val Ala Val Ser Gln Asn Met Gly
   1               5                   10                  15
   Ile Gly Lys Asn Gly Asp Leu Pro Trp Pro Pro Leu Arg Asn Glu Phe
               20                  25                  30
   Lys Tyr Phe Gln Arg Met Thr Thr Thr Ser Ser Val Glu Gly Lys Gln
           35                  40                  45
   Asn Leu Val Ile Met Gly Arg Lys Thr Trp Phe Ser Ile Pro Glu Lys
       50                  55                  60
   Asn Arg Pro Leu Lys Asp Arg Ile Asn Ile Val Leu Ser Arg Glu Leu
   65                  70                  75                  80
   Lys Glu Pro Pro Arg Gly Ala His Phe Leu Ala Lys Ser Leu Asp Asp
                   85                  90                  95
   Ala Leu Arg Leu Ile Glu Gln Pro Glu Leu Ala Ser Lys Val Asp Met
               100                 105                 110
   Val Trp Ile Val Gly Gly Ser Ser Val Tyr Gln Glu Ala Met Asn Gln
           115                 120                 125
   Pro Gly His Leu Arg Leu Phe Val Thr Arg Ile Met Gln Glu Phe Glu
       130                 135                 140
   Ser Asp Thr Phe Phe Pro Glu Ile Asp Leu Gly Lys Tyr Lys Leu Leu
   145                 150                 155                 160
   Pro Glu Tyr Pro Gly Val Leu Ser Glu Val Gln Glu Glu Lys Gly Ile
                   165                 170                 175
   Lys Tyr Lys Phe Glu Val Tyr Glu Lys Lys Asp
               180                 185
<210> 116
   <211> 1143
   <212> DNA
   <213> Artificial
<220>
   <223> wt hygromycin resistance gene
<220>
   <221> CDS
   <222> (1)..(1143)
<400> 116
   atg aaa aag cct gaa ctc acc gcg acg tct gtc gag aag ttt ctg atc      48
   Met Lys Lys Pro Glu Leu Thr Ala Thr Ser Val Glu Lys Phe Leu Ile
   1               5                   10                  15
   gaa aag ttc gac agc gtc tcc gac ctg atg cag ctc tcg gag ggc gaa      96
   Glu Lys Phe Asp Ser Val Ser Asp Leu Met Gln Leu Ser Glu Gly Glu
               20                  25                  30
   gaa tct cgt gct ttc agc ttc gat gta gga ggg cgt gga tat gtc ctg     144
   Glu Ser Arg Ala Phe Ser Phe Asp Val Gly Gly Arg Gly Tyr Val Leu
           35                  40                  45
   cgg gta aat agc tgc gcc gat ggt ttc tac aaa gat cgt tat gtt tat     192
   Arg Val Asn Ser Cys Ala Asp Gly Phe Tyr Lys Asp Arg Tyr Val Tyr
       50                  55                  60
   cgg cac ttt gca tcg gcc gcg ctc ccg att ccg gaa gtg ctt gac att      240
   Arg His Phe Ala Ser Ala Ala Leu Pro Ile Pro Glu Val Leu Asp Ile
   65                  70                  75                  80
   ggg gaa ttc agc gag agc ctg acc tat tgc atc tcc cgc cgt gca cag      288
   Gly Glu Phe Ser Glu Ser Leu Thr Tyr Cys Ile Ser Arg Arg Ala Gln
                   85                  90                  95
   ggt gtc acg ttg caa gac ctg cct gaa acc gaa ctg ccc gct gtt ctg      336
   Gly Val Thr Leu Gln Asp Leu Pro Glu Thr Glu Leu Pro Ala Val Leu
               100                 105                 110
   cag ccg gtc gcg gag gcc atg gat gcg atc gct gcg gcc gat ctt agc      384
   Gln Pro Val Ala Glu Ala Met Asp Ala Ile Ala Ala Ala Asp Leu Ser
           115                 120                 125
   cag acg agc ggg ttc ggc cca ttc gga ccg caa gga atc ggt caa tac      432
   Gln Thr Ser Gly Phe Gly Pro Phe Gly Pro Gln Gly Ile Gly Gln Tyr
       130                 135                 140
   act aca tgg cgt gat ttc ata tgc gcg att gct gat ccc cat gtg tat      480
   Thr Thr Trp Arg Asp Phe Ile Cys Ala Ile Ala Asp Pro His Val Tyr
   145                 150                 155                 160
   cac tgg caa act gtg atg gac gac acc gtc agt gcg tcc gtc gcg cag      528
   His Trp Gln Thr Val Met Asp Asp Thr Val Ser Ala Ser Val Ala Gln
                   165                 170                 175
   gct ctc gat gag ctg atg ctt tgg gcc gag gac tgc ccc gaa gtc cgg      576
   Ala Leu Asp Glu Leu Met Leu Trp Ala Glu Asp Cys Pro Glu Val Arg
               180                 185                 190
   cac ctc gtg cac gcg gat ttc ggc tcc aac aat gtc ctg acg gac aat      624
   His Leu Val His Ala Asp Phe Gly Ser Asn Asn Val Leu Thr Asp Asn
           195                 200                 205
   ggc cgc ata aca gcg gtc att gac tgg agc gag gcg atg ttc ggg gat      672
   Gly Arg Ile Thr Ala Val Ile Asp Trp Ser Glu Ala Met Phe Gly Asp
       210                 215                 220
   tcc caa tac gag gtc gcc aac atc ttc ttc tgg agg ccg tgg ttg gct      720
   Ser Gln Tyr Glu Val Ala Asn Ile Phe Phe Trp Arg Pro Trp Leu Ala
   225                 230                 235                 240
   tgt atg gag cag cag acg cgc tac ttc gag cgg agg cat ccg gag ctt      768
   Cys Met Glu Gln Gln Thr Arg Tyr Phe Glu Arg Arg His Pro Glu Leu
                   245                 250                 255
   gca gga tcg ccg cgg ctc cgg gcg tat atg ctc cgc att ggt ctt gac      816
   Ala Gly Ser Pro Arg Leu Arg Ala Tyr Met Leu Arg Ile Gly Leu Asp
               260                 265                 270
   caa ctc tat cag agc ttg gtt gac ggc aat ttc gat gat gca gct tgg      864
   Gln Leu Tyr Gln Ser Leu Val Asp Gly Asn Phe Asp Asp Ala Ala Trp
           275                 280                 285
   gcg cag ggt cga tgc gac gca atc gtc cga tcc gga gcc ggg act gtc      912
   Ala Gln Gly Arg Cys Asp Ala Ile Val Arg Ser Gly Ala Gly Thr Val
       290                 295                 300
   ggg cgt aca caa atc gcc cgc aga agc gcg gcc gtc tgg acc gat ggc      960
   Gly Arg Thr Gln Ile Ala Arg Arg Ser Ala Ala Val Trp Thr Asp Gly
   305                 310                 315                 320
   tgt gta gaa gta ctc gcc gat agt gga aac cga cgc ccc agc act cgt     1008
   Cys Val Glu Val Leu Ala Asp Ser Gly Asn Arg Arg Pro Ser Thr Arg
                   325                 330                 335
   ccg gag gca aag gaa ttc ggg aga tgg ggg agg cta act gaa aca cgg      1056
   Pro Glu Ala Lys Glu Phe Gly Arg Trp Gly Arg Leu Thr Glu Thr Arg
               340                 345                 350
   aag gag aca ata ccg gaa gga acc cgc gct atg acg gca ata aaa aga      1104
   Lys Glu Thr Ile Pro Glu Gly Thr Arg Ala Met Thr Ala Ile Lys Arg
           355                 360                 365
   cag aat aaa acg cac ggg tgt tgg gtc gtt tgt tca taa                  1143
   Gln Asn Lys Thr His Gly Cys Trp Val Val Cys Ser
       370                 375                 380
<210> 117
   <211> 380
   <212> PRT
   <213> Artificial
<220>
   <223> wt hygromycin resistance gene
<400> 117
   Met Lys Lys Pro Glu Leu Thr Ala Thr Ser Val Glu Lys Phe Leu Ile
   1               5                   10                  15
   Glu Lys Phe Asp Ser Val Ser Asp Leu Met Gln Leu Ser Glu Gly Glu
               20                  25                  30
   Glu Ser Arg Ala Phe Ser Phe Asp Val Gly Gly Arg Gly Tyr Val Leu
           35                  40                  45
   Arg Val Asn Ser Cys Ala Asp Gly Phe Tyr Lys Asp Arg Tyr Val Tyr
       50                  55                  60
   Arg His Phe Ala Ser Ala Ala Leu Pro Ile Pro Glu Val Leu Asp Ile
   65                  70                  75                  80
   Gly Glu Phe Ser Glu Ser Leu Thr Tyr Cys Ile Ser Arg Arg Ala Gln
                   85                  90                  95
   Gly Val Thr Leu Gln Asp Leu Pro Glu Thr Glu Leu Pro Ala Val Leu
               100                 105                 110
   Gln Pro Val Ala Glu Ala Met Asp Ala Ile Ala Ala Ala Asp Leu Ser
           115                 120                 125
   Gln Thr Ser Gly Phe Gly Pro Phe Gly Pro Gln Gly Ile Gly Gln Tyr
       130                 135                 140
   Thr Thr Trp Arg Asp Phe Ile Cys Ala Ile Ala Asp Pro His Val Tyr
   145                 150                 155                 160
   His Trp Gln Thr Val Met Asp Asp Thr Val Ser Ala Ser Val Ala Gln
                   165                 170                 175
   Ala Leu Asp Glu Leu Met Leu Trp Ala Glu Asp Cys Pro Glu Val Arg
               180                 185                 190
   His Leu Val His Ala Asp Phe Gly Ser Asn Asn Val Leu Thr Asp Asn
           195                 200                 205
   Gly Arg Ile Thr Ala Val Ile Asp Trp Ser Glu Ala Met Phe Gly Asp
       210                 215                 220
   Ser Gln Tyr Glu Val Ala Asn Ile Phe Phe Trp Arg Pro Trp Leu Ala
   225                 230                 235                 240
   Cys Met Glu Gln Gln Thr Arg Tyr Phe Glu Arg Arg His Pro Glu Leu
                   245                 250                 255
   Ala Gly Ser Pro Arg Leu Arg Ala Tyr Met Leu Arg Ile Gly Leu Asp
               260                 265                 270
   Gln Leu Tyr Gln Ser Leu Val Asp Gly Asn Phe Asp Asp Ala Ala Trp
           275                 280                 285
   Ala Gln Gly Arg Cys Asp Ala Ile Val Arg Ser Gly Ala Gly Thr Val
       290                 295                 300
   Gly Arg Thr Gln Ile Ala Arg Arg Ser Ala Ala Val Trp Thr Asp Gly
   305                 310                 315                 320
   Cys Val Glu Val Leu Ala Asp Ser Gly Asn Arg Arg Pro Ser Thr Arg
                   325                 330                 335
   Pro Glu Ala Lys Glu Phe Gly Arg Trp Gly Arg Leu Thr Glu Thr Arg
               340                 345                 350
   Lys Glu Thr Ile Pro Glu Gly Thr Arg Ala Met Thr Ala Ile Lys Arg
           355                 360                 365
   Gln Asn Lys Thr His Gly Cys Trp Val Val Cys Ser
       370                 375                 380
<210> 118
   <211> 804
   <212> DNA
   <213> Artificial
<220>
   <223> wt neomycin resistance gene
<220>
   <221> CDS
   <222> (1)..(804)
<400> 118
   atg gga tcg gcc att gaa caa gat gga ttg cac gca ggt tct ccg gcc      48
   Met Gly Ser Ala Ile Glu Gln Asp Gly Leu His Ala Gly Ser Pro Ala
   1               5                   10                  15
   gct tgg gtg gag agg cta ttc ggc tat gac tgg gca caa cag aca atc      96
   Ala Trp Val Glu Arg Leu Phe Gly Tyr Asp Trp Ala Gln Gln Thr Ile
               20                  25                  30
   ggc tgc tct gat gcc gcc gtg ttc cgg ctg tca gcg cag ggg cgc ccg     144
   Gly Cys Ser Asp Ala Ala Val Phe Arg Leu Ser Ala Gln Gly Arg Pro
           35                  40                  45
   gtt ctt ttt gtc aag acc gac ctg tcc ggt gcc ctg aat gaa ctg cag     192
   Val Leu Phe Val Lys Thr Asp Leu Ser Gly Ala Leu Asn Glu Leu Gln
       50                  55                  60
   gac gag gca gcg cgg cta tcg tgg ctg gcc acg acg ggc gtt cct tgc     240
   Asp Glu Ala Ala Arg Leu Ser Trp Leu Ala Thr Thr Gly Val Pro Cys
   65                  70                  75                  80
   gca gct gtg ctc gac gtt gtc act gaa gcg gga agg gac tgg ctg cta     288
   Ala Ala Val Leu Asp Val Val Thr Glu Ala Gly Arg Asp Trp Leu Leu
                   85                  90                  95
   ttg ggc gaa gtg ccg ggg cag gat ctc ctg tca tct cac ctt gct cct      336
   Leu Gly Glu Val Pro Gly Gln Asp Leu Leu Ser Ser His Leu Ala Pro
               100                 105                 110
   gcc gag aaa gta tcc atc atg gct gat gca atg cgg cgg ctg cat acg      384
   Ala Glu Lys Val Ser Ile Met Ala Asp Ala Met Arg Arg Leu His Thr
           115                 120                 125
   ctt gat ccg gct acc tgc cca ttc gac cac caa gcg aaa cat cgc atc      432
   Leu Asp Pro Ala Thr Cys Pro Phe Asp His Gln Ala Lys His Arg Ile
       130                 135                 140
   gag cga gca cgt act cgg atg gaa gcc ggt ctt gtc gat cag gat gat      480
   Glu Arg Ala Arg Thr Arg Met Glu Ala Gly Leu Val Asp Gln Asp Asp
   145                 150                 155                 160
   ctg gac gaa gag cat cag ggg ctc gcg cca gcc gaa ctg ttc gcc agg      528
   Leu Asp Glu Glu His Gln Gly Leu Ala Pro Ala Glu Leu Phe Ala Arg
                   165                 170                 175
   ctc aag gcg cgc atg ccc gac ggc gat gat ctc gtc gtg acc cat ggc      576
   Leu Lys Ala Arg Met Pro Asp Gly Asp Asp Leu Val Val Thr His Gly
               180                 185                 190
   gat gcc tgc ttg ccg aat atc atg gtg gaa aat ggc cgc ttt tct gga      624
   Asp Ala Cys Leu Pro Asn Ile Met Val Glu Asn Gly Arg Phe Ser Gly
           195                 200                 205
   ttc atc gac tgt ggc cgg ctg ggt gtg gcg gac cgc tat cag gac ata      672
   Phe Ile Asp Cys Gly Arg Leu Gly Val Ala Asp Arg Tyr Gln Asp Ile
       210                 215                 220
   gcg ttg gct acc cgt gat att gct gaa gag ctt ggc ggc gaa tgg gct      720
   Ala Leu Ala Thr Arg Asp Ile Ala Glu Glu Leu Gly Gly Glu Trp Ala
   225                 230                 235                 240
   gac cgc ttc ctc gtg ctt tac ggt atc gcc gct ccc gat tcg cag cgc      768
   Asp Arg Phe Leu Val Leu Tyr Gly Ile Ala Ala Pro Asp Ser Gln Arg
                   245                 250                 255
   atc gcc ttc tat cgc ctt ctt gac gag ttc ttc tga                      804
   Ile Ala Phe Tyr Arg Leu Leu Asp Glu Phe Phe
               260                 265
<210> 119
   <211> 267
   <212> PRT
   <213> Artificial
<220>
   <223> wt neomycin resistance gene
<400> 119
   Met Gly Ser Ala Ile Glu Gln Asp Gly Leu His Ala Gly Ser Pro Ala
   1               5                   10                  15
   Ala Trp Val Glu Arg Leu Phe Gly Tyr Asp Trp Ala Gln Gln Thr Ile
               20                  25                  30
   Gly Cys Ser Asp Ala Ala Val Phe Arg Leu Ser Ala Gln Gly Arg Pro
           35                  40                  45
   Val Leu Phe Val Lys Thr Asp Leu Ser Gly Ala Leu Asn Glu Leu Gln
       50                  55                  60
   Asp Glu Ala Ala Arg Leu Ser Trp Leu Ala Thr Thr Gly Val Pro Cys
   65                  70                  75                  80
   Ala Ala Val Leu Asp Val Val Thr Glu Ala Gly Arg Asp Trp Leu Leu
                   85                  90                  95
   Leu Gly Glu Val Pro Gly Gln Asp Leu Leu Ser Ser His Leu Ala Pro
               100                 105                 110
   Ala Glu Lys Val Ser Ile Met Ala Asp Ala Met Arg Arg Leu His Thr
           115                 120                 125
   Leu Asp Pro Ala Thr Cys Pro Phe Asp His Gln Ala Lys His Arg Ile
       130                 135                 140
   Glu Arg Ala Arg Thr Arg Met Glu Ala Gly Leu Val Asp Gln Asp Asp
   145                 150                 155                 160
   Leu Asp Glu Glu His Gln Gly Leu Ala Pro Ala Glu Leu Phe Ala Arg
                   165                 170                 175
   Leu Lys Ala Arg Met Pro Asp Gly Asp Asp Leu Val Val Thr His Gly
               180                 185                 190
   Asp Ala Cys Leu Pro Asn Ile Met Val Glu Asn Gly Arg Phe Ser Gly
           195                 200                 205
   Phe Ile Asp Cys Gly Arg Leu Gly Val Ala Asp Arg Tyr Gln Asp Ile
       210                 215                 220
   Ala Leu Ala Thr Arg Asp Ile Ala Glu Glu Leu Gly Gly Glu Trp Ala
   225                 230                 235                 240
   Asp Arg Phe Leu Val Leu Tyr Gly Ile Ala Ala Pro Asp Ser Gln Arg
                   245                 250                 255
   Ile Ala Phe Tyr Arg Leu Leu Asp Glu Phe Phe
               260                 265
<210> 120
   <211> 1121
   <212> DNA
   <213> Artificial
<220>
   <223> wt glutamine synthase gene (human)
<220>
   <221> CDS
   <222> (1)..(1119)
<400> 120
   atg acc acc tca gca agt tcc cac tta aat aaa ggc atc aag cag gtg         48
   Met Thr Thr Ser Ala Ser Ser His Leu Asn Lys Gly Ile Lys Gln Val
   1               5                   10                  15
   tac atg tcc ctg cct cag ggt gag aaa gtc cag gcc atg tat atc tgg         96
   Tyr Met Ser Leu Pro Gln Gly Glu Lys Val Gln Ala Met Tyr Ile Trp
               20                  25                  30
   atc gat ggt act gga gaa gga ctg cgc tgc aag acc cgg acc ctg gac        144
   Ile Asp Gly Thr Gly Glu Gly Leu Arg Cys Lys Thr Arg Thr Leu Asp
           35                  40                  45
   agt gag ccc aag tgt gtg gaa gag ttg cct gag tgg aat ttc gat ggc        192
   Ser Glu Pro Lys Cys Val Glu Glu Leu Pro Glu Trp Asn Phe Asp Gly
       50                  55                  60
   tcc agt act tta cag tct gag ggt tcc aac agt gac atg tat ctc gtg      240
   Ser Ser Thr Leu Gln Ser Glu Gly Ser Asn Ser Asp Met Tyr Leu Val
   65                  70                  75                  80
   cct gct gcc atg ttt cgg gac ccc ttc cgt aag gac cct aac aag ctg      288
   Pro Ala Ala Met Phe Arg Asp Pro Phe Arg Lys Asp Pro Asn Lys Leu
                   85                  90                  95
   gtg tta tgt gaa gtt ttc aag tac aat cga agg cct gca gag acc aat      336
   Val Leu Cys Glu Val Phe Lys Tyr Asn Arg Arg Pro Ala Glu Thr Asn
               100                 105                 110
   ttg agg cac acc tgt aaa cgg ata atg gac atg gtg agc aac cag cac      384
   Leu Arg His Thr Cys Lys Arg Ile Met Asp Met Val Ser Asn Gln His
           115                 120                 125
   ccc tgg ttt ggc atg gag cag gag tat acc ctc atg ggg aca gat ggg      432
   Pro Trp Phe Gly Met Glu Gln Glu Tyr Thr Leu Met Gly Thr Asp Gly
       130                 135                 140
   cac ccc ttt ggt tgg cct tcc aac ggc ttc cca ggg ccc cag ggt cca      480
   His Pro Phe Gly Trp Pro Ser Asn Gly Phe Pro Gly Pro Gln Gly Pro
   145                 150                 155                 160
   tat tac tgt ggt gtg gga gca gac aga gcc tat ggc agg gac atc gtg      528
   Tyr Tyr Cys Gly Val Gly Ala Asp Arg Ala Tyr Gly Arg Asp Ile Val
                   165                 170                 175
   gag gcc cat tac cgg gcc tgc ttg tat gct gga gtc aag att gcg ggg      576
   Glu Ala His Tyr Arg Ala Cys Leu Tyr Ala Gly Val Lys Ile Ala Gly
               180                 185                 190
   act aat gcc gag gtc atg cct gcc cag tgg gaa ttt cag att gga cct      624
   Thr Asn Ala Glu Val Met Pro Ala Gln Trp Glu Phe Gln Ile Gly Pro
           195                 200                 205
   tgt gaa gga atc agc atg gga gat cat ctc tgg gtg gcc cgt ttc atc      672
   Cys Glu Gly Ile Ser Met Gly Asp His Leu Trp Val Ala Arg Phe Ile
       210                 215                 220
   ttg cat cgt gtg tgt gaa gac ttt gga gtg ata gca acc ttt gat cct      720
   Leu His Arg Val Cys Glu Asp Phe Gly Val Ile Ala Thr Phe Asp Pro
   225                 230                 235                 240
   aag ccc att cct ggg aac tgg aat ggt gca ggc tgc cat acc aac ttc      768
   Lys Pro Ile Pro Gly Asn Trp Asn Gly Ala Gly Cys His Thr Asn Phe
                   245                 250                 255
   agc acc aag gcc atg cgg gag gag aat ggt ctg aag tac atc gag gag      816
   Ser Thr Lys Ala Met Arg Glu Glu Asn Gly Leu Lys Tyr Ile Glu Glu
               260                 265                 270
   gcc att gag aaa cta agc aag cgg cac cag tac cac atc cgt gcc tat      864
   Ala Ile Glu Lys Leu Ser Lys Arg His Gln Tyr His Ile Arg Ala Tyr
           275                 280                 285
   gat ccc aag gga ggc ctg gac aat gcc cga cgt cta act gga ttc cat      912
   Asp Pro Lys Gly Gly Leu Asp Asn Ala Arg Arg Leu Thr Gly Phe His
       290                 295                 300
   gaa acc tcc aac atc aac gac ttt tct ggt ggt gta gcc aat cgt agc      960
   Glu Thr Ser Asn Ile Asn Asp Phe Ser Gly Gly Val Ala Asn Arg Ser
   305                 310                 315                 320
   gcc agc ata cgc att ccc cgg act gtt ggc cag gag aag aag ggt tac     1008
   Ala Ser Ile Arg Ile Pro Arg Thr Val Gly Gln Glu Lys Lys Gly Tyr
                   325                 330                 335
   ttt gaa gat cgt cgc ccc tct gcc aac tgc gac ccc ttt tcg gtg aca      1056
   Phe Glu Asp Arg Arg Pro Ser Ala Asn Cys Asp Pro Phe Ser Val Thr
               340                 345                 350
   gaa gcc ctc atc cgc acg tgt ctt ctc aat gaa acc ggc gat gag ccc      1104
   Glu Ala Leu Ile Arg Thr Cys Leu Leu Asn Glu Thr Gly Asp Glu Pro
           355                 360                 365
   ttc cag tac aaa aat ta                                               1121
   Phe Gln Tyr Lys Asn
       370
<210> 121
   <211> 373
   <212> PRT
   <213> Artificial
<220>
   <223> wt glutamine synthase gene (human)
<400> 121
   Met Thr Thr Ser Ala Ser Ser His Leu Asn Lys Gly Ile Lys Gln Val
   1               5                   10                  15
   Tyr Met Ser Leu Pro Gln Gly Glu Lys Val Gln Ala Met Tyr Ile Trp
               20                  25                  30
   Ile Asp Gly Thr Gly Glu Gly Leu Arg Cys Lys Thr Arg Thr Leu Asp
           35                  40                  45
   Ser Glu Pro Lys Cys Val Glu Glu Leu Pro Glu Trp Asn Phe Asp Gly
       50                  55                  60
   Ser Ser Thr Leu Gln Ser Glu Gly Ser Asn Ser Asp Met Tyr Leu Val
   65                  70                  75                      80
   Pro Ala Ala Met Phe Arg Asp Pro Phe Arg Lys Asp Pro Asn Lys Leu
                   85                  90                  95
   Val Leu Cys Glu Val Phe Lys Tyr Asn Arg Arg Pro Ala Glu Thr Asn
               100                 105                 110
   Leu Arg His Thr Cys Lys Arg Ile Met Asp Met Val Ser Asn Gln His
           115                 120                 125
   Pro Trp Phe Gly Met Glu Gln Glu Tyr Thr Leu Met Gly Thr Asp Gly
       130                 135                 140
   His Pro Phe Gly Trp Pro Ser Asn Gly Phe Pro Gly Pro Gln Gly Pro
   145                 150                 155                     160
   Tyr Tyr Cys Gly Val Gly Ala Asp Arg Ala Tyr Gly Arg Asp Ile Val
                   165                 170                 175
   Glu Ala His Tyr Arg Ala Cys Leu Tyr Ala Gly Val Lys Ile Ala Gly
               180                 185                 190
   Thr Asn Ala Glu Val Met Pro Ala Gln Trp Glu Phe Gln Ile Gly Pro
           195                 200                 205
   Cys Glu Gly Ile Ser Met Gly Asp His Leu Trp Val Ala Arg Phe Ile
       210                 215                 220
   Leu His Arg Val Cys Glu Asp Phe Gly Val Ile Ala Thr Phe Asp Pro
   225                 230                 235                     240
   Lys Pro Ile Pro Gly Asn Trp Asn Gly Ala Gly Cys His Thr Asn Phe
   245 250 255
   Ser Thr Lys Ala Met Arg Glu Glu Asn Gly Leu Lys Tyr Ile Glu Glu
   260 265 270
   Ala Ile Glu Lys Leu Ser Lys Arg His Gln Tyr His Ile Arg Ala Tyr
   275 280 285
   Asp Pro Lys Gly Gly Leu Asp Asn Ala Arg Arg Leu Thr Gly Phe His
   290 295 300
   Glu Thr Ser Asn Ile Asn Asp Phe Ser Gly Gly Val Ala Asn Arg Ser
   305 310 315 320
   Ala Ser Ile Arg Ile Pro Arg Thr Val Gly Gln Glu Lys Lys Gly Tyr
   325 330 335
   Phe Glu Asp Arg Arg Pro Ser Ala Asn Cys Asp Pro Phe Ser Val Thr
   340 345 350
   Glu Ala Leu Ile Arg Thr Cys Leu Leu Asn Glu Thr Gly Asp Glu Pro
   355 360 365
   Phe Gln Tyr Lys Asn
   370
<210> 122
   <211> 43
   <212> DNA
   <213> Artificial
<220>
   <223> primer GTGspaceBamHIF
<400> 122
   gaattcggat ccaccgtggc gatccaaaga ctgccaaatc tag 43
<210> 123
   <211> 42
   <212> DNA
   <213> Artificial
<220>
   <223> primer ZEOTTTGTGBamHIF
<400> 123
   gaattcggat cctttgtggc caagttgacc agtgccgttc cg 42
<210> 124
   <211> 46
   <212> DNA
   <213> Artificial
<220>
   <223> primer ZEOForwardGTG-Thr9
<400> 124
   aattggatcc accgtggcca agttgaccag tgccgttacc gtgctc 46
<210> 125
   <211> 46
   <212> DNA
   <213> Artificial
<220>
   <223> pimer ZEOForward GTG-Phe9
<400> 125
   aattggatcc accgtggcca agttgaccag tgccgttttc gtgctc 46
<210> 126
   <211> 43
   <212> DNA
   <213> Artificial
<220>
   <223> primer TTGspaceBamHIF
<400> 126
   gaattcggat ccaccttggc gatccaaaga ctgccaaatc tag 43
<210> 127
   <211> 46
   <212> DNA
   <213> Artificial
<220>
   <223> primer ZEOForwardTTG-Thr9
<400> 127
   aattggatcc accttggcca agttgaccag tgccgttacc gtgctc 46
<210> 128
   <211> 46
   <212> DNA
   <213> Artificial
<220>
   <223> pimer ZEOForwardTTG-Phe9
<400> 128
   aattggatcc accttggcca agttgaccag tgccgttttc gtgctc 46
<210> 129
   <211> 37
   <212> DNA
   <213> Artificial
<220>
   <223> primer PURO BamHI F
<400> 129
   gatcggatcc atggttaccg agtacaagcc cacggtg 37
<210> 130
   <211> 35
   <212> DNA
   <213> Artificial
<220>
   <223> primer PURO300 R LEU
<400> 130
   cagccgggaa ccgctcaact cggccaggcg cgggc 35
<210> 131
   <211> 49
   <212> DNA
   <213> Artificial
<220>
   <223> primer PUR0300FLEU
<400> 131
   cgagttgagc ggttcccggc tggccgcgca gcaacagctg gaaggcctc 49
<210> 132
   <211> 44
   <212> DNA
   <213> Artificial
<220>
   <223> primer PURO600RLEU
<400> 132
   aagcttgaat tcaggcaccg ggcttgcggg tcaggcacca ggtc 44
<210> 133
   <211> 42
   <212> DNA
   <213> Artificial
<220>
   <223> primer PUROBamHI TTG1F
<400> 133
   gaattcggat ccaccttggt taccgagtac aagcccacgg tg 42
<210> 134
   <211> 804
   <212> DNA
   <213> Artificial
<220>
   <223> modified neomycin resistance gene lacking internal ATG sequences
<220>
   <221> CDS
   <222> (1)..(804)
<400> 134
   atg gga tcg gcc att gaa caa gac gga ttg cac gca ggt tct ccg gcc      48
   Met Gly Ser Ala Ile Glu Gln Asp Gly Leu His Ala Gly Ser Pro Ala
   1               5                   10                  15
   gct tgg gtg gag agg cta ttc ggc tac gac tgg gca caa cag aca atc      96
   Ala Trp Val Glu Arg Leu Phe Gly Tyr Asp Trp Ala Gln Gln Thr Ile
               20                  25                  30
   ggc tgc tct gac gcc gcc gtg ttc cgg ctg tca gcg cag ggg cgc ccg     144
   Gly Cys Ser Asp Ala Ala Val Phe Arg Leu Ser Ala Gln Gly Arg Pro
           35                  40                  45
   gtt ctt ttt gtc aag acc gac ctg tcc ggt gcc ctg aac gaa ctg cag     192
   Val Leu Phe Val Lys Thr Asp Leu Ser Gly Ala Leu Asn Glu Leu Gln
       50                  55                  60
   gac gag gca gcg cgg cta tcg tgg ctg gcc acg acg ggc gtt cct tgc     240
   Asp Glu Ala Ala Arg Leu Ser Trp Leu Ala Thr Thr Gly Val Pro Cys
   65                  70                  75                  80
   gca gct gtg ctc gac gtt gtc act gaa gcg gga agg gac tgg ctg cta       288
   Ala Ala Val Leu Asp Val Val Thr Glu Ala Gly Arg Asp Trp Leu Leu
                   85                  90                  95
   ttg ggc gaa gtg ccg ggg cag gat ctc ctg tca tct cac ctt gct cct       336
   Leu Gly Glu Val Pro Gly Gln Asp Leu Leu Ser Ser His Leu Ala Pro
               100                 105                 110
   gcc gag aaa gta tcc atc ctg gct gac gca ctg cgg cgg ctg cat acg       384
   Ala Glu Lys Val Ser Ile Leu Ala Asp Ala Leu Arg Arg Leu His Thr
           115                 120                 125
   ctt gat ccg gct acc tgc cca ttc gac cac caa gcg aaa cat cgc atc       432
   Leu Asp Pro Ala Thr Cys Pro Phe Asp His Gln Ala Lys His Arg Ile
       130                 135                 140
   gag cga gca cgt act cgg ctg gaa gcc ggt ctt gtc gat cag gac gat       480
   Glu Arg Ala Arg Thr Arg Leu Glu Ala Gly Leu Val Asp Gln Asp Asp
   145                 150                 155                 160
   ctg gac gaa gag cat cag ggg ctc gcg cca gcc gaa ctg ttc gcc agg       528
   Leu Asp Glu Glu His Gln Gly Leu Ala Pro Ala Glu Leu Phe Ala Arg
                   165                 170                 175
   ctc aag gcg cgc ctg ccc gac ggc gac gat ctc gtc gtg acc cac ggc       576
   Leu Lys Ala Arg Leu Pro Asp Gly Asp Asp Leu Val Val Thr His Gly
               180                 185                 190
   gac gcc tgc ttg ccg aat atc ctg gtg gaa aac ggc cgc ttt tct gga       624
   Asp Ala Cys Leu Pro Asn Ile Leu Val Glu Asn Gly Arg Phe Ser Gly
           195                 200                 205
   ttc atc gac tgt ggc cgg ctg ggt gtg gcg gac cgc tat cag gac ata       672
   Phe Ile Asp Cys Gly Arg Leu Gly Val Ala Asp Arg Tyr Gln Asp Ile
       210                 215                 220
   gcg ttg gct acc cgt gat att gct gaa gag ctt ggc ggc gag tgg gct       720
   Ala Leu Ala Thr Arg Asp Ile Ala Glu Glu Leu Gly Gly Glu Trp Ala
   225                 230                 235                 240
   gac cgc ttc ctc gtg ctt tac ggt atc gcc gct ccc gat tcg cag cgc       768
   Asp Arg Phe Leu Val Leu Tyr Gly Ile Ala Ala Pro Asp Ser Gln Arg
                   245                 250                 255
   atc gcc ttc tat cgc ctt ctt gac gag ttc ttc tga                       804
   Ile Ala Phe Tyr Arg Leu Leu Asp Glu Phe Phe
               260                 265
<210> 135
   <211> 267
   <212> PRT
   <213> Artificial
<220>
   <223> modified neomycin resistance gene lacking internal ATG sequences
<400> 135
   Met Gly Ser Ala Ile Glu Gln Asp Gly Leu His Ala Gly Ser Pro Ala
   1               5                   10                  15
   Ala Trp Val Glu Arg Leu Phe Gly Tyr Asp Trp Ala Gln Gln Thr Ile
               20                  25                  30
   Gly Cys Ser Asp Ala Ala Val Phe Arg Leu Ser Ala Gln Gly Arg Pro
           35                  40                  45
   Val Leu Phe Val Lys Thr Asp Leu Ser Gly Ala Leu Asn Glu Leu Gln
       50                  55                  60
   Asp Glu Ala Ala Arg Leu Ser Trp Leu Ala Thr Thr Gly Val Pro Cys
   65                  70                  75                  80
   Ala Ala Val Leu Asp Val Val Thr Glu Ala Gly Arg Asp Trp Leu Leu
                   85                  90                  95
   Leu Gly Glu Val Pro Gly Gln Asp Leu Leu Ser Ser His Leu Ala Pro
               100                 105                 110
   Ala Glu Lys Val Ser Ile Leu Ala Asp Ala Leu Arg Arg Leu His Thr
           115                 120                 125
   Leu Asp Pro Ala Thr Cys Pro Phe Asp His Gln Ala Lys His Arg Ile
       130                 135                 140
   Glu Arg Ala Arg Thr Arg Leu Glu Ala Gly Leu Val Asp Gln Asp Asp
   145                 150                 155                 160
   Leu Asp Glu Glu His Gln Gly Leu Ala Pro Ala Glu Leu Phe Ala Arg
                   165                 170                 175
   Leu Lys Ala Arg Leu Pro Asp Gly Asp Asp Leu Val Val Thr His Gly
               180                 185                 190
   Asp Ala Cys Leu Pro Asn Ile Leu Val Glu Asn Gly Arg Phe Ser Gly
           195                 200                 205
   Phe Ile Asp Cys Gly Arg Leu Gly Val Ala Asp Arg Tyr Gln Asp Ile
       210                 215                 220
   Ala Leu Ala Thr Arg Asp Ile Ala Glu Glu Leu Gly Gly Glu Trp Ala
   225                 230                 235                 240
   Asp Arg Phe Leu Val Leu Tyr Gly Ile Ala Ala Pro Asp Ser Gln Arg
                   245                 250                 255
   Ile Ala Phe Tyr Arg Leu Leu Asp Glu Phe Phe
               260                 265
<210> 136
   <211> 40
   <212> DNA
   <213> Artificial
<220>
   <223> primer NEO-F-HindIII
<400> 136
   gatcaagctt ttggatcggc cattgaaaca agacggattg 40
<210> 137
   <211> 36
   <212> DNA
   <213> Artificial
<220>
   <223> primer NEO EcoRI 800R
<400> 137
   aagcttgaat tctcagaaga actcgtcaag aaggcg 36
<210> 138
   <211> 564
   <212> DNA
   <213> Artificial
<220>
   <223> modified dhfr gene lacking internal ATG sequences
<220>
   <221> CDS
   <222> (1) .. (569)
   <400> 138
   atg gtt cga cca ttg aac tgc atc gtc gcc gtg tcc caa aat ctg ggg        48
   Met Val Arg Pro Leu Asn Cys Ile Val Ala Val Ser Gln Asn Leu Gly
   1               5                   10                  15
   att ggc aag aac gga gac cta ccc tgg cct ccg ctc agg aac gag ttc        96
   Ile Gly Lys Asn Gly Asp Leu Pro Trp Pro Pro Leu Arg Asn Glu Phe
               20                  25                  30
   aag tac ttc caa aga ctg acc aca acc tct tca gtg gaa ggt aaa cag       144
   Lys Tyr Phe Gln Arg Leu Thr Thr Thr Ser Ser Val Glu Gly Lys Gln
           35                  40                  45
   aat ctg gtg att ctg ggt agg aaa acc tgg ttc tcc att cct gag aag       192
   Asn Leu Val Ile Leu Gly Arg Lys Thr Trp Phe Ser Ile Pro Glu Lys
       50                  55                  60
   aat cga cct tta aag gac aga att aat ata gtt ctc agt aga gaa ctc       240
   Asn Arg Pro Leu Lys Asp Arg Ile Asn Ile Val Leu Ser Arg Glu Leu
   65                  70                  75                  80
   aaa gaa cca cca cga gga gct cat ttt ctt gcc aaa agt ttg gac gac       288
   Lys Glu Pro Pro Arg Gly Ala His Phe Leu Ala Lys Ser Leu Asp Asp
                   85                  90                  95
   gcc tta aga ctt att gaa caa ccg gaa ttg gca agt aaa gta gac ctg       336
   Ala Leu Arg Leu Ile Glu Gln Pro Glu Leu Ala Ser Lys Val Asp Leu
               100                 105                 110
   gtt tgg ata gtc gga ggc agt tct gtt tac cag gaa gcc ctg aat caa       384
   Val Trp Ile Val Gly Gly Ser Ser Val Tyr Gln Glu Ala Leu Asn Gln
           115                 120                 125
   cca ggc cac ctc aga ctc ttt gtg aca agg att ctg cag gaa ttt gaa       432
   Pro Gly His Leu Arg Leu Phe Val Thr Arg Ile Leu Gln Glu Phe Glu
       130                 135                 140
   agt gac acg ttt ttc cca gaa att gat ttg ggg aaa tat aaa ctt ctc       480
   Ser Asp Thr Phe Phe Pro Glu Ile Asp Leu Gly Lys Tyr Lys Leu Leu
   145                 150                 155                 160
   cca gaa tac cca ggc gtc ctc tct gag gtc cag gag gaa aaa ggc atc       528
   Pro Glu Tyr Pro Gly Val Leu Ser Glu Val Gln Glu Glu Lys Gly Ile
                   165                 170                 175
   aag tat aag ttt gaa gtc tac gag aag aaa gac taa                       564
   Lys Tyr Lys Phe Glu Val Tyr Glu Lys Lys Asp
               180                 185

<210> 139
   <211> 187
   <212> PRT
   <213> Artificial
<220>
   <223> modified dhfr gene lacking internal ATG sequences
<400> 139
   Met Val Arg Pro Leu Asn Cys Ile Val Ala Val Ser Gln Asn Leu Gly
   1               5                   10                  15
   Ile Gly Lys Asn Gly Asp Leu Pro Trp Pro Pro Leu Arg Asn Glu Phe
               20                  25                  30
   Lys Tyr Phe Gln Arg Leu Thr Thr Thr Ser Ser Val Glu Gly Lys Gln
           35                  40                  45
   Asn Leu Val Ile Leu Gly Arg Lys Thr Trp Phe Ser Ile Pro Glu Lys
       50                  55                  60
   Asn Arg Pro Leu Lys Asp Arg Ile Asn Ile Val Leu Ser Arg Glu Leu
   65                  70                  75                  80
   Lys Glu Pro Pro Arg Gly Ala His Phe Leu Ala Lys Ser Leu Asp Asp
                   85                  90                  95
   Ala Leu Arg Leu Ile Glu Gln Pro Glu Leu Ala Ser Lys Val Asp Leu
               100                 105                 110
   Val Trp Ile Val Gly Gly Ser Ser Val Tyr Gln Glu Ala Leu Asn Gln
           115                 120                 125
   Pro Gly His Leu Arg Leu Phe Val Thr Arg Ile Leu Gln Glu Phe Glu
       130                 135                 140
   Ser Asp Thr Phe Phe Pro Glu Ile Asp Leu Gly Lys Tyr Lys Leu Leu
   145                 150                 155                 160
   Pro Glu Tyr Pro Gly Val Leu Ser Glu Val Gln Glu Glu Lys Gly Ile
                   165                 170                 175
   Lys Tyr Lys Phe Glu Val Tyr Glu Lys Lys Asp
               180                 185
<210> 140
   <211> 36
   <212> DNA
   <213> Artificial
<220>
   <223> primer DHFR-F-HindIII
<400> 140
   gatcaagctt ttgttcgacc attgaactgc atcgtc 36
<210> 141
   <211> 36
   <212> DNA
   <213> Artificial
<220>
   <223> primer DHFR-EcoRI-600-R
<400> 141
   aagcttgaat tcttagtctt tcttctcgta gacttc 36
<210> 142
   <211> 154
   <212> DNA
   <213> Artificial
<220>
   <223> combined synthetic polyadenylation sequence and pausing signal from the human alpha2 globin gene
<220>
   <221> synthetic polyadenylation sequence
   <222> (1) .. (99)
<220>
   <221> cloning site
   <222> (50)..(62)
<220>
   <221> pausing signal from the human alpha2 globin gene
   <222> (63) .. (154)
<400> 142
   aataaaatat ctttattttc attacatctg tgtgttggtt ttttgtgtga atcgatagta     60
   ctaacatacg ctctccatca aaacaaaacg aaacaaaaca aactagcaaa ataggctgtc    120
   cccagtgcaa gtgcaggtgc cagaacattt ctct                                154